# EUROPEAN PATENT SPECIFICATION

(11) **EP 4 041 918 B1**
(45) Date of publication and mention of the grant of the patent: **10.12.2025**
(21) Application number: 20801126.2
(22) Date of filing: 09.10.2020
(51) Int. Cl.: C12Q 1/686, C12Q 1/689, G16B 10/00, G16B 20/40, G16B 30/10

(54) **METHODS AND SYSTEMS FOR ASSESSING MICROBIAL POPULATIONS**
VERFAHREN UND SYSTEME ZUR BEWERTUNG DER MIKROBIELLEN BEVÖLKERUNGEN
MÉTHODES ET SYSTÈMES D'ÉVALUATION DE POPULATIONS MICROBIENNES

(30) Priority: 11.10.2019 US 201962914366 P; 11.10.2019 US 201962914368 P; 06.12.2019 US 201962944877 P
(43) Date of publication of application: 17.08.2022
(73) Proprietor: Life Technologies Corporation, Carlsbad, CA 92008 (US)
(72) Inventor: SARDA, Shrutii, Carlsbad, California 92008 (US); MCGEACHY, Anna, Carlsbad, California 92008 (US); GOTTIMUKKALA, Rajesh, Fremont, CA 94555 (US); MERRILL, David, Millbrae, CA 94030 (US); SHIN, Heesun, Carlsbad, California 92008 (US); EWING, Aren, Carlsbad, CA 92008 (US); LEE, Wing, Carlsbad, California 92008 (US); DREWS, Birgit, Richmond, CA 92008 (US); HYLAND, Fiona, San Mateo, CA 94402 (US); AU-YOUNG, Janice, Brisbane, CA 94005 (US)
(74) Representative: HGF
(86) International application number: PCT/US2020/070643
(87) International publication number: WO 2021/072439

(56) References cited:
- FIONA FOUHY ET AL: "16S rRNA gene sequencing of mock microbial populations- impact of DNA extraction method, primer choice and sequencing platform", BMC MICROBIOLOGY, vol. 16, no. 1, 24 June 2016 (2016-06-24), XP055766287, DOI: 10.1186/s12866-016-0738-z
- M. J. CLAESSON ET AL: "Comparison of two next-generation sequencing technologies for resolving highly complex microbiota composition using tandem variable 16S rRNA gene regions", NUCLEIC ACIDS RESEARCH, vol. 38, no. 22, 29 September 2010 (2010-09-29), GB, pages e200 - 1, XP055250083, ISSN: 0305-1048, DOI: 10.1093/nar/gkq873
- SANCHIS-CHORDÀ JESÚS ET AL: "CECT 7765 supplementation improves inflammatory status in insulin-resistant obese children", EUROPEAN JOURNAL OF NUTRITION, STEINKOPFF VERLAG, DARMSTADT, DE, vol. 58, no. 7, 24 September 2018 (2018-09-24), pages 2789 - 2800, XP036895578, ISSN: 1436-6207, [retrieved on 20180924], DOI: 10.1007/S00394-018-1828-5
- SZYSZKOWICZ JOANNA KASIA ET AL: "Implications of the gut microbiota in vulnerability to the social avoidance effects of chronic social defeat in male mice", BRAIN, BEHAVIOR AND IMMUNITY, vol. 66, 17 June 2017 (2017-06-17), pages 45 - 55, XP085228069, ISSN: 0889-1591, DOI: 10.1016/J.BBI.2017.06.009

## Description

This application claims priority to and the benefit of U.S. Provisional App. Nos. 62/914,366, filed October 11, 2019, and 62/914,368, filed October 11, 2019, and 62/944,877, filed December 06, 2019.

### SEQUENCE LISTING

This application hereby incorporates by reference the material of the electronic Sequence Listing filed concurrently herewith in its entirety. The material in the electronic Sequence Listing is submitted as a text (.txt) file entitled LT01495_ST25.txt created October 8, 2020, and has a file size of 408 kilobytes.

### BACKGROUND

The diversity of the microbiota of a variety of environments has become an area of intensive research as the scientific and medical communities gain an increased understanding of the important role microbiota play in ecosystems and the health of individuals and populations. In one example, the microbiota of the gut (also referred to as the gut microbiome) is made up of trillions of bacteria, fungi and other microbes. One third of the gut microbiota in humans is common to most people while two thirds are specific to each person. A healthy human gut has a variety of commensal or mutualistic bacteria living in relative homeostasis. When a microbial imbalance or maladaptation occurs, changing the makeup and proportions of the normal flora of bacteria, the gut enters a state of dysbiosis. Dysbiosis typically causes inflammation of the intestinal cell wall, disrupting the mucus barrier, epithelial barrier, and immunosensitive cells that line the gastrointestinal tract. Imbalances in the gut microbiota are associated with diseases, chronic health conditions and response to immuno-oncology treatments. For example, imbalances in the gut microbiota have been associated with gut disorders such as irritable bowel syndrome (IBS), inflammatory bowel disease (IBD) and obesity, and autoimmune disorders such as celiac disease, lupus and rheumatoid arthritis (RA). Additionally, the composition of the gut microbiota may influence susceptibility to oncological conditions, such as cancer, and responsiveness to cancer therapies. Due to the involvement of gut microbiota in a wide range of disorders and diseases across animal species, including humans, animals and insects, characterization and study of the gut microbiome have emerged as key research focuses in advancing the understanding of health and disease and in the development of therapies for related conditions. Fouhy *et al.* (BMC Microbiology, Vol. 16, 123, 2016) describes 16S rRNA gene sequencing of mock microbial populations.

Several different techniques have been employed to attempt to identify microbes in various environmental and organismal samples. Initial techniques relied on microbial culture processes which are time-consuming and provide limited information due, in part, to varying growth conditions required to obtain different microbial cultures. Many more recent techniques that do not require culturing involve analysis of the genetic makeup of microbial cells contained in samples using nucleic acid analysis methods including, for example nucleic acid amplification (e.g., PCR) and/or sequencing. Typically, such methods involve amplification and analysis of microbial 16S rRNA gene segments. While analysis of 16S rRNA sequences has reduced the time and labor required in some other methods of evaluating microbial composition of samples, the comprehensiveness, accuracy, quality and depth of the information obtained through 16S rRNA gene sequence analysis-based methods can vary and be limited, for example, by the amplicons targeted and primers used in the methods. Therefore, there is a need for more sensitive and comprehensive methods for accurately characterizing the whole of a microbial population in a sample through identifying and distinguishing microbial species and levels thereof in samples containing multiple species. Such methods will factor significantly in many research areas including those directed to the causes, complications, and diagnosis of multifactorial disorders and diseases, and in advancing research into and understanding of the gut microbiota in health and disease.

### BRIEF SUMMARY

Reference to compositions and kits is provided for illustrative purposes only however they may be used in the method in accordance with the claims. Provided herein are methods, as well as systems in accordance with the claims. Compositions described herein include a nucleic acid, for example, a single-stranded nucleic acid, that is used as a primer and/or probe. Compositions described herein include a combination of a plurality of nucleic acids. In particular examples, the primers and/or probes are capable of binding to, hybridizing to, amplifying and/or detecting target nucleic acids of microorganisms (e.g., bacteria), such as may occur in a sample (e.g., biological sample), for example, a sample of contents of an alimentary canal of an animal. Such nucleic acids described herein include primers and probes that specifically or selectively amplify, bind to, hybridize to and/or detect a pre-determined unique nucleic acid sequence of a microorganism's genome, and primers and probes that amplify, bind to, hybridize to and/or detect a nucleic acid sequence in one or more genes that is homologous across most, or substantially all, members of a taxonomic category (e.g., domain, kingdom, phylum, class, order, genus, species) of organisms, e.g., microorganisms, but that varies between different organisms. Such nucleic acids contain one or more modifications that facilitate manipulation and/or multiplex amplification of nucleic acids. For example, such modifications include modifications that increase susceptibility of the nucleic acid to cleavage relative to the nucleic acid that does not include the modification. In some examples, the nucleic acids include one or more pairs of nucleic acids that are used as primers (e.g., primer pairs) for amplification of a target nucleic acid, such as, for example, a specific nucleic acid unique to a species of microorganism or one or more, or multiple, nucleic acids contained within a homologous gene (e.g., a 16S ribosomal RNA (rRNA) gene) common to multiple different microorganisms. For example, the nucleic acids include one or more primer pairs that separately amplify two or more regions, e.g., hypervariable regions, in a prokaryotic 16S rRNA gene. In some examples, the nucleic acids include a combination of a plurality of primer pairs. The combination of a plurality of primer pairs is designed to amplify nucleic acids in one, some, most or substantially all of the microorganisms, such as, e.g., bacteria, in a sample in a species-targeted and/or kingdom-encompassing manner. Also described herein are compositions containing a mixture of nucleic acids, in which most, or substantially all, of the nucleic acids contain sequence of a portion of the genome of a microorganism, e.g., a bacterium. In some examples, the sequences of portions of the genome of microorganisms are less than or about 250 nucleotides in length. In some examples, the nucleic acids include nucleotides containing a uracil nucleobase. In some examples, the composition contains one or more, or a plurality, of primers, e.g., nucleic acids and/or primer pairs of any of the examples described herein. In some examples, the composition includes a DNA polymerase, a DNA ligase, and/or at least one uracil cleaving or modifying enzyme.

In some examples of methods of amplification described herein, nucleic acids are subjected to amplification using nucleic acids described herein as amplification primers. In some examples, the nucleic acid amplification is a multiplex amplification. In some examples, the methods of amplification include a plurality of nucleic acid primers, e.g., primer pairs, that separately amplify two or more regions in one or more genes that is homologous across most, or substantially all, members of a taxonomic category (e.g., domain, kingdom, phylum, class, order, genus, species) of organisms. For example, a plurality of nucleic acid primers includes primers, or primer pairs, that separately amplify one or more or a plurality of hypervariable regions in a prokaryotic 16S rRNA gene. In some examples, the methods of amplification include one or more, or a plurality of, nucleic acid primers, e.g., primer pairs, that amplify a specific nucleic acid unique to a species of organism, e.g., a microorganism such as a bacterium. In some examples, the methods of amplification include a plurality of nucleic acid primers, e.g., primer pairs, that include a combination of primers that separately amplify two or more regions in one or more genes that is homologous across most, or substantially all, members of a taxonomic category of organisms and one or more, or a plurality of, nucleic acid primers, e.g., primer pairs, that amplify a specific nucleic acid unique to a species of organism. In some examples, primers used in a method of amplification include nucleic acids containing or consisting of nucleic acids provided herein and/or nucleic acids that are capable of amplifying nucleic acids containing or consisting essentially of target sequences provided herein.

In some examples of methods of detecting and/or measuring nucleic acids provided herein, nucleic acids described herein are used as primers and/or probes. For example, in some methods of detecting and/or measuring nucleic acids, nucleic acids are subjected to nucleic acid amplification using nucleic acids described herein as amplification primers, and the presence or absence of one or more nucleic acid amplification products is detected. In some examples, the amplification is performed using a plurality of nucleic acid primer pairs and is conducted in a single multiplex amplification reaction mixture. In some examples, the amplification is performed according to methods of amplification provided herein using any one or more primers, or combination of primers or primers pairs described herein. In some examples, nucleic acids are contacted with probes containing nucleic acids described herein under hybridizing conditions and the presence or absence of the hybridized probe is detected. In some examples, the presence or absence of one or more nucleic acid amplification products is detected using one or more nucleic acids provided herein as a probe (e.g., a detectable or labeled probe). In some examples, the presence or absence of one or more nucleic acid amplification products is detected by obtaining nucleotide sequence information of one or more nucleic acid amplification products. In some examples, the levels (absolute or relative) of detected amplification products are measured and determined. In some examples, the levels (absolute or relative) of detected hybridized probes are measured and determined. In some examples, the nucleic acids being detected and/or measured are nucleic acids of microorganisms, e.g., bacteria. In some examples, the nucleic acids being detected and/or measured are nucleic acids in or from a sample, e.g., a sample of contents of the alimentary canal of an organism.

Also described herein are methods, as well as systems that include the methods, for characterizing, assessing, profiling and/or measuring a population of microorganisms (e.g., bacteria), and/or components or constituents thereof, in a sample (e.g., biological sample), for example, a sample of contents of an alimentary canal of an animal. In some examples, a method for characterizing, assessing, profiling and/or measuring a population of microorganisms in a sample includes subjecting nucleic acids in or from the sample to nucleic acid amplification using a combination of nucleic acid primer pairs that specifically amplify a pre-determined unique nucleic acid sequence of a microorganism's genome, and/or primer pairs that amplify a nucleic acid sequence that occurs in a homologous gene or genome region common to multiple microorganisms but that varies between different microorganisms. In particular examples, the primer pairs that amplify a nucleic acid sequence that occurs in a homologous gene or genome region include one or more primer pairs that amplify nucleic acids comprising nucleotide sequences of one or more hypervariable regions of a prokaryotic 16S rRNA gene. In some examples, the amplification using a combination of nucleic acid primer pairs is conducted in a single multiplex amplification reaction mixture. In some examples, the method for characterizing, assessing, profiling and/or measuring a population of microorganisms includes obtaining sequence information from nucleic acid products of amplification using the combination of primer pairs and/or determining the levels (e.g., relative and/or absolute levels) of nucleic acid products of the amplification and using the sequence information and/or level determinations to identify genera of microorganisms in the sample and species of one or more microorganisms in the sample, and optionally relative and/or absolute levels thereof, to characterize, assess, profile and/or measure a population of microorganisms, and/or components or constituents thereof, in the sample.

Also described herein are methods, as well as systems that include the methods, for diagnosis and/or treatment, reduction in symptoms of, or prevention of microorganism (e.g., bacteria) imbalances and/or dysbiosis in a subject as well as conditions, disorders and diseases associated therewith. For example, in some instances, the microorganism imbalance and/or dysbiosis is in the alimentary canal, or gastrointestinal tract, of the subject. In some examples, diagnosis and/or treatment, reduction in symptoms of, or prevention of microorganism imbalances and/or dysbiosis in a subject includes subjecting nucleic acids in or from one or more samples from a subject to nucleic acid amplification, obtaining sequence information of the nucleic acid amplification products, detecting the presence or absence of one or more genus of microorganism in the sample, and detecting the presence or absence of a disproportionate level of one or more microorganisms in the sample, wherein the presence of a disproportionate level of one or more microorganisms is indicative of a microorganism imbalance and/or dysbiosis in the subject. In some examples of treating a subject having a microorganism imbalance and/or dysbiosis, a subject who has a disproportionate level of one or more microorganisms is treated to establish a balance of microorganisms or biosis in the subject. In some examples, the amplification is performed using a plurality of nucleic acid primer pairs. In some examples, detecting the presence or absence of one or more microorganisms in a sample includes identifying the genus of one or more microorganisms in the sample. In some examples, detecting the presence or absence of one or more microorganisms in a sample includes identifying the genus of one or more microorganisms in the sample and identifying one or more species of microorganism in the sample. In some examples, amplification is performed using a combination of nucleic acid primer pairs that specifically amplify a pre-determined unique nucleic acid sequence of a microorganism's genome, and/or primer pairs that amplify a nucleic acid sequence that occurs in a homologous gene or genome region common to multiple microorganisms but that varies between different microorganisms. In particular examples, the primer pairs that amplify a nucleic acid sequence that occurs in a homologous gene or genome region include one or more primer pairs that amplify nucleic acids comprising sequences of one or more hypervariable regions of a prokaryotic 16S rRNA gene. In some examples, the amplification is conducted in a single multiplex amplification reaction mixture. In some examples, obtaining nucleotide sequence information of nucleic acid amplification products includes detecting a nucleotide sequence using nucleic acids provided herein as a probe (e.g., a detectable or labeled probe). In some examples, obtaining nucleotide sequence information of nucleic acid amplification products includes conducting sequencing of the amplification products. In some examples, detecting the presence or absence of a disproportionate level of one or more microorganisms in the sample includes determining the relative levels of one or more microorganisms in the sample. Treating a subject having a disproportionate level of one or more microorganisms, in some examples, includes administering one or microorganisms to the subject and/or one or more compositions that reduce the levels of or eliminate certain microorganisms, e.g., an antibiotic-containing composition.

In accordance with the claims, new methods andsystems are provided to compress reference sequence databases used in mapping sequence reads for analysis and profiling of microbial populations.

### BRIEF DESCRIPTION OF THE DRAWINGS

The accompanying drawings, which are incorporated into and form a part of the specification, illustrate one or more exemplary embodiments and serve to explain the principles of various exemplary embodiments. The drawings are exemplary and explanatory only and are not to be construed as limiting or restrictive in any way.
FIG. 1 is an illustration depicting the structure of a prokaryotic 16S ribosomal RNA (rRNA) gene showing 9 hypervariable regions 101 (boxes labelled as V1-V9) that are interspersed between conserved regions (white unlabeled boxes) of the gene. Arrows above the gene depict forward and reverse primers that hybridize to sequences of 8 targeted conserved hypervariable segment regions 102 at the indicated positions to amplify the hypervariable region between the arrowheads.
FIG. 2 illustrates a workflow for use in analysis of nucleotide sequence information generated in methods provided herein.
FIG. 3 is a block diagram of an exemplary workflow for processing sequence read data obtained from sequencing of amplified nucleic acids generated in amplification of microbial nucleic acids using 16S rRNA gene primers.
FIG. 4 is a block diagram of an exemplary workflow for processing sequence read data obtained from sequencing of amplified nucleic acids generated in amplification of microbial nucleic acids using species-specific nucleic acid primers.
FIG. 5A and FIG. 5B are each a graphic representation of the results of analysis using Spearman's rho of a comparison of the data of sequencing of four replicate aliquots of DNA amplicon libraries generated from six bacterial samples using a pool of 16S rRNA gene primers for amplification (FIG. 5A) or using a pool of species specific primers for amplification (FIG. 5B).
FIG. 6A is a bar graph showing the results of an analysis of reads from sequencing of a DNA amplicon library generated from a mixed bacteria sample using a pool of 16S rRNA gene primers for nucleic acid amplification. The numbers of reads mapping to different bacterial genera are shown. FIG. 6B depicts analytics from the analysis.
FIG. 7 shows graphs of Spearman's rho analyses of the results of sequencing of four replicate aliquots of a library generated from a mixture of bacterial DNA (Sample no. 1 (MSA1002)) using a 16S primer pool for amplification.
FIG. 8A is a bar graph showing the results of an analysis of reads from sequencing of a DNA amplicon library generated from a mixed bacteria sample using a pool of species-specific gene primers for nucleic acid amplification. The numbers of reads mapping to different bacterial species are shown. FIG. 6B depicts analytics from the analysis.
FIG. 9 shows graphs of Spearman's rho analyses of the results of sequencing of four replicate aliquots of a library generated from a mixture of bacterial DNA (Sample no. 1 (MSA1002)) using a species-specific primer pool for amplification.
FIG. 10 is a block diagram depicting various examples of nucleic acid sequencing platforms, e.g., sequencing instrument 200 can include a fluidic delivery and control unit 202, a sample processing unit 204, a signal detection unit 206, and a data acquisition, analysis and control unit 208.

### DETAILED DESCRIPTION

Unless defined otherwise, all technical and scientific terms used herein have the same meaning as is commonly understood by one of ordinary skill in the art to which these inventions belong.

Described herein are compositions and methods, as well as combinations, kits, and systems that include the compositions and methods, for amplification, detection, characterization, assessment, profiling and/or measurement of nucleic acids, such as nucleic acids of microorganisms, including microbes, e.g., bacteria. The methods provided herein enable highly sensitive, specific, accurate, reproducible detection and identification of one or more microorganisms in sample containing a complex population of microorganisms and other biological materials (e.g., cells that are not microorganisms) in accordance with the claims. The methods further provide for accurate determination of relative and/or absolute levels or abundance of different microorganisms in a such a sample in accordance with the claims. These, and other, aspects of the methods provided herein make them ideally suited for use, for example, in a number of methods, including, but not limited to, accurate and comprehensive methods for assessing or characterizing a population of microorganisms in a sample (e.g., biological sample) or methods for diagnosing and/or treating, reducing symptoms of, or preventing microorganism imbalances and/or dysbiosis in a subject, including such methods described and provided herein. In some examples, the methods further enable multiplex, including highly multiplexed, amplification of nucleic acids of microorganisms in a single amplification reaction mixture and thereby provide for rapid and high-throughput, yet sensitive and readily discernable amplification of nucleic acids from large numbers of different microorganisms as may be found, for example, in numerous different samples, such as from food, water, soil, and animal, e.g., human, specimens such as biofluids (e.g., saliva, sputum, mucus, blood, urine, semen), tissues, skin, respiratory tract, genitourinary tract and the microbiota of an alimentary canal (e.g., gut) of an animal. In some examples, methods provided herein include a multiplex next generation sequencing workflow for accurate, sensitive, high-throughput assessment, characterization or profiling of a population of microorganisms that is used, for example, in correlating the microorganism composition of a subject (e.g., the microbiota of the alimentary canal, gastrointestinal tract, digestive tract or portion thereof of a subject) with states of health and diseases or disorders.

### Definitions

As used herein, the terms "comprises," "comprising," "includes," "including," "has," "having" or any other variation thereof, are intended to cover a non-exclusive inclusion. For example, a process, method, article, or apparatus that comprises a list of features is not necessarily limited only to those features but may include other features not expressly listed or inherent to such process, method, article, or apparatus. Further, unless expressly stated to the contrary, "or" refers to an inclusive-or and not to an exclusive-or.

As used herein, "organism" refers to a life form or living thing. Examples of organisms include microorganisms, unicellular organisms, multicellular organisms, plants and animals. Examples of animals include insects, fish, birds and mammals, including humans and non-human mammals.

As used herein, a "subject" refers to an organism, frequently an animal, e.g., a human or non-human animal, such as a mammal, that is a focus of study, investigation, treatment and/or from which information and/or material (e.g., a sample or specimen) is sought and/or obtained. In some instances, a subject can be a patient.

As used herein, "microorganism," used interchangeably with "microbe" herein, refers to an organism of microscopic or submicroscopic size. Examples of microorganisms include bacteria, archaea, protists and fungi. Many microorganisms are unicellular and capable of dividing and proliferating. Microorganisms include prokaryotes, e.g., bacteria, and non-prokaryotic, e.g., eukaryotic, organisms.

As used herein, "microbiota," refers to a collection, population or community of microbes inhabiting a particular biological niche or ecosystem. Environments in which microbiota are found include soil, water, hydrothermal vents, and hosts, e.g., animal hosts. For example, the human microbiota is made up of the array of microbes colonizing a human, such as on or within human tissues and biofluids. Within the human microbiota are several habitats such as the skin, oral mucosa, respiratory tract, conjunctiva, genitourinary tract and the alimentary canal or tract, or gastrointestinal tract, often referred to as the "gut" microbiota. The genetic component (e.g., genes and genomes) of all the microbial cells in the microbiota is referred to herein as the "microbiome."

As used herein, "sensitivity" with respect to detection and/or identification of a microorganism, e.g., bacterium, in a sample is a performance measure of methods of detecting or identifying a microorganism, for example at the genus and/or species level, that is based on calculating the true positive rate, i.e., the proportion of actual positives that are correctly identified as such. For example, one method of determining sensitivity of a nucleic acid sequencing and analysis method of detection or identification of a microorganism is to perform the method on a known control sample of microorganisms and then determining the percentage of sequence reads that are correctly and unambiguously assigned to a particular genus or species in the sample. The greater the sensitivity of detection or identification, the fewer the number of failures to detect the actual presence of a particular genus or species in a sample.

As used herein, "specificity" with respect to detection and/or identification of a microorganism, e.g., bacterium, in a sample is a performance measure of methods of detecting or identifying a microorganism, for example at the genus and/or species level, that is based on calculating the true negative rate, i.e., the proportion of actual negatives that are correctly identified as such. For example, one method of determining specificity of a nucleic acid sequencing and analysis method of detection or identification of a microorganism is to perform the method on a known control sample of microorganisms that is known to not include particular microorganisms and then determining the percentage of sequence reads that are incorrectly assigned to a particular genus or species that is absent from the sample. The greater the specificity of detection or identification, the fewer the number of errors in identification of a particular genus or species in a sample.

As used herein, the term "nucleic acid" refers to natural nucleic acids, artificial nucleic acids, analogs thereof, or combinations thereof, including polynucleotides and oligonucleotides. As used herein, the terms "polynucleotide" and "oligonucleotide" are used interchangeably and mean single-stranded, double-stranded, partially double-stranded polymers of nucleotides including, but not limited to, 2'-deoxyribonucleotides (DNA) and ribonucleotides (RNA) linked by internucleotide phosphodiester bond linkages, *e.g.* 3'-5' and 2'-5', inverted linkages, *e.g.* 3'-3' and 5'-5', branched structures, or analog nucleic acids. Examples of partially double-stranded nucleic acids include, for example, double-stranded molecules having a 5' and/or 3' single-stranded overhang. Polynucleotides have associated counter ions, such as H⁺, NH₄⁺, trialkylammonium, Mg²⁺, Na⁺ and the like. An oligonucleotide can be composed entirely of deoxyribonucleotides, entirely of ribonucleotides, or chimeric mixtures thereof. Oligonucleotides can be comprised of nucleobase and sugar analogs. Polynucleotides typically range in size from a few monomeric units, *e.g.* 5-40, when they are more commonly frequently referred to in the art as oligonucleotides, to several thousands of monomeric nucleotide units, when they are more commonly referred to in the art as polynucleotides; for purposes of this disclosure, however, both oligonucleotides and polynucleotides may be of any suitable length. Unless denoted otherwise, whenever a oligonucleotide sequence is represented, it will be understood that the nucleotides are in 5' to 3' order from left to right and that "A" denotes deoxyadenosine, "C" denotes deoxycytidine, "G" denotes deoxyguanosine, "T" denotes thymidine, and "U' denotes deoxyuridine. The letters A, C, G, and T may be used to refer to the bases themselves, to nucleosides, or to nucleotides comprising the bases, as is standard in the art. Oligonucleotides are said to have "5' ends" and "3' ends" because mononucleotides are typically reacted to form oligonucleotides via attachment of the 5' phosphate or equivalent group of one nucleotide to the 3' hydroxyl or equivalent group of its neighboring nucleotide, optionally via a phosphodiester or other suitable linkage.

As used herein, the term "nucleotide" and its variants comprises any compound, including without limitation any naturally occurring nucleotide or analog thereof, which is able to hybridize to another nucleotide and/or can bind to, or can be polymerized by, a polymerase. Typically, but not necessarily, selective binding of the nucleotide to the polymerase is followed by polymerization of the nucleotide into a nucleic acid strand by the polymerase; occasionally however the nucleotide may dissociate from the polymerase without becoming incorporated into the nucleic acid strand, an event referred to herein as a "non-productive" event. Such nucleotides include not only naturally occurring nucleotides but also any analogs, regardless of their structure, that can bind selectively to, or can be polymerized by, a polymerase. While naturally occurring nucleotides typically comprise base, sugar and phosphate moieties, the nucleotides of the present disclosure can include compounds lacking any one, some or all of such moieties. In some examples, the nucleotide can optionally include a chain of phosphorus atoms comprising three, four, five, six, seven, eight, nine, ten or more phosphorus atoms. In some examples, the phosphorus chain can be attached to any carbon of a sugar ring, such as the 5' carbon. The phosphorus chain can be linked to the sugar with an intervening O or S. In one example, one or more phosphorus atoms in the chain can be part of a phosphate group having P and O. In another example, the phosphorus atoms in the chain can be linked together with intervening O, NH, S, methylene, substituted methylene, ethylene, substituted ethylene, CNH₂, C(O), C(CH₂), CH₂CH₂, or C(OH)CH₂R (where R can be a 4-pyridine or 1-imidazole). In one example, the phosphorus atoms in the chain can have side groups having O, BH₃, or S. In the phosphorus chain, a phosphorus atom with a side group other than O can be a substituted phosphate group. In the phosphorus chain, phosphorus atoms with an intervening atom other than O can be a substituted phosphate group. Some examples of nucleotide analogs are described in Xu, U.S. Patent No. 7,405,281. In some examples, the nucleotide comprises a label and referred to herein as a "labeled nucleotide"; the label of the labeled nucleotide is referred to herein as a "nucleotide label". In some examples, the label can be in the form of a fluorescent dye attached to the terminal phosphate group, i.e., the phosphate group most distal from the sugar. Some examples of nucleotides that can be used in the disclosed methods and compositions include, but are not limited to, ribonucleotides, deoxyribonucleotides, modified ribonucleotides, modified deoxyribonucleotides, ribonucleotide polyphosphates, deoxyribonucleotide polyphosphates, modified ribonucleotide polyphosphates, modified deoxyribonucleotide polyphosphates, peptide nucleotides, modified peptide nucleotides, metallonucleosides, phosphonate nucleosides, and modified phosphate-sugar backbone nucleotides, analogs, derivatives, or variants of the foregoing compounds, and the like. In some examples, the nucleotide can comprise non-oxygen moieties such as, for example, thio- or borano- moieties, in place of the oxygen moiety bridging the alpha phosphate and the sugar of the nucleotide, or the alpha and beta phosphates of the nucleotide, or the beta and gamma phosphates of the nucleotide, or between any other two phosphates of the nucleotide, or any combination thereof. "Nucleotide 5'-triphosphate" refers to a nucleotide with a triphosphate ester group at the 5' position, and are sometimes denoted as "NTP", or "dNTP" and "ddNTP" to particularly point out the structural features of the ribose sugar. The triphosphate ester group can include sulfur substitutions for the various oxygens, *e.g.* .alpha.-thio-nucleotide 5'-triphosphates. For a review of nucleic acid chemistry, see: Shabarova, Z. and Bogdanov, A. Advanced Organic Chemistry of Nucleic Acids, VCH, New York, 1994.

As used herein, the term "hybridization" is consistent with its use in the art, and refers to the process whereby two nucleic acid molecules undergo base pairing interactions. Two nucleic acid molecule molecules are said to be hybridized when any portion of one nucleic acid molecule is base paired with any portion of the other nucleic acid molecule; it is not necessarily required that the two nucleic acid molecules be hybridized across their entire respective lengths and in some examples, at least one of the nucleic acid molecules can include portions that are not hybridized to the other nucleic acid molecule. "Hybridizing conditions" are conditions (e.g., temperature, ionic strength, etc.) suitable for hybridization of two nucleic acids containing sequences of nucleotides that are capable of undergoing base pairing interaction. The phrase "hybridizing under stringent conditions" and its variants refers to conditions under which hybridization of two nucleic acid sequence, e.g., a target-specific primer and a target sequence, occurs in the presence of high hybridization temperature and low ionic strength. In one exemplary example, stringent hybridization conditions include an aqueous environment containing about 30 mM magnesium sulfate, about 300 mM Tris-sulfate at pH 8.9, and about 90 mM ammonium sulfate at about 60-68°C., or equivalents thereof. As used herein, the phrase "standard hybridization conditions" and its variants refers to conditions under which hybridization of two nucleic acids occurs in the presence of low hybridization temperature and high ionic strength. In one exemplary example, standard hybridization conditions include an aqueous environment containing about 100 mM magnesium sulfate, about 500 mM Tris-sulfate at pH 8.9, and about 200 mM ammonium sulfate at about 50-55°C., or equivalents thereof.

The terms "identity" and "identical" and their variants, as used herein, when used in reference to two or more nucleic acid sequences, refer to similarity in sequence of the two or more sequences (e.g., nucleotide or polypeptide sequences). In the context of two or more homologous sequences, the percent identity, similarity or homology of the sequences or subsequences thereof indicates the percentage of all monomeric units (e.g., nucleotides or amino acids) that are the same (i.e., about 70% identity or more, about 75%, 80%, 85%, 90%, 95%, 98% or 99% identity). The percent identity can be over a specified region, when compared and aligned for maximum correspondence over a comparison window, or designated region as measured using a BLAST or BLAST 2.0 sequence comparison algorithms with default parameters described below, or by manual alignment and visual inspection. Sequences are said to be "substantially identical" when there is at least 85% identity at the amino acid level or at the nucleotide level. Preferably, the identity exists over a region that is at least about 25, 50, or 100 residues in length, or across the entire length of at least one compared sequence. A typical algorithm for determining percent sequence identity and sequence similarity are the BLAST and BLAST 2.0 algorithms, which are described in Altschul et al, Nuc. Acids Res. 25:3389-3402 (1977). Other methods include the algorithms of Smith & Waterman, Adv. Appl. Math. 2:482 (1981), and Needleman & Wunsch, J. Mol. Biol. 48:443 (1970), etc. Another indication that two nucleic acid sequences are substantially identical is that the two molecules or their complements hybridize to each other under stringent hybridization conditions.

The terms "complementary" and "complement" and their variants, as used herein, refer to any two or more nucleic acid sequences (e.g., portions or entireties of template nucleic acid molecules, target sequences and/or primers) that can undergo cumulative base pairing at two or more individual corresponding positions in antiparallel orientation, as in a hybridized duplex. Such base pairing can proceed according to any set of established rules, for example according to Watson-Crick base pairing rules or according to some other base pairing paradigm. Optionally there can be "complete" or "total" complementarity between a first and second nucleic acid sequence where each nucleotide in the first nucleic acid sequence can undergo a stabilizing base pairing interaction with a nucleotide in the corresponding antiparallel position on the second nucleic acid sequence. "Partial" complementarity describes nucleic acid sequences in which at least 20%, but less than 100%, of the residues of one nucleic acid sequence are complementary to residues in the other nucleic acid sequence. In some examples, at least 50%, but less than 100%, of the residues of one nucleic acid sequence are complementary to residues in the other nucleic acid sequence. In some examples, at least 70%, 80%, 90%, 95% or 98%, but less than 100%, of the residues of one nucleic acid sequence are complementary to residues in the other nucleic acid sequence. Sequences are said to be "substantially complementary" when at least 85% of the residues of one nucleic acid sequence are complementary to residues in the other nucleic acid sequence. In some examples, two complementary or substantially complementary sequences are capable of hybridizing to each other under standard or stringent hybridization conditions. "Non-complementary" describes nucleic acid sequences in which less than 20% of the residues of one nucleic acid sequence are complementary to residues in the other nucleic acid sequence. Sequences are said to be "substantially non-complementary" when less than 15% of the residues of one nucleic acid sequence are complementary to residues in the other nucleic acid sequence. In some examples, two non-complementary or substantially non-complementary sequences cannot hybridize to each other under standard or stringent hybridization conditions. A "mismatch" is present at any position in the two opposed nucleotides are not complementary. Complementary nucleotides include nucleotides that are efficiently incorporated by DNA polymerases opposite each other during DNA replication under physiological conditions. In a typical example, complementary nucleotides can form base pairs with each other, such as the A-T/U and G-C base pairs formed through specific Watson-Crick type hydrogen bonding, or base pairs formed through some other type of base pairing paradigm, between the nucleobases of nucleotides and/or polynucleotides in positions antiparallel to each other. The complementarity of other artificial base pairs can be based on other types of hydrogen bonding and/or hydrophobicity of bases and/or shape complementarity between bases.

As used herein, "sample" and its derivatives, is used in its broadest sense and includes any specimen, culture and the like that may include composition of interest, such as a target. In some embodiments, the sample comprises cDNA, RNA, PNA, LNA, chimeric, hybrid, or multiplex-forms of nucleic acids. The sample can include any biological, clinical, surgical, agricultural, atmospheric or aquatic-based specimen containing one or more organisms and/or nucleic acids. One example of a biological or clinical sample is a sample of the contents of the alimentary canal of an animal. The alimentary canal is the continuous passageway, beginning at the mouth and ending at the anus, through which food and liquids are ingested, digested and absorbed and waste is processed and eliminated. The alimentary canal or tract is also referred to herein as the gastrointestinal tract and gut, and includes multiple organs. An example of a sample from the alimentary canal is a fecal sample. In some instances, at least some nucleic acids in a sample may be contained within a cell. In some instances, nucleic acids may be extracted from one or more cells in a sample. In some instances, the term "nucleic acid sample" can refer to a sample containing nucleic acids within a cell or organism or not within a cell or organism and/or nucleic acids extracted from the sample. The term also includes any isolated nucleic acid sample such as expressed RNA, fresh-frozen or formalin-fixed paraffin-embedded nucleic acid specimen.

As used herein, "homologous" or "homolog" and derivatives thereof, when used in reference to a portion of a genome or gene, refers to genomic segments or genes that display conserved sequences of substantial sequence similarity in multiple organisms, e.g., multiple organisms of a domain, kingdom, phylum, class, order, family genus and/or species, but that also have differences in sequence. Examples of homologous genes include, but are not limited to, the 16S rRNA gene, 18S rRNA gene, 23S rRNA gene and ABC transporter genes.

As used herein, "unique" when used in reference to a nucleic acid sequence in an organism or group of organisms refers to a nucleotide sequence of a nucleic acid (e.g., a segment or portion of a genome) in an organism or group of organisms that is sufficiently different from sequences in the genomes of other organisms or other groups of organisms such that it can be used to selectively detect or identify the organism, or members of a group of organisms, and/or distinguish the organism, or members of a group of organisms, from some, most, the majority of or substantially all different organisms or organisms that are not in the group of organisms. Such unique sequences are also referred to herein as "signature sequences" or "signature regions" of nucleic acids of an organism or group of organisms. For example, a nucleic acid sequence of nucleotides may be unique to an individual organism, unique to members of a strain of a species of organism, unique to members of a species of organism, unique to members of a genus of organisms, unique to members of a family of organisms, unique to members of an order of organisms, unique to members of a class of organisms, unique to members of a phylum of organisms, unique to members of a kingdom of organisms and/or unique to members of a domain of organisms. Typically, the difference in a unique sequence is the identity and/or order of consecutive nucleotides or nucleobases in the sequence. In some examples, a unique sequence is unique to the organism in comparison to, or with respect to, some specified group of organisms (e.g., organisms in the same kingdom, phylum, class, order, family, genus, species) but may not be unique to the organism in comparison to the totality of all other organisms or all other organisms outside of the specified group. A unique nucleotide sequence can be any length, for example, between about 20 and 1000 nucleotides, 30 and 750 nucleotides, 40 and 500 nucleotides, 50 and 400 nucleotides, 50 and 350 nucleotides, 50 and 300 nucleotides, 50 and 250 nucleotides, 50 and 200 nucleotides, 50 and 150 nucleotides or 50 and 100 nucleotides. In some examples, a unique nucleotide sequence can be about 1000 nucleotides or less, about 750 nucleotides or less, about 500 nucleotides or less, about 400 nucleotides or less, about 350 nucleotides or less, about 300 nucleotides or less, about 250 nucleotides or less, about 200 nucleotides or less, about 150 nucleotides or less, about 100 nucleotides or less, or about 50 nucleotides or less in length. In some examples, a unique nucleotide sequence can be greater than about 25 nucleotides, greater than about 40 nucleotides, greater than about 50 nucleotides, greater than about 60 nucleotides, greater than about 70 nucleotides, greater than about 75 nucleotides, greater than about 90 nucleotides, greater than about 95 nucleotides, greater than about 100 nucleotides, greater than about 150 nucleotides, greater than about 175 nucleotides, greater than about 200 nucleotides, greater than about 250 nucleotides, greater than about 275 nucleotides, greater than about 300 nucleotides, greater than about 325 nucleotides, greater than about 350 nucleotides or greater than about 400 nucleotides in length. In some examples, the unique sequence is such that it can be used to selectively detect, identify and/or distinguish an organism, or members of a group of organisms, by binding to, hybridizing to and/or being amplified by specific nucleic acid probes and/or primers that specifically or selectively or uniquely bind to, hybridize to and/or amplify the unique sequence, particularly in the presence of nucleic acids of other organisms or organisms that are not members of the group of organisms. For example, in some examples, a unique, or signature, sequence of an organism (e.g., microorganism, such as bacterium), or group of organisms, is a sequence that has less than 60%, less than 65%, less than 70%, less than 75%, less than 80%, less than 81%, less than 82%, less than 83%, less than 84%, less than 85%, less than 86%, less than 87%, less than 88%, less than 89%, less than 90%, less than 91%, less than 92%, less than 93%, less than 94%, or less than 95% identity to a sequence of nucleotides in a different organism or specified group of organisms. In some examples, a unique sequence has less than 90% identity to a sequence of nucleotides in a different organism or specified group of organisms. In some examples, a unique, or signature, sequence of an organism (e.g., microorganism, such as bacterium), or group of organisms, has less than 25%, less than 20%, less than 19%, less than 18%, less than 17%, less than 16%, less than 15%, less than 14%, less than 13%, less than 12%, less than 10%, nucleotides that match nucleotides in a sequence of nucleotides of a similar length in a different organism or specified group of organisms. In some examples, a unique sequence has less than 17% nucleotides that match nucleotides in a sequence of nucleotides in a different organism or specified group of organisms. In some examples, a unique sequence has less than 90% identity to a sequence of nucleotides in a different organism or specified group of organisms and has less than 17% nucleotides that match nucleotides in a sequence of nucleotides in a different organism or specified group of organisms. In some examples, a unique sequence within a group of organisms (e.g., a species of bacteria) is at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical among the majority of or substantially all members (e.g., strains of a species) of the group (e.g., a species). In some examples, a unique sequence within a group of organisms is at least 95%, at least 96%, at least 97% identical among the majority of or substantially all members (e.g., strains of a species) of the group. In some examples, a specified identity of the unique sequence within a group of organisms is among at least or greater than 75%, at least or greater than 80%, at least or greater than 85%, at least or greater than 90%, or at least or greater than 95% of the members of the group. In some examples, the nucleotide sequence of a unique sequence within a group of organisms (e.g., a species of bacteria) has at least 85%, at least 86%, at least 87%, at least 88%, at least 89%, at least 90%, at least 91%, at least 92%, at least 93%, at least 94%, at least 95%, at least 96%, at least 97%, at least 98% or at least 99% matching nucleotides among the members of the group. In some examples, the nucleotide sequence of the unique sequence within a group of organisms has at least 95% nucleotides matching among the members of the group. In some examples, a unique sequence within a group of organisms (e.g., a species of bacteria) is at least 95% identical and has least 95% nucleotides matching among at least or greater than 90% of the members of the group.

As used herein, "synthesizing" and its derivatives, refers to a reaction involving nucleotide polymerization by a polymerase, optionally in a template-dependent fashion. Polymerases synthesize an oligonucleotide via transfer of a nucleoside monophosphate from a nucleoside triphosphate (NTP), deoxynucleoside triphosphate (dNTP) or dideoxynucleoside triphosphate (ddNTP) to the 3' hydroxyl of an extending oligonucleotide chain. For the purposes of this disclosure, synthesizing includes to the serial extension of a hybridized adapter or a target-specific primer via transfer of a nucleoside monophosphate from a deoxynucleoside triphosphate.

As used herein, "polymerase" and its derivatives, refers to any enzyme that can catalyze the polymerization of nucleotides (including analogs thereof) into a nucleic acid strand. Typically but not necessarily, such nucleotide polymerization can occur in a template-dependent fashion. Such polymerases can include without limitation naturally occurring polymerases and any subunits and truncations thereof, mutant polymerases, variant polymerases, recombinant, fusion or otherwise engineered polymerases, chemically modified polymerases, synthetic molecules or assemblies, and any analogs, derivatives or fragments thereof that retain the ability to catalyze such polymerization. Optionally, the polymerase can be a mutant polymerase comprising one or more mutations involving the replacement of one or more amino acids with other amino acids, the insertion or deletion of one or more amino acids from the polymerase, or the linkage of parts of two or more polymerases. Typically, the polymerase comprises one or more active sites at which nucleotide binding and/or catalysis of nucleotide polymerization can occur. Some exemplary polymerases include without limitation DNA polymerases and RNA polymerases. The term "polymerase" and its variants, as used herein, also refers to fusion proteins comprising at least two portions linked to each other, where the first portion comprises a peptide that can catalyze the polymerization of nucleotides into a nucleic acid strand and is linked to a second portion that comprises a second polypeptide. In some examples, the second polypeptide can include a reporter enzyme or a processivity-enhancing domain. Optionally, the polymerase can possess 5' exonuclease activity or terminal transferase activity. In some examples, the polymerase can be optionally reactivated, for example through the use of heat, chemicals or re-addition of new amounts of polymerase into a reaction mixture. In some examples, the polymerase can include a hot-start polymerase or an aptamer based polymerase that optionally can be reactivated.

As used herein, "amplify", "amplifying" or "amplification reaction" and their derivatives, refer to any action or process whereby at least a portion of a nucleic acid molecule (referred to as a template nucleic acid molecule, which can contain a target sequence) is replicated or copied into at least one additional nucleic acid molecule. The additional nucleic acid molecule optionally includes sequence that is substantially identical or substantially complementary to at least some portion of the template nucleic acid molecule. The template nucleic acid molecule can be single-stranded or double-stranded and the additional nucleic acid molecule can independently be single-stranded or double-stranded. In some examples, amplification includes a template-dependent in vitro enzyme-catalyzed reaction for the production of at least one copy of at least some portion of the nucleic acid molecule or the production of at least one copy of a nucleic acid sequence that is complementary to at least some portion of the nucleic acid molecule. Amplification optionally includes linear or exponential replication of a nucleic acid molecule. In some examples, such amplification is performed using isothermal conditions; in other examples, such amplification can include thermocycling. In some examples, the amplification is a multiplex amplification that includes the simultaneous amplification of a plurality of target sequences in a single amplification reaction. At least some of the target sequences can be situated on the same nucleic acid molecule or on different target nucleic acid molecules included in the single amplification reaction. In some examples, "amplification" includes amplification of at least some portion of DNA- and RNA-based nucleic acids alone, or in combination. The amplification reaction can include single- or double-stranded nucleic acid substrates and can further include any processes of amplification techniques known to one of ordinary skill in the art. In some examples, the amplification reaction includes polymerase chain reaction (PCR).

As used herein, "amplification conditions" and its derivatives, refers to conditions suitable for amplifying one or more nucleic acid sequences. Such amplification can be linear or exponential. In some examples, the amplification conditions can include isothermal conditions or alternatively can include thermocyling conditions, or a combination of isothermal and themocycling conditions. In some examples, the conditions suitable for amplifying one or more nucleic acid sequences includes polymerase chain reaction (PCR) conditions. Typically, the amplification conditions refer to a reaction mixture that is sufficient to amplify nucleic acids such as one or more target sequences, or to amplify an amplified target sequence ligated to one or more adapters, e.g., an adapter-ligated amplified target sequence. Amplification conditions include a catalyst for amplification or for nucleic acid synthesis, for example a polymerase; a primer that possesses some degree of complementarity to the nucleic acid to be amplified; and nucleotides, such as deoxyribonucleotide triphosphates (dNTPs) to promote extension of the primer once hybridized to the nucleic acid. The amplification conditions can require hybridization or annealing of a primer to a nucleic acid, extension of the primer and a dissociation step, e.g., denaturing, in which the extended primer is separated from the nucleic acid sequence undergoing amplification. Typically, but not necessarily, amplification conditions can include thermocycling; in some examples, amplification conditions include a plurality of cycles where the amplification steps of annealing, extending and separating are repeated. Typically, the amplification conditions include cations such as Mg⁺⁺ or Mn⁺⁺ (e.g., MgCl₂, etc) and can also include various modifiers of ionic strength.

As defined herein "multiplex amplification" refers to selective and non-random amplification of two or more target sequences within a sample using at least one specific primer. In some examples, multiplex amplification is performed such that some or all of the target sequences are amplified within a single reaction vessel. The "plexy" or "plex" of a given multiplex amplification refers to the number of different target-specific sequences that are amplified during that single multiplex amplification. In some examples, the plexy can be about 12-plex, 24-plex, 48-plex, 74-plex, 96-plex, 120-plex, 144-plex, 168-plex, 192-plex, 216-plex, 240-plex, 264-plex, 288-plex, 312-plex, 336-plex, 360-plex, 384-plex, or 398-plex.

As used herein, the term "polymerase chain reaction" ("PCR") refers to the method of K. B. Mullis U.S. Pat. Nos. 4,683,195 and 4,683,202, which describe a method for increasing the concentration of a segment of a polynucleotide of interest in a mixture of expressed RNA or cDNA without cloning or purification. This process for amplifying the polynucleotide of interest consists of introducing a large excess of two oligonucleotide primers to the DNA mixture containing the desired polynucleotide of interest, followed by a precise sequence of thermal cycling in the presence of a DNA polymerase. The two primers are complementary to their respective strands of the double stranded polynucleotide of interest. To effect amplification, the mixture is denatured and the primers then annealed to their complementary sequences within the polynucleotide of interest molecule. Following annealing, the primers are extended with a polymerase to form a new pair of complementary strands. The steps of denaturation, primer annealing and polymerase extension can be repeated many times (i.e., denaturation, annealing and extension constitute one "cycle"; there can be numerous "cycles") to obtain a high concentration of an amplified segment of the desired polynucleotide of interest. The length of the amplified segment of the desired polynucleotide of interest (amplicon) is determined by the relative positions of the primers with respect to each other, and therefore, this length is a controllable parameter. By virtue of repeating the process, the method is referred to as the "polymerase chain reaction" (hereinafter "PCR"). Because the desired amplified segments of the polynucleotide of interest become the predominant nucleic acid sequences (in terms of concentration) in the mixture, they are said to be "PCR amplified". As defined herein, target nucleic acid molecules within a sample including a plurality of target nucleic acid molecules are amplified via PCR. In a modification to the method discussed above, the target nucleic acid molecules can be PCR amplified using a plurality of different primer pairs, in some cases, one or more primer pairs per target nucleic acid molecule of interest, thereby forming a multiplex PCR reaction. Using multiplex PCR, it is possible to simultaneously amplify multiple nucleic acid molecules of interest from a sample to form amplified target sequences. It is also possible to detect the amplified target sequences by several different methodologies (e.g., quantitation with a bioanalyzer or qPCR, hybridization with a labeled probe; incorporation of biotinylated primers followed by avidin-enzyme conjugate detection; incorporation of ³²P-labeled deoxynucleotide triphosphates, such as dCTP or dATP, into the amplified target sequence). Any oligonucleotide sequence can be amplified with the appropriate set of primers, thereby allowing for the amplification of target nucleic acid molecules from RNA, cDNA, formalin-fixed paraffin-embedded DNA, fine-needle biopsies and various other sources. In particular, the amplified target sequences created by the multiplex PCR process as disclosed herein, are themselves efficient substrates for subsequent PCR amplification or various downstream assays or manipulations.

As used herein, "reamplifying" or "reamplification" and their derivatives refer to any process whereby at least a portion of an amplified nucleic acid molecule is further amplified via any suitable amplification process (referred to in some examples as a "secondary" amplification or "reamplification", thereby producing a reamplified nucleic acid molecule. The secondary amplification need not be identical to the original amplification process whereby the amplified nucleic acid molecule was produced; nor need the reamplified nucleic acid molecule be completely identical or completely complementary to the amplified nucleic acid molecule; all that is required is that the reamplified nucleic acid molecule include at least a portion of the amplified nucleic acid molecule or its complement. For example, the reamplification can involve the use of different amplification conditions and/or different primers, including different target-specific primers than the primary amplification.

The term "extension" and its variants, as used herein, when used in reference to a given primer, comprises any in vivo or in vitro enzymatic activity characteristic of a given polymerase that relates to polymerization of one or more nucleotides onto an end of an existing nucleic acid molecule. Typically but not necessarily such primer extension occurs in a template-dependent fashion; during template-dependent extension, the order and selection of bases is driven by established base pairing rules, which can include Watson-Crick type base pairing rules or alternatively (and especially in the case of extension reactions involving nucleotide analogs) by some other type of base pairing paradigm. In one non-limiting example, extension occurs via polymerization of nucleotides on the 3'OH end of the nucleic acid molecule by the polymerase.

The term "portion" and its variants, as used herein, when used in reference to a given nucleic acid molecule, for example a primer or a template nucleic acid molecule, comprises any number of contiguous nucleotides within the length of the nucleic acid molecule, including the partial or entire length of the nucleic acid molecule.

As used herein, "target sequence" or "target sequence of interest" and its derivatives, refers to any single or double-stranded nucleic acid sequence that can be bound to, hybridized to, amplified and/or synthesized according to the disclosure, including, for example, any nucleic acid sequence suspected to be, expected to be, or that could potentially be present in a sample. In some embodiments, the target sequence is present in double-stranded form and includes at least a portion of the particular nucleotide sequence to be bound, hybridized, amplified and/or synthesized, or its complement, prior to the addition of specific primers or appended adapters. In some embodiments, a target sequence is a part of a target. For example, a target nucleic acid sequence can be a sequence located in a target gene, a target genome and/or a target organism, e.g., bacteria, or a specific family, genus or species of a target organism, e.g., *Ruminococcaceae* family, *Ruminococcus* genus, and *R. gnavus* species. Target sequences can include the nucleic acids to which primers useful in an amplification or synthesis reaction can hybridize prior to extension by a polymerase. In some instances, a target sequence is a sequence adjacent to and contiguous with a sequence to which a primer used to amplify the target sequence hybridizes. In some embodiments, the term refers to a nucleic acid sequence whose sequence identity, ordering or location of nucleotides is determined by one or more of the methods of the disclosure.

As used herein, "amplified target sequence" and its derivatives, refers to a nucleic acid sequence produced by the amplification of/amplifying the target sequence using specific primers and the methods provided herein. The amplified target sequences may be either of the same sense (the positive strand produced in the second round and subsequent even-numbered rounds of amplification) or antisense (i.e., the negative strand produced during the first and subsequent odd-numbered rounds of amplification) with respect to the target sequences. In some examples, the amplified target sequences are typically less than 50% complementary to any portion of another amplified target sequence in the reaction. As used herein, "amplicon" refers to the total nucleic acid that results from an amplification using primers and methods such as provided herein. In some instances, an amplicon may be the same as a target sequence. In some instances, when a target nucleic acid sequence is defined as not including primer sequences, an amplicon includes an amplified target sequence as well as the primers used to amplify the target sequence located at each end of the amplified target sequence. In such cases, the target sequence can be referred to as the "insert" of the amplicon.

As used herein, the term "primer," "probe," and derivatives thereof refer to any polynucleotide that can hybridize to a target sequence of interest. In some embodiments, the primer can also serve to prime nucleic acid synthesis. Typically, the primer functions as a substrate onto which nucleotides can be polymerized by a polymerase; in some embodiments, however, the primer can become incorporated into the synthesized nucleic acid strand and provide a site to which another primer can hybridize to prime synthesis of a new strand that is complementary to the synthesized nucleic acid molecule. A primer or probe may be comprised of any combination of nucleotides or analogs thereof, which may be optionally linked to form a linear polymer of any suitable length. In some embodiments, the primer is a single-stranded oligonucleotide or polynucleotide. (For purposes of this disclosure, the terms 'polynucleotide" and "oligonucleotide" are used interchangeably herein and do not necessarily indicate any difference in length between the two). In some embodiments, the primer or probe is single-stranded but it can also be double-stranded. A primer or probe optionally occurs naturally, as in a purified restriction digest, or can be produced synthetically. In some embodiments, the primer acts as a point of initiation for amplification or synthesis when exposed to amplification or synthesis conditions; such amplification or synthesis can occur in a template-dependent fashion and optionally results in formation of a primer extension product that is complementary to at least a portion of the target sequence. Exemplary amplification or synthesis conditions can include contacting the primer with a polynucleotide template (e.g., a template including a target sequence), nucleotides and an inducing agent such as a polymerase at a suitable temperature and pH to induce polymerization of nucleotides onto an end of the target-specific primer. If double-stranded, a primer or probe can optionally be treated to separate its strands before being used to prepare primer extension products. In some embodiments, the primer probe is an oligodeoxyribonucleotide or an oligoribonucleotide. In some embodiments, the primer or probe can include one or more nucleotide analogs. The exact length and/or composition, including sequence, of a primer or probe can influence many properties, including melting temperature (Tm), GC content, formation of secondary structures, repeat nucleotide motifs, length of predicted primer extension products, extent of coverage across a nucleic acid molecule of interest, number of primers present in a single amplification or synthesis reaction, presence of nucleotide analogs or modified nucleotides within the primers, and the like. In some embodiments, a primer can be paired with a compatible primer within an amplification or synthesis reaction to form a primer pair made up of a forward primer and a reverse primer. In some embodiments, the forward primer of the primer pair includes a sequence that is substantially complementary to at least a portion of a strand of a nucleic acid molecule, and the reverse primer of the primer pair includes a sequence that is substantially identical to at least of portion of the strand. In some embodiments, the forward primer and the reverse primer are capable of hybridizing to opposite strands of a nucleic acid duplex. Optionally, the forward primer primes synthesis of a first nucleic acid strand, and the reverse primer primes synthesis of a second nucleic acid strand, wherein the first and second strands are substantially complementary to each other, or can hybridize to form a double-stranded nucleic acid molecule. In some embodiments, one end of an amplification or synthesis product is defined by the forward primer and the other end of the amplification or synthesis product is defined by the reverse primer. In some embodiments, where the amplification or synthesis of lengthy primer extension products is required, such as amplifying an exon, coding region, or gene, several primer pairs can be created that span the desired length to enable sufficient amplification of the region. In some embodiments, a primer or probe can include one or more cleavable groups. Primers and probes can be of any length. In some embodiments, a probe may be about about 200 or less nucleotides, 175 nucleotides or less, 150 or less nucleotides, 125 nucleotides or less, 100 or less nucleotides, 90 nucleotides or less, 80 or less nucleotides, 75 nucleotides or less, 70 or less nucleotides, 60 nucleotides or less, 55 or less nucleotides, 50 nucleotides or less, 40 or less nucleotides, 35 nucleotides or less, 30 or less nucleotides, 25 nucleotides or less, 20 or less nucleotides, 15 nucleotides or less, or 10 or less nucleotides in length. In some embodiments, primer lengths are in the range of about 10 to about 60 nucleotides, about 12 to about 50 nucleotides and about 15 to about 40 nucleotides in length. Typically, a primer is capable of hybridizing to a corresponding target sequence and undergoing primer extension when exposed to amplification conditions in the presence of dNTPs and a polymerase. In some instances, the particular nucleotide sequence or a portion of the primer is known at the outset of the amplification reaction or can be determined by one or more of the methods disclosed herein. In some embodiments, a primer includes one or more cleavable groups at one or more locations within the primer. In some embodiments, a mixture of primers can be degenerate primers. Degenerate primers are primers having similar sequences but that differ at one or more nucleotide positions such that one primer may have an A at the position, another may have a G at the same position, another may have a T at the same position and a fourth primer may have a C at the same position. Probes and/or primers may be labeled. Labels are frequently used in detecting a primer or probe that has bound to or hybridized to another nucleic acid, for example, for the purpose of detecting a particular sequence to which the primer or probe specifically binds. Compositions and methods for labeling nucleic acids for use as detectable probes are known in the art and include attaching a reporter or signal-generating moiety to the probe. Examples of detectable labels include, but are not limited to, fluorescent, luminescent, chemiluminescent, chromogenic, radioactive and colorimetric moieties. The labels can be directly detectable or can be part of a system for generating a detectable signal.

As used herein, "capable of" when used with reference to processes such as amplifying, binding to or hybridizing to, refers to the ability of a nucleic acid, e.g., a primer or primer pair, to interact with another nucleic acid (e.g., target nucleic acid, target sequence, template) in such a way as to perform, participate in performing and/or accomplishing the stated process. For example, a nucleic acid capable of binding to another nucleic acid or other molecule through intermolecular forces or bonds is able to form a stable attachment to the other nucleic acid or molecule. A nucleic acid capable of hybridizing to another nucleic acid is able to undergo base pairing interactions with the other nucleic acid. In some examples, the nucleic acid is capable of hybridizing under low or high stringency conditions. Nucleic acids capable of amplifying another nucleic acid are able to serve as primers in a polymerization reaction that results in extension of the nucleic acid and generation of a complement of a template nucleic acid strand which can be a copy of an opposing strand of the template nucleic acid strand. A nucleic acid is specifically or selectively capable of binding to, hybridizing to and/or amplifying if it is capable of binding to a certain target molecule, hybridizing to a certain target nucleic acid and/or amplifying a certain target nucleic acid without substantially binding to, hybridizing to and/or amplifying a molecule or nucleic acid that is not the target molecule or nucleic acid. In some instances, such binding, hybridizing and/or amplifying is referred to as "uniquely" binding, hybridizing and/or amplifying a target molecule or nucleic acid.

As used herein, the term "separately" when used in reference to amplifying a nucleic acid refers to a primer or primer pair that is used to amplify a particular defined region of a nucleic acid, e.g., a gene, without amplifying another region of the nucleic acid. For example, primer pairs that separately amplify different hypervariable regions of a 16S rRNA gene each amplify only a single hypervariable region to generate separate amplicons for each different region and do not generate amplicons that contain more than one hypervariable region.

As defined herein, a "cleavable group" refers to any moiety that once incorporated into a nucleic acid can be cleaved under appropriate conditions. For example, a cleavable group can be incorporated into a target-specific primer, an amplified sequence, an adapter or a nucleic acid molecule of the sample. In an exemplary example a target-specific primer can include a cleavable group that becomes incorporated into the amplified product and is subsequently cleaved after amplification, thereby removing a portion, or all, of the target-specific primer from the amplified product. The cleavable group can be cleaved or otherwise removed from a target-specific primer, an amplified sequence, an adapter or a nucleic acid molecule of the sample by any acceptable means. For example, a cleavable group can be removed from a target-specific primer, an amplified sequence, an adapter or a nucleic acid molecule of the sample by enzymatic, thermal, photo-oxidative or chemical treatment. In one aspect, a cleavable group can include a nucleobase that is not naturally occurring. For example, an oligodeoxyribonucleotide can include one or more RNA nucleobases, such as uracil that can be removed by a uracil glycosylase. In some examples, a cleavable group can include one or more modified nucleobases (such as 7-methylguanine, 8-oxo-guanine, xanthine, hypoxanthine, 5,6-dihydrouracil or 5-methylcytosine) or one or more modified nucleosides (i.e., 7-methylguanosine, 8-oxo-deoxyguanosine, xanthosine, inosine, dihydrouridine or 5-methylcytidine). The modified nucleobases or nucleotides can be removed from the nucleic acid by enzymatic, chemical or thermal means. In one example, a cleavable group can include a moiety that can be removed from a primer after amplification (or synthesis) upon exposure to ultraviolet light (i.e., bromodeoxyuridine). In another example, a cleavable group can include methylated cytosine. Typically, methylated cytosine can be cleaved from a primer for example, after induction of amplification (or synthesis), upon sodium bisulfite treatment. In some examples, a cleavable moiety can include a restriction site. For example, a primer or target sequence can include a nucleic acid sequence that is specific to one or more restriction enzymes, and following amplification (or synthesis), the primer or target sequence can be treated with the one or more restriction enzymes such that the cleavable group is removed. Typically, one or more cleavable groups can be included at one or more locations with a target-specific primer, an amplified sequence, an adapter or a nucleic acid molecule of the sample.

As used herein, "cleavage step" and its derivatives, refers to any process by which a cleavable group is cleaved or otherwise removed from a target-specific primer, an amplified sequence, an adapter or a nucleic acid molecule of the sample. In some examples, the cleavage steps involves a chemical, thermal, photo-oxidative or digestive process.

In some embodiments, a primer is a single-stranded or double-stranded polynucleotide, typically an oligonucleotide, that includes at least one sequence that is at least 50% complementary, typically at least 75% complementary or at least 85% complementary, more typically at least 90% complementary, more typically at least 95% complementary, more typically at least 98% or at least 99% complementary, or 100% complementary or identical, to at least a portion of a nucleic acid molecule that includes a target sequence. In such instances, the primer and target sequence are described as "corresponding" to each other and, in some instances, the primer may be referred to as being "directed to" the target sequence. In some embodiments, a primer is capable of hybridizing to at least a portion of its corresponding target sequence (or to a complement of the target sequence); such hybridization can optionally be performed under standard hybridization conditions or under stringent hybridization conditions. In some embodiments, a primer is not capable of hybridizing to the target sequence, or to its complement, but is capable of hybridizing to a portion of a nucleic acid strand including the target sequence, or to its complement, e.g., sequence upstream or downstream or adjacent to the target sequence. In some embodiments, a primer includes at least one sequence that is at least 75% complementary, typically at least 85% complementary, more typically at least 90% complementary, more typically at least 95% complementary, more typically at least 98% complementary, or more typically at least 99% complementary, to at least a portion of the target sequence itself; in other embodiments, a primer includes at least one sequence that is at least 75% complementary, typically at least 85% complementary, more typically at least 90% complementary, more typically at least 95% complementary, more typically at least 98% complementary, or more typically at least 99% complementary, to at least a portion of the nucleic acid molecule other than the target sequence. In some embodiments, such primers are referred to as a "specific primer" or "selective primer" which is substantially non-complementary to target sequences other than the target sequence to which it corresponds or portion of a nucleic acid to which it corresponds that includes the target sequence; optionally, a specific primer, or selective primer, is substantially non-complementary to other nucleic acid molecules that may be present in a mixture of nucleic acids, e.g, in a sample. In some embodiments, nucleic acid molecules present in a sample that do not include or correspond to a target sequence (or to a complement of the target sequence) are referred to as "non-specific" sequences or "non-specific nucleic acids". In some embodiments, a specific primer or selective primer is designed to include a nucleotide sequence that is substantially complementary to at least a portion of its corresponding target sequence. In some embodiments, a specific primer or selective primer is at least 95% complementary, or at least 99% complementary, 100% complementary or identical, across its entire length to at least a portion of a nucleic acid molecule that includes its corresponding target sequence. In some embodiments, a specific primer or selective primer can be at least 90%, at least 95% complementary, at least 98% complementary or at least 99% complementary, 100% complementary or identical, across its entire length to at least a portion of its corresponding target sequence. In some embodiments, a forward specific primer and a reverse specific primer define a specific primer pair (or selective primer pair) that can be used to amplify the target sequence via template-dependent primer extension. Typically, each primer of a specific primer pair includes at least one sequence that is substantially complementary to at least a portion of a nucleic acid molecule including a corresponding target sequence but that is less than 50% complementary to at least one other target sequence in a mixture or sample. In some embodiments, amplification can be performed using multiple specific primer pairs in a single amplification reaction, wherein each primer pair includes a forward specific primer and a reverse specific primer, each including at least one sequence that is substantially complementary or substantially identical to a corresponding target sequence in the mixture or sample, and each specific primer pair having a different corresponding target sequence. In some embodiments, a specific primer can be substantially non-complementary at its 3' end or its 5' end to any other specific primer present in an amplification reaction. In some embodiments, a specific primer can include minimal cross hybridization to other specific primers in an amplification reaction. In some embodiments, specific primers include minimal cross-hybridization to non-specific sequences in an amplification reaction mixture. In some embodiments, specific primers include minimal self-complementarity. In some embodiments, specific primers can include one or more cleavable groups located at the 3' end. In some embodiments, specific primers can include one or more cleavable groups located near or about a central nucleotide of the specific primer. In some embodiments, one of more specific primers includes only non-cleavable nucleotides at the 5' end of the specific primer. In some embodiments, a specific primer includes minimal nucleotide sequence overlap at the 3'end or the 5' end of the primer as compared to one or more different specific primers, optionally in the same amplification reaction. In some embodiments 1, 2, 3, 4, 5, 6, 7, 8, 9, 10 or more, specific primers in a single reaction mixture include one or more of the above embodiments. In some embodiments, substantially all of a plurality of specific primers in a single reaction mixture includes one or more of the above embodiments.

As used herein, the terms "ligating", "ligation" and their derivatives refer to the act or process for covalently linking two or more molecules together, for example, covalently linking two or more nucleic acid molecules to each other. In some examples, ligation includes joining nicks between adjacent nucleotides of nucleic acids. In some examples, ligation includes forming a covalent bond between an end of a first and an end of a second nucleic acid molecule. In some examples, for example wherein the nucleic acid molecules to be ligated include conventional nucleotide residues, the litgation can include forming a covalent bond between a 5' phosphate group of one nucleic acid and a 3' hydroxyl group of a second nucleic acid thereby forming a ligated nucleic acid molecule. In some examples, any means for joining nicks or bonding a 5'phosphate to a 3' hydroxyl between adjacent nucleotides can be employed. In an exemplary example, an enzyme such as a ligase can be used. For the purposes of this disclosure, an amplified target sequence can be ligated to an adapter to generate an adapter-ligated amplified target sequence.

As used herein, "ligase" and its derivatives, refers to any agent capable of catalyzing the ligation of two substrate molecules. In some examples, the ligase includes an enzyme capable of catalyzing the joining of nicks between adjacent nucleotides of a nucleic acid. In some examples, the ligase includes an enzyme capable of catalyzing the formation of a covalent bond between a 5' phosphate of one nucleic acid molecule to a 3' hydroxyl of another nucleic acid molecule thereby forming a ligated nucleic acid molecule. Suitable ligases may include, but not limited to, T4 DNA ligase, T4 RNA ligase, and *E. coli* DNA ligase.

As used herein, "ligation conditions" and its derivatives, refers to conditions suitable for ligating two molecules to each other. In some examples, the ligation conditions are suitable for sealing nicks or gaps between nucleic acids. As defined herein, a "nick" or "gap" refers to a nucleic acid molecule that lacks a directly bound 5' phosphate of a mononucleotide pentose ring to a 3' hydroxyl of a neighboring mononucleotide pentose ring within internal nucleotides of a nucleic acid sequence. As used herein, the term nick or gap is consistent with the use of the term in the art. Typically, a nick or gap can be ligated in the presence of an enzyme, such as ligase at an appropriate temperature and pH. In some examples, T4 DNA ligase can join a nick between nucleic acids at a temperature of about 70-72°C.

As used herein, "blunt-end ligation" and its derivatives, refers to ligation of two blunt-end double-stranded nucleic acid molecules to each other. A "blunt end" refers to an end of a double-stranded nucleic acid molecule wherein substantially all of the nucleotides in the end of one strand of the nucleic acid molecule are base paired with opposing nucleotides in the other strand of the same nucleic acid molecule. A nucleic acid molecule is not blunt ended if it has an end that includes a single-stranded portion greater than two nucleotides in length, referred to herein as an "overhang". In some examples, the end of nucleic acid molecule does not include any single stranded portion, such that every nucleotide in one strand of the end is based paired with opposing nucleotides in the other strand of the same nucleic acid molecule. In some examples, the ends of the two blunt ended nucleic acid molecules that become ligated to each other do not include any overlapping, shared or complementary sequence. Typically, blunted-end ligation excludes the use of additional oligonucleotide adapters to assist in the ligation of the double-stranded amplified target sequence to the double-stranded adapter, such as patch oligonucleotides as described in Mitra and Varley, US2010/0129874, published May 27, 2010. In some examples, blunt-ended ligation includes a nick translation reaction to seal a nick created during the ligation process.

As used herein, the terms "adapter" or "adapter and its complements" and their derivatives, refers to any linear oligonucleotide which can be ligated to a nucleic acid molecule of the disclosure. Optionally, the adapter includes a nucleic acid sequence that is not substantially complementary to the 3' end or the 5' end of at least one target sequences within the sample. In some examples, the adapter is substantially non-complementary to the 3' end or the 5' end of any target sequence present in the sample. In some examples, the adapter includes any single stranded or double-stranded linear oligonucleotide that is not substantially complementary to an amplified target sequence. In some examples, the adapter is substantially non-complementary to at least one, some or all of the nucleic acid molecules of the sample. In some examples, suitable adapter lengths are in the range of about 10-100 nucleotides, about 12-60 nucleotides and about 15-50 nucleotides in length. An adapter can include any combination of nucleotides and/or nucleic acids. In some aspects, the adapter can include one or more cleavable groups at one or more locations. In another aspect, the adapter can include a sequence that is substantially identical, or substantially complementary, to at least a portion of a primer, for example a universal primer. In some examples, the adapter can include a barcode or tag to assist with downstream cataloguing, identification or sequencing. In some examples, a single-stranded adapter can act as a substrate for amplification when ligated to an amplified target sequence, particularly in the presence of a polymerase and dNTPs under suitable temperature and pH.

As used herein, "DNA barcode" or "DNA tagging sequence" and its derivatives, refers to a unique short (6-14 nucleotide) nucleic acid sequence within an adapter that can act as a 'key' to distinguish or separate a plurality of amplified target sequences in a sample. For the purposes of this disclosure, a DNA barcode or DNA tagging sequence can be incorporated into the nucleotide sequence of an adapter.

As used herein, "GC content" and its derivatives, refers to the cytosine and guanine content of a nucleic acid molecule. In some examples, the GC content of a specific primer (or adapter) of is 85% or lower. In some examples, the GC content of a specific primer or adapter is between 15-85%.

### Compositions

Compositions described herein include compositions containing one or more nucleic acids, including, for example, but not limited to, double-stranded, partially double-stranded, single-stranded, modified and unmodified nucleic acids. In some examples, the nucleic acid is single-stranded, e.g., a single-stranded oligonucleotide that can be used as a primer and/or probe. In some examples, a composition provided herein contains two nucleic acids, e.g., a nucleic acid primer pair, that are capable of amplifying a particular nucleic acid in a nucleic acid amplification process or reaction. Compositions containing or consisting of a plurality of nucleic acids, e.g., primers and/or probes, including, for example, a plurality of primer pairs, are also provided herein. In some examples, a nucleic acid and/or nucleic acid pair (e.g., primer pair) in a composition provided herein is capable of binding to, hybridizing to and/or amplifying a nucleic acid contained within the genome of one or more microorganisms, such as, for example, bacteria or archaea. In some examples, a nucleic acid or nucleic acids (e.g., primer pair) in a composition provided herein is/are capable of binding to, hybridizing to and/or amplifying, or specifically binding to, hybridizing to and/or amplifying, a nucleic acid (e.g., a nucleic acid from a microorganism, such as a bacterium) that contains a nucleotide sequence set forth in SEQ ID NOS: 1605-1979 in Table 17, or SEQ ID NOS: 1605-1826 in Table 17, or SEQ ID NOS: 1605-1820 in Table 17A, or SEQ ID NOS: 1827-1979 in Table 17C, or SEQ ID NOS: 1827-1976 in Table 17C, or a substantially identical or similar sequence of any of these sequences. In some examples, a nucleic acid or nucleic acids (e.g., primer pair) in a composition provided herein is capable of amplifying, or specifically amplifying, a nucleic acid, such as a nucleic acid from a microorganism, e.g., bacteria, that contains a nucleotide sequence set forth in SEQ ID NOS: 1605-1979 in Table 17, or SEQ ID NOS: 1605-1826 in Table 17, or SEQ ID NOS: 1605-1820 in Table 17A, or SEQ ID NOS: 1827-1979 in Table 17C, or SEQ ID NOS: 1827-1976 in Table 17C to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence that consists essentially of a sequence selected from SEQ ID NOS: 1605-1979 in Table 17, or SEQ ID NOS: 1605-1826 in Table 17, or SEQ ID NOS: 1605-1820 in Table 17A, or SEQ ID NOS: 1827-1979 in Table 17C, or SEQ ID NOS: 1827-1976 in Table 17C, or a substantially identical or similar sequence, and optionally containing nucleic acid primer sequences at the 5' and 3' ends of the sequence. In some examples, a composition contains a plurality of nucleic acids that are capable of binding to, hybridizing to and/or amplifying, or specifically of binding to, hybridizing to and/or amplifying, a plurality of nucleic acids each of which contains a nucleotide sequence selected from SEQ ID NOS: 1605-1979 in Table 17, or SEQ ID NOS: 1605-1826 in Table 17, or SEQ ID NOS: 1605-1820 in Table 17A, or SEQ ID NOS: 1827-1979 in Table 17C, or SEQ ID NOS: 1827-1976 in Table 17C. In some examples, a composition contains a plurality of nucleic acids (e.g., primer pairs) that are capable of amplifying, or specifically amplifying, a plurality of nucleic acids (such as a nucleic acids from a microorganism, e.g., bacteria) each of which contains a nucleotide sequence selected from SEQ ID NOS: 1605-1979 in Table 17, or SEQ ID NOS: 1605-1826 in Table 17, or SEQ ID NOS: 1605-1820 in Table 17A, or SEQ ID NOS: 1827-1979 in Table 17C, or SEQ ID NOS: 1827-1976 in Table 17C, or a substantially identical or similar sequence, to generate amplicon sequences that are less than about 500, less than about 475, less than about 450, less than about 400, less than about 375, less than about 350, less than about 300, less than about 275, less than about 250, less than about 200, less than about 175, less than about 150, or less than about 100 nucleotides in length, or amplicon sequences that consist essentially of a sequence selected from SEQ ID NOS: 1605-1979 in Table 17, or SEQ ID NOS: 1605-1826 in Table 17, or SEQ ID NOS: 1605-1820 in Table 17A, or SEQ ID NOS: 1827-1979 in Table 17C, or SEQ ID NOS: 1827-1976 in Table 17C and optionally containing the nucleic acid primer sequences at the 5' and 3' ends of the sequence. In some examples, a composition provided herein contains a plurality of nucleic acids each of which comprises a nucleotide sequence selected from SEQ ID NOS: 1605-1979 in Table 17, or SEQ ID NOS: 1605-1826 in Table 17, or SEQ ID NOS: 1605-1820 in Table 17A, or SEQ ID NOS: 1827-1979 in Table 17C, or SEQ ID NOS: 1827-1976 in Table 17C, or a substantially identical or similar sequence. In some examples, the composition contains a plurality of nucleic acids each of which contains, or consists essentially of, a nucleotide sequence selected from SEQ ID NOS: 1605-1826 in Table 17, or SEQ ID NOS: 1605-1820 in Table 17A, or a substantially identical or similar sequence, and optionally containing nucleic acid primer sequences at the 5' and 3' ends of the sequence, and is less than about 500, less than about 475, less than about 450, less than about 400, less than about 375, less than about 350, less than about 300, less than about 275, less than about 250, less than about 200, less than about 175, less than about 150, or less than about 100 nucleotides in length.

In some examples, a nucleic acid in a composition provided herein includes or consists essentially of a nucleotide sequence in Table 15 or Table 16, or a nucleotide sequence in Table 15 or Table 16 in which one or more thymine bases is substituted with a uracil base. In some examples, a nucleic acid provided herein includes or consists essentially of a nucleotide sequence selected from SEQ ID NOS: 11-16, 23 and 24 of Table 15, SEQ ID NOS: 35-40, 47 and 48 of Table 15, SEQ ID NOS: 49-480 of Table 16A, SEQ ID NOS: 49-452 and 457-472 of Table 16A, SEQ ID NOS: 521-820 of Table 16C, SEQ ID NOS: 827-1258 of Table 16D, SEQ ID NOS: 827-1230 and 1235-1250 of Table 16D, or SEQ ID NOS: 1299-1598 of Table 16F or a substantially identical or similar sequence. In some examples, a composition contains or consists essentially of a plurality of nucleic acids each of which contains or consists essentially of a sequence selected from the sequences in Table 15, SEQ ID NOS: 1-24 of Table 15, SEQ ID NOS: 11-16, 23 and 24 of Table 15, SEQ ID NOS: 25-48 of Table 15, SEQ ID NOS: 35-40, 47 and 48 of Table 15, the sequences in Table 16, SEQ ID NOS: 49-520 of Table 16, SEQ ID NOS: 49-452, 457-472 and 481-520 of Table 16, SEQ ID NOS: 49-492 of Table 16, SEQ ID NOS: 49-452, 457-472 and 481-492 of Table 16, SEQ ID NOS: 49-480 of Table 16A, SEQ ID NOS: 49-452 and 457-472 of Table 16A, SEQ ID NOS: 521-826 of Table 16C, SEQ ID NOS: 521-820 of Table 16C, SEQ ID NOS: 827-1298 of Table 16, SEQ ID NOS: 827-1230, 1235-1250 and 1259-1298 of Table 16, SEQ ID NOS: 827-1270 of Table 16, SEQ ID NOS: 827-1230, 1235-1250 and 1259-1270 of Table 16, SEQ ID NOS: 827-1258 of Table 16D, SEQ ID NOS: 827-1230 and 1235-1250 of Table 16D, SEQ ID NOS: 1299-1604 of Table 16F, SEQ ID NOS: 1299-1598 of Table 16F, substantially identical or similar sequences and/or or any of the aforementioned nucleotide sequences in in which one or more thymine bases is substituted with a uracil base. In some examples, nucleic acids in a composition provided herein include one or more pairs of nucleic acids (e.g., primer pairs). Primer pairs include pairs of (i.e., 2) nucleic acids (polynucleotides) which can be used to amplify nucleic acids. Examples of primer pairs are shown in Tables 15 and 16 as "Primer 1" and "Primer 2" in each row of the tables that are capable of amplifying a nucleic acid sequence contained in the corresponding region (hypervariable region) of a prokaryotic (e.g., bacterial) 16S rRNA gene (Table 15) or contained in the corresponding species of microorganism (Table 16). In some examples, nucleic acids in a composition provided herein include, or consist essentially of, one or more pairs of nucleic acids that contain or consist essentially of the nucleotide sequences of one or more pairs of nucleotide sequences in Table 15 or Table 16, one or more pairs of nucleotide sequences selected from the pairs of sequences set forth in SEQ ID NOS: 1-24 of Table 15, SEQ ID NOS: 25-48 of Table 15, SEQ ID NOS: 11-16, 23 and 24 of Table 15, SEQ ID NOS: 35-40, 47 and 48 of Table 15, SEQ ID NOS: 49-520 of Table 16, SEQ ID NOS: 49-452, 457-472 and 481-520 of Table 16, SEQ ID NOS: 49-492 of Table 16, SEQ ID NOS: 49-452, 457-472 and 481-492 of Table 16, SEQ ID NOS: 49-480 of Table 16A, SEQ ID NOS: 49-452 and 457-472 of Table 16A, SEQ ID NOS: 521-826 of Table 16C, SEQ ID NOS: 521-820 of Table 16C, SEQ ID NOS: 827-1298 of Table 16, SEQ ID NOS: 827-1230, 1235-1250 and 1259-1298 of Table 16, SEQ ID NOS: 827-1270 of Table 16, SEQ ID NOS: 827-1230, 1235-1250 and 1259-1270 of Table 16, SEQ ID NOS: 827-1258 of Table 16D, SEQ ID NOS: 827-1230 and 1235-1250 of Table 16D, SEQ ID NOS: 1299-1604 of Table 16F, or SEQ ID NOS: 1299-1598 of Table 16F, substantially identical or similar sequences and/or or any of the aforementioned nucleotide sequences of primer pairs in which one or more thymine bases is substituted with a uracil base. In some examples, a composition contains, or consists essentially of, a plurality of pairs of nucleic acids (e.g., primer pairs) that contain or consist essentially of the nucleotide sequences of two or more pairs of nucleotide sequences in Table 15 or Table 16, two or more pairs of nucleotide sequences selected from the pairs of sequences set forth in SEQ ID NOS: 1-24 of Table 15, SEQ ID NOS: 25-48 of Table 15, SEQ ID NOS: 11-16, 23 and 24 of Table 15, SEQ ID NOS: 35-40, 47 and 48 of Table 15, SEQ ID NOS: 49-520 of Table 16, SEQ ID NOS: 49-452, 457-472 and 481-520 of Table 16, SEQ ID NOS: 49-492 of Table 16, SEQ ID NOS: 49-452, 457-472 and 481-492 of Table 16, SEQ ID NOS: 49-480 of Table 16A, SEQ ID NOS: 49-452 and 457-472 of Table 16A, SEQ ID NOS: 521-826 of Table 16C, SEQ ID NOS: 521-820 of Table 16C, SEQ ID NOS: 827-1298 of Table 16, SEQ ID NOS: 827-1230, 1235-1250 and 1259-1298 of Table 16, SEQ ID NOS: 827-1270 of Table 16, SEQ ID NOS: 827-1230, 1235-1250 and 1259-1270 of Table 16, SEQ ID NOS: 827-1258 of Table 16D, SEQ ID NOS: 827-1230 and 1235-1250 of Table 16D, SEQ ID NOS: 1299-1604 of Table 16F, or SEQ ID NOS: 1299-1598 of Table 16F, or substantially identical or similar sequences, and/or or any of the aforementioned nucleotide sequences of primer pairs in which one or more thymine bases is substituted with a uracil base.

In some examples, a nucleic acid or nucleic acid pair, and optionally degenerate sequences thereof, binds to, hybridizes and/or amplifies a specific nucleic acid sequence unique to a particular microorganism (e.g., a species of bacteria). Such a nucleic acid or nucleic acid pair, and optionally degenerate sequences thereof, is referred to herein as "microorganism-specific" or "species-specific" and amplifies nucleic acids in a microorganism-specific or species-specific manner to produce a single amplification product having a unique sequence among microorganisms (e.g., bacteria) or a group of microorganisms in the presence of nucleic acids from the microorganism in an amplification reaction. Nonlimiting examples of sequences of such nucleic acids and nucleic acid primer pairs are provided in Table 16.

In some examples, a nucleic acid pair, and optionally degenerate sequences thereof, is capable of amplifying a sequence in a homologous gene or genomic region common to multiple, most, a majority, substantially all, or all microorganisms in a taxonomic group, but that varies between different microorganisms. Taxonomic groups include kingdom, domain, phylum, class, order, family and species. In one example, the taxonomic group is the *Bacteria* kingdom. Such a nucleic acid pair, or primer pair, and optionally degenerate sequences thereof, that is capable of amplifying a sequence in a homologous gene or genomic region common to multiple, most, a majority, substantially all, or all microorganisms in a kingdom, but that varies between different microorganisms in the kingdom, is referred to herein as "kingdom-encompassing" and amplifies nucleic acid in microorganisms in the kingdom in a kingdom-encompassing manner to produce multiple amplification products having different nucleotide sequences of different microorganisms (e.g., different bacteria) in the kingdom in the presence of nucleic acids from microorganisms (e.g., bacteria) in an amplification reaction. Conserved sequences of nucleic acids can be found in the genomes of different organisms or microbes. Such sequences can be identical or share substantial similarity in the different genomes (see, e.g., Isenbarger et al. (2008) *Orig Life Evol Biosph* doi:10.1007/s11084-008-9148-z). In many instances, conserved sequences are located in essential genes, e.g., housekeeping genes, that encode elements required across a category or group of organisms or microbes for carrying out basic biochemical functions of survival. However, through evolution and adaptation of organisms and microbes to diverse conditions, even homologous genes diverged and contain sequences that vary between different organisms and microbes and that may be so divergent as to be unique to specific organisms or microbes such that they can be used to identify an individual organism or microbe or a related group (e.g., species) of organisms or microbes. Homologous genes include, for example, some essential genes required for basic functioning and survival of microorganisms. In some examples, the homologous gene is a 16S rRNA gene, 18S rRNA gene or an 23S rRNA gene common to multiple different organisms, or microorganisms (e.g., multiple different bacteria). For example, the nucleic acids include one or more primer pairs that separately amplify two or more regions, e.g., hypervariable regions, in a prokaryotic, e.g., bacterial, 16S rRNA gene. Nonlimiting examples of such nucleic acid primer pairs are provided in Table 15.

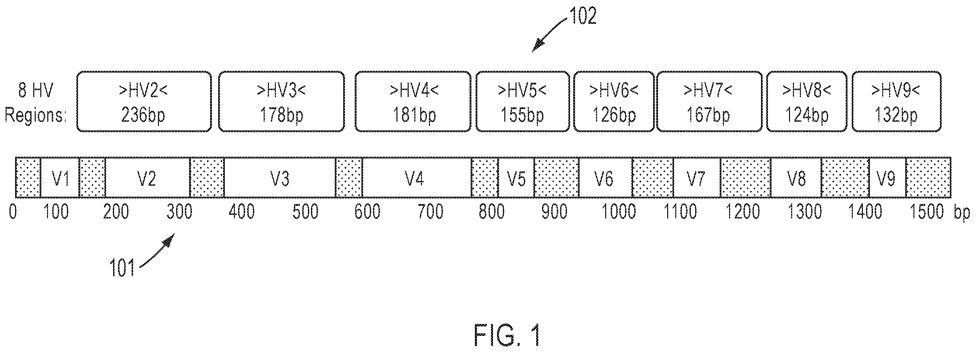

Variable region analysis has been used for taxonomic classification of prokaryotes, for example, in methods using nucleic acid primers that hybridize to conserved sequences flanking a variable region. Homologous genes that contain multiple variable regions interspersed between conserved regions are particularly useful in such methods because they provide multiple sequences that can be analyzed to more accurately and definitively identify individual constituents of a population of targeted elements. One example of such a gene is the prokaryotic 16S rRNA gene encoding ribosomal RNAs which are the main structural and catalytic components of ribosomes. The 16S ribosomal RNA (rRNA) gene of bacteria and archaea is about 1500-1700 base pairs long and includes 9 hypervariable regions of varying conservation, which are commonly referred to a V1-V9 (FIG. 1), that are interspersed between conservative or conserved regions (see, e.g., Wang and Qian (2009) PloS ONE 4:e7401 and Kim et al. (2011) J Microbiol Meth 84:81-87). Exemplary 16S rRNA gene sequences are known and include those contained in the Greengenes database (http://greengenes.lbl.gov), SILVA database (www.arb-silva.de) and GRD-Genomic-Based 16 Ribosomal RNA Database (https://metasystems.riken.jp/grd/). Sequences of the hypervariable regions of 16S rRNA genes which differ in different microorganisms can be used to identify microorganisms in a sample. Instead of specifically amplifying a hypervariable region of every possible microorganism that could be present in a sample by using many oligonucleotide primers, each specific to the hypervariable region of each organism, it is possible to utilize the conserved, highly similar or identical sequences flanking the hypervariable regions as primer-binding sequences to which one, or a small number of, primer pair(s) will bind and amplify a hypervariable region in substantially all of the microorganisms, e.g., bacteria, in a sample. This allows specific nucleic acids that can be used to identify a microorganism to be amplified from substantially all the microorganisms which can then be sequenced for efficient profiling of the population. However, the results of such methods tend to be inconsistent, and often incomplete in determining most or all microorganisms present in a sample, particularly samples containing multiple different microorganisms. Furthermore, such methods typically do not reliably or accurately discriminate between species of microorganisms, if they are able to distinguish species at all. Most such methods utilize primers intended for amplification of one or a few, and less than all, hypervariable regions of the 16S rRNA gene. If more than a limited number of hypervariable regions are targeted for amplification in these methods, the method typically requires multiple separate amplification reactions for different primers due to overlap of primer sequences, which introduces inefficiencies in resource use and time into the methods. Also, such methods often include primer pairs designed to amplify two or more hypervariable regions (e.g., V2-V3 or V3-V4) as a single amplicon which results in longer amplicons for sequencing.

### Kingdom-Encompassing Nucleic Acids

Nucleic acid primer pairs are provided herein that separately amplify nucleic acids comprising sequences located in multiple hypervariable regions of the prokaryotic 16S rRNA gene. In some examples, there is little (e.g., less than or equal to 7 nucleotides) to no overlap of the nucleotide sequences of any two of the 16s rRNA gene primers that separately amplify nucleic acids comprising sequences located in multiple hypervariable regions. In some aspects, the primer pairs amplify 16s rRNA gene sequences less than or equal to about 200 nucleotides in length, for example, between about 125 and 200 nucleotides in length. In some examples, compositions provided herein contain a plurality of nucleic acid primer pairs that includes at least 2, at least 3, at least 4, at least 5, at least 6, or at least 7 separate primer pairs, and optionally degenerate variants thereof, which separately amplify nucleic acids comprising sequences of a different one of 2, 3, 4, 5, 6, or 7 different hypervariable regions, respectively, in a prokaryotic 16s rRNA gene in a nucleic acid amplification reaction. In some examples, compositions provided herein contain a plurality of nucleic acid primer pairs that includes at least 8 separate primer pairs, and optionally degenerate variants thereof, which separately amplify a nucleic acid comprising a sequence of one of 8 different hypervariable regions in a prokaryotic 16s rRNA gene in a nucleic acid amplification reaction. In some examples, a composition includes a combination of primer pairs, wherein the primer pairs in the combination of primer pairs separately amplify nucleic acids comprising sequences located in 3 or more hypervariable regions of a prokaryotic 16S rRNA gene and wherein one of the 3 or more regions is a V5 region. Degenerate primer variants, containing, for example, different nucleotides at 1 or 2 positions in the primer sequences, are included in some compositions to ensure amplification of 16S rRNA genes containing minor variations in conserved regions. Non-limiting examples of nucleotide sequences of primer pairs that separately amplify 8 hypervariable regions (V2, V3, V4, V5, V6, V7, V8 and V9) of the prokaryotic 16S rRNA gene are listed in Table 15. In some examples, compositions provided herein contain or consist essentially of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24 nucleic acids, or primer pairs, in which the nucleic acids or primer pairs contain or consist essentially of sequences selected from those in Table 15 or from SEQ ID NOS: 1-24 of Table 15, SEQ ID NOS: 25-48 of Table 15, SEQ ID NOS: 11-16, 23 and 24 of Table 15, or SEQ ID NOS: 35-40, 47 and 48 of Table 15, or substantially identical or similar sequences. In some examples, compositions provided herein contain or consist essentially of nucleic acids, or primer pairs, in which the nucleic acids or primer pairs separately contain, or consist essentially of, all sequences of SEQ ID NOS: 1-24 of Table 15 and/or all sequences of SEQ ID NOS: 25-48 of Table 15. In some of the examples of compositions provided herein containing a plurality of nucleic acid primer pairs that separately amplify nucleic acids comprising sequences located in multiple hypervariable regions of a prokaryotic 16S rRNA gene, the plurality of primer pairs provide at least 85%, or at least 90%, or at least 92%, or at least 95% or at least 98%, or at least 99% or 100% coverage of different bacterial 16S rRNA gene sequences in a given database containing bacterial 16S rRNA gene sequences. In some examples of compositions provided herein containing a plurality of nucleic acid primer pairs that separately amplify nucleic acids comprising sequences located in multiple hypervariable regions of a prokaryotic 16S rRNA gene, the plurality of primer pairs are capable of amplifying all or substantially all microbial (e.g., bacterial) nucleic acids in a sample containing a mixture of microorganisms (e.g. bacteria) of at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, or at least 100 or more different genera.

### Species/Microorganism-Specific Nucleic Acids

Nucleic acids and nucleic acid pairs (e.g., primer pairs) are provided herein that bind to, hybridize to and/or amplify a specific nucleic acid sequence unique to a particular microorganism (e.g., a species, subspecies or strain of bacteria). Such microorganism-specific (e.g., bacteria-specific or species-specific) nucleic acids can be used, for example, as specific, selective probes and/or primers to greatly increase the depth and exactness of the detection and identification of microorganisms in a sample and significantly enhance characterization, assessment, measuring and/or profiling of a population or community of microorganisms as well as the components or constituents thereof. Such information is required to gain a complete understanding of the biodiversity of a community of microorganisms, e.g., microbiota of the alimentary tract of an animal. Exemplary nucleic acid sequences provided in Table 16 bind to, hybridize to and/or amplify a specific nucleic acid sequence unique to species in more than 40 different genera of microorganism (bacteria), or at least 43 different genera of microorganism, and unique to more than 70, or at least 73, or at least 74, or at least 75, different species of microorganism (bacteria).

Microorganism-specific nucleic acids provide many advantages, for example, in completely and accurately assessing, characterizing, measuring and/or profiling the composition of a population of microorganisms, e.g., microbiota, and determining relationships of individual microorganisms, as well as relating and/or correlating a community of microorganisms, and a state (e.g., health, degree of balance, susceptibility to certain conditions, responsiveness to treatment) of a subject and/or environment. The microbiota of a human, i.e., microorganisms, including bacteria, associated with different areas of a human subject, contains more than 10 times more microorganism cells than human cells. The microbiota includes commensal microorganisms, in addition to occurrences of pathogenic microorganisms. While the significance of identifying pathogenic microbes within an animal is relatively clear, profiling the complex composition of all types of microorganisms in the microbiota is also of great significance in understanding health of an animal and potential therapeutic interventions in disorders and disease. For example, microorganisms residing in the alimentary tract of animals, often referred to as the "gut microbiome," contribute to animal metabolism, and evidence supports roles of the gut microbiome in inflammatory bowel diseases, autoimmune disorders, cardiometabolic disorders, cancer and neuropsychiatric disorders and diseases.

Compositions and methods described herein, including microorganism-specific and kingdom-encompassing nucleic acids, and use of them in sample analysis, enable not only a comprehensive survey of the entirety and relative levels of genera of microorganisms (e.g., bacteria), but also detailed identification of species of microorganisms that can be tailored to focus on one or more particular microorganisms of interest that may be significant in certain states of health and disease or imbalance. For example, provided herein are nucleic acids and/or nucleic acid primer pairs that bind to, hybridize to and/or amplify a specific nucleic acid sequence unique to a particular microorganism (e.g., a species, subspecies or strain of bacteria), i.e., microorganism-specific nucleic acids. In some examples, the nucleic acids are capable of specifically binding to and/or hybridizing to a target nucleic acid sequence contained within the genome of the microorganism in a mixture comprising nucleic acids of multiple different microorganisms, for example, in a mixture comprising nucleic acids of the genome of a different microorganism that is in the same genus of the microorganism containing the target nucleic acid sequence. In some examples, the nucleic acids that specifically bind to and/or hybridize to a nucleic acid sequence contained with the genome of a microorganism do not bind to or hybridize to a nucleic acid contained within any other genus of microorganism or within any other species of microorganism. In some examples, a primer pair specifically amplifies a specific target nucleic acid sequence unique to a particular microorganism in an amplification reaction mixture comprising nucleic acids of the genomes of multiple different microorganisms, and in particular examples, in an amplification reaction mixture comprising nucleic acid of the genome of a different microorganism that is in the same genus of the microorganism containing the target nucleic acid sequence. In some examples, the primer pair does not amplify a nucleic acid sequence contained within any other genus of microorganism or within any other species of organism. In some examples, combinations of nucleic acids include microorganism-specific nucleic acids and/or primer pairs that specifically bind to, hybridize to and/or amplify a nucleic acid sequence contained in the genome of one or more microorganisms (e.g., bacteria) implicated in one or more conditions, disorders and/or diseases. In particular examples of compositions provided herein, the composition includes a nucleic acid and/or a primer pair that specifically binds to, hybridizes to and/or amplifies a target nucleic acid sequence contained within a genome of a microorganism selected from the microorganisms in Table 1. In particular examples, the target nucleic acid sequence contained in the genome of a microorganism selected from the microorganisms in Table 1 is unique to the microorganism. In some examples, the composition includes, or consists essentially of, a plurality of nucleic acids and/or primer pairs that include at least one nucleic acid that specifically binds to and/or hybridizes to a target nucleic acid for each of the microorganisms in Table 1 and/or at least one primer pair that specifically amplifies a genomic target nucleic acid for each of the microorganisms in Table 1. In some examples, the composition includes, or consists essentially of, a plurality of nucleic acids and/or primer pairs that include at least one nucleic acid that specifically binds to and/or hybridizes to a target nucleic acid for each of the microorganisms in Table 1, except for, or excluding, *Actinomyces viscosus* and/or *Blautia coccoides,* or except for, or excluding, *Actinomyces viscosus, Blautia coccoides* and/or *Helicobacter salomonis.* In some examples, the composition includes, or consists essentially of, a plurality of nucleic acids and/or primer pairs that include at least one primer pair that specifically amplifies a genomic target nucleic acid for each of the microorganisms in Table 1 except for, or excluding, *Actinomyces viscosus* and/or *Blautia coccoides,* or except for, or excluding, *Actinomyces viscosus, Blautia coccoides* and/or *Helicobacter salomonis.* In some examples, the plurality of primer pairs includes, or consists essentially of, different primer pairs that specifically and separately amplify different genomic target nucleic acids contained within, or within at least, 5, 10, 15, 20, 25, 30, 35, 40, 45, 50, 55, 60, 70, 71, 72, 73, 74, 75 or more of the microorganisms in Table 1. In some examples, the plurality of nucleic acid primer pairs includes, or consists essentially of, a set of nucleic acid primer pairs in which each different nucleic acid primer pair specifically amplifies a different unique nucleic acid sequence contained in a different one of each of the genomes of the group of microorganisms in Table 1 or the group of microorganisms in Table 1 except for, or excluding, *Actinomyces viscosus* and/or *Blautia coccoides,* or except for, or excluding, *Actinomyces viscosus, Blautia coccoides* and/or *Helicobacter salomonis.* In particular examples, the target nucleic acid sequences contained in the genome of the different microorganisms are unique to each of the microorganisms.

| **TABLE 1 - Microorganisms** | |
|---|---|
| **GENUS** | **SPECIES** |
| *Actinomyces* | *Viscosus* |
| *Akkermansia* | *Muciniphila* |
| *Anaerococcus* | *Vaginalis* |
| *Atopobium* | *Parvulum* |
| *Bacteroides* | *fragilis, nordii, thetaiotaomicron, vulgatus* |
| *Barnesiella* | *Intestinihominis* |
| *Bifidobacterium* | *adolescentis, animalis, bifidum, longum* |
| *Blautia* | *coccoides, obeum* |
| *Borreliella* | *Burgdorferi* |
| *Campylobacter* | *concisus, curvus, gracilis, hominis, jejuni, rectus* |
| *Chlamydia* | *pneumoniae, trachomatis* |
| *Citrobacter* | *Rodentium* |
| *Cloacibacillus* | *Porcorum* |
| *Clostridioides* | *Difficile* |
| *Collinsella* | *aerofaciens, stercoris* |
| *Cutibacterium* | *Acnes* |
| *Desulfovibrio* | *Alaskensis* |
| *Dorea* | *Formicigenerans* |
| *Enterococcus* | *faecium, faecalis, gallinarum, hirae* |
| *Escherichia* | *Coli* |
| *Eubacterium* | *limosum, rectale* |
| *Faecalibacterium* | *Prausnitzii* |
| *Fusobacterium* | *Nucleatum* |
| *Gardnerella* | *Vaginalis* |
| *Gemmiger* | *Formicilis* |
| *Helicobacter* | *bilis, bizzozeronii, hepaticus, pylori, salomonis* |
| *Holdemania* | *Filiformis* |
| *Klebsiella* | *Pneumoniae* |
| *Lactobacillus* | *acidophilus, delbrueckii, johnsonii, murinus, reuteri, rhamnosus* |
| *Lactococcus* | *Lactis* |
| *Mycoplasma* | *fermentans, penetrans* |
| *Parabacteroides* | *distasonis, merdae* |
| *Parvimonas* | *Micra* |
| *Peptostreptococcus* | *anerobius, stomatis* |
| *Phascolarctobacterium* | *Faecium* |
| *Porphyromonas* | *Gingivalis* |
| *Prevotella* | *copri, histicola* |
| *Proteus* | *Mirabilis* |
| *Roseburia* | *Intestinalis* |
| *Ruminococcus* | *bromii, gnavus* |
| *Slackia* | *Exigua* |
| *Streptococcus* | *gallolyticus, infantarius* |
| *Veillonella* | *Parvula* |

In some examples, nucleic acids and/or nucleic acid primer pairs provided herein bind to, hybridize to and/or amplify, or specifically bind to, hybridize to and/or amplify, a nucleic acid (such as a nucleic acid from a microorganism, e.g., bacteria) that contains, or consists essentially of, a nucleotide sequence selected from among SEQ ID NOS: 1605-1979 in Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C and/or substantially identical or similar nucleotide sequences. In some examples, nucleic acid primer pairs provided herein are capable of amplifying, or specifically amplifying, a nucleic acid (such as a nucleic acid from a microorganism, e.g., bacteria) containing, or consisting essentially of, a nucleotide sequence selected from among SEQ ID NOS: 1605-1979 in Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon that consists essentially of a nucleotide sequence selected from among SEQ ID NOS: 1605-1979 in Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, and optionally containing primer sequences attached at the 5' and 3' ends. In some examples, compositions provided herein contain a combination of a plurality of microorganism-specific nucleic acids and/or primer pairs in which within the plurality of nucleic acids and/or primer pairs, there are different nucleic acids and/or primer pairs that bind to, hybridize to and/or amplify (or specifically bind to, hybridize to and/or amplify) at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 125, at least 150, at least 175, at least 200, at least 225, at least 250, at least 275, or at least 300 or more different nucleic acids containing or consisting essentially of a different one of the sequences of SEQ ID NOS: 1605-1979 in Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, and optionally containing primers attached at the 3' and 5' ends. In some examples, such different nucleic acids and/or primer pairs amplify different nucleic acids containing a different one of the sequences of SEQ ID NOS: 1605-1979 in Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C to generate amplicon sequences that are less than about 500, less than about 475, less than about 450, less than about 400, less than about 375, less than about 350, less than about 300, less than about 275, less than about 250, less than about 200, less than about 175, less than about 150, or less than about 100 nucleotides in length, or amplicon sequences that consist essentially of a nucleotide sequence selected from SEQ ID NOS: 1605-1979 in Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C and optionally primer sequences attached at the 5' and 3' end of the sequence. In some examples, a combination of microorganism-specific nucleic acids or nucleic acid primer pairs includes or consists essentially of two or more nucleic acids or primer pairs containing, or consisting essentially of, a nucleotide sequence or pair of sequences (for primer pairs) selected from Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a sequence or sequences substantially identical or similar thereto, or any of the aforementioned nucleotide sequences of nucleic acids or primer pairs in which one or more thymine bases is substituted with a uracil base. In some examples, a combination of microorganism-specific nucleic acids or nucleic acid primer pairs includes or consists essentially of a plurality of nucleic acids or primer pairs wherein there is at least one nucleic acid or primer pair separately containing, or consisting essentially of, each nucleotide sequence or pair of sequences (for primer pairs) of SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or substantially identical or similar sequences, and/or or any of the aforementioned nucleotide sequences of nucleic acids or primer pairs in which one or more thymine bases is substituted with a uracil base. In some examples, compositions provided herein contain or consist essentially of at least 1, at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 125, at least 150, at least 175, at least 200, at least 225, at least 250, at least 275, at least 300 or more, or all of the nucleic acids, or all of the primer pairs, containing or consisting essentially of sequences selected from Table 16 or from SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a sequence or sequences substantially identical or similar thereto, or any of the aforementioned nucleotide sequences of nucleic acids or primer pairs in which one or more thymine bases is substituted with a uracil base.

In some examples, a combination of nucleic acids and/or nucleic acid primer pairs includes two or more nucleic acids and/or nucleic acid primer pairs that bind to, hybridize to and/or amplify, or specifically bind to, hybridize to and/or amplify, a unique nucleic acid sequence contained in the genome of one or more of *Akkermansia muciniphila, Bacteroides vulgatus, Bifidobacterium adolescentis, Campylobacter concisus, Campylobacter jejuni, Clostridioides difficile, Escherichia coli, Eubacterium rectale, Helicobacter bilis, Helicobacter hepaticus, Lactobacillus delbrueckii, Parabacteroides distasonis, Ruminococcus bromii, Streptococcus gallolyticus,* and *Streptococcus infantarius* (referred to herein as "Group A" microorganisms; see Table 2A), which are species implicated as having a role in multiple conditions, diseases and/or disorders, including, for example oncological conditions including, for example, response to immuno-oncology treatment and cancer, gastrointestinal disorders, including, for example, irritable bowel syndrome, inflammatory bowel disease and coeliac disease, and autoimmune diseases, including, for example, lupus and rheumatoid arthritis. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group A. In some examples, the combination of nucleic acids and/or nucleic acid primer pairs includes or consists essentially of nucleic acids and/or nucleic acid primer pairs that bind to, hybridize to and/or amplify, or specifically bind to, hybridize to and/or amplify, a nucleic acid (such as a nucleic acid from a microorganism, e.g., bacteria) containing a sequence selected from SEQ ID NOS: 1607-1609, 1619, 1620, 1635-1637, 1643-1645, 1663-1670, 1679-1684, 1699-1701, 1728-1730, 1752, 1753, 1801, 1802, 1809, 1810, 1827, 1828, 1831-1833, 1844, 1845, 1852-1856, 1864, 1876-1885, 1889-1891, 1899, 1900, 1932, 1933, 1968 and 1972 of Table 17 or a sequence that is substantially identical or similar to any of the aforementioned sequences. In some examples, the combination of nucleic acids and/or nucleic acid primer pairs includes or consists essentially of nucleic acids and/or nucleic acid primer pairs that bind to, hybridize to and/or amplify, or specifically bind to, hybridize to and/or amplify, a nucleic acid (such as a nucleic acid from a microorganism, e.g., bacteria) containing a sequence selected from SEQ ID NOS: 1607-1609, 1619, 1620, 1635-1637, 1643-1645, 1663-1670, 1679-1684, 1699-1701, 1728-1730, 1752, 1753, 1801, 1802, 1809 and 1810 of Table 17 or a sequence that is substantially identical or similar to any of the aforementioned sequences. In some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from SEQ ID NOS: 1607-1609, 1619, 1620, 1635-1637, 1643-1645, 1663-1670, 1679-1684, 1699-1701, 1728-1730, 1752, 1753, 1801, 1802, 1809, 1810, 1827, 1828, 1831-1833, 1844, 1845, 1852-1856, 1864, 1876-1885, 1889-1891, 1899, 1900, 1932, 1933, 1968 and 1972 of Table 17 (or a sequence that is substantially identical or similar to any of the aforementioned sequences) to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from SEQ ID NOS: 1607-1609, 1619, 1620, 1635-1637, 1643-1645, 1663-1670, 1679-1684, 1699-1701, 1728-1730, 1752, 1753, 1801, 1802, 1809, 1810, 1827, 1828, 1831-1833, 1844, 1845, 1852-1856, 1864, 1876-1885, 1889-1891, 1899, 1900, 1932, 1933, 1968 and 1972 of Table 17, or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from SEQ ID NOS: 1607-1609, 1619, 1620, 1635-1637, 1643-1645, 1663-1670, 1679-1684, 1699-1701, 1728-1730, 1752, 1753, 1801, 1802, 1809, and 1810 of Table 17 (or a sequence that is substantially identical or similar to any of the aforementioned sequences) to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from SEQ ID NOS: 1607-1609, 1619, 1620, 1635-1637, 1643-1645, 1663-1670, 1679-1684, 1699-1701, 1728-1730, 1752, 1753, 1801, 1802, 1809 and 1810 of Table 17, or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from SEQ ID NOS: 53-58, 77-80, 109-114, 125-130, 165-180, 197-208, 237-242, 295-300, 343-346, 441-444, 457-460, 493-498, 511-520, 521-524, 529-534, 555-558, 571-580, 595, 596, 619-638, 645-650, 665-668, 731-734, 803, 804, 811, 812 and/or SEQ ID NOS: 831-836, 855-858, 887-892, 903-908, 943-958, 975-986, 1015-1020, 1073-1078, 1121-1124, 1219-1222, 1235-1238, 1271-1276, 1289-1298, 1299-1302, 1307-1312, 1333-1336, 1349-1358, 1373, 1374, 1397-1416, 1423-1428, 1443-1446, 1509-1512, 1581, 1582, 1589, and 1590 in Table 16, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from SEQ ID NOS: 53-58, 77-80, 109-114, 125-130, 165-180, 197-208, 237-242, 295-300, 343-346, 441-444, 457-460, 493-498 and 511-520 in Table 16 and/or SEQ ID NOS: 831-836, 855-858, 887-892, 903-908, 943-958, 975-986, 1015-1020, 1073-1078, 1121-1124, 1219-1222, 1235-1238, 1271-1276, 1289-1298 in Table 16, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from SEQ ID NOS: 53-58, 77-80, 109-114, 125-130, 165-180, 197-208, 237-242, 295-300, 343-346, 441-444, 457-460 in Table 16A and/or SEQ ID NOS: 831-836, 855-858, 887-892, 903-908, 943-958, 975-986, 1015-1020, 1073-1078, 1121-1124, 1219-1222, 1235-1238 in Table 16D, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences of SEQ ID NOS: 53-58, 77-80, 109-114, 125-130, 165-180, 197-208, 237-242, 295-300, 343-346, 441-444, 457-460, 493-498 and 511-520 in Table 16 and/or SEQ ID NOS: 831-836, 855-858, 887-892, 903-908, 943-958, 975-986, 1015-1020, 1073-1078, 1121-1124, 1219-1222, 1235-1238, 1271-1276, 1289-1298 in Table 16, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences of SEQ ID NOS: 53-58, 77-80, 109-114, 125-130, 165-180, 197-208, 237-242, 295-300, 343-346, 441-444, 457-460 in Table 16A and/or SEQ ID NOS: 831-836, 855-858, 887-892, 903-908, 943-958, 975-986, 1015-1020, 1073-1078, 1121-1124, 1219-1222, 1235-1238 in Table 16D, or substantially identical or similar sequences, or any of the aforementioned nucleotide sequences of nucleic acids or primer pairs in which one or more thymine bases is substituted with a uracil base.

| **TABLE 2 - MICROORGANISM GROUPS:** | | |
|---|---|---|
| **Exemplary Combinations Of Nucleic Acids, Primers And Primer Pairs That Bind To, Hybridize To and/or Amplify A Unique Nucleic Acid Sequence Contained In The Genomes Of One Or More Of The Microorganisms in the Group for Groups A (Table 2A), B (Table 2B), C (Table 2C), D (Table 2D) and E (Table 2E)** | | |
| I. Combination includes or consists essentially of nucleic acids and/or primer pairs that bind to, hybridize to and/or amplify, or specifically bind to, hybridize to and/or amplify, a nucleic acid containing a sequence selected from SEQ ID NOS:__________ (SEE FIRST COLUMN OF TABLES 2A-2E) | II. Combination includes or consists essentially of primers and/or primer pairs capable of amplifying or specifically amplifying a nucleic acid containing a sequence selected from (a) SEQ ID NOS:__________ to generate an amplicon sequence <about 500, <about 475, <about 450, <about 400, <about 375, <about 350, <about 300, <about 275, <about 250, <about 200, <about 175, <about 150, <about 100 nucleotides in length, or an amplicon sequence consisting essentially of a nucleotide sequence selected from (b) SEQ ID NOS:__________ (SEE SECOND COLUMN OF TABLES 2A-2E) | III. Combination includes or consists essentially of primers nucleic acids and/or primer pairs containing, or consisting essentially of, nucleic acids and/or nucleic acid primer pairs containing, or consisting essentially of, a nucleotide sequence or sequences (in the case of primer pairs) selected from SEQ ID NOS:__________ (SEE THIRD COLUMN OF TABLES 2A-2E) |

| **TABLE 2A - GROUP A MICROORGANISMS** | | |
|---|---|---|
| *Akkermansia muciniphila, Bacteroides vulgatus, Bifidobacterium adolescentis, Campylobacter concisus, Campylobacter jejuni, Clostridioides difficile, Escherichia coli, Eubacterium rectale, Helicobacter bilis, Helicobacter hepaticus, Lactobacillus delbrueckii, Parabacteroides distasonis, Ruminococcus bromii, Streptococcus gallolyticus, and Streptococcus infantarius* | | |
| SEQ ID NOS: 1607-1609, 1619, 1620, 1635-1637, 1643-1645, 1663-1670, 1679-1684, 1699-1701, 1728-1730, 1752, 1753, 1801, 1802, 1809, 1810, 1827, 1828, 1831-1833, 1844, 1845, 1852-1856, 1864, 1876-1885, 1889-1891, 1899, 1900, 1932, 1933, 1968 and 1972 of Table 17, or a substantially identical or similar sequence OR SEQ ID NOS: 1607-1609, 1619, 1620, 1635-1637, 1643-1645, 1663-1670, 1679-1684, 1699- | (a) SEQ ID NOS: 1607-1609, 1619, 1620, 1635-1637, 1643-1645, 1663-1670, 1679-1684, 1699-1701, 1728-1730, 1752, 1753, 1801, 1802, 1809, 1810, 1827, 1828, 1831-1833, 1844, 1845, 1852-1856, 1864, 1876-1885, 1889-1891, 1899, 1900, 1932, 1933, 1968 and 1972 of Table 17, or a substantially identical or similar sequence, | SEQ ID NOS: 53-58, 77-80, 109-114, 125-130, 165-180, 197-208, 237-242, 295-300, 343-346, 441-444, 457-460, 493-498, 511-520, 521-524, 529-534, 555-558, 571-580, 595, 596, 619-638, 645-650, 665-668, 731-734, 803, 804, 811, 812 and/or SEQ ID NOS: 831-836, 855-858, 887-892, 903-908, 943-958, 975-986, 1015-1020, 1073-1078, 1121-1124, 1219-1222, 1235-1238, 1271-1276,1289-1298, 1299-1302, 1307-1312, 1333-1336, 1349-1358, 1373, 1374, 1397-1416, 1423-1428, 1443-1446, 1509-1512, 1581, 1582, 1589, and 1590 in Table 16, OR |
| | (b) SEQ ID NOS: 1607-1609, 1619, 1620, 1635-1637, 1643-1645, 1663-1670, 1679-1684, 1699-1701, 1728-1730, 1752, 1753, 1801, 1802, 1809, 1810, 1827, 1828, 1831-1833, 1844, 1845, 1852-1856, 1864, 1876-1885, 1889-1891, 1899, 1900, 1932, 1933, 1968 and 1972 of Table 17, or a substantially identical or similar sequence, or a sequence that is substantially identical or similar to any of the aforementioned sequences, and optionally containing the nucleic acid primer sequences at the 5' and 3' ends of the sequence OR | |
| | (a) SEQ ID NOS: 1607-1609, 1619, 1620, 1635-1637, 1643-1645, 1663-1670, 1679-1684, 1699-1701, 1728- | |

| **TABLE 2A - GROUP A MICROORGANISMS** | | |
|---|---|---|
| *Akkermansia muciniphila, Bacteroides vulgatus, Bifidobacterium adolescentis, Campylobacter concisus, Campylobacter jejuni, Clostridioides difficile, Escherichia coli, Eubacterium rectale, Helicobacter bilis, Helicobacter hepaticus, Lactobacillus delbrueckii, Parabacteroides distasonis, Ruminococcus bromii, Streptococcus gallolyticus, and Streptococcus infantarius* | | |
| 1701, 1728-1730, 1752, 1753, 1801, 1802, 1809 and 1810 of Table 17, or or a substantially identical or similar sequence | 1730, 1752, 1753, 1801, 1802, 1809, and 1810 of Table 17, or a substantially identical or similar sequence, | SEQ ID NOS: 53-58, 77-80, 109-114, 125-130, 165-180, 197-208, 237-242, 295-300, 343-346, 441-444, 457-460, 493-498, 511-520 in Table 16 and/or SEQ ID NOS: 831-836, 855-858, 887-892, 903-908, 943-958, 975-986, 1015-1020, 1073-1078, 1121-1124, 1219-1222, 1235-1238, 1271-1276, 1289-1298 in Table 16, OR |
| | (b) SEQ ID NOS: 1607-1609, 1619, 1620, 1635-1637, 1643-1645, 1663-1670, 1679-1684, 1699-1701, 1728-1730, 1752, 1753, 1801, 1802, 1809 and 1810 of Table 17, or a substantially identical or similar sequence, or a sequence that is substantially identical or similar to any of the aforementioned sequences, and optionally containing the nucleic acid primer sequences at the 5' and 3' ends of the sequence | |
| | | SEQ ID NOS: 53-58, 77-80, 109-114, 125-130, 165-180, 197-208, 237-242, 295-300, 343-346, 441-444, 457-460 in Table 16A and/or SEQ ID NOS: 831-836, 855-858, 887-892, 903-908, 943-958, 975-986, 1015-1020, 1073-1078, 1121-1124, 1219-1222, 1235-1238 in Table 16D, |
| | | or substantially identical or similar sequences of any of the above, or any of the aforementioned nucleotide sequences of nucleic acids or primer pairs in which one or more thymine bases is substituted with a uracil base |

| **TABLE 2B - GROUP B MICROORGANISMS** | | |
|---|---|---|
| *Akkermansia muciniphila, Anaerococcus vaginalis, Atopobium parvulum, Bacteroides nordii, Bacteroides thetaiotaomicron, Bacteroides vulgatus, Bifidobacterium adolescentis, Bifidobacterium longum, Collinsella aerofaciens, Collinsella stercoris, Desulfovibrio alaskensis, Dorea formicigenerans, Enterococcus faecium, Eubacterium rectale, Faecalibacterium prausnitzii, Gardnerella vaginalis, Gemmiger formicilis, Holdemania filiformis, Klebsiella pneumoniae, Parabacteroides distasonis, Parabacteroides merdae, Phascolarctobacterium faecium, Prevotella histicola, Roseburia intestinalis, Ruminococcus bromii, Slackia exigua, Streptococcus infantarius, and Veillonella parvula* | | |
| SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816, 1821-1826, 1829, 1830, 1864, 1869-1871, 1874-1882, 1890-1896, 1901-1903, 1910-1915, 1920- | (a) SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816, 1821-1826, 1829, 1830, 1864, 1869-1871, 1874-1882, 1890-1896, 1901-1903, 1910-1915, 1920-1922, 1930-1931, 1934-1939, 1954, 1955, 1961-1964, 1968, 1972-1974 and 1977-1979 of Table 17, or a substantially identical or similar sequence, | SEQ ID NOS: 49-52, 125-130, 135-140, 157-174, 203-208, 217-228, 243-248, 275-286, 295-300, 309-324, 335-342, 347-372, 399-406, 421-424, 441-444, 457-472, 481-492, 525-528, 595, 596, 605-610, 615-632, 647-660, 669-674, 687-698, 707-712, 727-730, 735-746, 775-778, 789-796, 803, 804, 811-816, 821-826 and/or SEQ ID NOS: 827-830, 903-908, 913-918, 935-952, 981-986, 995-1006, 1021-1026, 1053-1064, 1073-1078, 1087- |
| | (b) SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816, 1821- | |

| **TABLE 2B - GROUP B MICROORGANISMS** | | |
|---|---|---|
| *Akkermansia muciniphila, Anaerococcus vaginalis, Atopobium parvulum, Bacteroides nordii, Bacteroides thetaiotaomicron, Bacteroides vulgatus, Bifidobacterium adolescentis, Bifidobacterium longum, Collinsella aerofaciens, Collinsella stercoris, Desulfovibrio alaskensis, Dorea formicigenerans, Enterococcus faecium, Eubacterium rectale, Faecalibacterium prausnitzii, Gardnerella vaginalis, Gemmiger formicilis, Holdemania filiformis, Klebsiella pneumoniae, Parabacteroides distasonis, Parabacteroides merdae, Phascolarctobacterium faecium, Prevotella histicola, Roseburia intestinalis, Ruminococcus bromii, Slackia exigua, Streptococcus infantarius, and Veillonella parvula* | | |
| 1922, 1930-1931, 1934-1939, 1954, 1955, 1961-1964, 1968, 1972-1974 and 1977-1979 of Table 17, or or a substantially identical or similar sequence | 1826, 1829, 1830, 1864, 1869-1871, 1874-1882, 1890-1896, 1901-1903, 1910-1915, 1920-1922, 1930-1931, 1934-1939, 1954, 1955, 1961-1964, 1968, 1972-1974 and 1977-1979 of Table 17, or a substantially identical or similar sequence, or a sequence that is substantially identical or similar to any of the aforementioned sequences, and optionally containing the nucleic acid primer sequences at the 5' and 3' ends of the sequence | 1102, 1113-1120, 1125-1150, 1177-1184, 1199-1202, 1219-1222, 1235-1250, 1259-1270, 1303-1306, 1373, 1374, 1383-1388, 1393-1410, 1425-1438, 1447-1452, 1465-1476, 1485-1490, 1505-1508, 1513-1524, 1553-1556, 1567-1574, 1581, 1582, 1589-1594, 1599-1604 in Table 16, OR SEQ ID NOS: 49-52, 125-130, 135-140, 157-174, 203-208, 217-228, 243-248, 275-286, 295-300, 309-324, 335-342, 347-372, 399-406, 421-424, 441-444, 457-472, 481-492 and/or SEQ ID NOS: 827-830, 903-908, 913-918, 935-952, 981-986, 995-1006, 1021-1026, 1053-1064, 1073-1078, 1087-1102, 1113-1120, 1125-1150, 1177-1184, 1199-1202, 1219-1222, 1235-1250, 1259-1270 in Table 16, OR SEQ ID NOS: 49-52, 125-130, 135-140, 157-174, 203-208, 217-228, 243-248, 275-286, 295-300, 309-324, 335-342, 347-372, 399-406, 421-424, 441-444, 457-472 in Table 16A and/or SEQ ID NOS: 827-830, 903-908, 913-918, 935-952, 981-986, 995-1006, 1021-1026, 1053-1064, 1073-1078, 1087-1102, 1113-1120, 1125-1150, 1177-1184, 1199-1202, 1219-1222, 1235-1250 in Table 16D, |
| OR SEQ ID NOS: | OR | |
| 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816 and 1821-1826 of Table 17, or a substantially identical or similar sequence | (a) SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816 and 1821-1826 of Table 17, or a substantially identical or similar sequence, | |
| | (b) SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816 and 1821-1826 of Table 17, or a substantially identical or similar sequence, or a sequence that is substantially identical or similar to any of the aforementioned sequences, and optionally containing the nucleic acid primer sequences at the 5' and 3' ends of the sequence OR | |
| OR SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802 and 1809-1816 of Table 17A, or or a substantially identical or similar sequence | | |
| | (a) SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802 and 1809-1816 of Table 17A, or a substantially identical or similar sequence, | |
| | (b) SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802 and 1809-1816 of Table 17A, or a substantially identical or similar sequence, or a sequence that is substantially identical or similar to any of the aforementioned sequences, and optionally containing the nucleic acid primer sequences at the 5' and 3' ends of the sequence | |
| | | or substantially identical or similar sequences of any of the above, or any of the aforementioned nucleotide sequences of nucleic acids or primer pairs in which one or more thymine bases is substituted with a uracil base |

| **TABLE 2C - GROUP C MICROORGANISMS** | | |
|---|---|---|
| *Bacteroides fragilis, Campylobacter jejuni, Cutibacterium acnes, Escherichia coli, Fusobacterium nucleatum, Helicobacter bilis, Helicobacter bizzozeronii, Helicobacter hepaticus, Helicobacter pylori, Helicobacter salomonis, Peptostreptococcus stomatis, and Streptococcus gallolyticus* | | |
| SEQ ID NOS: 1616, 1619, 1620, 1625-1628, 1635-1640, 1699, 1700, 1705-1708, 1752, 1753, 1784-1786, 1817-1820, 1827, 1828, 1840, 1841, 1844, 1845, 1852-1859, 1899, 1900, 1904, 1905, 1932, 1933, 1956-1958, 1975, 1976 of Table 17, | (a) SEQ ID NOS: 1616, 1619, 1620, 1625-1628, 1635-1640, 1699, 1700, 1705-1708, 1752, 1753, 1784-1786, 1817-1820, 1827, 1828, 1840, 1841, 1844, 1845, 1852-1859, 1899, 1900, 1904, 1905, 1932, 1933, 1956-1958, 1975, 1976 of Table 17, or a substantially identical or similar sequence, | SEQ ID NOS: 71, 72, 77-80, 89-96, 109-120, 237-242, 249-256, 343-346, 407-412, 473-480, 493-496, 511-520, 521-524, 547-550, 555-558, 561-568, 571-586, 665-668, 675-678, 731-734, 779-784, 817-820 and/or SEQ ID NOS: 849, 850, 855-858, 867-874, 887-898, 1012-1020, 1025-1034, 1121-1124, 1185-1190, 1251-1258, 1271-1276, 1289-1298, 1299-1302, 1325-1328, 1333-1336, 1339-1346, 1349-1364, 1443-1446, 1453-1456, 1509-1512, 1557-1562, 1595-1598 in Table 16, OR SEQ ID NOS: 71, 72, 77-80, 89-96, 109-120, 237-242, 249-256, 343-346, 407-412, 473-480, 493-496, 511-520 and/or SEQ ID NOS: 849, 850, 855-858, 867-874, 887-898, 1012-1020, 1025-1034, 1121-1124, 1185-1190, 1251-1258, 1271-1276, 1289-1298 in Table 16, OR |
| or a substantially identical or similar sequence | | |
| OR SEQ ID NOS: 1616, 1619, 1620, 1625-1628, 1635-1640, 1699, 1700, 1705-1708, 1752, 1753, 1784-1786, 1817-1820 of Table 17, or a substantially identical or similar sequence | (b) SEQ ID NOS: 1616, 1619, 1620, 1625-1628, 1635-1640, 1699, 1700, 1705-1708, 1752, 1753, 1784-1786, 1817-1820, 1827, 1828, 1840, 1841, 1844, 1845, 1852-1859, 1899, 1900, 1904, 1905, 1932, 1933, 1956-1958, 1975, 1976 of Table 17, or a substantially identical or similar sequence, or a sequence that is substantially identical or similar to any of the aforementioned sequences, and optionally containing the nucleic acid primer sequences at the 5' and 3' ends of the sequence OR | |
| | | SEQ ID NOS: 71, 72, 77-80, 89-96, 109-120, 237-242, 249-256, 343-346, 407-412, 473-480 and/or SEQ ID NOS: 849, 850, 855-858, 867-874, 887-898, 1012-1020, 1025-1034, 1121-1124, 1185-1190, 1251-1258 |
| | a) SEQ ID NOS: 1616, 1619, 1620, 1625-1628, 1635-1640, 1699, 1700, 1705-1708, 1752, 1753, 1784-1786, 1817-1820 of Table 17, or a substantially identical or similar sequence, | |
| | | or substantially identical or similar sequences of any of the above, or any of the aforementioned nucleotide sequences of nucleic acids or primer pairs in which one or more thymine bases is substituted with a uracil base |
| | (b) SEQ ID NOS: 1616, 1619, 1620, 1625-1628, 1635-1640, 1699, 1700, 1705-1708, 1752, 1753, 1784-1786, 1817-1820 of Table 17, or a substantially identical or similar sequence, or a sequence that is substantially identical or similar to any of the aforementioned sequences, and optionally containing the nucleic acid primer sequences at the 5' and 3' ends of the sequence | |

| **TABLE 2D - GROUP D MICROORGANISMS** | | |
|---|---|---|
| *Akkermansia muciniphila, Bifidobacterium bifidum, Bifidobacterium longum, Blautia coccoides, Campylobacter concisus, Campylobacter curvus, Campylobacter jejuni, Campylobacter rectus, Clostridioides difficile, Escherichia coli, Eubacterium rectale, Fusobacterium nucleatum, Helicobacter bilis, Helicobacter hepaticus, Helicobacter pylori, Klebsiella pneumoniae, Lactobacillus delbrueckii, Parabacteroides distasonis, Proteus mirabilis, Ruminococcus bromii and Ruminococcus gnavus* | | |
| SEQ ID NOS in Table 17, or Table 17A and Table 17B, which correspond to Group D microorganisms, or a substantially identical or similar sequence | (a) SEQ ID NOS in Table 17, or Table 17A and Table 17B, which correspond to Group D microorganisms, or a substantially identical or similar sequence, (b) SEQ ID NOS SEQ ID NOS in Table 17, or Table 17A and Table 17B, which correspond to Group D microorganisms, or a substantially identical or similar sequence, or a sequence that is substantially identical or similar to any of the aforementioned sequences, and optionally containing the nucleic acid primer sequences at the 5' and 3' ends of the sequence | SEQ ID NOS corresponding to Group D microorganisms in Table 16, SEQ ID NOS: 49-520 of Table 16, SEQ ID NOS: 49-492 of Table 16, SEQ ID NOS: 49-480 of Table 16A, SEQ ID NOS: 521-826 of Table 16C, SEQ ID NOS: 521-820 of Table 16C, SEQ ID NOS: 827-1298 of Table 16, SEQ ID NOS: 827-1258 of Table 16D, SEQ ID NOS: 1299-1604 of Table 16F, or SEQ ID NOS: 1299-1598 of Table 16F,or substantially identical or similar sequences of any of the above, or any of the aforementioned nucleotide sequences of nucleic acids or primer pairs in which one or more thymine bases is substituted with a uracil base |

| **TABLE 2E - GROUP E MICROORGANISMS** | | |
|---|---|---|
| *Akkermansia muciniphila, Bacteroides fragilis, Bacteroides vulgatus, Bifidobacterium adolescentis, Campylobacter concisus, Campylobacter jejuni, Citrobacter rodentium, Clostridioides difficile, Enterococcus gallinarum, Escherichia coli, Helicobacter bilis, Lactobacillus delbrueckii, Lactobacillus murinus, Lactobacillus reuteri, Lactobacillus rhamnosus, Lactococcus lactis, and Prevotella copri* | | |
| SEQ ID NOS in Table 17, or Table 17A and Table 17B, which correspond to Group E microorganisms, or a substantially identical or similar sequence | (a) SEQ ID NOS in Table 17, , or Table 17A and Table 17B, which correspond to Group E microorganisms, or a substantially identical or similar sequence, (b) SEQ ID NOS SEQ ID NOS in Table 17, or Table 17A and Table 17B, which correspond to Group E microorganisms, or a substantially identical or similar sequence, or a sequence that is substantially identical or similar to any of the aforementioned sequences, and optionally containing the nucleic acid primer sequences at the 5' and 3' ends of the sequence | SEQ ID NOS corresponding to Group D microorganisms in Table 16, SEQ ID NOS: 49-520 of Table 16, SEQ ID NOS: 49-492 of Table 16, SEQ ID NOS: 49-480 of Table 16A, SEQ ID NOS: 521-826 of Table 16C, SEQ ID NOS: 521-820 of Table 16C, SEQ ID NOS: 827-1298 of Table 16, SEQ ID NOS: 827-1258 of Table 16D, SEQ ID NOS: 1299-1604 of Table 16F, or SEQ ID NOS: 1299-1598 of Table 16F,or substantially identical or similar sequences of any of the above, or any of the aforementioned nucleotide sequences of nucleic acids or primer pairs in which one or more thymine bases is substituted with a uracil base |

In some examples, a combination of nucleic acids and/or nucleic acid primer pairs includes two or more nucleic acids and/or nucleic acid primer pairs that specifically bind to, hybridize to and/or amplify a unique nucleic acid sequence contained in the genome of one or more *of Akkermansia muciniphila, Anaerococcus vaginalis, Atopobium parvulum, Bacteroides nordii, Bacteroides thetaiotaomicron, Bacteroides vulgatus, Bifidobacterium adolescentis, Bifidobacterium longum, Collinsella aerofaciens, Collinsella stercoris, Desulfovibrio alaskensis, Dorea formicigenerans, Enterococcus faecium, Eubacterium rectale, Faecalibacterium prausnitzii, Gardnerella vaginalis, Gemmiger formicilis, Holdemania filiformis, Klebsiella pneumoniae, Parabacteroides distasonis, Parabacteroides merdae, Phascolarctobacterium faecium, Prevotella histicola, Roseburia intestinalis, Ruminococcus bromii, Slackia exigua, Streptococcus infantarius, and Veillonella parvula* (referred to herein as "Group B" microorganisms; see Table 2B), which are species implicated as having a role in response to immuno-oncology treatment. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group B. In some examples, the combination of nucleic acids and/or nucleic acid primer pairs includes or consists essentially of nucleic acids and/or nucleic acid primer pairs that bind to, hybridize to and/or amplify, or specifically bind to, hybridize to and/or amplify, a nucleic acid (such as a nucleic acid from a microorganism, e.g., bacteria) containing a sequence selected from SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816, 1821-1826, 1829, 1830, 1864, 1869-1871, 1874-1882, 1890-1896, 1901-1903, 1910-1915, 1920-1922, 1930-1931, 1934-1939, 1954, 1955, 1961-1964, 1968, 1972-1974 and 1977-1979 of Table 17, and/or a substantially identical or similar sequence. In some examples, the combination of nucleic acids and/or nucleic acid primer pairs includes or consists essentially of nucleic acids and/or nucleic acid primer pairs that bind to, hybridize to and/or amplify, or specifically bind to, hybridize to and/or amplify, a nucleic acid (such as a nucleic acid from a microorganism, e.g., bacteria) containing a sequence selected from SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816 and 1821-1826, of Table 17, and/or a substantially identical or similar sequence. In some examples, the combination of nucleic acids and/or nucleic acid primer pairs includes or consists essentially of nucleic acids and/or nucleic acid primer pairs that bind to, hybridize to and/or amplify, or specifically bind to, hybridize to and/or amplify, a nucleic acid (such as a nucleic acid from a microorganism, e.g., bacteria) containing a sequence selected from SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802 and 1809-1816 of Table 17A, and/or a substantially identical or similar sequence. In some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816, 1821-1826, 1829, 1830, 1864, 1869-1871, 1874-1882, 1890-1896, 1901-1903, 1910-1915, 1920-1922, 1930-1931, 1934-1939, 1954, 1955, 1961-1964, 1968, 1972-1974 and 1977-1979 of Table 17 (or a sequence that is substantially identical or similar to any of the aforementioned sequences) to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from SEQ **ID** NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816, 1821-1826, 1829, 1830, 1864, 1869-1871, 1874-1882, 1890-1896, 1901-1903, 1910-1915, 1920-1922, 1930-1931, 1934-1939, 1954, 1955, 1961-1964, 1968, 1972-1974 and 1977-1979 of Table 17, or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816 and 1821-1826 of Table 17 (or a sequence that is substantially identical or similar to any of the aforementioned sequences) to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802, 1809-1816 and 1821-1826 of Table 17, or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802 and 1809-1816 of Table 17A (or a sequence that is substantially identical or similar to any of the aforementioned sequences) to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from SEQ ID NOS: 1605, 1606, 1643-1645, 1648-1650, 1659-1667, 1682-1684, 1689-1694, 1702-1704, 1718-1723, 1728-1730, 1735-1742, 1748-1751, 1754-1766, 1780-1783, 1791, 1792, 1801, 1802 and 1809-1816 of Table 17A, or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from SEQ ID NOS: 49-52, 125-130, 135-140, 157-174, 203-208, 217-228, 243-248, 275-286, 295-300, 309-324, 335-342, 347-372, 399-406, 421-424, 441-444, 457-472, 481-492, 525-528, 595, 596, 605-610, 615-632, 647-660, 669-674, 687-698, 707-712, 727-730, 735-746, 775-778, 789-796, 803, 804, 811-816, 821-826 and/or SEQ ID NOS: 827-830, 903-908, 913-918, 935-952, 981-986, 995-1006, 1021-1026, 1053-1064, 1073-1078, 1087-1102, 1113-1120, 1125-1150, 1177-1184, 1199-1202, 1219-1222, 1235-1250, 1259-1270, 1303-1306, 1373, 1374, 1383-1388, 1393-1410, 1425-1438, 1447-1452, 1465-1476, 1485-1490, 1505-1508, 1513-1524, 1553-1556, 1567-1574, 1581, 1582, 1589-1594, 1599-1604 in Table 16, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from SEQ ID NOS: 49-52, 125-130, 135-140, 157-174, 203-208, 217-228, 243-248, 275-286, 295-300, 309-324, 335-342, 347-372, 399-406, 421-424, 441-444, 457-472, 481-492 and/or SEQ ID NOS: 827-830, 903-908, 913-918, 935-952, 981-986, 995-1006, 1021-1026, 1053-1064, 1073-1078, 1087-1102, 1113-1120, 1125-1150, 1177-1184, 1199-1202, 1219-1222, 1235-1250, 1259-1270 in Table 16, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from SEQ ID NOS: 49-52, 125-130, 135-140, 157-174, 203-208, 217-228, 243-248, 275-286, 295-300, 309-324, 335-342, 347-372, 399-406, 421-424, 441-444, 457-472 in Table 16A and/or SEQ ID NOS: 827-830, 903-908, 913-918, 935-952, 981-986, 995-1006, 1021-1026, 1053-1064, 1073-1078, 1087-1102, 1113-1120, 1125-1150, 1177-1184, 1199-1202, 1219-1222, 1235-1250 in Table 16D, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences of SEQ ID NOS: 49-52, 125-130, 135-140, 157-174, 203-208, 217-228, 243-248, 275-286, 295-300, 309-324, 335-342, 347-372, 399-406, 421-424, 441-444, 457-472, 481-492 and/or SEQ ID NOS: 827-830, 903-908, 913-918, 935-952, 981-986, 995-1006, 1021-1026, 1053-1064, 1073-1078, 1087-1102, 1113-1120, 1125-1150, 1177-1184, 1199-1202, 1219-1222, 1235-1250, 1259-1270 in Table 16, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences of SEQ ID NOS: 49-52, 125-130, 135-140, 157-174, 203-208, 217-228, 243-248, 275-286, 295-300, 309-324, 335-342, 347-372, 399-406, 421-424, 441-444, 457-472 in Table 16A and/or SEQ ID NOS: 827-830, 903-908, 913-918, 935-952, 981-986, 995-1006, 1021-1026, 1053-1064, 1073-1078, 1087-1102, 1113-1120, 1125-1150, 1177-1184, 1199-1202, 1219-1222, 1235-1250 in Table 16D, or substantially identical or similar sequences, or any of the aforementioned nucleotide sequences of nucleic acids or primer pairs in which one or more thymine bases is substituted with a uracil base.

In some examples, a combination of nucleic acids and/or nucleic acid primer pairs includes two or more nucleic acids and/or nucleic acid primer pairs that specifically bind to, hybridize to and/or amplify a unique nucleic acid sequence contained in the genome of one or more of *Bacteroides fragilis, Campylobacter jejuni, Cutibacterium acnes, Escherichia coli, Fusobacterium nucleatum, Helicobacter bilis, Helicobacter bizzozeronii, Helicobacter hepaticus, Helicobacter pylori, Helicobacter salomonis, Peptostreptococcus stomatis,* and *Streptococcus gallolyticus* (referred to herein as "Group C" microorganisms; see Table 2C), which are species implicated as having a role in cancer. In some examples, a combination of nucleic acids and/or nucleic acid primer pairs includes two or more nucleic acids and/or nucleic acid primer pairs that specifically bind to, hybridize to and/or amplify a unique nucleic acid sequence contained in the genome of one or more of *Bacteroides fragilis, Campylobacter jejuni, Cutibacterium acnes, Escherichia coli, Fusobacterium nucleatum, Helicobacter bilis, Helicobacter bizzozeronii, Helicobacter hepaticus, Helicobacter pylori, Peptostreptococcus stomatis,* and *Streptococcus gallolyticus* (referred to herein as "Subgroup 1" of the Group C microorganisms). In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group C or in Group C excluding *Helicobacter salomonis* (i.e., Subgroup 1 of Group C). In some examples, the combination of nucleic acids and/or nucleic acid primer pairs includes or consists essentially of nucleic acids and/or nucleic acid primer pairs that bind to, hybridize to and/or amplify, or specifically bind to, hybridize to and/or amplify, a nucleic acid (such as a nucleic acid from a microorganism, e.g., bacteria) containing a sequence selected from SEQ ID NOS: 1616, 1619, 1620, 1625-1628, 1635-1640, 1699, 1700, 1705-1708, 1752, 1753, 1784-1786, 1817-1820, 1827, 1828, 1840, 1841, 1844, 1845, 1852-1859, 1899, 1900, 1904, 1905, 1932, 1933, 1956-1958, 1975, 1976 of Table 17, and/or a substantially identical or similar sequence, or a nucleic acid containing a sequence selected from SEQ ID NOS: 1616, 1619, 1620, 1625-1628, 1635-1640, 1699, 1700, 1705-1708, 1752, 1753, 1784-1786, 1827, 1828, 1840, 1841, 1844, 1845, 1852-1859, 1899, 1900, 1904, 1905, 1932, 1933, 1956, 1957, 1958 of Table 17, and/or a substantially identical or similar sequence. In some examples, the combination of nucleic acids and/or nucleic acid primer pairs includes or consists essentially of nucleic acids and/or nucleic acid primer pairs that bind to, hybridize to and/or amplify, or specifically bind to, hybridize to and/or amplify, a nucleic acid (such as a nucleic acid from a microorganism, e.g., bacteria) containing a sequence selected from SEQ ID NOS: 1616, 1619, 1620, 1625-1628, 1635-1640, 1699, 1700, 1705-1708, 1752, 1753, 1784-1786, 1817-1820 of Table 17A, and/or a substantially identical or similar sequence, or a nucleic acid containing a sequence selected from SEQ ID NOS: 1616, 1619, 1620, 1625-1628, 1635-1640, 1699, 1700, 1705-1708, 1752, 1753, 1784-1786 of Table 17A, and/or a substantially identical or similar sequence. In some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from SEQ ID NOS: 1616, 1619, 1620, 1625-1628, 1635-1640, 1699, 1700, 1705-1708, 1752, 1753, 1784-1786, 1817-1820, 1827, 1828, 1840, 1841, 1844, 1845, 1852-1859, 1899, 1900, 1904, 1905, 1932, 1933, 1956-1958, 1975, 1976 of Table 17 (or a sequence that is substantially identical or similar to any of the aforementioned sequences) to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from SEQ **ID** NOS: 1616, 1619, 1620, 1625-1628, 1635-1640, 1699, 1700, 1705-1708, 1752, 1753, 1784-1786, 1817-1820, 1827, 1828, 1840, 1841, 1844, 1845, 1852-1859, 1899, 1900, 1904, 1905, 1932, 1933, 1956-1958, 1975, 1976 of Table 17, or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from SEQ ID NOS: 1616, 1619, 1620, 1625-1628, 1635-1640, 1699, 1700, 1705-1708, 1752, 1753, 1784-1786, 1817-1820 of Table 17A (or a sequence that is substantially identical or similar to any of the aforementioned sequences) to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from SEQ ID NOS: 1616, 1619, 1620, 1625-1628, 1635-1640, 1699, 1700, 1705-1708, 1752, 1753, 1784-1786, 1817-1820 of Table 17A, or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from SEQ ID NOs: 71, 72, 77-80, 89-96, 109-120, 237-242, 249-256, 343-346, 407-412, 473-480, 493-496, 511-520, 521-524, 547-550, 555-558, 561-568, 571-586, 665-668, 675-678, 731-734, 779-784, 817-820 and/or SEQ ID NOS: 849, 850, 855-858, 867-874, 887-898, 1012-1020, 1025-1034, 1121-1124, 1185-1190, 1251-1258, 1271-1276, 1289-1298, 1299-1302, 1325-1328, 1333-1336, 1339-1346, 1349-1364, 1443-1446, 1453-1456, 1509-1512, 1557-1562, 1595-1598 in Table 16, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from SEQ ID NOs: 71, 72, 77-80, 89-96, 109-120, 237-242, 249-256, 343-346, 407-412, 473-480, 493-496, 511-520 and/or SEQ ID NOS: 849, 850, 855-858, 867-874, 887-898, 1012-1020, 1025-1034, 1121-1124, 1185-1190, 1251-1258, 1271-1276, 1289-1298 in Table 16, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from SEQ ID NOs: 71, 72, 77-80, 89-96, 109-120, 237-242, 249-256, 343-346, 407-412, 473-480 and/or SEQ ID NOS: 849, 850, 855-858, 867-874, 887-898, 1012-1020, 1025-1034, 1121-1124, 1185-1190, 1251-1258 in Table 16, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences of SEQ ID NOS: 71, 72, 77-80, 89-96, 109-120, 237-242, 249-256, 343-346, 407-412, 473-480, 493-496, 511-520, 521-524, 547-550, 555-558, 561-568, 571-586, 665-668, 675-678, 731-734, 779-784, 817-820 and/or SEQ ID NOS: 849, 850, 855-858, 867-874, 887-898, 1012-1020, 1025-1034, 1121-1124, 1185-1190, 1251-1258, 1271-1276, 1289-1298, 1299-1302, 1325-1328, 1333-1336, 1339-1346, 1349-1364, 1443-1446, 1453-1456, 1509-1512, 1557-1562, 1595-1598 in Table 16, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences of SEQ ID NOS: 71, 72, 77-80, 89-96, 109-120, 237-242, 249-256, 343-346, 407-412, 493-496, 511-520, 521-524, 547-550, 555-558, 561-568, 571-586, 665-668, 675-678, 731-734, 779-784, and/or SEQ ID NOS: 849, 850, 855-858, 867-874, 887-898, 1012-1020, 1025-1034, 1121-1124, 1185-1190, 1271-1276, 1289-1298, 1299-1302, 1325-1328, 1333-1336, 1339-1346, 1349-1364, 1443-1446, 1453-1456, 1509-1512, 1557-1562, in Table 16, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences of SEQ ID NOS: 71, 72, 77-80, 89-96, 109-120, 237-242, 249-256, 343-346, 407-412, 473-480, 493-496, 511-520 and/or SEQ ID NOS: 849, 850, 855-858, 867-874, 887-898, 1012-1020, 1025-1034, 1121-1124, 1185-1190, 1251-1258, 1271-1276, 1289-1298 in Table 16, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences of SEQ ID NOS: 71, 72, 77-80, 89-96, 109-120, 237-242, 249-256, 343-346, 407-412, 493-496, 511-520 and/or SEQ ID NOS: 849, 850, 855-858, 867-874, 887-898, 1012-1020, 1025-1034, 1121-1124, 1185-1190, 1271-1276, 1289-1298 in Table 16, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences of SEQ ID NOS: 71, 72, 77-80, 89-96, 109-120, 237-242, 249-256, 343-346, 407-412, 473-480 and/or SEQ ID NOS: 849, 850, 855-858, 867-874, 887-898, 1012-1020, 1025-1034, 1121-1124, 1185-1190, 1251-1258 in Table 16, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences of SEQ ID NOS: 71, 72, 77-80, 89-96, 109-120, 237-242, 249-256, 343-346, 407-412 and/or SEQ ID NOS: 849, 850, 855-858, 867-874, 887-898, 1012-1020, 1025-1034, 1121-1124, 1185-1190 in Table 16, or substantially identical or similar sequences, or any of the aforementioned nucleotide sequences of nucleic acids or primer pairs in which one or more thymine bases is substituted with a uracil base.

**In** some examples, a combination of nucleic acids and/or nucleic acid primer pairs includes two or more nucleic acids and/or nucleic acid primer pairs that specifically bind to, hybridize to and/or amplify a unique nucleic acid sequence contained in the genome of one or more *of Akkermansia muciniphila, Bifidobacterium bifidum, Bifidobacterium longum, Blautia coccoides, Campylobacter concisus, Campylobacter curvus, Campylobacter jejuni, Campylobacter rectus, Clostridioides difficile, Escherichia coli, Eubacterium rectale, Fusobacterium nucleatum, Helicobacter bilis, Helicobacter hepaticus, Helicobacter pylori, Klebsiella pneumoniae, Lactobacillus delbrueckii, Parabacteroides distasonis, Proteus mirabilis, Ruminococcus bromii* and *Ruminococcus gnavus* (referred to herein as "Group D" microorganisms; see Table 2D), which are species implicated as having a role in gastrointestinal disorders, including, for example, irritable bowel syndrome, inflammatory bowel disease and coeliac disease. **In** some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group D. In some examples, the combination of nucleic acids and/or nucleic acid primer pairs includes or consists essentially of nucleic acids and/or nucleic acid primer pairs that bind to, hybridize to and/or amplify, or specifically bind to, hybridize to and/or amplify, a nucleic acid (such as a nucleic acid from a microorganism, e.g., bacteria) containing a sequence selected from the sequences in Table 17, or Table 17 excluding SEQ ID NOS: 1807, 1808 and 1971, or Table 17A and Table 17B, or Table 17 B and Table 17A that excludes SEQ ID NOS: 1807 and 1808, and/or a substantially identical or similar sequence, which correspond to a Group D microorganism. In some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from the sequences in Table 17, or Table 17 excluding SEQ ID NOS: 1807, 1808 and 1971, or Table 17A and Table 17B, or Table 17 B and Table 17A that excludes SEQ ID NOS: 1807 and 1808, and/or a substantially identical or similar sequence, which correspond to a Group D microorganism (or a sequence that is substantially identical or similar to any of the aforementioned sequences) to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from the sequences in Table 17, or Table 17 excluding SEQ ID NOS: 1807, 1808 and 1971, or Table 17A and Table 17B, or Table 17 B and Table 17A that excludes SEQ ID NOS: 1807 and 1808, and/or a substantially identical or similar sequence, which correspond to a Group D microorganism, or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from sequences in Table 17, or Table 17 excluding SEQ ID NOS: 1807, 1808 and 1971, or Table 17A and Table 17B, or Table 17 B and Table 17A that excludes SEQ ID NOS: 1807 and 1808, (or a sequence that is substantially identical or similar to any of the aforementioned sequences) which correspond to a Group D microorganism to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from sequences in Table 17, or Table 17A and Table 17B, which correspond to a Group D microorganism or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from sequences corresponding to Group D microorganisms in Table 16, or Table 16 excluding SEQ ID NOS: 453-456, 809, 810, 1231-1234 and 1587-1588, or SEQ ID NOS: 49-520 of Table 16, or SEQ ID NOS: 49-452 and 457-520 of Table 16, or SEQ ID NOS: 49-492 of Table 16, or SEQ ID NOS: 49-452 and 457-492 of Table 16, or SEQ ID NOS: 49-480 of Table 16A, or SEQ ID NOS: 49-452 and 457-480 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230 and 1235-1298 of Table 16, or SEQ ID NOS: 827-1258 of Table 16D, or SEQ ID NOS: 827-1230 and 1235-1258 of Table 16D, or SEQ ID NOS: 1299-1604 of Table 16F, or SEQ ID NOS: 1299-1598 of Table 16F, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from sequences corresponding to Group D microorganisms in Table 16, or Table 16 excluding SEQ ID NOS: 453-456, 809, 810, 1231-1234 and 1587-1588, or SEQ ID NOS: 49-520 of Table 16, or SEQ ID NOS: 49-452 and 457-520 of Table 16, or SEQ ID NOS: 49-492 of Table 16, or SEQ ID NOS: 49-452 and 457-492 of Table 16, or SEQ ID NOS: 49-480 of Table 16A, or SEQ ID NOS: 49-452 and 457-480 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230 and 1235-1298 of Table 16, or SEQ ID NOS: 827-1258 of Table 16D, or SEQ ID NOS: 827-1230 and 1235-1258 of Table 16D, or SEQ ID NOS: 1299-1604 of Table 16F, or SEQ ID NOS: 1299-1598 of Table 16F, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from sequences corresponding to Group D microorganisms in Table 16, or Table 16 excluding SEQ ID NOS: 453-456, 809, 810, 1231-1234 and 1587-1588, or SEQ ID NOS: 49-520 of Table 16, or SEQ ID NOS: 49-452 and 457-520 of Table 16, or SEQ ID NOS: 49-492 of Table 16, or SEQ ID NOS: 49-452 and 457-492 of Table 16, or SEQ ID NOS: 49-480 of Table 16A, or SEQ ID NOS: 49-452 and 457-480 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230 and 1235-1298 of Table 16, or SEQ ID NOS: 827-1258 of Table 16D, or SEQ ID NOS: 827-1230 and 1235-1258 of Table 16D, or SEQ ID NOS: 1299-1604 of Table 16F, or SEQ ID NOS: 1299-1598 of Table 16F, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences corresponding to Group D microorganisms in Table 16, or Table 16 excluding SEQ ID NOS: 453-456, 809, 810, 1231-1234 and 1587-1588, or SEQ ID NOS: 49-520 of Table 16, or SEQ ID NOS: 49-452 and 457-520 of Table 16, or SEQ ID NOS: 49-492 of Table 16, or SEQ ID NOS: 49-452 and 457-492 of Table 16, or SEQ ID NOS: 49-480 of Table 16A, or SEQ ID NOS: 49-452 and 457-480 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230 and 1235-1298 of Table 16, or SEQ ID NOS: 827-1258 of Table 16D, or SEQ ID NOS: 827-1230 and 1235-1258 of Table 16D, or SEQ ID NOS: 1299-1604 of Table 16F, or SEQ ID NOS: 1299-1598 of Table 16F, or substantially identical or similar sequences, or any of the aforementioned nucleotide sequences of nucleic acids or primer pairs in which one or more thymine bases is substituted with a uracil base.

**In** some examples, a combination of nucleic acids and/or nucleic acid primer pairs includes two or more nucleic acids and/or nucleic acid primer pairs that specifically bind to, hybridize to and/or amplify a unique nucleic acid sequence contained in the genome of one or more *of Akkermansia muciniphila, Bacteroides fragilis, Bacteroides vulgatus, Bifidobacterium adolescentis, Campylobacter concisus, Campylobacter jejuni, Citrobacter rodentium, Clostridioides difficile, Enterococcus gallinarum, Escherichia coli, Helicobacter bilis, Lactobacillus delbrueckii, Lactobacillus murinus, Lactobacillus reuteri, Lactobacillus rhamnosus, Lactococcus lactis,* and *Prevotella copri* (referred to herein as "Group E" microorganisms; see Table 2E), which are species implicated as having a role in autoimmune disorders, including, for example, lupus and rheumatoid arthritis. **In** some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group E. **In** some examples, the combination of nucleic acids and/or nucleic acid primer pairs includes or consists essentially of nucleic acids and/or nucleic acid primer pairs that bind to, hybridize to and/or amplify, or specifically bind to, hybridize to and/or amplify, a nucleic acid (such as a nucleic acid from a microorganism, e.g., bacteria) containing a sequence selected from the sequences in Table 17, or Table 17A and Table 17B, and/or a substantially identical or similar sequence, which correspond to a Group E microorganism. **In** some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from the sequences in Table 17, or Table 17A and Table 17B, and/or a substantially identical or similar sequence, which correspond to a Group E microorganism (or a sequence that is substantially identical or similar to any of the aforementioned sequences) to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from the sequences in Table 17, or Table 17A and Table 17B, and/or a substantially identical or similar sequence, which correspond to a Group E microorganism, or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of primers and/or primer pairs capable of amplifying, or specifically amplifying, a nucleic acid (such as a nucleic acid from a microorganism, e.g., bacteria) containing a sequence selected from sequences in Table 17, or Table 17A and Table 17B, (or a sequence that is substantially identical or similar to any of the aforementioned sequences) which correspond to a Group E microorganism to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or an amplicon sequence consisting essentially of a nucleotide sequence selected from sequences in Table 17, or Table 17A and Table 17B, which correspond to a Group E microorganism or a sequence that is substantially identical or similar to any of the aforementioned sequences, and optionally containing the nucleic acid primer sequences at the 5' and 3' ends of the sequence. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from sequences corresponding to Group E microorganisms in Table 16, SEQ ID NOS: 49-520 of Table 16, SEQ ID NOS: 49-492 of Table 16, SEQ ID NOS: 49-480 of Table 16A, SEQ ID NOS: 521-826 of Table 16C, SEQ ID NOS: 521-820 of Table 16C, SEQ ID NOS: 827-1298 of Table 16, SEQ ID NOS: 827-1258 of Table 16D, SEQ ID NOS: 1299-1604 of Table 16F, or SEQ ID NOS: 1299-1598 of Table 16F, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from sequences corresponding to Group E microorganisms in Table 16, SEQ ID NOS: 49-520 of Table 16, SEQ ID NOS: 49-492 of Table 16, SEQ ID NOS: 49-480 of Table 16A, SEQ ID NOS: 521-826 of Table 16C, SEQ ID NOS: 521-820 of Table 16C, SEQ ID NOS: 827-1298 of Table 16, SEQ ID NOS: 827-1258 of Table 16D, SEQ ID NOS: 1299-1604 of Table 16F, or SEQ ID NOS: 1299-1598 of Table 16F, or substantially identical or similar sequences, or any of the aforementioned nucleotide sequences of nucleic acids or primer pairs in which one or more thymine bases is substituted with a uracil base. **In** some examples, the combination of nucleic acids and/or nucleic acid primer pairs includes or consists essentially of nucleic acids and/or nucleic acid primer pairs containing, or consisting essentially of, a nucleotide sequence or sequences (in the case of primer pairs) selected from sequences corresponding to Group E microorganisms in Table 16, SEQ ID NOS: 49-520 of Table 16, SEQ ID NOS: 49-492 of Table 16, SEQ ID NOS: 49-480 of Table 16A, SEQ ID NOS: 521-826 of Table 16C, SEQ ID NOS: 521-820 of Table 16C, SEQ ID NOS: 827-1298 of Table 16, SEQ ID NOS: 827-1258 of Table 16D, SEQ ID NOS: 1299-1604 of Table 16F, or SEQ ID NOS: 1299-1598 of Table 16F, or substantially identical or similar sequences, or any of the aforementioned nucleotide sequences of nucleic acids or primer pairs in which one or more thymine bases is substituted with a uracil base. In some examples, the combination includes, or consists essentially of, different nucleic acids or primers separately containing, or consisting essentially of, each of the different sequences corresponding to Group E microorganisms in Table 16, SEQ ID NOS: 49-520 of Table 16, SEQ ID NOS: 49-492 of Table 16, SEQ ID NOS: 49-480 of Table 16A, SEQ ID NOS: 521-826 of Table 16C, SEQ ID NOS: 521-820 of Table 16C, SEQ ID NOS: 827-1298 of Table 16, SEQ ID NOS: 827-1258 of Table 16D, SEQ ID NOS: 1299-1604 of Table 16F, or SEQ ID NOS: 1299-1598 of Table 16F, or substantially identical or similar sequences, or any of the aforementioned nucleotide sequences of nucleic acids or primer pairs in which one or more thymine bases is substituted with a uracil base.

### Nucleic Acid Combinations

**In** order to accurately assess, profile and characterize a population of microorganisms as is necessary in order to establish meaningful correlations between an animal's or environment's microbiome and state of health or homeostasis and imbalance or disease, and then assess and characterize a microbiome sample to detect and/or diagnose an imbalance, susceptibility, disorder and/or disease, it is essential to be able to perform comprehensive, specific and proportional evaluation of the constituent microorganisms in a microbiome populations. Accurate analysis of a microbiome population relies on comprehensively detecting and identifying all microorganisms, e.g., bacteria, present in a population, at least at the genus level, and detecting and identifying some, for example microorganisms of particular significance in health and disease, most, the majority of, or substantially all of the species of microorganisms present in the population to achieve a sufficient depth of constituent microorganisms of the population. Provided herein are methods, as well as systems that include the methods, for accurate, comprehensive, informative, sensitive, specific, rapid, high-throughput and cost-effective assessment, profiling or characterization of a mixture or population of microorganisms, e.g., bacteria. In some examples, the mixture or population of microorganisms is in a sample (e.g., biological sample), for example, a sample of contents of an alimentary tract of an organism, such as an animal. In some examples, compositions provided herein for such assessment, profiling or characterization of a mixture or population of microorganisms, e.g., bacteria, include a combination of (1) one or more kingdom-encompassing nucleic acid primer pairs capable of amplifying a sequence in a homologous gene or genomic region common to multiple, most, a majority, substantially all, or all microorganisms in a kingdom (e.g., bacteria), but that varies between different microorganisms in the kingdom, and/or (2) microorganism-specific nucleic acids and/or nucleic acid primer pairs that are capable of amplifying, or specifically or selectively amplifying, a specific nucleic acid sequence unique to a particular microorganism (e.g., a species, subspecies or strain of microorganism, such as bacteria). Numerous examples of kingdom-encompassing nucleic acid primer pairs and microorganism-specific nucleic acid primer pairs that can be used in combinations of nucleic acids are provided herein.

For example, in some examples, the kingdom-encompassing nucleic acids in the combination of nucleic acids include one or more primer pairs that separately amplify two or more regions, e.g., hypervariable regions, in a prokaryotic, e.g., bacterial, 16S rRNA gene. In some examples, there is little (e.g., less than or equal to 7 nucleotides, or 6 nucleotides, or 5 nucleotides, or 4 nucleotides, or 3 nucleotides, or 2 nucleotides, or 1 nucleotide) to no overlap of the nucleotide sequences of any two of the 16s rRNA gene primers that separately amplify nucleic acids comprising sequences located in multiple hypervariable regions. In some aspects, kingdom-encompassing nucleic acid primer pairs amplify 16s rRNA gene sequences less than or equal to about 200 nucleotides in length, for example, between about 125 and 200 nucleotides in length. In some examples, the kingdom-encompassing nucleic acids in the combination of nucleic acids include a plurality of nucleic acid primer pairs that includes at least 2, at least 3, at least 4, at least 5, at least 6, at least 7, at least 8 or at least 9 separate primer pairs, and optionally degenerate variants thereof, which separately amplify nucleic acids containing sequences located in 2, 3, 4, 5, 6, 7, 8 or 9 different hypervariable regions, respectively, in a prokaryotic 16s rRNA gene in a nucleic acid amplification reaction. In some examples, the kingdom-encompassing nucleic acids in the combination of nucleic acids include at least 8 separate primer pairs, and optionally degenerate variants thereof, which separately amplify nucleic acids containing sequences located in 8 different hypervariable regions in a prokaryotic 16s rRNA gene in a nucleic acid amplification reaction. In some examples, the kingdom-encompassing nucleic acids in the combination of nucleic acids include a plurality of primer pairs that separately amplify nucleic acids containing sequences located in 3 or more hypervariable regions of a prokaryotic 16S rRNA gene and wherein one of the 3 or more regions is a V5 region. Degenerate primer variants, containing, for example, different nucleotides at 1 or 2 positions in the primer sequences, are included in some compositions to ensure amplification of 16S rRNA genes containing minor variations in conserved regions. Nonlimiting examples of nucleotide sequences of primer pairs that separately amplify 8 hypervariable regions (V2, V3, V4, V5, V6, V7, V8 and V9) of the prokaryotic 16S rRNA gene are listed in Table 15. In some examples, the kingdom-encompassing nucleic acids in a combination of nucleic acids include at least 1, at least 2, at least 3, at least 4, at least 5, at least 6, at least 7, at least 8, at least 9, at least 10, at least 15, at least 20, at least 24, at least 30, at least 35, at least 40, at least 45 or more, or all of the primers, or of the primer pairs, having or consisting essentially of the sequences listed in Table 15 or SEQ ID NOS: 1-24 in Table 15 and/or SEQ ID NOS: 25-48 in Table 15 or SEQ ID NOS: 11-16, 23 and 24 in Table 15 and/or SEQ ID NOS: 35-40, 47 and 48 in Table 15. In some examples, the kingdom-encompassing nucleic acids in the combination of nucleic acids, include one or more primer pairs that provide at least 85%, or at least 90%, or at least 92%, or at least 95%, or at least 98%, or at least 99%, or 100% coverage of different bacterial 16S rRNA gene sequences in a given database (e.g., GreenGenes bacterial 16S rRNA gene sequence; www.greengenes.lbl.gov; SILVA database (www.arb-silva.de)) containing bacterial 16S rRNA gene sequences. In some examples, each of one or more microorganism-specific nucleic acid primer pairs contained in a combination of primer pairs is capable of amplifying, or specifically amplifying, a specific nucleic acid (e.g., a nucleic acid sequence from a microorganism such as a bacterium) containing, or consisting essentially of, a nucleotide sequence selected from among SEQ ID NOS: 1605-1979 of Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C. In some examples, each of one or more microorganism-specific nucleic acid primer pairs contained in a combination of primer pairs is capable of amplifying, or specifically amplifying, a specific nucleic acid sequence containing a nucleotide sequence selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, to generate amplicon sequences that are less than about 500, less than about 475, less than about 450, less than about 400, less than about 375, less than about 350, less than about 300, less than about 275, less than about 250, less than about 200, less than about 175, less than about 150, or less than about 100 nucleotides in length, or amplicon sequences that consist essentially of a sequence selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or substantially identical or similar sequence, and optionally containing the nucleic acid primer sequences at the 5' and 3' ends of the sequence. In some examples, the collection of microorganism-specific nucleic acid primer pairs in a combination are capable of amplifying, or specifically amplifying, in a multiplex reaction at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 125, at least 150, at least 175, at least 200, at least 225, or at least 230 or more different nucleic acids containing a different one of SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C. In some such examples, the microorganism-specific nucleic acid primer pairs in the combination can amplify the different nucleic acids containing a different one of SEQ ID NOS: 1605-1979 in Table 17, or SEQ ID NOS: 1605-1806, 1809-1816, 1821-1970 and 1972-1974 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or that consists essentially of a nucleotide sequence selected from among SEQ **ID** NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or substantially identical or similar sequence, and optionally containing the nucleic acid primer sequences at the 5' and 3' ends of the sequence. In some examples, microorganism-specific nucleic acid primer pairs in the combination include one or more primer pairs having or consisting essentially of a nucleotide sequence or pair of sequences (for primer pairs) selected from Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a sequence or sequences substantially identical or similar thereto, or any of the aforementioned nucleotide sequences of nucleic acids or primer pairs in which one or more thymine bases is substituted with a uracil base. In some examples, microorganism-specific nucleic acid primer pairs in the combination include at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 125, at least 150, at least 175, at least 200, at least 225, or at least 230, or all of the nucleic acid primer pairs having or consisting essentially of sequences selected from Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a sequence or sequences substantially identical or similar thereto, or any of the aforementioned nucleotide sequences of nucleic acids or primer pairs in which one or more thymine bases is substituted with a uracil base. In some examples, the combinations include one or more microorganism-specific nucleic acid primer pairs that amplifies a specific nucleic acid sequence unique to one or more microorganisms (e.g., bacteria) implicated in one or more conditions, disorders and/or diseases.

Described herein for compositions that include one or more, or a plurality of, or combinations of nucleic acids, primers or nucleic acid primer pairs, one or more of the nucleic acids, or one or more primers or primer pairs may include a modification. In some examples, a modification is one that facilitates nucleic acid manipulation, amplification, ligation and/or sequencing of amplification products and/or reduction or elimination of primer dimers. In particular examples, a modification is one that facilitates multiplex nucleic acid amplification, ligation and/or sequencing of products of multiplex amplification. In some examples, at least one primer of a primer pair or both primers of a primer pair contains a modification relative to the nucleic acid sequence to be amplified that increases the susceptibility of the primer to cleavage. For example, in some examples, one or more nucleic acids, or primers, or both primers of a primer pair has at least one cleavable group located at either a) the 3' end or the 5' end, and/or b) at about the central nucleotide position of the nucleic acid or primer, and wherein the nucleic acids, primers or primer pairs can be substantially non-complementary to other nucleic acids, primers or primer pairs in the composition. In some examples, the composition comprises at least 50, 100, 150, 200, 250, 300, 350, 398, or more primer pairs. In some examples, the primer pairs comprise about 15 nucleotides to about 40 nucleotides in length. In some examples, at least one nucleotide of one or more primers is replaced with a cleavable group. In some examples the cleavable group can be a uridine nucleotide. In some examples, the template, one or more primers and/or amplification product includes nucleotides or nucleobases that can be recognized by specific enzymes. In some examples, the nucleotides or nucleobases can be bound by specific enzymes. Optionally, the specific enzymes can also cleave the template, one or more primers and/or amplification product at one or more sites. In some examples, such cleavage can occur at specific nucleotides within the template, one or more primers and/or amplification product. For example, the template, one or more primers and/or amplification product can include one or more nucleotides or nucleobases including uracil, which can be recognized and/or cleaved by enzymes such as uracil DNA glycosylase (UDG, also referred to as UNG) or formamidopyrimidine DNA glycosylase (Fpg). The template, one or more primers and/or amplification product can include one or more nucleotides or nucleobases including RNA-specific bases, which can be recognized and/or cleaved by enzymes such as RNAseH. In some examples, the template, one or more primers and/or amplification product can include one or more abasic sites, which can be recognized and/or cleaved using various proofreading polymerases or apyrase treatments. In some examples, the template, one or more primers and/or amplification product can include 7,8-dihydro-8-oxoguanine (8-oxoG) nucleobases, which can be recognized or cleaved by enzymes such as Fpg. In some examples, one or more amplified target sequences can be partially digested by a FuPa reagent. In some examples, the primer includes a sufficient number of modified nucleotides to allow functionally complete degradation of the primer by the cleavage treatment, but not so many as to interfere with the primer's specificity or functionality prior to such cleavage treatment, for example in the amplification reaction. In some examples, the primer includes at least one modified nucleotide, but no greater than 75% of nucleotides of the primer are modified. For example, the primers can include uracil-containing nucleobases that can be selectively cleaved using UNG/UDG (optionally with heat and/or alkali). In some examples, the primers can include uracil-containing nucleotides that can be selectively cleaved using UNG and Fpg. In some examples, the cleavage treatment includes exposure to oxidizing conditions for selective cleavage of dithiols, treatment with RNAseH for selective cleavage of modified nucleotides including RNA-specific moieties (e.g., ribose sugars, etc.), and the like. This cleavage treatment can effectively fragment the original amplification primers and non-specific amplification products into small nucleic acid fragments that include relatively few nucleotides each. Such fragments are typically incapable of promoting further amplification at elevated temperatures. Such fragments can also be removed relatively easily from the reaction pool through the various post-amplification cleanup procedures known in the art (e.g., spin columns, NaEtOH precipitation, etc).

A composition described herein includes a sample containing a plurality of microorganisms, or nucleic acids from such a sample that contains a plurality of microorganisms, and one or more nucleic acids, primers and/or primer pairs described herein and, optionally, a polymerase, e.g., a DNA polymerase. In some examples, the sample is a biological sample, such as, for example, an environmental sample or a sample from an animal subject, e.g., a human. Samples include, but are not limited to, biological fluid samples, blood samples, skin samples, mucus samples, saliva samples, sputum samples, samples from a subject's oral or nasal cavity, respiratory tract samples, vaginal samples, alimentary tract samples and fecal samples. In some examples, the sample is from the alimentary tract of an animal, such as, for example, a fecal or stool sample. In particular examples, the composition includes one or more kingdom-encompassing nucleic acid primer pairs capable of amplifying a sequence in a homologous gene or genomic region common to multiple, most, a majority, substantially all, or all microorganisms in a kingdom (e.g., bacteria), but that varies between different microorganisms in the kingdom, and/or one or more microorganism-specific nucleic acid primer pairs that amplify a specific nucleic acid sequence unique to a particular microorganism (e.g., a species, subspecies or strain of microorganism, such as bacteria). Numerous examples of kingdom-encompassing nucleic acid primer pairs and microorganism-specific nucleic acid primer pairs that can be used in combinations of nucleic acids are provided herein. For example, in some examples, the kingdom-encompassing nucleic acids in the combination of nucleic acids include one or more primer pairs that separately amplify two or more, three or more, four or more, five or more, 6 or more, 7 or more, or 8 or more regions, e.g., hypervariable regions, in a prokaryotic, e.g., bacterial, 16S rRNA gene. In some examples, at least one of the one or more microorganism-specific nucleic acid primer pairs is capable of amplifying, or specifically amplifying, a specific nucleic acid sequence containing a nucleotide sequence selected from among SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, to generate amplicon sequences that are less than about 500, less than about 475, less than about 450, less than about 400, less than about 375, less than about 350, less than about 300, less than about 275, less than about 250, less than about 200, less than about 175, less than about 150, or less than about 100 nucleotides in length, or amplicon sequences that consist essentially of a sequence selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or substantially identical or similar sequence, and optionally containing the nucleic acid primer sequences at the 5' and 3' ends of the sequence. In some examples, the one or more microorganism-specific nucleic acid primer pairs is a plurality of such primer pairs wherein each of the primer pairs is capable of amplifying, or specifically amplifying, a specific nucleic acid sequence containing a nucleotide sequence selected from among SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C. In some examples, the one or more microorganism-specific nucleic acid primer pairs is a plurality of such primer pairs wherein each of the primer pairs is capable of amplifying, or specifically amplifying, a specific nucleic acid sequence containing a nucleotide sequence selected from among SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, to generate an amplicon sequence that is less than about 500, less than about 475, less than about 450, less than about 400, less than about 375, less than about 350, less than about 300, less than about 275, less than about 250, less than about 200, less than about 175, less than about 150, or less than about 100 nucleotides in length, or that consists essentially of a nucleotide sequence selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or substantially identical or similar sequence, and optionally containing the nucleic acid primer sequences at the 5' and 3' ends of the sequence.

Also described herein are compositions containing a mixture of nucleic acids, in which most, or substantially all of the nucleic acids contain sequence of a portion of the genome of a microorganism, e.g., a bacterium. In some examples, the mixture of nucleic acids includes nucleic acids containing sequences of a portion of at least 2, at least 5, at least 10, at least 20, at least 25, at least 30, at least 35, at least 40, at least 50, at least 75, at least 100, at least 150, at least 200, at least 250, at least 300, at least 350, at least 400, or at least 500 or more different microorganisms, e.g., different species of microorganisms such as bacteria. In some examples, the sequences of portions of the genome of microorganisms are each less than about 1000 nucleotides, less than about 900 nucleotides, less than about 1000 nucleotides, less than about 900 nucleotides, less than about 800 nucleotides, less than about 700 nucleotides, less than about 600 nucleotides, less than about 500 nucleotides, less than about 450 nucleotides, less than about 400 nucleotides, less than about 350 nucleotides, less than about 300 nucleotides, less than about 250 nucleotides, or less than about 200 nucleotides in length. In some examples, the sequences of portions of the genome of microorganisms are each less than or about 250 nucleotides in length. In some examples, the nucleic acids include double-stranded, partially double-stranded and/or single-stranded nucleic acids. In some examples, the nucleic acids include amplicons generated in a nucleic acid amplification reaction of nucleic acids from one or more, or a plurality of microorganisms, such as a plurality of different microorganisms, e.g., bacteria. In some examples, the nucleic acids include nucleotides containing a uracil nucleobase. In some examples, the nucleic acids contain 5' and/or 3' overhangs. In some examples, the composition contains one or more, or a plurality, of primers, e.g., nucleic acids and/or primer pairs of any of the examples described herein. In some examples, the composition includes a DNA polymerase, a DNA ligase, and/or at least one uracil cleaving or modifying enzyme. In some examples, the nucleic acids include any one or more of the following:
(1) one or more nucleic acids containing, or consisting essentially of, a nucleotide sequence of a hypervariable region of a prokaryotic 16S rRNA gene, e.g., a V1, V2, V3, V4, V5, V6, V7, V8 and/or V9 region,
(2) a plurality of nucleic acids containing, or consisting essentially of, a nucleotide sequence of a hypervariable region of a prokaryotic 16S rRNA gene, e.g., a V1, V2, V3, V4, V5, V6, V7, V8 and/or V9 region,
(3) one or more or a plurality of nucleic acids containing, or consisting essentially of, a nucleotide sequence of a hypervariable region of a prokaryotic 16S rRNA gene, e.g., a V1, V2, V3, V4, V5, V6, V7, V8 and/or V9 region, wherein the sequence has the sequence from only one hypervariable region,
(4) one or more nucleic acids containing, or consisting essentially of, a nucleotide sequence of a hypervariable region of a prokaryotic 16S rRNA gene, e.g., a V1, V2, V3, V4, V5, V6, V7, V8 and/or V9 region, wherein the sequence includes one or more sequences selected from among sequences listed in Table 15 or SEQ ID NOS: 1-24 in Table 15 and/or SEQ ID NOS: 25-48 in Table 15 or SEQ ID NOS: 11-16, 23 and 24 in Table 15 and/or SEQ ID NOS: 35-40, 47 and 48 in Table 15, and/or
(5) one or more single-stranded nucleic acids containing, or consisting essentially of, a nucleotide sequence selected from among SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, optionally containing one or more primer sequences at the 3' and/or 5' end (e.g., sequences selected from Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F), or the complement thereof, and/or one or more double-stranded or partially double-stranded nucleic acids containing, or consisting essentially of, a nucleotide sequence selected from among SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, optionally containing one or more primer sequences at the 3' and/or 5' end (e.g., sequences selected from Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F), and a complementary nucleotide sequence hybridized thereto.

In some examples, the nucleic acids include any combination of nucleic acids of (1), (2), (3) or (4) above with nucleic acids of (5) above. In some examples of the compositions containing a mixture of nucleic acids, in which most, or substantially all of the nucleic acids contain sequence of a portion of the genome of a microorganism, e.g., a bacterium, provided herein, the composition is or contains one or more libraries of microorganism, e.g., bacteria, nucleic acids. In some examples, the mixture of nucleic acids is generated by amplifying nucleic acids in or from a sample containing microorganisms (e.g., bacteria) using primers and/or primer pairs provided herein. For example, a mixture of nucleic acids can be generated by amplifying nucleic acids using (1) one or more kingdom-encompassing nucleic acid primer pairs capable of amplifying a sequence in a homologous gene or genomic region common to multiple, most, a majority, substantially all, or all microorganisms in a kingdom (e.g., bacteria), but that varies between different microorganisms in the kingdom, and/or (2) one or more microorganism-specific nucleic acids and/or nucleic acid primer pairs that are capable of amplifying, or specifically or selectively amplifying, a specific nucleic acid sequence unique to a particular microorganism (e.g., a species, subspecies or strain of microorganism, such as bacteria). Numerous examples of kingdom-encompassing nucleic acid primer pairs and microorganism-specific nucleic acid primer pairs that can be used in generating combinations of nucleic acids are described herein. In some examples, the mixture is generated by amplifying microorganism nucleic acids using kingdom-encompassing nucleic acid primer pairs and microorganism-specific nucleic acid primers and/or nucleic acid primer pairs in a single reaction mixture. In some examples, the mixture is generated by separately amplifying microorganism nucleic acids, e.g., from a single sample, using kingdom-encompassing nucleic acid primer pairs in one amplification reaction and microorganism-specific nucleic acid primers and/or nucleic acid primer pairs in a separate amplification reaction and then combining the products of both amplification reactions. In some examples, the mixture of nucleic acids comprises or consists essentially of portions of a prokaryotic 16S rRNA gene, such as nucleotide sequences of a hypervariable region of a prokaryotic (e.g., bacteria) 16S rRNA gene (e.g., a V1, V2, V3, V4, V5, V6, V7, V8 and/or V9 region), from one or more, or a plurality of, microorganisms and portions of a microorganism (e.g., bacteria) genome from one or more, or a plurality of, microorganisms that are not contained within a prokaryotic 16S rRNA gene.

### Methods for Amplification of Nucleic Acids

Methods described herein include methods for amplification and/or detection of nucleic acids. In particular examples, the nucleic acids being amplified and/or detected are from microorganisms, including, for example, bacteria and archaea. As described further herein, methods for amplifying and/or detecting nucleic acids from microorganisms provided herein represent significant improvements over previous methods including, but not limited to, improvements in microorganism nucleic acid amplification and/or detection coverage, sensitivity, efficiency, scale, cost-effectiveness and/or application to or use in other methods. In some examples nucleic acids are subjected to nucleic acid hybridization and/or amplification, for example, using any of the nucleic acids provided herein as probes and/or amplification primers. In some examples, the presence or absence of one or more hybridization and/or nucleic acid amplification products is detected. In some examples, the nucleic acid amplification is a multiplex amplification. In some examples, the amplification is performed using a plurality of nucleic acid primer pairs and is conducted in a single multiplex amplification reaction mixture. In some examples, the presence or absence of one or more nucleic acids and/or amplification products is detected using one or more nucleic acids provided herein as a probe (e.g., a detectable or labeled probe). In some examples, the presence or absence of one or more nucleic acid amplification products is detected by obtaining nucleotide sequence information of one or more nucleic acid amplification products.

### Methods for Amplification of Nucleic Acids of Selected Microorganisms

In some examples, a method describedherein for amplifying a target nucleic acid of one or more microorganisms includes (a) obtaining nucleic acids of one or more microorganisms selected from the microorganisms listed in Table 1 (or Table 1, except for, or excluding, *Actinomyces viscosus* and/or *Blautia coccoides,* or Table 1, except for, or excluding, *Actinomyces viscosus, Blautia coccoides* and/or *Helicobacter salomonis)* and (b) subjecting the nucleic acids to nucleic acid amplification using at least one primer pair that is capable of specifically amplifying a target nucleic acid sequence contained within a genome of a microorganism selected from the microorganisms of Table 1 (or Table 1, except for, or excluding, *Actinomyces viscosus* and/or *Blautia coccoides,* or Table 1, except for, or excluding, *Actinomyces viscosus, Blautia coccoides* and/or *Helicobacter salomonis),* thereby producing amplified copies of the target nucleic acid. In some examples, the target nucleic acid is unique to the microorganism. In some examples, the target nucleic acid is not contained within a prokaryotic 16S rRNA gene. In some examples, the nucleic acids subjected to amplification include nucleic acids from a plurality of different microorganisms listed in Table 1. In some such examples, amplified copies of a plurality of different microorganisms in Table 1 (or Table 1, except for, or excluding, *Actinomyces viscosus* and/or *Blautia coccoides,* or Table 1, except for, or excluding, *Actinomyces viscosus, Blautia coccoides* and/or *Helicobacter salomonis)* is produced, for example in a multiplex nucleic acid amplification. In some examples, the nucleic acids subjected to amplification include a mixture of nucleic acids of one or more, or a plurality of, microorganisms selected from among the microorganisms listed in Table 1 (or Table 1, except for, or excluding, *Actinomyces viscosus* and/or *Blautia coccoides,* or Table 1, except for, or excluding, *Actinomyces viscosus, Blautia coccoides* and/or *Helicobacter salomonis)* and one or more microorganisms, e.g., bacteria, not listed in Table 1. In some examples, nucleic acids of one or more microorganisms selected from the microorganisms listed in Table 1 are obtained from a biological sample, such as, for example, a sample of contents of the alimentary canal of an animal. In some examples, the sample is a fecal sample. In some examples, at least one, or one or more, target nucleic acid sequence(s) comprises or consists essentially of a nucleotide sequence selected from the nucleotide sequences of SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence. In some examples, at least one, or one or more, product(s) of the nucleic acid amplification comprises, or consists essentially of, a nucleotide sequence selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, or the complement thereof, and optionally having one or more primer sequences at the 5' and/or 3' end(s) of the sequence, such as any of the primer sequences provided herein. In some examples, the at least one primer pair does not detectably amplify a nucleic acid sequence contained within any genus other than the genus of the microorganism containing the target nucleic acid sequence. In some examples, the at least one primer pair does not detectably amplify a nucleic acid sequence contained within any species other than the species of the microorganism containing the target nucleic acid sequence. In some examples, at least one primer of the primer pair, or at least one primer pair, contains, or consists essentially of, the sequence or sequences of a primer or primer pair in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the nucleic acids are subjected to nucleic acid amplification using a plurality of primers or primer pairs, each containing, or consisting essentially of, a sequence or sequences of a primer pair in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some of the examples in which the nucleic acids are subjected to nucleic acid amplification using more than one, or a plurality of primers or primer pairs, the amplification is a multiplex amplification conducted in a single reaction mixture. In some examples, at least one primer or one primer pair includes a modification that facilitates nucleic acid manipulation, amplification, ligation and/or sequencing of amplification products and/or reduction or elimination of primer dimers. In particular examples, a modification is one that facilitates multiplex nucleic acid amplification, ligation and/or sequencing of products of multiplex amplification.

### Methods for Multiplex Amplification of Multiple Regions of a Gene

In some examples, a multiplex amplification method described herein is for amplifying multiple regions of a gene of one or more microorganisms, e.g., bacteria. In one example, the method includes (a) obtaining nucleic acids of one or more microorganisms comprising a 16S rRNA gene and (b) subjecting the nucleic acids to nucleic acid amplification using a combination of primer pairs that includes at least two primer pairs that separately amplify nucleic acids containing sequences of different hypervariable regions of a prokaryotic 16S rRNA gene thereby producing amplified copies of the nucleic acid sequences containing sequences of different hypervariable regions of the 16S rRNA gene of one or more microorganisms. In some examples, the microorganism(s) is/are bacteria. In some examples, the prokaryotic 16S rRNA gene is a bacterial gene. In some examples, the nucleic acids subjected to amplification include nucleic acids from a plurality of different microorganisms. In some examples, nucleic acids of one or more microorganisms comprising a 16S rRNA gene are obtained from a biological sample, such as, for example, a sample of contents of the alimentary canal of an animal. In some examples, the sample is a fecal sample. In some examples, the primers of the combination of primer pairs are directed to, or bind to, or hybridize to nucleic acid sequences contained in conserved regions of a 16S rRNA gene. In some examples, each primer of the combination of primer pairs contains less than 10, less than 9, less than 8, less than 7, less than 6, less than 5, less than 4, less than 3, or less than 2 contiguous nucleotides of sequence identical to a sequence of contiguous nucleotides of another primer in the combination of primer pairs. In some examples, the nucleic acid sequences being amplified are less than about 300 bp, less than about 250 bp, less than about 200 bp, less than about 175 bp, less than about 150 bp, or less than about 125 bp in length. In some examples, the combination of primer pairs separately amplify nucleic acids containing sequences of 3 or more, 4 or more, 5 or more, 6 or more, 7 or more, 8 or more or 9 different hypervariable regions of a prokaryotic 16S rRNA gene thereby producing amplified copies of the nucleic acids containing sequences of the 3 or more, 4 or more, 5 or more, 6 or more, 7 or more, 8 or more or 9 different hypervariable regions of the 16S rRNA gene of one or more microorganisms, wherein the amplified copies of different hypervariable regions are separate amplicons. In some examples, the combination of primer pairs separately amplify 8 different nucleic acids separately containing sequences of 8 different hypervariable regions of a prokaryotic 16S rRNA gene. In some examples, the 8 different hypervariable regions are V2-V9. In some examples, the combination of primer pairs separately amplify at least 3 different nucleic acids each of which separately contains a sequence of a different hypervariable region of a prokaryotic 16S rRNA gene wherein one of the 3 or more regions is a V5 region thereby producing amplified copies of the nucleic acids separately containing sequences of 3 or more hypervariable regions of the 16S rRNA gene of one or more microorganisms. In some examples, the combination of primer pairs includes degenerate sequences of one or more primers in one or more primer pairs. For example, in some examples, for at least one of the hypervariable regions amplified by the combination of primer pairs, at least two different primer pairs in the combination of primer pairs separately amplify nucleic acid sequence within the same hypervariable region for 2 or more species of the same prokaryotic genus, or for 2 or more strains of the same prokaryotic species, having differences in nucleic acid sequences at the same hypervariable region. In some such instances, at least two different primer pairs in the combination of primer pairs separately amplify nucleic acid sequence within the V2 hypervariable region for 2 or more species of the same prokaryotic genus, or 2 or more strains of the same prokaryotic species, having differences in nucleic acid sequences at the V2 hypervariable region, and/or at least two different primer pairs in the combination of primer pairs separately amplify nucleic acid sequence within the V8 hypervariable region for 2 or more species of the same prokaryotic genus, or 2 or more strains of the same prokaryotic species, having differences in nucleic acid sequences at the V8 hypervariable region. In some examples, the combination of primer pairs that amplifies nucleic acids containing sequences of hypervariable regions of a prokaryotic 16S rRNA gene comprises primers and/or primer pairs containing, or consisting essentially of, a sequence or sequences of a primer or primer pair in Table 15, or SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base. In some examples, the amplification is a multiplex amplification conducted in a single reaction mixture. In some examples, at least one primer or primer pair in the combination includes a modification that facilitates nucleic acid manipulation, amplification, ligation and/or sequencing of amplification products and/or reduction or elimination of primer dimers. In particular examples, a modification is one that facilitates multiplex nucleic acid amplification, ligation and/or sequencing of products of multiplex amplification.

### Methods for Amplification of Multiple Regions of a Genome of a Microorganism

In some examples, an amplification method is described for amplifying multiple regions of the genome of one or more microorganisms. In some examples, the method includes (a) obtaining nucleic acids of one or more microorganisms comprising a 16S rRNA gene and (b) subjecting the nucleic acids to nucleic acid amplification using a combination of primer pairs comprising (i) one or more primer pairs that amplifies a nucleic acid containing a sequence of a hypervariable region of a prokaryotic 16S rRNA gene (referred to as the "16S rRNA gene primers and primer pairs"), and (ii) one or more primer pairs that amplify a target nucleic acid sequence contained within the genome of a microorganism that is not contained within a hypervariable region of a prokaryotic 16S rRNA gene, wherein different primer pairs amplify different target nucleic acid sequences contained within the genome of different microorganisms (referred to as the "non-16S rRNA gene primers and primer pairs"), thereby generating amplified copies of at least two different regions of the genome of one or more microorganisms. In some examples, the microorganism(s) is/are bacteria. In some examples, the prokaryotic 16S rRNA gene is a bacterial gene and/or the prokaryotic microorganism is a bacterium. In some examples, the one or more primer pairs that amplifies a nucleic acid containing a sequence of a hypervariable region of a prokaryotic 16S rRNA gene separately amplify nucleic acid sequences of different hypervariable regions. In some examples, the primers of the one or more primer pairs of (i) are directed to, or bind to, or hybridize to nucleic acid sequences contained in conserved regions of a prokaryotic 16S rRNA gene. In some examples, the amplification is a multiplex amplification conducted in a single reaction mixture. In some examples, an amplification method for amplifying multiple regions of the genome of one or more microorganisms includes (a) obtaining nucleic acids of one or more microorganisms comprising a 16S rRNA gene and (b) subjecting the nucleic acids to two or more separate nucleic acid amplification reactions using a first set of primer pairs for one nucleic acid amplification reaction and a second set of primer pairs for the other nucleic acid amplification reaction, wherein (i) the first set of primer pairs comprises one or more primer pairs that amplifies a nucleic acid containing a sequence of a hypervariable region of a prokaryotic 16S rRNA gene (referred to as the "16S rRNA gene primers and primer pairs"), and (ii) the second set of primer pairs comprises one or more primer pairs that amplify a target nucleic acid sequence contained within the genome of a microorganism that is not contained within a hypervariable region of a prokaryotic 16S rRNA gene, wherein different primer pairs amplify different target nucleic acid sequences contained within the genome of different microorganisms (referred to as the "non-16S rRNA gene primers and primer pairs"), thereby generating amplified copies of at least two different regions of the genome of one or more microorganisms. In some examples, the microorganism(s) is/are bacteria. In some examples, the prokaryotic 16S rRNA gene is a bacterial gene and/or the prokaryotic microorganism is a bacterium. In some examples, the one or more primer pairs that amplifies a nucleic acid containing a sequence of a hypervariable region of a prokaryotic 16S rRNA gene separately amplify nucleic acid sequences of different hypervariable regions. In some examples, the primers of the one or more primer pairs of (i) are directed to, or bind to, or hybridize to nucleic acid sequences contained in conserved regions of a prokaryotic 16S rRNA gene. In some examples, the amplification is a multiplex amplification conducted in a single reaction mixture.

In some examples, of the amplification methods for amplifying multiple regions of the genome of one or more microorganisms, the target nucleic acid sequence contained within a genome of a prokaryotic microorganism, e.g., bacteria, is unique to the microorganism. In some examples, the one or more 16S rRNA gene primer pairs amplify a nucleic acid sequence in a plurality of microorganisms, e.g., bacteria, from different genera. In some examples, a mixture of nucleic acids of at least two different microorganisms, e.g., bacteria, is obtained and subjected to nucleic acid amplification, and the genome of only one of the microorganisms contains a target sequence specifically amplified by the non-16S rRNA gene primer pair. In some such examples, the generated amplified copies contain copies of a target nucleic acid sequence amplified by a non-16S rRNA gene primer pair from the nucleic acid of the genome of one microorganism but do not contain copies of a target nucleic acid sequence amplified by a non-16S rRNA gene primer pair from the nucleic acid of the genome of any other microorganism that was subjected to nucleic acid amplification. Also in some such examples, the generated amplified copies contain copies of a nucleic acid sequence of a hypervariable region amplified by a 16S rRNA gene primer pair from the nucleic acids of the genome of a plurality of microorganisms. In some examples, the nucleic acids subjected to nucleic acid amplification include nucleic acids from a plurality of different microorganisms. In some examples, nucleic acids of one or more microorganisms, e.g., bacteria, comprising a 16S rRNA gene are obtained from a biological sample, such as, for example, a sample of contents of the alimentary tract of an animal. In some examples, the sample is a fecal sample. In some examples, each primer of the one or more 16S rRNA gene primer pairs contains less than 10, less than 9, less than 8, less than 7, less than 6, less than 5, less than 4, less than 3, or less than 2 contiguous nucleotides of sequence identical to a sequence of contiguous nucleotides of another primer in the combination of primer pairs. In some examples, the nucleic acid sequences being amplified by the one or more 16S rRNA gene primer pairs are less than about 300 bp, less than about 250 bp, less than about 200 bp, less than about 175 bp, less than about 150 bp, or less than about 125 bp in length. In some examples, the 16S rRNA gene primer pairs separately amplify nucleic acids separately containing a different one of 3 or more, 4 or more, 5 or more, 6 or more, 7 or more, 8 or more or 9 different hypervariable regions of a prokaryotic 16S rRNA gene thereby producing amplified copies of the nucleic acids separately containing sequences of one of 3 or more, 4 or more, 5 or more, 6 or more, 7 or more, 8 or more or 9 different hypervariable regions of the 16S rRNA gene of one or more microorganisms, wherein the amplified copies of different hypervariable regions are separate amplicons. In some examples, the 16S rRNA gene primer pairs separately amplify nucleic acids separately containing sequences of 8 different hypervariable regions of a prokaryotic 16S rRNA gene. In some examples, the 8 different hypervariable regions are V2-V9. In some examples, the 16S rRNA gene primer pairs separately amplify nucleic acids separately containing sequences of 3 or more different hypervariable regions of a prokaryotic 16S rRNA gene wherein one of the 3 or more regions is a V5 region thereby producing amplified copies of the nucleic acids separately containing sequences of 3 or more different hypervariable regions of the 16S rRNA gene of one or more microorganisms. In some examples, the combination of primer pairs includes degenerate sequences of one or more primers in one or more primer pairs. In some examples, the 16S rRNA gene primer pair(s) comprise primers and/or primer pairs containing, or consisting essentially of, a sequence or sequences of a primer or primer pair in Table 15, or SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base. In some examples, the at least one non-16S rRNA gene primer pair specifically amplifies a target nucleic acid sequence contained within a genome of a microorganism selected from the microorganisms of Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the target nucleic acid is unique to the microorganism. In some examples, the nucleic acids subjected to amplification include nucleic acids from a plurality of different microorganisms listed in Table 1. In some such examples, amplified copies of a plurality of different microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis,* is produced. In some examples, the nucleic acids subjected to amplification include a mixture of nucleic acids of one or more, or a plurality of, microorganisms selected from among the microorganisms listed in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis,* and one or more microorganisms, e.g., bacteria, not listed in Table 1. In some examples, at least one, or one or more, target nucleic acid sequence(s) comprises or consists essentially of a nucleotide sequence selected from the nucleotide sequences of SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence. In some examples, at least one, or one or more, product(s) of the nucleic acid amplification comprises, or consists essentially of, a nucleotide sequence selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, or the complement thereof, and optionally having one or more primer sequences at the 5' and/or 3' end(s) of the sequence, such as any of the primer sequences provided herein. In some examples, at least one, or one or more, product(s) of the nucleic acid amplification is less than about 500, less than about 475, less than about 450, less than about 400, less than about 375, less than about 350, less than about 300, less than about 275, less than about 250, less than about 200, less than about 175, less than about 150, or less than about 100 nucleotides in length. In some examples, the at least one non-16S rRNA gene primer pair does not detectably amplify a nucleic acid sequence contained within any genus other than the genus of the microorganism containing the target nucleic acid sequence. In some examples, the at least one non-16S rRNA gene primer pair does not detectably amplify a nucleic acid sequence contained within any species other than the species of the microorganism containing the target nucleic acid sequence. In some examples, at least one primer of the non-16S rRNA gene primer pair, or at least one non-16S rRNA gene primer pair, contains, or consists essentially of, the sequence or sequences of a primer or primer pair in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the nucleic acids are subjected to nucleic acid amplification using a plurality of non-16S rRNA gene primers or primer pairs, each containing, or consisting essentially of, a sequence or sequences of a primer pair in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, at least one primer or one primer pair in the combination of primer pairs includes a modification that facilitates nucleic acid manipulation, amplification, ligation and/or sequencing of amplification products and/or reduction or elimination of primer dimers. In particular examples, a modification is one that facilitates multiplex nucleic acid amplification, ligation and/or sequencing of products of multiplex amplification.

### Procedures/Techniques for Use in Methods for Amplification of Nucleic Acids

Methods for obtaining nucleic acids, for example, from a sample are described herein and/or known to those of skill in the art. Samples containing microorganisms can come from a variety of sources, including, for example, environmental sources, e.g., water, soil, and organismal sources, e.g., animals, including, without limitation, insects, domestic animals (e.g., cattle, sheep, pigs, horses, dogs, cats, etc.), mammals (e.g., humans). Common animal samples include, without limitation, saliva, biopsies, tumors, scrapings, swabs, blood, mucus, urine, plasma, semen, hair, laser capture micro-dissections, surgical resections, feces and other clinical or laboratory obtained samples. Fecal samples are commonly used as sources of microorganisms from an animal's alimentary tract or gut. Kits, protocols and instruments for use in extracting nucleic acids from animal samples are available from commercial public sources and include, for example, the MagMAX^{™} Microbiome Ultra Nucleic Acid Isolation Kit (Thermo Fisher Scientific; catalog no. A42357 (with plate) or A42358 (with tubes)) which can be used with the Thermo Scientific^{™} Kingfisher^{™} Flex Magnetic Particle Processor with 96 deep well heads (Thermo Fisher Scientific; catalog no. 5400630). The amount of nucleic acid material required for successful multiplex amplification reactions as can be conducted in the examples of the methods described herein, can be about 1 ng. In some examples, the amount of nucleic acid material can be about 10 ng to about 50 ng, about 10 ng to about 100 ng, or about 1 ng to about 200 ng of nucleic acid material. Higher amounts of input material can be used, however one aspect of the disclosure is to selectively amplify a plurality of target sequence from a low (ng) about of starting material.

Amplification methods provided herein typically include preparation of an amplification reaction mixture containing reagents for conducting the reaction and subjecting the mixture to conditions to achieve repeated cycles of primer annealing to a template nucleic acid, primer extension and dissociation of the extended primer and template strands (e.g., denaturation). Various techniques for use in amplifying nucleic acids can be employed in the amplification methods, for example, polymerase chain reaction (PCR)-based techniques, helicase-dependent amplification (HDA), loop-mediated isothermal amplification (LAMP) and strand displacement amplification. In some examples, the method comprises hybridizing one or more primers of a primer pair to a target template sequence, extending a first primer of the primer pair, denaturing the extended first primer product from the population of nucleic acid molecules, hybridizing to the extended first primer product the second primer of the primer pair, extending the second primer to form a double stranded product, and, in some examples, digesting the target-specific primer pair away from the double stranded product to generate a plurality of amplified target sequences. In some examples, the digesting includes partial digesting of one or more of the target-specific primers from the amplified target sequence. In some examples, the method of performing multiplex PCR amplification includes contacting a plurality of primer pairs having a forward and reverse primer, with a population of template nucleic acid sequences, e.g., in or from a sample, to form a plurality of template/primer duplexes; adding a DNA polymerase and a mixture of dNTPs to the plurality of template/primer duplexes for sufficient time and at sufficient temperature to extend either (or both) the forward or reverse primer in each target-specific primer pair via template-dependent synthesis thereby generating a plurality of extended primer product/template duplexes; denaturing the extended primer product/template duplexes; annealing to the extended primer product the complementary primer from the target-specific primer pair; and extending the annealed primer in the presence of a DNA polymerase and dNTPs to form a plurality of target-specific double-stranded nucleic acid molecules. In some examples, the steps of the amplification PCR method can be performed in any order. In some instances, the methods disclosed herein can be further optimized to remove one or more steps and still obtain sufficient amplified target sequences to be used in a variety of downstream processes. For example, the number of purification or clean-up steps can be modified to include more or less steps than disclose herein, providing the amplified target sequences are generated in sufficient yield. In some examples the multiplex PCR comprises hybridizing one or more target-specific primer pairs to a nucleic acid molecule, extending the primers of the target-specific primer pairs via template dependent synthesis in the presence of a DNA polymerase and dNTPs; repeating the hybridization and extension steps for sufficient time and sufficient temperature there generating a plurality of amplified target sequences. In some examples, the steps of the multiplex amplification reaction method can be performed in any order. The multiplex PCR amplification reactions disclosed herein can include a plurality of "cycles" typically performed on a thermocycler. Each cycle includes at least one annealing step and at least one extension step. In one example, a mutliplex PCR amplification reaction is performed wherein target-specific primer pairs are hybridized to a target sequence; the hybridized primers are extended generating an extended primer product/nucleic acid duplex; the extended primer product/nucleic acid duplex is denatured allowing the complementary primer to hybridize to the extended primer product, wherein the complementary primer is extended to generate a plurality of amplified target sequences. In one example, the methods disclosed herein have about 5 to about 18 cycles per preamplification reaction. The annealing temperature and/or annealing duration per cycle can be identical; can include incremental increases or decreases, or a combination of both. The extension temperature and/or extension duration per cycle can be identical; can include incremental increases or decreases, or a combination of both. For example, the annealing temperature or extension temperature can remain constant per cycle. In some examples, the annealing temperature can remain constant each cycle and the extension duration can incrementally increase per cycle. In some examples, increases or decreases in duration can occur in 15 second, 30 second, 1 minute, 2 minute or 4 minute increments. In some examples, increases or decrease in temperature can occur as 0.5, 1, 2, 3, or 4 Celsius deviations. In some examples, the amplification reaction can be conducted using hot-start PCR techniques. These techniques include the use of a heating step (>60°C) before polymerization begins to reduce the formation of undesired PCR products. Other techniques such as the reversible inactivation or physical separation of one or more critical reagents of the reaction, for example the magnesium or DNA polymerase can be sequestered in a wax bead, which melts as the reaction is heated during the denaturation step, releasing the reagent only at higher temperatures. The DNA polymerase can also be kept in an active state by binding to an aptamer or an antibody. This binding is disrupted at higher temperatures, releasing the functional DNA polymerase that can proceed with the PCR unhindered.

In some examples, the amplified target sequences can be ligated to one or more adapters. In some examples, adapters can include one or more nucleic acid barcodes or tagging sequences. In some examples, amplified target sequences once ligated to an adapter can undergo a nick translation reaction and/or further amplification to generate a library of adapter-ligated amplified target sequences. In one example, the amplification method involves performing multiplex PCR on a nucleic acid sample using a plurality of primers having a cleavable group. In some examples, a multiplex PCR amplification reaction is conducted using a plurality of primers provided herein that have a cleavable group, and includes a DNA polymerase, an adapter, dATP, dCTP, dGTP and dTTP. In some examples, the cleavable group can be a uracil nucleotide. In some examples, forward and reverse primer pairs contain a uracil nucleotide as the one or more cleavable groups. In one example, a primer pair can include a uracil nucleotide in each of the forward and reverse primers of each primer pair. In one example, a forward or reverse primer contains one, two, three or more uracil nucleotides. In some examples, methods involve amplifying at least 10, 50, 100, 150, 200, 250, 300, 350, 398 or more, target sequences from a population of nucleic acids having a plurality of target sequences using target-specific forward and reverse primer pairs containing at least two uracil nucleotides. The reaction can also include one or more antibodies and/or nucleic acid barcodes. In some examples, the methods include processes for reducing the formation of amplification artifacts in a multiplex PCR. In some examples, primer-dimers or non-specific amplification products are obtained in lower number or yield as compared to standard multiplex PCR of the prior art. In some examples, the reduction in amplification artifacts is in part, governed by the use of specific primer pairs in the multiplex PCR reaction. In one example, the number of specific primer pairs in the multiplex PCR reaction can be greater than 50, 100, 150, 200, 250, 300 or more. In some examples, multiplex PCR is performed using primers that contain a cleavable group. In one example, primers containing a cleavable group can include one or more cleavable moieties per primer of each primer pair. In some examples, a primer containing a cleavable group includes a nucleotide neither normally present in a sample nor native to the population of nucleic acids undergoing multiplex PCR. For example, a primer can include one or more non-native nucleic acid molecules such as, but not limited to thymine dimers, 8-oxo-2'-deoxyguanosine, inosine, deoxyuridine, bromodeoxyuridine, apurinic nucleotides, and the like.

In some examples, the describedmethods can optionally include destroying one or more primer-containing amplification artifacts, e.g., primer-dimers, dimer-dimers or superamplicons. In some examples, the destroying can optionally include treating the primer and/or amplification product so as to cleave specific cleavable groups present in the primer and/or amplification product. In some examples, the treating can include partial or complete digestion of one or more target-specific primers. In one example, the treating can include removing at least 40% of the target specific primer from the amplification product. The cleavable treatment can include enzymatic, acid, alkali, thermal, photo or chemical activity. The cleavable treatment can result in the cleavage or other destruction of the linkages between one or more nucleotides of the primer, or between one or more nucleotides of the amplification product. The primer and/or the amplification product can optionally include one or more modified nucleotides or nucleobases. In some examples, the cleavage can selectively occur at these sites, or adjacent to the modified nucleotides or nucleobases. In some examples, the primer includes a sufficient number of modified nucleotides to allow functionally complete degradation of the primer by the cleavage treatment, but not so many as to interfere with the primer's specificity or functionality prior to such cleavage treatment, for example in the amplification reaction. In some examples, the primer includes at least one modified nucleotide, but no greater than 75% of nucleotides of the primer are modified. In some examples, the cleavage or treatment of the amplified target sequence can result in the formation of a phosphorylated amplified target sequence. In some examples, the amplified target sequence is phosphorylated at the 5' terminus.

In some examples, primers can be designed *de novo* using algorithms that generate oligonucleotide sequences according to specified design criteria. For example, the primers may be selected according to any one or more of criteria specified herein. In some examples, one or more of the primers are selected or designed to satisfy any one or more of the following criteria: (1) inclusion of two or more modified nucleotides within the primer sequence, at least one of which is included near or at the termini of the primer and at least one of which is included at, or about the center nucleotide position of the primer sequence; (2) primer length of about 15 to about 40 bases in length; (3) Tₘ of from about 60°C to about 70°C; (4) low cross-reactivity with non-target sequences present in the target genome or sample of interest; (5) for each primer in a given reaction, the sequence of at least the first four nucleotides (going from 3' to 5' direction) are not complementary to any sequence within any other primer present in the same reaction; and (6) no amplicon includes any consecutive stretch of at least 5 nucleotides that is complementary to any sequence within any other amplicon. In some examples, the primers include one or more primer pairs designed to amplify target sequences from the sample that are about 100 base pairs to about 500 base pairs in length. In some examples, the primers include a plurality of primer pairs designed to amplify target sequences, where the amplified target sequences are predicted to vary in length from each other by no more than 50%, typically no more than 25%, even more typically by no more than 10%, or 5%. For example, if one primer pair is selected or predicted to amplify a product that is 100 nucleotides in length, then other primer pairs are selected or predicted to amplify products that are between 50-150 nucleotides in length, typically between 75-125 nucleotides in length, even more typically between 90-110 nucleotides, or 95-105 nucleotides, or 99-101 nucleotides in length. In some examples, at least one primer pair in the amplification reaction is not designed *de novo* according to any predetermined selection criteria. For example, at least one primer pair can be an oligonucleotide sequence selected or generated at random, or previously selected or generated for other applications. In one exemplary example, the amplification reaction can include at least one primer pair selected from the TaqMan^{®} probe reagents (Roche Molecular Systems). The TagMan^{®} reagents include labeled probes and can be useful, *inter alia,* for measuring the amount of target sequence present in the sample, optionally in real time. Some examples of TaqMan technology are disclosed in U.S. Pat. Nos. 5,210,015, 5,487,972, 5,804,375, 6,214,979, 7,141,377 and 7,445,900. In some examples, at least one primer within the amplification reaction can be labeled, for example with an optically detectable label, to facilitate a particular application of interest. For example, labeling may facilitate quantification of target template and/or amplification product, isolation of the target template and/or amplification, product, and the like. In some examples, the primers do not contain a carbon-spacer or terminal linker. In some examples, the primers or amplified target sequences do not contain an enzymatic, magnetic, optical or fluorescent label.

In some examples, primers are synthesized that are complementary to, and can hybridize with, discrete segments of a nucleic acid template strand, including: a primer that can hybridize to the 5' region of the template, which encompasses a sequence that is complementary to either the forward or reverse amplification primer. In some examples, the forward primers, reverse primers, or both, share no common nucleic acid sequence, such that they hybridize to distinct nucleic acid sequences. For example, target-specific forward and reverse primers can be prepared that do not compete with other primer pairs within the primer pool to amplify the same nucleic acid sequence. In this example, primer pairs that do not compete with other primer pairs in the primer pool assist in the reduction of non-specific or spurious amplification products. In some examples, the forward and reverse primers of each primer pair are unique, in that the nucleotide sequence for each primer is non-complementary and non-identical to the other primer in the primer pair. In some examples, the primer pair can differ by at least 10 %, at least 20%, at least 30%, at least 40%, at least 50%, at least 60%, at least 70%, at least 75%, at least 80%, at least 85%, or at least 90% nucleotide identity. In some examples, the forward and reverse primers in each primer pair are non-complementary or non-identical to other primer pairs in the primer pool or multiplex reaction. For example, the primer pairs within a primer pool or multiplex reaction can differ by at least 5%, at least 10%, at least 20%, at least 30%, at least 40%, at least 50%, at least 60%, or at least 70% nucleotide identity to other primer pairs within the primer pool or multiplex reaction. Primers are designed to minimize the formation of primer-dimers, dimer-dimers or other non-specific amplification products. Typically, primers are optimized to reduce GC bias and low melting temperatures (Tₘ) during the amplification reaction. In some examples, the primers are designed to possess a Tₘ of about 55°C to about 72°C. In some examples, the primers of a primer pool can possess a Tₘ of about 59°C to about 70°C, 60°C to about 68°C, or 60°C to about 65°C. In some examples, the primer pool can possess a Tₘ that does not deviate by more than 5°C.

In some examples, the primer pairs used to produce an amplicon library can result in the amplification of target-specific nucleic acid molecules possessing one or more of the following metrics: greater than 97% target coverage at 20x if normalized to 100x average coverage depth; greater than 97% of bases with greater than 0.2x mean; greater than 90% base without strand bias; greater than 95% of all reads on target; greater than 99% of bases with greater than 0.01x mean; and greater than 99.5% per base accuracy.

In some examples, the primers can be provided as a set of primer pairs in a single amplification vessel. In some examples, the primers can be provided in one or more aliquots of primer pairs that can be pooled prior to performing the multiplex PCR reaction in a single amplification vessel or reaction chamber. In one example, the primers can be provided as a pool of forward primers and a separate pool of reverse primers. In another example, primer pairs can be pooled into subsets such as non-overlapping primer pairs. In some examples, the pool of primer pairs can be provided in a single reaction chamber or microwell, for example on a PCR plate to perform multiplex PCR using a thermocycler. In some examples, the forward and reverse primer pairs can be substantially complementary to the target sequences. In some examples, the primer pairs do not contain a common extension (tail) at the 3' or 5' end of the primer. In another example, the primers do not contain a Tag or universal sequence. In some examples, the primer pairs are designed to eliminate or reduce interactions that promote the formation of non-specific amplification.

### Methods for Detecting and/or Measuring the Presence or Absence of Microorganisms in a Sample

Also described herein are nucleic acid-based methods of detecting and/or measuring the presence or absence of a microorganism in a sample. In some examples of the detection and/or measurement methods, nucleic acids in or from a sample are subjected to nucleic acid hybridization and/or amplification, for example, using any of the nucleic acids provided herein as probes and/or amplification primers. In some examples, the presence or absence of one or more hybridization and/or nucleic acid amplification products is detected, thereby detecting the presence or absence of a microorganism.

In some examples, a method provided herein for detecting, determining the presence or absence of, and/or measuring one or more microorganisms in a sample includes (a) subjecting nucleic acids in or from the sample to nucleic acid amplification using one or more primer pairs that specifically amplifies a target nucleic acid sequence contained within a genome of a microorganism selected from the microorganisms listed in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis,* and (b) detecting one or more amplification products (or the presence or absence thereof), thereby detecting and/or measuring one or more microorganisms selected from the microorganisms listed in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis,* or determining the presence or absence of one or more microorganisms selected from the microorganisms listed in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the target nucleic acid is unique to the microorganism. In some examples, the target nucleic acid is not contained within a prokaryotic 16S rRNA gene. Any of the examples provided herein for subjecting nucleic acids to amplification using one or more primer pairs that specifically amplifies a target nucleic acid sequence contained within a genome of a microorganism listed in Table 1 can be used in any example of this method for detecting the presence or absence of a microorganism in a sample. In some examples, the at least one primer pair does not detectably amplify a nucleic acid sequence contained within any genus other than the genus of the microorganism. In some examples, the at least one primer pair does not detectably amplify a nucleic acid sequence contained within any species other than the species of the microorganism. In some examples, the nucleic acids in or from the sample include nucleic acids from a plurality of different microorganisms listed in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis,* and/or a plurality of different microorganisms, e.g., bacteria, not listed in Table 1. In some examples, the sample is a biological sample, such as, for example, a sample of contents of the alimentary canal of an animal. In some examples, the sample is a fecal sample. In some examples, the target nucleic acid sequence comprises or consists essentially of a nucleotide sequence selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence. In some examples, detecting the presence or absence of one or more amplification products comprises detecting the presence or absence of one or more nucleotide sequences selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, or the complement thereof. In some examples, the at least one primer pair does not detectably amplify a nucleic acid sequence contained within any genus other than the genus of the microorganism containing the target nucleic acid sequence. In some examples, the at least one primer pair does not detectably amplify a nucleic acid sequence contained within any species other than the species of the microorganism containing the target nucleic acid sequence. In some examples, at least one primer of the primer pair, or at least one primer pair, contains, or consists essentially of, a sequence, or sequences of a primer or primer pair, in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the nucleic acids are subjected to nucleic acid amplification using a plurality of primers or primer pairs, each containing, or consisting essentially of, a sequence or sequences selected from the sequences of primers in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some of the examples in which the nucleic acids are subjected to nucleic acid amplification using more than one, or a plurality of primers or primer pairs, the amplification is a multiplex amplification conducted in a single reaction mixture. In some examples, at least one primer or one primer pair includes a modification that facilitates nucleic acid manipulation, amplification, ligation and/or sequencing of amplification products and/or reduction or elimination of primer dimers. In particular examples, a modification is one that facilitates multiplex nucleic acid amplification, ligation and/or sequencing of products of multiplex amplification.

### Methods for Detecting and/or Measuring a Microorganism Group

**In** some examples of the methods for detecting, determining the presence or absence of, and/or measuring one or more microorganisms in a sample, the method is designed to focus on detection and/or measuring a certain group of microorganisms. In such examples, the one or more primer pairs used in the method is a combination of primers and/or primer pairs that include a selected group or sub-group of microorganism-specific nucleic acids enable a directed survey of the sample for identification of species of microorganisms that may be significant, for example, in certain states of health and disease or microbiota imbalance, e.g., dysbiosis. In some examples, combinations of nucleic acids include microorganism-specific nucleic acids, and/or primer pairs, that specifically amplify a nucleic acid sequence contained in the genome of one or more microorganisms (e.g., bacteria) implicated in one or more conditions, disorders and/or diseases (referred to herein as a "condition-attendant group" of microorganisms. In particular examples, the combination of nucleic acid primers and/or primer pairs includes a nucleic acid and/or a primer pair that specifically amplifies a target nucleic acid sequence contained within a genome of a microorganism selected from the microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the combination includes a plurality of nucleic acids and/or primer pairs that include at least one nucleic acid primer pair that specifically amplifies a target nucleic acid in each of the microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the plurality of primer pairs includes primer pairs that specifically amplify genomic target nucleic acids contained within at least 5, 10, 15, 20, 25, 30, 35, 40, 45, 50, 55, 60 or 70 of the microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In particular examples, the target nucleic acid sequences contained in the genome of the different microorganisms are unique to each of the microorganisms. In some examples, a combination of nucleic acids and/or nucleic acid primer pairs includes two or more nucleic acids and/or nucleic acid primer pairs that specifically amplify a unique nucleic acid sequence contained in the genome of one or more of the Group A microorganisms (see Table 2A), which are species implicated as having a role in multiple conditions, diseases and/or disorders, including, for example oncological conditions including, for example, response to immune-oncology treatment and cancer, gastrointestinal disorders, including, for example, irritable bowel syndrome, inflammatory bowel disease and coeliac disease, and autoimmune diseases, including, for example, lupus and rheumatoid arthritis. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group A. In some examples, the combination of nucleic acids and/or nucleic acid primer pairs includes nucleic acids and/or nucleic acid primer pairs that specifically bind to, hybridize to and/or amplify sequences as set forth in Table 2A for exemplary nucleic acids, primers and primer pairs for genomes of Group A microorganisms. In some examples, the combination of nucleic acids and/or nucleic acid primer pairs includes nucleic acids and/or nucleic acid primer pairs having a nucleotide sequence or sequences as set forth in Table 2A for exemplary nucleic acids, primers and primer pairs for genomes of Group A microorganisms.

In some examples, a combination of nucleic acids and/or nucleic acid primer pairs for use in a method of detecting and/or measuring a certain group of microorganisms includes two or more nucleic acids and/or nucleic acid primer pairs that specifically amplify a unique nucleic acid sequence contained in the genome of one or more of the Group B microorganisms (see Table 2B), which are species implicated as having a role in response to immuno-oncology treatment. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group B. In some examples, the combination of nucleic acids and/or nucleic acid primer pairs includes nucleic acids and/or nucleic acid primer pairs that specifically bind to, hybridize to and/or amplify sequences as set forth in Table 2B for exemplary nucleic acids, primers and primer pairs for genomes of Group B microorganisms. In some examples, the combination of nucleic acids and/or nucleic acid primer pairs includes nucleic acids and/or nucleic acid primer pairs having a nucleotide sequence or sequences as set forth in Table 2B for exemplary nucleic acids, primers and primer pairs for genomes of Group B microorganisms.

In some examples, a combination of nucleic acids and/or nucleic acid primer pairs for use in a method of detecting and/or measuring a certain group of microorganisms includes two or more nucleic acids and/or nucleic acid primer pairs that specifically amplify a unique nucleic acid sequence contained in the genome of one or more of the Group C microorganisms (see Table 2C), or the Group C microorganisms excluding *Helicobacter salomonis* (Subgroup 1 of the Group C microorganisms) which are species implicated as having a role in cancer. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group C, or the Group C microorganisms excluding *Helicobacter salomonis* (Subgroup 1 of the Group C microorganisms). In some examples, the combination of nucleic acids and/or nucleic acid primer pairs includes nucleic acids and/or nucleic acid primer pairs that specifically bind to, hybridize to and/or amplify sequences as set forth in Table 2C for exemplary nucleic acids, primers and primer pairs for genomes of Group C microorganisms. In some examples, the combination of nucleic acids and/or nucleic acid primer pairs includes nucleic acids and/or nucleic acid primer pairs that specifically bind to, hybridize to and/or amplify sequences selected from SEQ ID NOS: 1616, 1619, 1620, 1625-1628, 1635-1640, 1699, 1700, 1705-1708, 1752, 1753, 1784-1786, 1817-1820, 1827, 1828, 1840, 1841, 1844, 1845, 1852-1859, 1899, 1900, 1904, 1905, 1932, 1933, 1956-1958, 1975, 1976 of Table 17, and/or a substantially identical or similar sequence, or sequences selected from SEQ ID NOS: 1616, 1619, 1620, 1625-1628, 1635-1640, 1699, 1700, 1705-1708, 1752, 1753, 1784-1786, 1827, 1828, 1840, 1841, 1844, 1845, 1852-1859, 1899, 1900, 1904, 1905, 1932, 1933, 1956, 1957, 1958 of Table 17, and/or a substantially identical or similar sequence. In some examples, the combination of nucleic acids and/or nucleic acid primer pairs includes nucleic acids and/or nucleic acid primer pairs having a nucleotide sequence or sequences as set forth in Table 2C for exemplary nucleic acids, primers and primer pairs for genomes of Group C microorganisms. In some examples, the combination of nucleic acids and/or nucleic acid primer pairs includes nucleic acids and/or nucleic acid primer pairs having a nucleotide sequence or sequences selected from SEQ ID NOS: 71, 72, 77-80, 89-96, 109-120, 237-242, 249-256, 343-346, 407-412, 493-496, 511-520, 521-524, 547-550, 555-558, 561-568, 571-586, 665-668, 675-678, 731-734, 779-784, and/or SEQ ID NOS: 849, 850, 855-858, 867-874, 887-898, 1012-1020, 1025-1034, 1121-1124, 1185-1190, 1271-1276, 1289-1298, 1299-1302, 1325-1328, 1333-1336, 1339-1346, 1349-1364, 1443-1446, 1453-1456, 1509-1512, 1557-1562, in Table 16, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes nucleic acids and/or nucleic acid primer pairs having a nucleotide sequence or sequences selected from SEQ ID NOS: 71, 72, 77-80, 89-96, 109-120, 237-242, 249-256, 343-346, 407-412, 493-496, 511-520 and/or SEQ ID NOS: 849, 850, 855-858, 867-874, 887-898, 1012-1020, 1025-1034, 1121-1124, 1185-1190, 1271-1276, 1289-1298 in Table 16, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes nucleic acids and/or nucleic acid primer pairs having a nucleotide sequence or sequences selected from SEQ ID NOS: 71, 72, 77-80, 89-96, 109-120, 237-242, 249-256, 343-346, 407-412 and/or SEQ ID NOS: 849, 850, 855-858, 867-874, 887-898, 1012-1020, 1025-1034, 1121-1124, 1185-1190 in Table 16, or substantially identical or similar sequences, or any of the aforementioned nucleotide sequences of nucleic acids or primer pairs in which one or more thymine bases is substituted with a uracil base.

**In** some examples, a combination of nucleic acids and/or nucleic acid primer pairs for use in a method of detecting and/or measuring a certain group of microorganisms includes two or more nucleic acids and/or nucleic acid primer pairs that specifically amplify a unique nucleic acid sequence contained in the genome of one or more of the Group D microorganisms (see Table 2D), which are species implicated as having a role in gastrointestinal disorders, including, for example, irritable bowel syndrome, inflammatory bowel disease and coeliac disease. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group D. In some examples, the combination of nucleic acids and/or nucleic acid primer pairs includes nucleic acids and/or nucleic acid primer pairs that specifically bind to, hybridize to and/or amplify sequences as set forth in Table 2D for exemplary nucleic acids, primers and primer pairs for genomes of Group D microorganisms. In some examples, the combination of nucleic acids and/or nucleic acid primer pairs includes nucleic acids and/or nucleic acid primer pairs having a nucleotide sequence or sequences as set forth in Table 2D for exemplary nucleic acids, primers and primer pairs for genomes of Group D microorganisms.

In some examples, a combination of nucleic acids and/or nucleic acid primer pairs for use in a method of detecting and/or measuring a certain group of microorganisms includes two or more nucleic acids and/or nucleic acid primer pairs that specifically amplify a unique nucleic acid sequence contained in the genome of one or more of the Group E microorganisms (see Table 2E), which are species implicated as having a role in autoimmune disorders, including, for example, lupus and rheumatoid arthritis. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group E. In some examples, the combination of nucleic acids and/or nucleic acid primer pairs includes nucleic acids and/or nucleic acid primer pairs that specifically bind to, hybridize to and/or amplify sequences as set forth in Table 2E for exemplary nucleic acids, primers and primer pairs for genomes of Group E microorganisms. In some examples, the combination of nucleic acids and/or nucleic acid primer pairs includes nucleic acids and/or nucleic acid primer pairs having a nucleotide sequence or sequences as set forth in Table 2E for exemplary nucleic acids, primers and primer pairs for genomes of Group E microorganisms.

In some examples, detecting, determining the presence or absence of, and/or measuring as provided herein, the one or more nucleic acid amplification products is detected via a labeled probe having a nucleic acid sequence of compositions provided herein that specifically identifies a particular nucleic acid product. For example, such methods can employ a nucleic acid microarray of oligonucleotides attached to a substrate, e.g., a chip, to capture amplification products which is then contacted with labeled (e.g., fluorescently labeled) specific probes under conditions suitable for hybridization which can be detected upon binding to a complementary product thereby detecting the presence of a microorganism in the sample. Methods for detection of labels are known in the art and include, for example, optical methods such as scanning using confocal laser microscopy or a CCD camera. Such methods also allow for quantitation of hybridization to assess abundance of the labeled nucleic acid product. In some examples, the presence or absence of one or more nucleic acid amplification products is detected by obtaining nucleotide sequence information of one or more nucleic acid amplification products. Methods for sequencing nucleic acids are described herein and/or known in the art. The sequence of an amplification product can also be used to identify the microorganism at various levels of specificity, e.g., kingdom, phylum, class, order, family, genus and/or species. If a microorganism of Table 1 or 2 for which presence or absence is being determined is present in the sample, the sequence of at least one amplification product will be the target sequence specifically amplified by the one or more primer pairs from the genome of the microorganism and, thus, the presence of the amplification product is detected. If the microorganism is absent from the sample, no amplification product will be produced that contains the target sequence specifically amplified by the one or more primer pairs, and, thus, the absence of the amplification product is detected. In some examples, detecting the presence or absence of a nucleic acid amplification product includes comparing the sequence of the one or more nucleic acid amplification products to nucleic acid sequences of the genome of one or more of the microorganisms in Table 1 or 2. Genome sequences for microorganisms in Table 1 or 2 are available in public databases (e.g., NCBI public database; www.ncbi.nlm.nih.gov/genome/microbes/). In some examples, comparing the sequence of a nucleic acid amplification product to reference genome sequences includes conducting computer-assisted alignment of the sequence and mapping it to a reference genome. Exemplary nucleotide sequence analysis workflows for mapping sequence reads of amplification products are provided herein. In particular examples, detecting the presence or absence of a nucleic acid amplification product includes detecting the presence or absence of an amplification product containing a sequence in SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence.

### Methods for Increasing the Accuracy, Specificity and/or Sensitivity of Detecting and/or Measuring Microorganisms in a Sample

In some examples, a method described herein for detecting, determining the presence or absence of, and/or measuring one or more microorganisms in a sample includes (a) subjecting nucleic acids in or from the sample to nucleic acid amplification using one or more, or a combination of, primer pairs capable of separately amplifying nucleic acids separately containing sequences of one or more different hypervariable regions of a prokaryotic 16S rRNA gene and (b) detecting one or more amplification products, thereby detecting, or determining the presence or absence of, one or more microorganisms in the sample. In some examples, the microorganism(s) is/are bacteria. In some examples, the presence or absence of one or microorganisms is detected at the level of the genus of the microorganisms. In some examples, the presence or absence of one or more microorganisms is detected at the level of the species of the microorganism. In some examples, the prokaryotic 16S rRNA gene is a bacterial gene. In some examples, the one or more, or combination of, primer pairs separately amplify nucleic acids containing sequences of different variable regions. In some examples, the primers of the primer pairs are directed to, or bind to, or hybridize to nucleic acid sequences contained in conserved regions of a prokaryotic 16S rRNA gene. In some examples, the one or more, or combination of primer pairs amplify, or separately amplify, nucleic acids separately containing sequences of 2 or more, 3 or more, 4 or more, 5 or more, 6 or more, 7 or more, 8 or more, or 9 different hypervariable regions of a prokaryotic 16S rRNA gene thereby producing amplified copies of the nucleic acids separately containing sequences of the 2 or more, 3 or more, 4 or more, 5 or more, 6 or more, 7 or more, 8 or more or 9 different hypervariable regions of the 16S rRNA gene of one or more microorganisms, wherein, in some examples, the amplified copies of different hypervariable regions are separate amplicons. In some examples, the one or more, or combination of, primer pairs separately amplify nucleic acids separately containing sequences of 8 different hypervariable regions of a prokaryotic 16S rRNA gene. In some examples, the 8 different hypervariable regions are V2-V9. In some examples, the one or more, or combination of, primer pairs separately amplify nucleic acids separately containing sequences of 3 or more different hypervariable regions of a prokaryotic 16S rRNA gene wherein one of the 3 or more different regions is a V5 region thereby producing amplified copies of the nucleic acids separately containing sequences of 3 or more different hypervariable regions of the 16S rRNA gene of one or more microorganisms. In some examples, the primer pairs includes degenerate sequences of one or more primers in one or more primer pairs. In some examples, the one or more, or combination, of primer pairs is selected from any of the primer pairs described herein that separately amplify nucleic acids containing sequences of one or more hypervariable regions of a prokaryotic 16S rRNA gene. For example, in some examples, the one or more, or a combination of, primer pairs that are capable of separately amplifying nucleic acids containing sequences of one or more hypervariable regions of a prokaryotic 16S rRNA gene comprises one or more primer pairs containing sequences selected from Table 15, or SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base. In some examples, the one or more, or a combination of, primer pairs that separately amplify nucleic acids containing sequences of one or more hypervariable regions of a prokaryotic 16S rRNA gene comprises one or more primer pairs containing sequences selected from SEQ ID NOS: 25-48 in Table 15 and/or SEQ ID NOS: 35-40, 47 and 48 in Table 15, or substantially identical or similar sequences in which one or more thymine bases is substituted with a uracil base. In some examples, the presence or absence of one or more nucleic acid amplification products is detected by obtaining nucleotide sequence information of one or more nucleic acid amplification products. If only one sequence is obtained for each of one or more hypervariable regions amplified, the sequence information is indicative of the presence of only one microorganism in the sample. If 2 or more different sequences are obtained for each of one or more hypervariable regions amplified, the sequence information is indicative of the presence of 2 or more different microorganisms in the sample. Additionally, the combined results of the amplifications using 16S rRNA gene primers that separately amplify multiple different hypervariable regions yield increased accuracy in the detection and/or measurement of one or more microorganisms in a sample by reducing false negatives or false positivies that may occur in basing a determination solely on the results of amplification using 16S rRNA primers that amplify one or only a few (e.g., less than 8 or less than 5) hypervariable regions or that amplify combined regions as a single amplicon, e.g., V2-V3, or V3-V4, etc., if the amplification primers are not directed to highly conserved sequences flanking the multiple regions. For example, due to possible sequence variations in 16S rRNA gene conserved regions in some species or strains of microorganisms (e.g., bacteria), primers designed to amplify a particular hypervariable region of all bacteria may fail to amplify some bacterial nucleic acids present in a sample, thereby yielding a false negative result. This failure may be compounded if the primers are designed to amplify combined regions as a single amplicon and are not directed to highly conserved sequences flanking the multiple regions, because not only is one region potentially not amplified, two or more regions may not amplified. However, amplification of the same nucleic acids using 16S rRNA gene primers designed to separately amplify multiple hypervariable regions in such a microorganism would be likely to amplify at least one hypervariable sequence in the microorganism and enable detection of the presence of the microorganism in the sample. Separately amplifying multiple hypervariable regions increases the coverage of the sequence and provide more useful information and increases accuracy and resolution of detection. Also, when 16S rRNA gene primers that separately amplify multiple (e.g., 3, 4, 5, 6, 7, 8 or 9) hypervariable regions are used, the number (and sequences) of amplification products using the different hypervariable region primers provides a basis on which to filter out and eliminate sequences of hypervariable regions of a particular microorganism that are amplified in only one (or less than 3, or less than 4, or less than 5, or less than 6, or another threshold amount) 16S rRNA gene hypervariable region amplification(s) from consideration as unreliable since 16S rRNA gene nucleic acids from a bacterial microorganism that is truly present in a sample should be amplified in the majority of amplifications using primers that separately amplify multiple hypervariable regions. The sequence of an amplification product can also be used to identify the microorganism at various levels of specificity, e.g., kingdom, phylum, class, order, family, genus and/or species. In some examples, detecting, determining the presence or absence of, and/or measuring a nucleic acid amplification product includes comparing the sequence of the one or more nucleic acid amplification products to nucleic acid sequences of prokaryotic, e.g., bacterial, 16S rRNA genes of one or more of the microorganisms. Sequences of prokaryotic 16S rRNA genes are available in public databases (see, e.g., the GreenGenes bacterial 16S rRNA gene sequence; e.g., www.greengenes.lbl.gov). In some examples, comparing the sequence of a nucleic acid amplification product to reference genome sequences includes conducting computer-assisted alignment of the sequence and mapping it to a reference genome. Exemplary nucleotide sequence analysis workflows for mapping sequence reads of amplification products are provided herein. In some examples, relative and/or absolute levels of one or more microorganisms are determined or measured. For example, in some examples, the level of abundance of one or more nucleic acid amplification products and/or sequence reads can be measured to provide relative and/or absolute levels of one or microorganisms. Techniques for quantifying nucleic acids (e.g., amplification products) and/or sequence reads are known in the art and/or provided herein.

In some examples, a method provided herein for detecting, determining the presence or absence, and/or measuring one or more microorganisms in a sample includes (a) subjecting nucleic acids in or from the sample to nucleic acid amplification using a combination of primer pairs comprising (i) one or more primer pairs capable of amplifying nucleic acids containing sequences of one or more hypervariable regions of a prokaryotic 16S rRNA gene (referred to as the "16S rRNA gene primers and primer pairs"), and (ii) one or more primer pairs capable of amplifying a target nucleic acid sequence contained within the genome of a microorganism that is not contained within a hypervariable region of a prokaryotic 16S rRNA gene, wherein different primer pairs amplify different target nucleic acid sequences contained within the genome of different microorganisms (referred to as the "non-16S rRNA gene primers and primer pairs"); and (b) detecting one or more amplification products, thereby detecting or determining the presence or absence of a microorganism. In some examples, the microorganism(s) is/are bacteria. In some examples, the prokaryotic 16S rRNA gene is a bacterial gene and/or the prokaryotic microorganism is a bacterium. In some examples, the one or more primer pairs that amplifies a nucleic acid containing a sequence of a hypervariable region of a prokaryotic 16S rRNA gene separately amplify nucleic acid sequences of different hypervariable regions. In some examples, the primers of the one or more primer pairs of (i) are directed to, or bind to, or hybridize to nucleic acid sequences contained in conserved regions of a prokaryotic 16S rRNA gene. In some examples, the amplification is a multiplex amplification conducted in a single reaction mixture. In some examples a method provided herein for detecting one or more microorganisms in a sample includes (a) subjecting nucleic acids in or from a sample to two or more separate nucleic acid amplification reactions using a first set of primer pairs for one nucleic acid amplification reaction and a second set of primer pairs for the other nucleic acid amplification reaction, wherein: (i) the first set of primer pairs comprises one or more primer pairs capable of amplifying a nucleic acid containing a sequence of a hypervariable region of a prokaryotic 16S rRNA gene (referred to as the "16S rRNA gene primers and primer pairs"), and (ii) the second set of primer pairs comprises one or more primer pairs capable of amplifying, or specifically amplifying, a target nucleic acid sequence contained within the genome of a microorganism that is not contained within a hypervariable region of a prokaryotic 16S rRNA gene, wherein different primer pairs amplify different target nucleic acid sequences contained within the genome of different microorganisms (referred to as the "non-16S rRNA gene primers and primer pairs"); and (b) detecting one or more amplification products, thereby detecting or determining the presence or absence of a microorganism. In some examples, the microorganism(s) is/are bacteria. In some examples, the prokaryotic 16S rRNA gene is a bacterial gene and/or the prokaryotic microorganism is a bacterium. In some examples, the one or more primer pairs that amplifies a nucleic acid containing a sequence of a hypervariable region of a prokaryotic 16S rRNA gene separately amplify nucleic acids containing sequences of different hypervariable regions. In some examples, the primers of the one or more primer pairs of (i) are directed to, or bind to, or hybridize to nucleic acid sequences contained in conserved regions of a prokaryotic 16S rRNA gene. In some examples, the amplification is a multiplex amplification conducted in a single reaction mixture.

In any examples of methods for detecting and/or measuring the presence or absence of one or more microorganisms in a sample wherein the method includes subjecting nucleic acids in or from the sample to nucleic acid amplification using a combination of, or first and second sets of, 16S rRNA primer pairs and non-16S rRNA gene primer pairs, respectively, the nucleic acid amplification can be performed according to any of the examples provided herein for such amplification. For example, in some examples the target nucleic acid sequence contained within a genome of a prokaryotic microorganism, e.g., bacteria, is unique to the microorganism. In some examples, the one or more 16S rRNA gene primer pairs amplify a nucleic acid sequence in a plurality of microorganisms, e.g., bacteria, from different genera. In some examples, sample includes nucleic acids from a plurality of different microorganisms. In some examples, the sample is a biological sample, such as, for example, a sample of contents of the alimentary tract of an animal. In some examples, the sample is a fecal sample. In some examples, each primer of the one or more 16S rRNA gene primer pairs contains less than 10, less than 9, less than 8, less than 7, less than 6, less than 5, less than 4, less than 3, or less than 2 contiguous nucleotides of sequence identical to a sequence of contiguous nucleotides of another primer in the combination of primer pairs. In some examples, the nucleic acid sequences being amplified by the one or more 16S rRNA gene primer pairs are less than about 300 bp, less than about 250 bp, less than about 200 bp, less than about 175 bp, less than about 150 bp, or less than about 125 bp in length. In some examples, the 16S rRNA gene primer pairs separately amplify nucleic acids separately containing sequences of 3 or more, 4 or more, 5 or more, 6 or more, 7 or more, 8 or more or 9 different hypervariable regions of a prokaryotic 16S rRNA gene thereby producing amplified copies of the nucleic acids containing sequences of the 3 or more, 4 or more, 5 or more, 6 or more, 7 or more, 8 or more or 9 different hypervariable regions of the 16S rRNA gene of one or more microorganisms, wherein the amplified copies of different hypervariable regions are separate amplicons. In some examples, the 16S rRNA gene primer pairs separately amplify nucleic acids separately containing sequences of 8 different hypervariable regions of a prokaryotic 16S rRNA gene. In some examples, the 8 different hypervariable regions are V2-V9. In some examples, the 16S rRNA gene primer pairs separately amplify nucleic acids separately containing sequences of 3 or more hypervariable regions of a prokaryotic 16S rRNA gene wherein one of the 3 or more regions is a V5 region thereby producing amplified copies of the nucleic acids containing sequences of the 3 or more hypervariable regions of the 16S rRNA gene of one or more microorganisms. In some examples, the combination of primer pairs includes degenerate sequences of one or more primers in one or more primer pairs. In some examples, the 16S rRNA gene primer pair(s) comprise primers and/or primer pairs containing, or consisting essentially of, a sequence or sequences of a primer or primer pair in Table 15, or SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base. In some examples, the one or more non-16S rRNA gene primer pairs specifically amplifies a target nucleic acid sequence contained within a genome of a microorganism selected from the microorganisms of Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the target nucleic acid is unique to the microorganism. In some examples, the sample includes nucleic acids from a plurality of different microorganisms listed in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some such examples, amplified copies of a plurality of different microorganisms in Table 1 are produced. In some examples, the sample includes a mixture of nucleic acids of one or more, or a plurality of, microorganisms selected from among the microorganisms listed in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis,* and one or more microorganisms, e.g., bacteria, not listed in Table 1. In some examples, at least one, or one or more, target nucleic acid sequence(s) comprises or consists essentially of a nucleotide sequence selected from the nucleotide sequences of SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence. In some examples, at least one, or one or more, product(s) of the nucleic acid amplification comprises, or consists essentially of, a nucleotide sequence selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, or the complement thereof, and optionally having one or more primer sequences at the 5' and/or 3' end(s) of the sequence, such as any of the primer sequences provided herein. In some examples, at least one, or one or more, product(s) of the nucleic acid amplification is less than about 500, less than about 475, less than about 450, less than about 400, less than about 375, less than about 350, less than about 300, less than about 275, less than about 250, less than about 200, less than about 175, less than about 150, or less than about 100 nucleotides in length. In some examples, the at least one non-16S rRNA gene primer pair does not detectably amplify a nucleic acid sequence contained within any genus other than the genus of the microorganism containing the target nucleic acid sequence. In some examples, the at least one non-16S rRNA gene primer pair does not detectably amplify a nucleic acid sequence contained within any species other than the species of the microorganism containing the target nucleic acid sequence. In some examples, at least one primer of the non-16S rRNA gene primer pair, or at least one non-16S rRNA gene primer pair, contains, or consists essentially of, the sequence or sequences of a primer or primer pair in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the nucleic acids are subjected to nucleic acid amplification using a plurality of non-16S rRNA gene primers or primer pairs, each containing, or consisting essentially of, a sequence or sequences of a primer pair in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, at least one primer or one primer pair in the combination of primer pairs includes a modification that facilitates nucleic acid manipulation, amplification, ligation and/or sequencing of amplification products and/or reduction or elimination of primer dimers. In particular examples, a modification is one that facilitates multiplex nucleic acid amplification, ligation and/or sequencing of products of multiplex amplification.

In some examples of methods for detecting, determining the presence or absence of, and/or measuring one or more microorganisms in a sample wherein the method includes subjecting nucleic acids in or from the sample to nucleic acid amplification using a combination of, or first and second sets of, 16S rRNA primer pairs and non-16S rRNA gene primer pairs, the one or more nucleic acid amplification products is/are detected by obtaining nucleotide sequence information of one or more nucleic acid amplification products. The detecting, determining the presence or absence of and/or measuring of one or more microorganisms in a sample may be based on nucleotide sequence information of products from one, some, a minority, most, the majority, or substantially all, or less than the majority, or less than substantially all of the amplifications performed using each of the different primer pairs in a combination of primer pairs employed in the method. In some examples, detecting and/or measuring only one, or more, particular microorganism(s) in a sample is without regard to the presence or absence of any other, or some other, or most other, microorganisms (or nucleic acids from other sources) in the sample. Thus, in some examples of the methods of detecting, determining presence or absence of, and/or measuring a microorganism, a determination of presence or absence may be made based on nucleotide sequence information of products from only some of the amplifications performed with different primer pairs, or of products from all of a smaller or limited number of amplifications. In some instances, even without a determination of the identities of some or all of the microorganisms, the number of different sequences in the products of amplification of sample nucleic acids with the combination of different primers (e.g, 16S rRNA gene primers (and/or the number of and particular hypervariable regions amplified by the primers) vs. non-16S rRNA gene primers (and/or the of the number of and particular target nucleic acids amplified by the primers)) provides useful information not only regarding the presence or absence of a microorganism of interest but also whether other microorganisms are present and relative abundance of a microorganism. For example, in some instances, no amplification products are detected from amplification of sample nucleic acids using one or more non-16S rRNA gene primer pairs. A lack of a particular product from the amplification indicates that one or more microorganisms containing a target nucleic acid that is amplified by the one or more non-16S rRNA gene primer pairs is absent from the sample or present in an amount below the limit of detection. In this case, if amplification products are detected from amplification of sample nucleic acids using one or more 16S rRNA gene primers, this indicates the presence of other microorganisms in the sample. Furthermore, if 2 or more amplification products are detected from amplification of a particular hypervariable region using the one or more 16S rRNA gene primers in this case, and the sequences of the 2 or more amplification products are different, this indicates the presence of a plurality of microorganisms in the sample that are not a microorganism containing a target nucleic acid that is amplified by the one or more non-16S rRNA gene primer pairs. In another example, if two or more different non-16S rRNA gene primer pairs that amplify target nucleic acids in different microorganisms are used in the amplification, and the amplification products contain copies of only a single target nucleic acid sequence, this is indicative of the presence of one microorganism and the absence of the other microorganism from the sample. Also, if in this example two or more amplification products are detected from amplification of a particular hypervariable region using the one or more 16S rRNA gene primers, and the sequences of the two or more amplification products are different, this indicates the presence of a plurality of microorganisms in the sample only one of which is the microorganism that contains target nucleic acid sequence amplified by a pair of the non-16S primers. Additionally, the combined results of the amplifications using the 16S rRNA gene primers (and particularly primers that separately amplify multiple different hypervariable regions) and the non-16S rRNA gene primers yield increased accuracy and specificity in the detection and/or measurement of one or more microorganisms in a sample by reducing false negatives that may occur in basing a determination solely on the results of amplification using 16S rRNA primers and/or reducing or eliminating the number of false positives that may occur in basing a determination solely on the results of amplification using a non-16S rRNA gene primer pair that amplifies a species-specific nucleic acid. For example, due to possible sequence variations in 16S rRNA gene conserved regions in some species or strains of microorganisms (e.g., bacteria), primers designed to amplify a 16S rRNA gene hypervariable region of all bacteria may fail to amplify some bacterial nucleic acids present in a sample, thereby yielding a false negative result. However, the results of amplification of the same nucleic acids using non-16S rRNA gene primers that amplify a specific target nucleic acid in such a microorganism would enable detection of the presence of the microorganism in the sample. In another example, a false positive result can occur in amplification using a non-16S rRNA gene primer pair that amplifies a target nucleic acid sequence that is not unique to the genome of the microorganism intended to be detected by amplification using the primer pair. However, sequence information obtained from products of amplification of the same nucleic acids using one, or typically multiple, 16S rRNA gene primer pairs would reveal the absence of any 16S rRNA gene hypervariable sequences for the intended microorganism, thereby yielding a result of detection of the absence of the microorganism in the sample. In some examples, detecting the presence or absence of a nucleic acid amplification product includes comparing the sequence of the one or more nucleic acid amplification products to reference nucleic acid sequences the genomes of microorganisms, e.g., bacteria, and/or of prokaryotic, e.g., bacterial, 16S rRNA genes. In some examples, comparing the sequence of a nucleic acid amplification product to reference genome sequences includes conducting computer-assisted alignment of the sequence and mapping it to a reference genome. Exemplary nucleotide sequence analysis workflows for mapping sequence reads of amplification products are provided herein. In some examples, relative and/or absolute levels of one or more microorganisms are determined or measured. For example, in some examples, the level of abundance of one or more nucleic acid amplification products and/or sequence reads can be measured to provide relative and/or absolute levels of one or microorganisms. Techniques for quantifying nucleic acids (e.g., amplification products) and/or sequence reads are known in the art and/or provided herein.

### Methods for Assessing, Characterizing, Profiling and/or Measuring a Population of Microorganisms

Also described herein are methods for characterizing, profiling, assessing and/or measuring a population of microorganisms, e.g., bacteria, in a sample. In some examples of the methods, nucleic acids in or from a sample are subjected to nucleic acid hybridization, annealing and/or amplification, for example, using any of the nucleic acids provided herein as probes and/or amplification primers.

In some examples, a method for characterizing, profiling, assessing and/or measuring a population of microorganisms, e.g., bacteria, and/or the composition or components thereof, in a sample provided herein includes (a) subjecting nucleic acids in or from a sample to nucleic acid amplification using a combination of primer pairs comprising (i) one or more primer pairs capable of amplifying nucleic acids containing sequences of one or more hypervariable regions of a prokaryotic 16S rRNA gene (referred to as the "16S rRNA gene primers or primer pairs") and (ii) one or more primer pairs capable of amplifying a target nucleic acid sequence contained within the genome of a microorganism that is not contained within a hypervariable region of a prokaryotic 16S rRNA gene, wherein different primer pairs amplify different target nucleic acid sequences contained within the genome of different microorganisms (referred to as the "non-16S rRNA gene primers or primer pairs"); (b) obtaining sequence information from nucleic acid products amplified by the combination of primer pairs of (i) and (ii); and (c) identifying genera of microorganisms in the sample and species of one or more of the microorganisms in the sample, thereby characterizing a population of microorganisms in the sample. In some examples, the method further includes determining levels, e.g. relative and/or absolute levels, of nucleic acid products amplified by one or more primer pairs of (i), i.e., the 16S rRNA gene primer pairs, and/or (ii), i.e., the non-16S rRNA gene primer pairs, or sequence reads thereof. In some examples, a method for characterizing, profiling, assessing and/or measuring a population of microorganisms in a sample provided herein includes (a) subjecting the nucleic acids to two or more separate nucleic acid amplification reactions using a first set of primer pairs for one nucleic acid amplification reaction and a second set of primer pairs for the other nucleic acid amplification reaction, wherein (i) the first set of primer pairs comprises one or more primer pairs that amplifies a nucleic acid containing a sequence of one or more hypervariable regions of a prokaryotic 16S rRNA gene (referred to as the "16S rRNA gene primers or primer pairs") and (ii) the second set of primer pairs comprises one or more primer pairs that amplify a target nucleic acid sequence contained within the genome of a microorganism that is not contained within a hypervariable region of a prokaryotic 16S rRNA gene, wherein different primer pairs amplify different target nucleic acid sequences contained within the genome of different microorganisms (referred to as the "non-16S rRNA gene primers or primer pairs"); (b) obtaining sequence information from nucleic acid products amplified by primer pairs of (i) and (ii); and (c) identifying genera of microorganisms in the sample and species of one or more of the microorganisms in the sample, thereby characterizing a population of microorganisms in the sample. In some examples, the method further includes determining levels, e.g. relative and/or absolute levels, of nucleic acid products amplified by one or more primer pairs of (i) and/or (ii) or sequence reads thereof.

In any examples of methods provided herein for characterizing, profiling, assessing and/or measuring a population of microorganisms and/or the composition or components thereof, in a sample, the methods can include any examples of the methods for detecting and/or measuring the presence or absence of one or more microorganisms in a sample as described herein. For example, in some examples, the microorganism(s) is/are bacteria. In some examples, the prokaryotic 16S rRNA gene is a bacterial gene and/or the prokaryotic microorganism is a bacterium. In some examples the target nucleic acid sequence contained within a genome of a prokaryotic microorganism, e.g., bacteria, is unique to the microorganism. In some examples, the one or more 16S rRNA gene primer pairs amplify a nucleic acid sequence in a plurality of microorganisms, e.g., bacteria, from different genera. In some examples, the sample is a biological sample, such as, for example, a sample of contents of the alimentary tract of an animal. In some examples, the sample is a fecal sample.

In any examples of methods for characterizing, profiling, assessing and/or measuring a population of microorganisms, in a sample, a nucleic acid amplification can be performed according to any of the examples provided herein for such amplification. For example, in some examples, the one or more primer pairs that amplifies a nucleic acid containing a sequence of a hypervariable region of a prokaryotic 16S rRNA gene separately amplify nucleic acids containing sequences of different hypervariable regions. In some examples, the primers of the one or more 16S rRNA gene primer pairs are directed to, or bind to, or hybridize to nucleic acid sequences contained in conserved regions of a prokaryotic 16S rRNA gene. In some examples, the one or more 16S rRNA gene primer pairs and/or non-16S rRNA gene primer pairs comprise a plurality of primer pairs. For example, the one or more 16S rRNA gene primer pairs of can comprise a plurality of primer pairs that amplify nucleic acids containing sequences of multiple hypervariable regions of a prokaryotic 16S rRNA gene and/or the one or more non-16S rRNA gene primer pairs can comprise a plurality of primer pairs that amplify target nucleic acid sequences contained in the genomes of a plurality of microorganisms. In some examples, the amplification, or two or more separate nucleic acid amplification reactions, is/are multiplex amplification conducted in a single reaction mixture. In some examples, each primer of the one or more 16S rRNA gene primer pairs contains less than 10, less than 9, less than 8, less than 7, less than 6, less than 5, less than 4, less than 3, or less than 2 contiguous nucleotides of sequence identical to a sequence of contiguous nucleotides of another primer in the combination of primer pairs. In some examples, the nucleic acid sequences being amplified by the one or more 16S rRNA gene primer pairs are less than about 300 bp, less than about 250 bp, less than about 200 bp, less than about 175 bp, less than about 150 bp, or less than about 125 bp in length. In some examples, the 16S rRNA gene primer pairs separately amplify nucleic acids separately containing sequences of 3 or more, 4 or more, 5 or more, 6 or more, 7 or more, 8 or more or 9 different hypervariable regions of a prokaryotic 16S rRNA gene thereby producing amplified copies of the nucleic acids containing sequences of the 3 or more, 4 or more, 5 or more, 6 or more, 7 or more, 8 or more or 9 different hypervariable regions of the 16S rRNA gene of one or more microorganisms, wherein the amplified copies of different hypervariable regions are separate amplicons. In some examples, the 16S rRNA gene primer pairs separately amplify nucleic acids containing sequences of 8 different hypervariable regions of a prokaryotic 16S rRNA gene. In some examples, the 8 different hypervariable regions are V2-V9. In some examples, the 16S rRNA gene primer pairs separately amplify nucleic acids separately containing sequences of 3 or more different hypervariable regions of a prokaryotic 16S rRNA gene wherein one of the 3 or more regions is a V5 region thereby producing amplified copies of the nucleic acids containing sequences of the 3 or more different hypervariable regions of the 16S rRNA gene of one or more microorganisms. In some examples, the combination of primer pairs includes degenerate sequences of one or more primers in one or more primer pairs. In some examples, the 16S rRNA gene primer pair(s) comprise primers and/or primer pairs containing, or consisting essentially of, a sequence or sequences of a primer or primer pair in Table 15, or SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base.

In some examples of methods for characterizing, profiling, assessing and/or measuring a population of microorganisms, and/or the composition or components thereof, in a sample, the one or more non-16S rRNA gene primer pairs specifically amplifies a target nucleic acid sequence contained within a genome of a microorganism selected from the microorganisms of Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the target nucleic acid is unique to the microorganism. In some examples, the sample includes nucleic acids from a plurality of different microorganisms listed in Table 1. In some such examples, amplified copies of a plurality of different microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis* is produced. In some examples, the sample includes a mixture of nucleic acids of one or more, or a plurality of, microorganisms selected from among the microorganisms listed in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis,* and one or more microorganisms, e.g., bacteria, not listed in Table 1. In some examples, at least one, or one or more, target nucleic acid sequence(s) comprises or consists essentially of a nucleotide sequence selected from the nucleotide sequences of SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, or the complement thereof. In some examples, at least one, or one or more, product(s) of the nucleic acid amplification comprises, or consists essentially of, a nucleotide sequence selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, or the complement thereof, and optionally having one or more primer sequences at the 5' and/or 3' end(s) of the sequence, such as any of the primer sequences provided herein, and is less than about 500, less than about 475, less than about 450, less than about 400, less than about 375, less than about 350, less than about 300, less than about 275, less than about 250, less than about 200, less than about 175, less than about 150, or less than about 100 nucleotides in length. In some examples, the at least one non-16S rRNA gene primer pair does not detectably amplify a nucleic acid sequence contained within any genus other than the genus of the microorganism containing the target nucleic acid sequence. In some examples, the at least one non-16S rRNA gene primer pair does not detectably amplify a nucleic acid sequence contained within any species other than the species of the microorganism containing the target nucleic acid sequence. In some examples, at least one primer of the non-16S rRNA gene primer pair, or at least one non-16S rRNA gene primer pair, contains, or consists essentially of, the sequence or sequences of a primer or primer pair in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the nucleic acids are subjected to nucleic acid amplification using a plurality of non-16S rRNA gene primers or primer pairs, each containing, or consisting essentially of, a sequence or sequences of a primer pair in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, at least one primer or one primer pair in the combination of primer pairs includes a modification that facilitates nucleic acid manipulation, amplification, ligation and/or sequencing of amplification products and/or reduction or elimination of primer dimers. In particular examples, a modification is one that facilitates multiplex nucleic acid amplification, ligation and/or sequencing of products of multiplex amplification.

In some examples of the methods, the characterizing, profiling, assessing and/or measuring of a population of microorganisms (e.g., bacteria), is designed to focus on the make-up of the population of microorganisms particularly with respect to certain groups of microorganisms and the proportionate presence of the group and/or group members in the population. In such examples, the combination of primers and/or primer pairs include a selected group or sub-group of microorganism-specific nucleic acids and include kingdom-encompassing nucleic acids (e.g., 16S rRNA gene primers and primer pairs), and enable not only a comprehensive survey of the entirety and relative levels of genera of microorganisms (e.g., bacteria), but also detailed identification of species of microorganisms that can be tailored, for example, to focus on one or more particular microorganisms of interest that may be significant in certain states of health and disease or microbiota imbalance, e.g., dysbiosis. In some examples, combinations of nucleic acids include microorganism-specific nucleic acids, and/or primer pairs, that specifically amplify a nucleic acid sequence contained in the genome of one or more microorganisms (e.g., bacteria) implicated in one or more conditions, disorders and/or diseases. In particular examples, the combination of nucleic acid primers and/or primer pairs includes a nucleic acid and/or a primer pair that specifically amplifies a target nucleic acid sequence contained within a genome of a microorganism selected from the microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the combination includes a plurality of nucleic acids and/or primer pairs that include at least one nucleic acid primer pair that specifically amplifies a target nucleic acid in each of the microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the plurality of primer pairs includes primer pairs that specifically amplify genomic target nucleic acids contained within at least 5, 10, 15, 20, 25, 30, 35, 40, 45, 50, 55, 60 or 70 of the microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In particular examples, the target nucleic acid sequences contained in the genome of the different microorganisms are unique to each of the microorganisms. In some examples, a combination of nucleic acids and/or nucleic acid primer pairs includes two or more nucleic acids and/or nucleic acid primer pairs that specifically amplify a unique nucleic acid sequence contained in the genome of one or more of the Group A microorganisms (see Table 2A), which are species implicated as having a role in multiple conditions, diseases and/or disorders, including, for example oncological conditions including, for example, response to immune-oncology treatment and cancer, gastrointestinal disorders, including, for example, irritable bowel syndrome, inflammatory bowel disease and coeliac disease, and autoimmune diseases, including, for example, lupus and rheumatoid arthritis. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group A. In some examples, the combination of nucleic acids and/or nucleic acid primer pairs includes nucleic acids and/or nucleic acid primer pairs that specifically bind to, hybridize to and/or amplify sequences as set forth in Table 2A for exemplary nucleic acids, primers and primer pairs for genomes of Group A microorganisms. **In** some examples, the combination of nucleic acids and/or nucleic acid primer pairs includes nucleic acids and/or nucleic acid primer pairs having a nucleotide sequence or sequences as set forth in Table 2A for exemplary nucleic acids, primers and primer pairs for genomes of Group A microorganisms.

In some examples, a combination of nucleic acids and/or nucleic acid primer pairs includes two or more nucleic acids and/or nucleic acid primer pairs that specifically amplify a unique nucleic acid sequence contained in the genome of one or more of the Group B microorganisms (see Table 2B), which are species implicated as having a role in response to immuno-oncology treatment. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group B. In some examples, the combination of nucleic acids and/or nucleic acid primer pairs includes nucleic acids and/or nucleic acid primer pairs that specifically bind to, hybridize to and/or amplify sequences as set forth in Table 2B for exemplary nucleic acids, primers and primer pairs for genomes of Group B microorganisms. In some examples, the combination of nucleic acids and/or nucleic acid primer pairs includes nucleic acids and/or nucleic acid primer pairs having a nucleotide sequence or sequences as set forth in Table 2B for exemplary nucleic acids, primers and primer pairs for genomes of Group B microorganisms.

In some examples, a combination of nucleic acids and/or nucleic acid primer pairs includes two or more nucleic acids and/or nucleic acid primer pairs that specifically amplify a unique nucleic acid sequence contained in the genome of one or more of the Group C microorganisms (see Table 2C), or the Group C microorganisms excluding *Helicobacter salomonis* (Subgroup 1 of the Group C microorganisms), which are species implicated as having a role in cancer. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group C, or the Group C microorganisms excluding *Helicobacter salomonis* (Subgroup 1 of the Group C microorganisms). In some examples, the combination of nucleic acids and/or nucleic acid primer pairs includes nucleic acids and/or nucleic acid primer pairs that specifically bind to, hybridize to and/or amplify sequences as set forth in Table 2C for exemplary nucleic acids, primers and primer pairs for genomes of Group C microorganisms. In some examples, the combination of nucleic acids and/or nucleic acid primer pairs includes nucleic acids and/or nucleic acid primer pairs that specifically bind to, hybridize to and/or amplify sequences selected from SEQ ID NOS: 1616, 1619, 1620, 1625-1628, 1635-1640, 1699, 1700, 1705-1708, 1752, 1753, 1784-1786, 1817-1820, 1827, 1828, 1840, 1841, 1844, 1845, 1852-1859, 1899, 1900, 1904, 1905, 1932, 1933, 1956-1958, 1975, 1976 of Table 17, and/or a substantially identical or similar sequence, or sequences selected from SEQ ID NOS: 1616, 1619, 1620, 1625-1628, 1635-1640, 1699, 1700, 1705-1708, 1752, 1753, 1784-1786, 1827, 1828, 1840, 1841, 1844, 1845, 1852-1859, 1899, 1900, 1904, 1905, 1932, 1933, 1956, 1957, 1958 of Table 17, and/or a substantially identical or similar sequence. In some examples, the combination of nucleic acids and/or nucleic acid primer pairs includes nucleic acids and/or nucleic acid primer pairs having a nucleotide sequence or sequences as set forth in Table 2C for exemplary nucleic acids, primers and primer pairs for genomes of Group C microorganisms. In some examples, the combination of nucleic acids and/or nucleic acid primer pairs includes nucleic acids and/or nucleic acid primer pairs having a nucleotide sequence or sequences selected from SEQ ID NOS: 71, 72, 77-80, 89-96, 109-120, 237-242, 249-256, 343-346, 407-412, 493-496, 511-520, 521-524, 547-550, 555-558, 561-568, 571-586, 665-668, 675-678, 731-734, 779-784, and/or SEQ ID NOS: 849, 850, 855-858, 867-874, 887-898, 1012-1020, 1025-1034, 1121-1124, 1185-1190, 1271-1276, 1289-1298, 1299-1302, 1325-1328, 1333-1336, 1339-1346, 1349-1364, 1443-1446, 1453-1456, 1509-1512, 1557-1562, in Table 16, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes nucleic acids and/or nucleic acid primer pairs having a nucleotide sequence or sequences selected from SEQ ID NOS: 71, 72, 77-80, 89-96, 109-120, 237-242, 249-256, 343-346, 407-412, 493-496, 511-520 and/or SEQ ID NOS: 849, 850, 855-858, 867-874, 887-898, 1012-1020, 1025-1034, 1121-1124, 1185-1190, 1271-1276, 1289-1298 in Table 16, or substantially identical or similar sequences, or any of the aforementioned nucleotide sequences of nucleic acids or primer pairs in which one or more thymine bases is substituted with a uracil base. In some examples, the combination of nucleic acids and/or nucleic acid primer pairs includes nucleic acids and/or nucleic acid primer pairs having a nucleotide sequence or sequences selected from SEQ ID NOS: 71, 72, 77-80, 89-96, 109-120, 237-242, 249-256, 343-346, 407-412 and/or SEQ ID NOS: 849, 850, 855-858, 867-874, 887-898, 1012-1020, 1025-1034, 1121-1124, 1185-1190 in Table 16, or substantially identical or similar sequences, or any of the aforementioned nucleotide sequences of nucleic acids or primer pairs in which one or more thymine bases is substituted with a uracil base.

In some examples, a combination of nucleic acids and/or nucleic acid primer pairs includes two or more nucleic acids and/or nucleic acid primer pairs that specifically amplify a unique nucleic acid sequence contained in the genome of one or more of the Group D microorganisms (see Table 2D), which are species implicated as having a role in gastrointestinal disorders, including, for example, irritable bowel syndrome, inflammatory bowel disease and coeliac disease. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group D. In some examples, the combination of nucleic acids and/or nucleic acid primer pairs includes nucleic acids and/or nucleic acid primer pairs that specifically bind to, hybridize to and/or amplify sequences as set forth in Table 2D for exemplary nucleic acids, primers and primer pairs for genomes of Group D microorganisms. In some examples, the combination of nucleic acids and/or nucleic acid primer pairs includes nucleic acids and/or nucleic acid primer pairs having a nucleotide sequence or sequences as set forth in Table 2D for exemplary nucleic acids, primers and primer pairs for genomes of Group D microorganisms.

In some examples, a combination of nucleic acids and/or nucleic acid primer pairs includes two or more nucleic acids and/or nucleic acid primer pairs that specifically amplify a unique nucleic acid sequence contained in the genome of one or more of the Group E microorganisms (see Table 2E), which are species implicated as having a role in autoimmune disorders, including, for example, lupus and rheumatoid arthritis. In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group E. In some examples, the combination of nucleic acids and/or nucleic acid primer pairs includes nucleic acids and/or nucleic acid primer pairs that specifically bind to, hybridize to and/or amplify sequences as set forth in Table 2E for exemplary nucleic acids, primers and primer pairs for genomes of Group E microorganisms. In some examples, the combination of nucleic acids and/or nucleic acid primer pairs includes nucleic acids and/or nucleic acid primer pairs having a nucleotide sequence or sequences as set forth in Table 2E for exemplary nucleic acids, primers and primer pairs for genomes of Group E microorganisms.

### Nucleotide Sequence Information

Embodiments of the methods, the characterizing, profiling, assessing and/or measuring of a population of microorganisms and/or the composition or components thereof, involve utilization of nucleotide sequence information of amplification products in accordance with the claims. The nucleotide sequences provide information used in assessing or measuring the diversity (e.g., number of different microorganisms, such as number of different genera and/or species of microorganisms), the levels or abundance of different microorganisms, the proportionate amounts of different microorganisms and/or the identities (e.g., genus, species, etc.) of the microorganisms that make up a microorganism population in a sample. Typically, the characterizing, profiling, assessing and/or measuring of a population of microorganisms and/or the composition or components thereof, is based on nucleotide sequence information of products from the majority of, or substantially all, the amplifications performed using each of the different primer pairs in a combination of primer pairs employed in the method. One reason for this is that the microorganism population, as defined by the different microorganisms, and/or relative abundances thereof, is being elucidated in the method. In contrast, a method of amplifying, detecting and/or measuring only one, or more, particular microorganism(s) in a sample can be without regard to the presence or absence of any other, or some other, microorganisms (or nucleic acids from other sources) in the sample, and thus a determination may be made based on nucleotide sequence information of products from only some of the amplifications performed with different primer pairs, or of products from all of a smaller or limited number of amplifications.

In some embodiments, the methods for characterizing, profiling, assessing and/or measuring a population of microorganisms and/or the composition or components thereof, in a sample, include obtaining sequence information from nucleic acid products amplified by the combination of primer pairs employed in the method. Examples of sequence information include, but are not limited to, the identities of the nucleotides and the order thereof in a contiguous polynucleotide sequence (the nucleotide sequence determination) of an amplification product (which can include, for example, barcode sequence, e.g., corresponding to amplicon library source, primers used, etc.), alignment and/or mapping of nucleotide sequence to a reference sequence (and identity of the genus or species of the reference sequence), the number of sequence reads that map to a reference sequence and/or portions thereof (e.g., hypervariable regions of a 16S rRNA gene), the number of sequence reads that map uniquely to a reference sequence, and the number of regions (e.g., target sequences) of a reference sequence to which sequence reads map. In some embodiments, sequence information provides the identities (e.g., genus, species) of microorganisms and/or the number of different microorganisms in a population which is indicative of the diversity of the population. In some embodiments, sequence information provides a measure of the levels (e.g., relative and/or absolute) of microorganisms in a population (abundance and proportionate contribution or presence in a population).

Techniques for sequencing nucleic acids, e.g., nucleic acids of a prepared library of nucleic acids, such as amplicon libraries that can be generated using compositions and methods described herein, are provided herein and/or known in the art and include, for example, next generation sequencing-by-synthesis and Sanger sequencing methods. Any such methods can include use of microarrays for massively parallel sequencing of nucleic acids, e.g., on a substrate, such as a chip. In some embodiments, nucleic acids, e.g., an amplicon library, can be sequenced using an Ion Torrent Sequencer (Life Technologies), e.g., the Ion Torrent PGM 318^{™} or Ion Torrent S5 520^{™}, Ion Torrent S5 530^{™}, or Ion Torrent S5 XL^{™} system. Other sequencing systems include, but are not limited to, systems employing solid-phase PCR involving bridge amplification of nucleic acids using oligonucleotide adapters (e.g., Illumina MiSeq, NextSeq or HiSeq platforms). In some embodiments, nucleic acid templates to be sequenced can be prepared from a population of nucleic acid molecules using amplification methods provided herein. In some embodiments, a sequencer can be coupled to a server that applies parameters or software to determine the sequence of the amplified nucleic acid molecules.

In some embodiments, an amplicon library prepared using primers provided herein can be used in downstream enrichment applications. For example, following amplification of sample nucleic acids using nucleic acid composition, e.g., primers, primer pairs, provided herein, a secondary and/or tertiary amplification process including, but not limited to, a library amplification step and/or a clonal amplification step, including, for example, isothermal nucleic acid amplification, emulsion PCR or bridge PCR, can be performed. In some embodiments, the amplicon library can be used in an enrichment application and a sequencing application. For example, an amplicon library can be sequenced using any suitable DNA sequencing platform. In some embodiments, amplification and templating of amplified amplicons can be performed according to the Ion PGM^{™} Template IA 500 Kit user guide (see, e.g., Thermo Fisher Scientific Catalog no. A24622 and Publication no. MAN0009347), Ion 540^{™} Kit-Chef user guide (see, e.g., Thermo Fisher Scientific Catalog no. A30011 and Publication no. MAN0010851), or Ion 550^{™} Kit-Chef user guide (see, e.g., Thermo Fisher Scientific Catalog no. A34541 and Publication no. MAN0017275). In some embodiments, at least one of the amplified targets sequences to be clonally amplified can be attached to a support or particle (see, for example, U.S. Patent Application Publication No. US2019/0194719). The support can be comprised of any suitable material and have any suitable shape, including, for example, planar, spheroid or particulate. In some embodiments, the support is a scaffolded polymer particle as described in U.S. Published App. No. 20100304982. In some embodiments, the amplicon library can be prepared, enriched and sequenced in less than 24 hours. In some embodiments, the amplicon library can be prepared, enriched and sequenced in approximately 9 hours. In some embodiments, an amplicon library can be a paired or combined library, e.g.,, a library that contains amplicons generated from amplification of sample nucleic acids using 16S rRNA gene primers and amplicons generated from amplification of sample nucleic acids using species (e.g., bacterial species)-specific primers. In some embodiments, a library and/or template preparation to be sequenced can be prepared for sequencing automatically with an automated system, e.g., the Ion Chef^{™} system (Thermo Fisher Scientific, Inc.). Amplification products generated by the methods disclosed herein can be ligated to an adapter that may be used downstream as a platform for clonal amplification. The adapter can function as a template strand for subsequent amplification using a second set of primers and therefore allows universal amplification of the adapter-ligated amplification product. In some embodiments, adapters ligated to amplicons include one or more barcodes. In one embodiment, one barcode can be ligated to amplicons generated in amplification of sample nucleic acids using 16S rRNA gene primers and a different barcode can be ligated to amplicons generated in amplification of sample nucleic acids using species (e.g., bacterial species)-specific primers. The ability to incorporate barcodes enhances sample throughput and allows for analysis of multiple samples or sources of material concurrently. In one example, amplified nucleic acid molecules prepared using compositions and methods provided herein can be ligated to Ion Torrent^{™} Sequencing Adapters (A and P1 adapters, sold as a component of the Ion Fragment Library Kit, Life Technologies, Part No. 4466464) or Ion Torrent^{™} DNA Barcodes (Life Technologies, Part No. 4468654). In some embodiments, a barcode or key can be incorporated into each of the amplification products to assist with data analysis and for example, cataloging.

In some embodiments, an amplicon library produced by the teachings of the present disclosure is sufficient in yield to be used in a variety of downstream applications including the Ion Xpress^{™} Template Kit using an Ion Torrent^{™} PGM system (e.g., PCR-mediated addition of the nucleic acid fragment library onto Ion Sphere^{™} Particles)(Life Technologies, Part No. 4467389). For example, instructions to prepare a template library from the amplicon library can be found in the Ion Xpress Template Kit User Guide (Thermo Fisher Scientific). Instructions for loading the subsequent template library onto the Ion Torrent^{™} Chip for nucleic acid sequencing are described in the Ion Sequencing User Guide (Thermo Fisher Scientific). In some embodiments, the amplicon library produced by the teachings of the present disclosure can be used in paired end sequencing (e.g., paired-end sequencing on the Ion Torrent^{™} PGM system (Thermo Fisher Scientific). It will be apparent to one of ordinary skill in the art that numerous other techniques, platforms or methods for clonal amplification such as wildfire, PCR and bridge amplification can be used in conjunction with the amplification products of the present disclosure. It is also envisaged that one of ordinary skill in art upon further refinement or optimization of the conditions provided herein can proceed directly to nucleic acid sequencing (for example using the Ion Torrent PGM^{™} or Proton^{™} sequencers, Life Technologies) without performing a clonal amplification step. Sequence data processing and analysis programs to obtain the sequence of nucleotides of nucleic acids, e.g., amplicons, are available and include, for example, the Torrent Suite^{™} Software product for use with Ion sequencers (Thermo Fisher Scientific, Inc.).

In some embodiments, relative and/or absolute levels of one or more microorganisms are determined or measured. For example, in some embodiments, the level of abundance of one or more nucleic acid amplification products and/or sequence reads can be measured to provide relative and/or absolute levels of one or microorganisms. Techniques for quantifying nucleic acids (e.g., amplification products) and/or sequence reads are known in the art and/or provided herein.

Utilizing sequence information, for example, in detecting microorganisms and/or identifying genera and/or species of sample microorganisms, in the methods includes comparing the sequence of the one or more nucleic acid amplification products to each other and/or to reference nucleic acid sequences of the genomes of microorganisms, e.g., bacteria, and/or of prokaryotic, and 16S rRNA genes in accordance with the claims. Comparing the sequence of a nucleic acid amplification product includes computer-assisted alignment of the sequence to other sequences in accordance with the claims. There are several software products that can be used in conducting the computational processing involved in aligning and mapping nucleic acid sequences. For example, some products utilize a Burrows-Wheeler Transform (BWT; see, e.g., Li and Durbin (2009) Bioinformatics 25:1754-1760) algorithm in mapping sequence reads to sequences in a reference database. One implementation of BWT is provided by the Burrows-Wheeler Aligner (see, e.g., https://sourceforge.net/projects/bio-bwa/files/). Some products utilize hashing algorithms (e.g., SSAHA; sanger.ac.uk/science/tools/ssaha; see, e.g., Ning et al. (2001) *Genome Res 11(10)*:1725-11729) and/or dynamic programming algorithms (e.g., Needleman-Wunsch or Smith-Waterman) implemented, for example, in software tools available through the European Bioinformatics Institute (see, e.g., ebi.ac.uk/services/all). Another tool available for aligning nucleotide sequences is the Basic Local Alignment Search Tool (BLAST) available through the National Center for Biotechnology Information (NCBI) (see, e.g., https://blast.ncbi.nlm.nih.gov/Blast.cgi). This program can be used to search sequence databases (e.g., microbial genome databases) for similar sequences using metrics of read identity, alignment length and other parameters, and the sequence reads mapping to a particular genus or species can be calculated. An example of a program providing several options for mapping/alignment of nucleotide sequences is the Torrent Mapping Alignment Program (TMAP) module (see, e.g., Torrent Suite^{™} Software User Guide; Thermo Fisher Scientific Publication number MAN0017972) for use with the Torrent Suite^{™} Software product that is optimized for sequence data generated using Ion Torrent^{™} sequencer systems.

Claimed herein are nucleotide sequence analysis workflows for aligning and/or mapping sequence reads of amplification products in accordance with the claims. These methods can be used to compress reference sequence databases used in mapping sequence reads for analysis and profiling of microbial populations in accordance with the claims. There can be over 100,000 nucleotide sequences in a database of genome and gene (e.g., 16S rRNA gene) sequences from numerous microorganisms (e.g., bacteria). In methods provided herein in which 16S rRNA gene hypervariable regions are amplified, the more of the nine hypervariable regions amplified and sequenced, the more the number of alignments that have to be performed. Thus, an analysis of multiple amplified nucleic acid regions of multiple microorganisms in a sample can require extensive processor memory and time and potentially introduce errors and uncertainties into the analysis. Methods of performing such analyses that reduce computational requirements, reduce memory requirements and improve the quality of characterization of nucleic acids in a sample are described herein. In some embodiments, an unaligned BAM file including sequence read information may be provided to a processor for analyzing the sequence reads corresponding to marker regions. Reads obtained from sequencing of library DNA templates may be analyzed to identify, and determine the levels of, microbial constituents of the samples. Analysis may be conducted using a workflow incorporating Ion Torrent Suite^{™} Software (Thermo Fisher Scientific) with a run plan template designed to facilitate microbial DNA sequence read analysis, and an AmpliSeq microbiome analysis software plugin which generates counts for amplicons targeted in the assay. Reference genome files used for alignment in read mapping aspects of the analysis may be included in the plugin. Compressed 16S reference sequences may be derived from the GreenGenes or other 16S rRNA gene sequence database. The compressed 16S reference sequences comprise a plurality of the hypervariable regions of the 16S rRNA gene. Primers, such as those described herein, targeting multiple of the 9 hypervariable regions for amplification, e.g., V2 - V9, yield a set of hypervariable segment sequences through amplification of microbial nucleic acids. The set of hypervariable segments may be generated by applying an in silico PCR simulation using the primer pairs to the full length 16S rRNA gene sequences contained in the database to extract expected target segments for variable regions, e.g., V2 - V9. The in silico PCR simulation may use available computational tools for calculation theoretical PCR results using a given set of primers and a target DNA sequence input by the user. One such tool is Primer-BLAST (https://www.ncbi.nlm.nih.gov/tools/primer-blast/index.cgi?LINK_LOC=BlastHome), described in Ye J, Coulouris G, Zaretskaya I, Cutcutache I, Rozen S, Madden TL. (2012) Primer-BLAST: a tool to design target-specific primers for polymerase chain reaction. BMC Bioinformatics. 13:134. The set of hypervariable segments derived from the in silico simulation provides a compressed reference containing only hypervariable region sequences of those full-length 16S rRNA sequences in the complete database that would be expected to be amplified by the primers. For example, the number of base pairs of the reference may be reduced from 1500 bp of the full length sequence to 8 hypervariable segments having a total of 1299 bp. The GreenGenes database contains about 150,000 16S rRNA gene sequences.

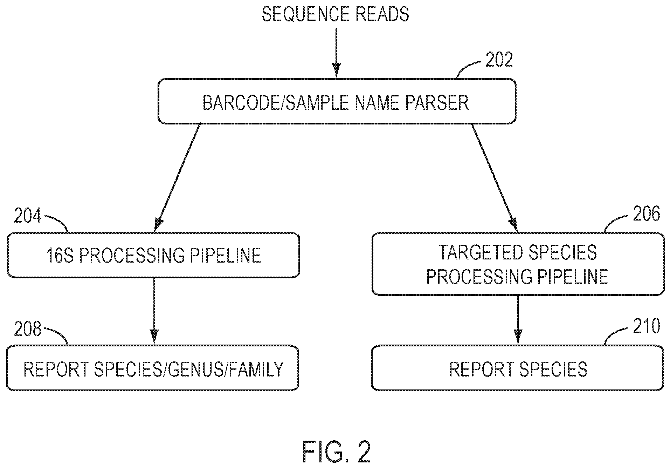

FIG. 2 illustrates a workflow for use in analysis of sequence information generated in methods provided herein. For example, the workflow can be used as a method for processing the sequence reads to assess microbial composition of a sample. The barcode/sample name parser separates the sequence reads into a set corresponding to the amplicons generated in amplification using 16S rRNA gene primers and a set corresponding to the amplicons generated in amplification using non-16S rRNA gene primers (e.g., targeted species-specific primers). Quality read trimming and short reads may be removed by the base caller. The base calls may be made by analyzing any suitable signal characteristics (e.g., signal amplitude or intensity). The structure and/or design of a sensor array, signal processing and base calling for use with the present teachings may include one or more features described in U.S. Pat. Appl. Publ. No. 2013/0090860, published April 11, 2013. For example, sequence reads having lengths less than 60 bases or greater than 350 may be removed. The sequence reads and targeted species of sequences generated through amplification of sample nucleic acids using 16S rRNA gene primers and species-specific primers may be analyzed independently. In some embodiments, more than one primer pair may target a given hypervariable region. The sequence of each primer of a primer pair for amplifying each region is directed to "conserved" sequence on each side of the particular V region so that the primer pair will theoretically amplify that variable region in the genome of every bacterium. However, some of the "conserved" regions on either side of each variable region, particularly the conserved regions on either side of V2 and V8, are not sufficiently conserved in order to amplify the V2 and V8 regions of all bacteria. Thus, for amplifying V2 and V8, the 3 primer pairs (instead of 1 primer pair) may be used with the sequences of each primer pair being almost identical but having one or two nucleotides different (referred to as "degenerate primers"). Using those 3 primer pairs is a means to amplify the V2 and V8 regions for all bacteria even though the conserved regions on either side of V2 and V8 may be slightly different for different bacteria.

The hypervariable segments generated by the in silico simulation for the species and strains in the 16S rRNA gene sequences database are further processed to identify expected patterns, or signatures, in the hypervariable segments characteristic of the species and strain. An expected signature is generated for each species and strain based on the presence (=1) or absence (=0) of each of the targeted hypervariable segments in the amplification results of the in silico simulation. Matrix A gives an example of expected signatures when there is one primer pair per hypervariable region V2 - V9 for two species A and B.

### MATRIX A

| SPECIES | SEQ. # | V2 | V3 | V4 | V5 | V6 | V7 | V8 | V9 |
|---|---|---|---|---|---|---|---|---|---|
| A | 1 | 0 | 1 | 1 | 1 | 0 | 0 | 1 | 0 |
| A | 2 | 0 | 0 | 0 | 1 | 0 | 0 | 1 | 0 |
| A | 3 | 0 | 1 | 1 | 1 | 0 | 0 | 1 | 0 |
| B | 4 | 0 | 1 | 1 | 1 | 0 | 0 | 1 | 0 |

Matrix B gives an example of expected signatures when more than one primer pair targets two of the hypervariable regions, V2 and V8, for species C and D. In this example, three primer pairs target hypervariable region V2 and three primer pairs target hypervariable region V8.

### MATRIX B

| SPECIES | SEQ. # | V2 | V2 | V2 | V3 | V4 | V5 | V6 | V7 | V8 | V8 | V8 | V9 |
|---|---|---|---|---|---|---|---|---|---|---|---|---|---|
| C | 1 | 0 | 1 | 1 | 1 | 0 | 0 | 0 | 0 | 0 | 1 | 1 | 1 |
| C | 2 | 0 | 1 | 1 | 1 | 0 | 0 | 0 | 0 | 0 | 1 | 1 | 1 |
| C | 3 | 0 | 0 | 1 | 1 | 1 | 0 | 0 | 0 | 0 | 0 | 1 | 1 |
| D | 4 | 0 | 1 | 1 | 1 | 0 | 0 | 0 | 0 | 0 | 1 | 1 | 1 |

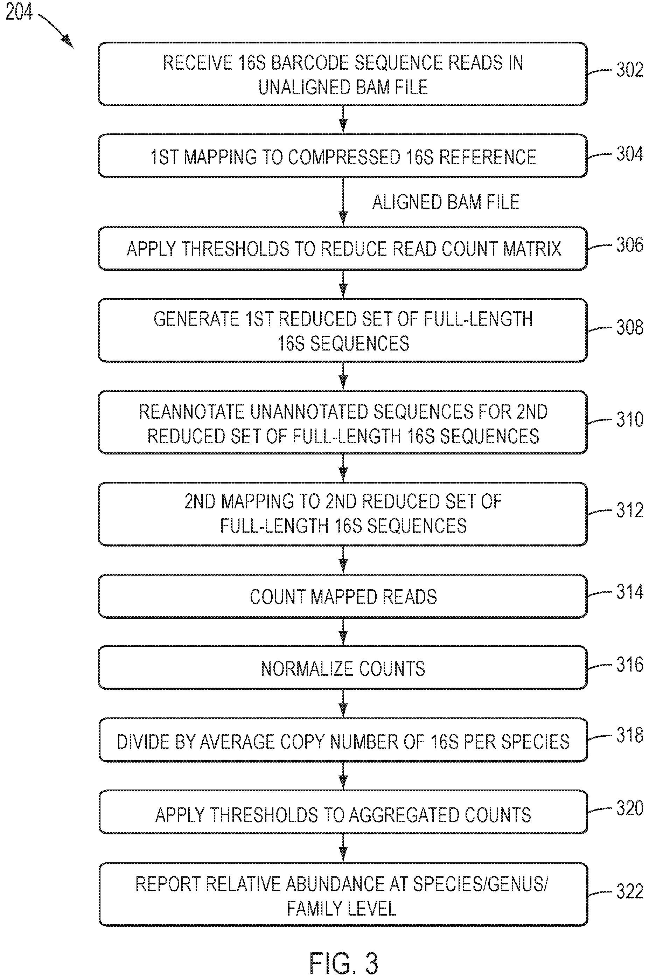

For example, for the GreenGenes database, 150,000 expected signatures may be determined, one for each gene sequence in the database. The expected signatures, such as the examples shown in Matrix A and Matrix B, may be used for combining counts of aligned reads for 16S, as described with respect to FIG. 3.

FIG. 2 is a block diagram of a method for processing the sequence reads to determine microbial composition. The sequence reads are obtained from sequencing of the amplicons generated using the 16S primer pool and a species primer pool to amplify nucleic acids extracted from a sample. The barcode/sample name parser 202 separates the sequence reads into a set of sequence reads corresponding to the 16S amplicons, or 16S sequence reads, and a set of sequence reads corresponding to targeted species amplicons, or targeted species sequence reads. Quality read trimming and short reads may be removed by the base caller. The base calls may be made by analyzing any suitable signal characteristics (e.g., signal amplitude or intensity). The structure and/or design of a sensor array, signal processing and base calling for use with the present teachings may include one or more features described in U.S. Pat. Appl. Publ. No. 2013/0090860, published April 11, 2013. For example, sequence reads having lengths less than 60 bases or greater than 350 may be removed. The 16S sequence reads may be analyzed by a16S processing pipeline 204 and the targeted species sequence reads may be analyzed independently by a targeted species processing pipeline 206. The 16S processing pipeline 204 may provide information on the species/genus/family detected in the sample for a report 208. The targeted species processing pipeline 206 may provide information on the species detected in the sample for a report 210.

FIG. 3 is a block diagram of the 16S read data processing pipeline, in accordance with an embodiment. In step 302, the 16S sequence reads identified by the barcode/sample name parser 202 are received in an unaligned BAM file. The 16S sequence reads are subjected to two mapping steps 304 and 312. In a first mapping step 304, the 16S sequence reads are aligned to the reference hypervariable segments of the compressed 16S reference set, with multi-mapping and end-to-end mapping enabled. The mapping steps 304 and 312 determine aligned sequence reads and associated mapping quality parameters. Methods for aligning sequence reads for use with the present teachings may include one or more features described in U.S. Pat. Appl. Publ. No. 2012/0197623, published August 2, 2012. The mapped reads are filtered based on alignment quality. For example, a minimum local alignment score may be set to 35. With multi-mapping enabled, one read may align to more than one of the reference hypervariable segments. The alignments having equal best scores may be included in observed read counts for subsequent steps.

In step 306, a matrix of observed read counts for each of the targeted hypervariable regions for each species and strain is formed from the aligned reads information in the aligned BAM file and operations to reduce the read count matrix are applied. For example, the matrix may have dimensions of 150,000 x (number of targeted hypervariable regions per gene). Matrix C gives an example of a portion of a matrix of observed read counts, or read count matrix, for targeted hypervariable regions corresponding to the example of Matrix B.

### MATRIX C

| SPECIES | SEQ. # | V2 | V2 | V2 | V3 | V4 | V5 | V6 | V7 | V8 | V8 | V8 | V9 |
|---|---|---|---|---|---|---|---|---|---|---|---|---|---|
| C | 1 | 5 | 10 | 200 | 1000 | 0 | 0 | 0 | 1 | 2 | 100 | 2000 | 400 |
| C | 2 | 15 | 20 | 100 | 1000 | 5 | 10 | 0 | 0 | 0 | 200 | 5000 | 500 |

In step 306, a first reduction of the read count matrix reduces the number of rows. The read counts corresponding to the hypervariable regions for each row may be added to form a sum and a threshold T_{RS} applied to the sum. For example, the row sum threshold T_{RS} may be set to 500 so that rows with less than 500 reads are eliminated. The row sum threshold T_{RS} may be set in a range between 100 and 1000. The same row sum threshold T_{RS} is applied to the sum for each row. Since each row corresponds to a species and strain, the eliminated rows correspond to species and strains that are eliminated from further consideration.

In step 306, a second reduction of the read count matrix combines the read counts of the rows based on the expected signatures determined from the in silico simulations, such as the expected signatures given in the examples of Matrix A or Matrix B. For species and strains (rows) having identical expected signatures, the read counts per hypervariable region (column) of the same species are added to give a column sum of read counts corresponding to a single species. For example, in Matrix B the expected signatures for species C, seq. # 1 and seq. # 2 are identical. Matrix C gives the read count matrix for row sums > T_{RS} for species C, seq. # 1 and seq. # 2. The read counts per hypervariable region (column) for species C, seq. # 1 and seq. # 2 may be added because they correspond to identical expected signatures within the same species C in Matrix B. Note that Species D also has the same expected signature as seq. # 1 and seq. # 2 of species C, but read counts for species D will not be added to read counts for species C because of the different species. The column sums for the combined read counts may be added to form a combined sum. A threshold T_{COMB} is applied to the combined sum. If the combined sum is greater than T_{COMB} the corresponding rows of the row count matrix are retained, otherwise the corresponding rows of the row count matrix are eliminated, thus reducing the size of the row count matrix. The threshold T_{COMB} for the combined sum may be set to 10,000, for example and is configurable by the user.

A signature threshold T_{S} is applied to each of the column sums to give binary values by assigning a "1" if the column sum ≥ T_{S} and assigning "0" if the column sum < T_{S}. The resulting array of binary values provides the observed signature. For example, the column sum threshold T_{S} can be set to 10.

Exemplary results of the reduction operations and observed signature are given in Matrix D, corresponding to the examples of Matrix B and Matrix C. In this example, seq. #1 and seq. #2 of species C are retained because the combined sum is greater than the combined threshold T_{COMB} of 10,000.

### MATRIX D

| SPECIES | SEQ . # | V 2 | V 2 | V2 | V3 | V 4 | V 5 | V 6 | V 7 | V 8 | V8 | V8 | V9 | COMB . SUM |
|---|---|---|---|---|---|---|---|---|---|---|---|---|---|---|
| C | 1 | 5 | 10 | 20 0 | 100 0 | 0 | 0 | 0 | 1 | 2 | 10 0 | 200 0 | 40 0 | |
| C | 2 | 15 | 20 | 10 0 | 100 0 | 5 | 10 | 0 | 0 | 0 | 20 0 | 500 0 | 50 0 | |
| COLUMN SUM | | 20 | 30 | 30 0 | 200 0 | 5 | 10 | 0 | 1 | 2 | 30 0 | 700 0 | 90 0 | 10, 568 |
| OBSERVED SIGNATUR E | | 1 | 1 | 1 | 1 | 0 | 1 | 0 | 0 | 0 | 1 | 1 | 1 | |
| EXPECTED SIGNATUR E | | 0 | 1 | 1 | 1 | 0 | 0 | 0 | 0 | 0 | 1 | 1 | 1 | |

In step 308, a first reduced set of full-length 16S sequence is generated as follows. The binary values of the observed signature and corresponding expected signature (e.g. from Matrix B, species C, seq. #1 and seq. #2) are compared and the ratio of matching categories, or matching binary values, to total categories is determined. If the ratio meets a minimum threshold T_{R}, the full-length 16S rRNA gene sequences corresponding to the expected signatures in the given species are selected for a first reduced set of full-length reference sequences. Otherwise, the full-length 16S rRNA gene sequences corresponding to the expected signature in the given species are not included in the first reduced set of full-length reference sequences. The ratio threshold T_{R} may be set to 0.75, and is configurable by the user. In the example of Matrix D, the observed and expected signature's binary values agree for 10 categories out of 12 total categories, which is greater than 0.75% of the total categories (9 of the 12 categories corresponds to 75%). The full-length 16S rRNA gene sequences corresponding to seq. #1 and seq. #2 of species C are selected for a first reduced set of full-length reference sequences.

In step 310, the first reduced set of full-length 16S rRNA gene sequences may be further reduced by a reassignment of unannotated species strains based on a sequence similarity metric to form a second reduced set of full-length reference sequences. The second reduced set of full-length reference sequences is used for the second mapping step. The gene sequence for an unannotated species in the first reduced set is compared to each annotated sequence in the first reduced set having the same genus. The levenshtein distance is calculated between the unannotated sequence and the each of the annotated sequences having the same genus. The levenshtein distance is a count of differences between two sequences, including substitutions, insertions and deletions. If the levenshtein distance between an unannotated sequence and an annotated sequence in the first reduced set is less than a threshold T_{LEV} then the annotated sequence is identified as candidate annotated sequence. For example, the threshold T_{LEV} may be set to 80. The threshold T_{LEV} of 80 counts corresponds to 5% of a 16S gene length of 1600 bp. In some situations, there may be a single candidate annotated sequence. For a single candidate annotated sequence, the unannotated sequence is reannotated with the annotation of the candidate annotated sequence. In some situations, there may be multiple candidate annotated sequences associated with a given unannotated sequence. When there are multiple candidate annotated sequences, the candidate annotated sequence with the lowest levenshtein distance is selected. The given unannotated sequence is reannotated with the annotation corresponding to the selected candidate annotated sequence. When more than one candidate annotated sequence associated with the given unannotated sequence have equal levenshtein distances, the given unannotated sequence is included as is in the second reduced set of full-length reference sequences. The reannotated sequences are represented by the annotated sequences to which they were matched and the unannotated versions are removed to form the second reduced set of full-length reference sequences. The size of the first reduced set of full-length sequences is reduced by the number of previously unannotated sequences that were removed, to produce the second reduced set of full-length reference sequences.

The second reduced set of full-length reference sequences may have substantially fewer full-length 16S rRNA sequences than the original number in the database. For example, the 150,000 16S rRNA sequences in the GreenGenes database may be reduced to a few thousand full-length sequences. An advantage of the smaller size of the second reduced set of full-length reference sequences is a smaller memory requirement. Another advantage is a faster search time for the second mapping step because there are fewer full-length reference sequences to match with the sequence reads. Another advantage is that the reannotated sequences allow more species level resolution because the reannotated sequences are associated with a species level rather than a genus level for unannotated sequences. In the second mapping step, sequence reads will be associated with reannotated reference sequences that indicate a species. This results in more sequence reads mapped to a given species for improved read depth at the species level. Furthermore, the second reduced set of full-length reference sequences are more likely to match the sequence reads in the second mapping step, since they are determined based on the observed read counts resulting from the first mapping step.

In a second mapping step, 312, the sequence reads are mapped to the second reduced set of full-length 16S rRNA gene sequences. In step 314, one best hit per sequence read is counted (i.e., multi-mapping is disabled). In a first normalizing step, 316, the read count for each 16S reference sequence obtained after the second mapping step, 312 is normalized by dividing the read count by the number of 1's in the expected signature to form first normalized counts. For the example of Matrix D, the expected signature has six 1's and corresponds to two 16S reference sequences for Species C. The read counts for the each of the 16S reference sequences corresponding to the same expected signature for species C are divided by six to give the corresponding first normalized counts. In a second normalizing step, 318, the first normalized counts are divided by an average copy number of the 16S gene for the species to form second normalized counts. The copy numbers for the species may be obtained from a 16S copy number database, such as rrnDB (https://rrndb.umms.med.umich.edu/; Stoddard S.F, Smith B.J., Hein R., Roller B.R.K. and Schmidt T.M. (2015) rmDB: improved tools for interpreting rRNA gene abundance in bacteria and archaea and a new foundation for future development. Nucleic Acids Research 2014; doi: 10.1093/nar/gkul201). For a given species, the copy numbers for the 16S gene given in the database records may be averaged to form the average copy number used for the second normalizing step 318. The second normalizing step 318 may be optional.

In step 320, the second normalized counts are aggregated, or added, for the species level, genus level and family level. The percentage of aggregated counts to the total number of mapped reads is calculated and thresholds may be applied for species detection, genus detection and family detection to give relative abundances if the threshold criteria are met. The species, genus and/or family may be reported as present if the percentage value is greater than the respective threshold. For example, the thresholds applied may be set to the values shown in the threshold table below. The thresholds can be set by the user. The threshold for detection may also be referred to as a noise threshold. In step 322, a report of the relative abundance at the species/genus/family level may be reported to the user.

**TABLE E. Example Thresholds**

| AGGREGATED COUNTS / TOTAL MAPPED READS (%) | THRESHOLD |
|---|---|
| SPECIES | 0.1% |
| GENUS | 0.5% |
| FAMILY | 1.0% |

The above methods may provide greater than 90% sensitivity at the genus level and greater than 85% PPV at the genus level. The threshold may be used to call respective species, genus or family having the percent of aggregated counts/mapped reads above the threshold as existing in a sample. This threshold may be adjusted by user to optimize for sensitivity and specificity according to the user's application.

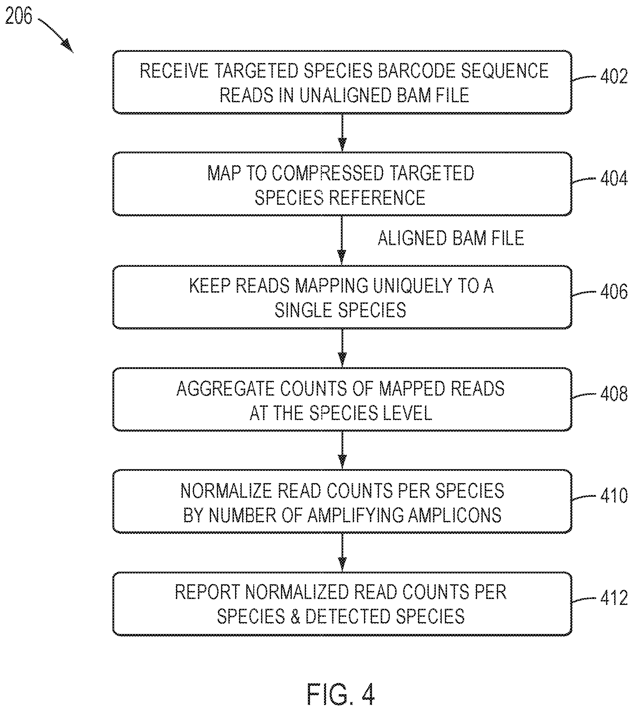

Referring to FIG. 2, the reads from the amplicons generated using the species primer pool are separately analyzed by the targeted species analysis pipeline 206. Prior to mapping step 404 in FIG 4, the microbial genome database, e.g. the NCBI public database, may be pre-processed to provide segmented reference sequences corresponding to expected amplicons. In this pre-processing, the microbial genome sequences in the database may be subjected to an in silico PCR simulation conducted using primers of the species primer pool to generate expected amplicon (primers + inserts) sequences from the whole genomes of all microbial strains in the database. The in silico PCR results identify genomes in the database that contain sequences that will be amplified by the primers in the species primer pool. The in silico simulation provides segmented reference sequences corresponding to the expected amplicons for the targeted species and possibly off-target species. Any genomes that do not contain sequence that would be amplified by the species primers were eliminated from the database. Any genomes that contain sequence that would be amplified using the species primers but would not be expected to contain such sequence were evaluated to determine the average nucleotide identity (ANI) between the genome and a genome that was expected to be amplified by the primers to assess possible misclassification and reannotation of the genome, and retainment of the genome in the database. For possible misclassified genomes, histograms of identities with known strains were created. A genome was reclassified only if it had greater than 95% identity to the known genome to which it was being reclassified. The segmented reference sequences may be generated once for a given set of primers and applied in multiple experiments. The segmented reference sequences may be used instead of the full-length reference genomes for the species in the mapping step. A compressed reference database for the targeted species includes the segmented reference sequences for each strain, including any reannotated strains. For example, a segmented reference sequence for a strain may include 1 to 8 segments and each segment may include 6 to 300 bases, giving a total number of at most a few thousand bases. For example, a full-length reference genome for a strain of E. coli may include 4.6 to 5.3 Mbases.

FIG. 4 is a block diagram of the targeted species processing pipeline, in accordance with an embodiment. In step 402, the targeted species sequence reads identified by the barcode/sample name parser 202 are received in an unaligned BAM file. Following pre-processing of the reference database, in the mapping step 404 the targeted species sequence reads are mapped to the to the segmented reference sequences for the species and strains in the compressed targeted species reference, with end-to-end mapping enabled. The mapped reads are filtered based on alignment quality. For example, a minimum local alignment score may be set to 25. In step 406, those reads that uniquely mapped to a single species (either uniquely to one segmented reference sequence for a single strain of a species or to multiple segmented reference sequences for multiple strains of the same species) are included in a read count. Matrix E gives examples of reads W, X, Y and Z mapping to strains of species A, B and C.

### MATRIX E

| SPECIES | SEQ. # | READ W | READ X | READ Y | READ Z |
|---|---|---|---|---|---|
| A | 1 | MAPPED | | | |
| A | 2 | MAPPED | MAPPED | | |
| B | 1 | | MAPPED | | |
| B | 2 | | | | MAPPED |
| C | 1 | | | MAPPED | |
| C | 2 | | | MAPPED | |

In the example of Matrix E, reads W, Y and Z would be included in the read counts and read X would not be included in read counts. Reads W and Y mapped to segmented reference sequences corresponding to two strains of the same species, A and C, respectively. Read Z mapped to a single strain of a segmented reference sequence for a strain of species B. Read X mapped to a strain of species A and also to a strain of species B, so it will not be counted.

In step 408, the number of sequence reads mapped to segmented reference sequences of a species, including strains of the species, are calculated to form an aggregate read count per species. In step 410, the aggregate read count per species is, normalized by the number of amplifying amplicons for the species. An amplifying amplicon has a minimum number of mapped reads. For example, the minimum number of mapped reads can be set to 10. Dividing the aggregate read count per species by the number of total amplifying amplicons for the species gives a normalized read count per species. The normalized read counts across all species are added to form a total of normalized read counts. The normalized read count per species is divided by the total of normalized read counts to form a ratio of normalized read counts. The ratio of normalized read counts for a species may be compared to a threshold T_{target} to decide whether a species was present in the sample. For example, the threshold T_{target} may be set to 0.1% and a species may be determined as present if the ratio of normalized read counts is greater than 0.1%. The threshold T_{target} may be set by the user. In step 412, the results of the normalized read counts for each species and the detected species may be reported to the user.

### Methods for Detecting, Diagnosing, Preventing and/or Treating Microorganism Imbalances

Also described herein are methods for detection, diagnosis, prevention and/or treatment, reduction in symptoms, and/or prevention of microorganism (e.g., bacteria) imbalances and/or dysbiosis in a subject as well as of conditions, disorders and diseases associated therewith. In some examples, the subject is an animal, for example, an insect or a mammal, such as a domestic or agricultural mammal or a human. In some instances, the microorganism imbalance and/or dysbiosis is in the alimentary canal, or gastrointestinal tract (often referred to as the "gut microbiota"), of the subject. The gut microbiota includes a diverse population of bacteria having a symbiotic relationship with a subject. The majority of bacteria in the gut microbiota are from seven phyla: Firmicutes, Bacteroidetes, Proteobacteria, Fusobacteria, Verrucomicrobia, Cynaobacteria and Actinobaceria, with greater than 90% of the bacteria human gut microbiota being from the Firmicutes and Bacteroidetes phyla. The composition of the gut microbiota is associated with and/or plays a role in a number of conditions, as well as the state of health or disease of animal subjects, and contributes to the development of disorders including, for example, irritable bowel syndrome (IBS), inflammatory bowel disease (IBD) and obesity, and autoimmune disorders such as celiac disease, lupus and rheumatoid arthritis (RA). Additionally, the composition of the gut microbiome may influence susceptibility to oncological conditions, such as cancer, and responsiveness to cancer therapies. For example, the composition of the gut microbiome has been implicated as a biomarker for cancer immunotherapies, including, for example, immune checkpoint inhibitors. Methods of detecting, diagnosing, preventing and/or treating a condition relating to microorganism imbalances and/or dysbiosis provided herein can include detecting, measuring, characterizing, profiling, assessing and/or monitoring the bacterial composition of the microbiota of a subject. In some examples, methods of detecting, diagnosing, preventing and/or treating a condition provided herein are based on detecting, measuring, characterizing, profiling, assessing and/or monitoring the bacterial composition of the microbiota of the alimentary tract which has been associated with conditions, diseases and disorders affecting animal health and responsiveness to therapies.

In some examples, a method provided herein of detecting and/or diagnosing an imbalance of microorganisms or dysbiosis in a subject includes amplifying nucleic acids in or from a sample from the subject, obtaining sequence information of the nucleic acid amplification products, and, optionally determining the levels of nucleic acid amplification products, determining the microorganism composition of the sample by identifying genera of microorganisms in the sample, and optionally the relative levels thereof, and species of one or more of the microorganisms in the sample, comparing the microorganism composition of the sample to a reference microorganism composition, and detecting an imbalance of microorganisms in the subject if the level of one or more microorganisms in the sample differ from the level of the microorganism(s) in the reference microorganism composition, one or more microorganisms in the reference composition is not present in the sample, and/or one or more microorganisms present in the sample is not present in the reference microorganism composition. In some examples of the method, the sample from the subject is a sample from the alimentary canal of the subject, e.g., a fecal sample. In some examples, a reference microorganism composition comprises a bacterial population (representative types and relative levels of bacteria) characteristic of the microbiota of a normobiotic subject. A normobiotic subject is healthy and does not have a microorganism (e.g., bacteria) imbalance and thus is in a state of normobiosis, as opposed to dysbiosis. Typically, in a normobiotic state, microorganisms with a potential health benefit predominate in number over potentially harmful microorganisms in the microbiota. An imbalance of one or more microorganisms in the microbiota of a subject can also be relative to the composition of microorganisms in a reference microbiota of the same subject when not in a state of imbalance or dysbiosis or when in a healthy state free of a disorder, disease, condition and/or symptoms of an unhealthy state associated with an imbalance or dysbiosis. An imbalance of one or microorganisms in a subject's microbiota can be relative to the average levels of the microorganism(s) typically present in any subject who is not in a state of imbalance or dysbiosis or who is in a healthy state free of a disorder, disease, condition and/or symptoms of an unhealthy state associated with an imbalance or dysbiosis. Significant deviations in the types and relative levels of constituent bacteria in a subject's microbiota from those of a bacterial population of a normobiotic subject is indicative of dysbiosis. Furthermore, the composition of different types of bacteria, and levels thereof, in the microbiota of a subject having an imbalance of microorganisms (i.e., the microbiota profile of the subject) can be indicative of susceptibility to or the occurrence of a particular condition, disorder or disease. Thus, a comparison of the bacterial constituents, and relative levels thereof, in the microbiota of a subject having an imbalance of microorganisms to microbiota profiles characteristic of certain disorders and diseases can be a consideration in diagnosing a related disorder or disease of the subject. For example, bacteria that may contribute to dysbiosis in gut microbiota in irritable bowel syndrome (IBS) include Firmicuties, Proteobacteria (*Shigella* and *Escherichia*), Actinobacteria and *Ruminococcus gnavus,* whereas bacteria that may contribute to dysbiosis in gut microbiota in inflammatory bowel disease (IBD) include Proteobacteria (*Shigella* and *Escherichia*), Firmicuties (specifically *F. prausnitzii*) and Bacteroidetes (*Bacteroides* and *Prevotella*) (see, e.g., Casen et al. (2015) Aliment Pharmacol Ther 42:71-83). Typically, a reduction in the diversity of the gut microbiota occurs in IBD which includes an expansion of pro-inflammatory bacteria (e.g., *Enterobacteriaceae* and *Fusobacteriaceae*) and a reduction in phyla with anti-inflammatory properties (e.g., Firmicuties). In another example, *Desulfococcus, Enterobacter, Prevotella* and *Veillonella* may be increased in gut microbiota in primary hepatocellular carcinoma compared to healthy controls (see, e.g., Ni et al. (2019) Front Microbiol Volume10 Article 1458). In some examples of the methods provided herein of detecting and/or diagnosing an imbalance of microorganisms or dysbiosis in a subject, an imbalance of microorganisms in the subject is detected if the level (relative and/or absolute) of one or more microorganisms differs from the level of one or more microorganisms in the reference microorganism composition. In some examples, an imbalance of microorganisms in the subject is detected if one or more microorganisms in the reference composition is not present in the sample, and/or one or more microorganisms present in the sample is not present in the reference microorganism composition. In some examples, the relative level of one or more microorganisms in a sample from a subject is determined by counting the number of sequence reads for nucleic acid products amplified from nucleic acids in the sample and normalizing the sequence read counts as described herein.

Also described herein are methods of treating an imbalance of microorganisms or dysbiosis in a subject. In some examples of treating a subject having a microorganism imbalance or dysbiosis, a subject who has a disproportionate level of one or more microorganisms is treated to establish a balance of microorganisms or biosis in the subject. In some examples, a method provided herein of treating a subject having an imbalance of microorganisms or dysbiosis includes amplifying nucleic acids in or from a sample from the subject, obtaining sequence information of the nucleic acid amplification products, and, optionally determining the levels of nucleic acid amplification products, determining the microorganism composition of the sample by identifying genera of microorganisms in the sample, and optionally the relative levels thereof, and species of one or more of the microorganisms in the sample, detecting an imbalance of microorganisms in the subject and treating the subject to establish a balance of microorganisms or biosis (or normobiosis) in the subject. In some examples of the method, the sample from the subject is a sample from the alimentary canal of the subject, e.g., a fecal sample. In some examples, detecting an imbalance of microorganisms includes comparing the microorganism composition of the sample to a reference microorganism composition, and detecting an imbalance of microorganisms in the subject if the level of one or more microorganisms in the sample differ from the level of the microorganism(s) in the reference microorganism composition, one or more microorganisms in the reference composition is not present in the sample, and/or one or more microorganisms present in the sample is not present in the reference microorganism composition. In some examples, treating the subject to establish a balance of microorganisms in the subject includes, but is not limited to, administering to the subject microorganisms, e.g., bacteria, that are under-represented in or absent from the microbiota, creating conditions unfavorable to the survival or growth of a microorganism over-represented in the microbiota and/or creating conditions favorable to the survival or growth of a microorganism under-represented in or absent from the microbiota. For example, in the case of a microorganism imbalance or dysbiosis of microbiota of the alimentary tract, administering bacteria to a subject may include ingestion of probiotics, which are live microorganisms that are typically delivered in food. Another technique for administering bacteria to a subject is fecal microbial transplantation, typically through transcolonoscopic infusion, of a population of bacteria from a healthy donor to supplant the imbalanced microbiota of the subject (see, e.g, van Nood et al (2014) Curr Opin Gastroenterol 30(1):34-39*).* Creating conditions favorable to the survival and/or growth of a microorganism in a subject's microbiota include, for example, administration of prebiotics. Prebiotics are compositions, typically delivered as food ingredients or supplements, that selectively stimulate growth and/or activities of one or a select group of microorganisms and include, for example, inulin-type fructans (ITF) and galactooligosaccharides (GOS). Prebiotics such as ITF and GOS have been shown to have growth-promoting effects on *Bifidobacteria* and *Lactobacilli.* Creating conditions unfavorable to the survival and/or growth of a microorganism in a subject's microbiota include, for example, administration of antibiotics, e.g., rifaximin, and altering diet to eliminate or reduce intake of compositions that are favorable to growth of certain microorganisms, such as sulfates, animal proteins and refined sugars. Any of these and other possible interventions useful for establishing biosis or gut microorganism homeostasis (see, e.g., Bull and Plummer (2015) Integrative Medicine 14(1):25-33) can be used in treating a subject in methods described herein.

In some examples of the methods described herein for detection, diagnosis, prevention and/or treatment, reduction in symptoms, and/or prevention of microorganism (e.g., bacteria) imbalances and/or dysbiosis in a subject as well as of conditions, disorders and diseases associated therewith, the step of amplifying nucleic acids in or from a sample from the subject includes (a) subjecting nucleic acids in or from a sample from the subject to nucleic acid amplification using a combination of primer pairs comprising (i) one or more primer pairs capable of amplifying nucleic acid sequences of one or more hypervariable regions of a prokaryotic 16S rRNA gene (referred to as the "16S rRNA gene primers or primer pairs") and (ii) one or more primer pairs capable of amplifying a target nucleic acid sequence contained within the genome of a microorganism that is not contained within a hypervariable region of a prokaryotic 16S rRNA gene, wherein different primer pairs amplify different target nucleic acid sequences contained within the genome of different microorganisms (referred to as the "non-16S rRNA gene primers or primer pairs"). In some examples, obtaining sequence information from amplified nucleic acid products comprises obtaining sequence information from nucleic acid products amplified by the combination of primer pairs of (i) and (ii), and optionally determining the levels of nucleic acid products amplified by the one or more primer pairs of (i). In some examples, the method includes determining levels, e.g. relative and/or absolute levels, of nucleic acid products amplified by one or more primer pairs of (i), i.e., the 16S rRNA gene primer pairs, and/or (ii), i.e., the non-16S rRNA gene primer pairs, or sequence reads thereof. In some examples, the step of amplifying nucleic acids in or from a sample from the subject includes includes (a) subjecting the nucleic acids to two or more separate nucleic acid amplification reactions using a first set of primer pairs for one nucleic acid amplification reaction and a second set of primer pairs for the other nucleic acid amplification reaction, wherein (i) the first set of primer pairs comprises one or more primer pairs that amplifies a nucleic acid sequence of one or more hypervariable regions of a prokaryotic 16S rRNA gene (referred to as the "16S rRNA gene primers or primer pairs") and (ii) the second set of primer pairs comprises one or more primer pairs that amplify a target nucleic acid sequence contained within the genome of a microorganism that is not contained within a hypervariable region of a prokaryotic 16S rRNA gene, wherein different primer pairs amplify different target nucleic acid sequences contained within the genome of different microorganisms (referred to as the "non-16S rRNA gene primers or primer pairs"), and obtaining sequence information comprises obtaining sequence information from nucleic acid products amplified by primer pairs of (i) and (ii). In some examples, the method includes determining levels, e.g. relative and/or absolute levels, of nucleic acid products amplified by one or more primer pairs of (i) and/or (ii) or sequence reads thereof.

In any examples of the methods provided herein for detection, diagnosis, prevention and/or treatment, reduction in symptoms, and/or prevention of microorganism (e.g., bacteria) imbalances and/or dysbiosis in a subject as well as of conditions, disorders and diseases associated therewith, the methods can include any examples of the methods for detecting and/or measuring the presence or absence of one or more microorganisms in a sample as described herein. For example, in some examples, the microorganism(s) is/are bacteria. In some examples, the prokaryotic 16S rRNA gene is a bacterial gene and/or the microorganism is a bacterium. In some examples the target nucleic acid sequence contained within a genome of a microorganism, e.g., bacteria, is unique to the microorganism. In some examples, the one or more 16S rRNA gene primer pairs amplify a nucleic acid sequence in a plurality of microorganisms, e.g., bacteria, from different genera. In some examples, the sample is a sample of contents of the alimentary tract of an animal. In some examples, the sample is a fecal sample.

In any examples of the methods provided herein for detection, diagnosis, prevention and/or treatment, reduction in symptoms, and/or prevention of microorganism (e.g., bacteria) imbalances and/or dysbiosis in a subject as well as of conditions, disorders and diseases associated therewith, a nucleic acid amplification can be performed according to any of the examples provided herein for such amplification. For example, in some examples, the one or more primer pairs that amplifies a nucleic acid containing a sequence of a hypervariable region of a prokaryotic 16S rRNA gene separately amplify nucleic acids containing sequences of different hypervariable regions. In some examples, the primers of the one or more 16S rRNA gene primer pairs are directed to, or bind to, or hybridize to nucleic acid sequences contained in conserved regions of a prokaryotic 16S rRNA gene. In some examples, the one or more 16S rRNA gene primer pairs and/or non-16S rRNA gene primer pairs comprise a plurality of primer pairs. For example, the one or more 16S rRNA gene primer pairs of can comprise a plurality of primer pairs that amplify nucleic acid sequences of multiple hypervariable regions of a prokaryotic 16S rRNA gene and/or the one or more non-16S rRNA gene primer pairs can comprise a plurality of primer pairs that amplify different target nucleic acid sequences contained in the genomes of a plurality of different microorganisms. In some examples, the amplification, or two or more separate nucleic acid amplification reactions, is/are multiplex amplification conducted in a single reaction mixture. In some examples, each primer of the one or more 16S rRNA gene primer pairs contains less than 10, less than 9, less than 8, less than 7, less than 6, less than 5, less than 4, less than 3, or less than 2 contiguous nucleotides of sequence identical to a sequence of contiguous nucleotides of another primer in the combination of primer pairs. In some examples, the nucleic acid sequences being amplified by the one or more 16S rRNA gene primer pairs are less than about 300 bp, less than about 250 bp, less than about 200 bp, less than about 175 bp, less than about 150 bp, or less than about 125 bp in length. In some examples, the 16S rRNA gene primer pairs separately amplify nucleic acids separately containing sequences of 3 or more, 4 or more, 5 or more, 6 or more, 7 or more, 8 or more or 9 different hypervariable regions of a prokaryotic 16S rRNA gene thereby producing amplified copies of the nucleic acids containing sequences of the 3 or more, 4 or more, 5 or more, 6 or more, 7 or more, 8 or more or 9 different hypervariable regions of the 16S rRNA gene of one or more microorganisms, wherein the amplified copies of different hypervariable regions are separate amplicons. In some examples, the 16S rRNA gene primer pairs separately amplify nucleic acids containing sequences of 8 different hypervariable regions of a prokaryotic 16S rRNA gene. In some examples, the 8 different hypervariable regions are V2-V9. In some examples, the 16S rRNA gene primer pairs separately amplify nucleic acids containing sequences of 3 or more different hypervariable regions of a prokaryotic 16S rRNA gene wherein one of the 3 or more regions is a V5 region thereby producing amplified copies of the nucleic acids containing sequences of the 3 or more hypervariable regions of the 16S rRNA gene of one or more microorganisms. In some examples, the combination of primer pairs includes degenerate sequences of one or more primers in one or more primer pairs. In some examples, the 16S rRNA gene primer pair(s) comprise primers and/or primer pairs containing, or consisting essentially of, a sequence or sequences of a primer or primer pair in Table 15, or SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base.

In some examples of the methods provided herein for detection, diagnosis, prevention and/or treatment, reduction in symptoms, and/or prevention of microorganism (e.g., bacteria) imbalances and/or dysbiosis in a subject as well as of conditions, disorders and diseases associated therewith, the one or more non-16S rRNA gene primer pairs specifically amplifies a target nucleic acid sequence contained within a genome of a microorganism selected from the microorganisms of Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the target nucleic acid is unique to the microorganism. In some such examples, amplified copies of nucleic acids from a plurality of different microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis,* is produced. In some examples, at least one, or one or more, target nucleic acid sequence(s) comprises or consists essentially of a nucleotide sequence selected from the nucleotide sequences of SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, or the complement thereof. In some examples, at least one, or one or more, target nucleic acid sequences comprises a nucleotide sequence selected from the sequences of SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, or the complement thereof and is less than about 500, less than about 475, less than about 450, less than about 400, less than about 375, less than about 350, less than about 300, less than about 275, less than about 250, less than about 200, less than about 175, less than about 150, or less than about 100 nucleotides in length. In some examples, at least one, or one or more, product(s) of the nucleic acid amplification comprises, or consists essentially of, a nucleotide sequence selected from SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, or the complement thereof, and optionally having one or more primer sequences at the 5' and/or 3' end(s) of the sequence, such as any of the primer sequences provided herein, and is less than about 500, less than about 475, less than about 450, less than about 400, less than about 375, less than about 350, less than about 300, less than about 275, less than about 250, less than about 200, less than about 175, less than about 150, or less than about 100 nucleotides in length. In some examples, the at least one non-16S rRNA gene primer pair does not detectably amplify a nucleic acid sequence contained within any genus other than the genus of the microorganism containing the target nucleic acid sequence. In some examples, the at least one non-16S rRNA gene primer pair does not detectably amplify a nucleic acid sequence contained within any species other than the species of the microorganism containing the target nucleic acid sequence. In some examples, at least one primer of the non-16S rRNA gene primer pair, or at least one non-16S rRNA gene primer pair, contains, or consists essentially of, the sequence or sequences of a primer or primer pair in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the nucleic acids are subjected to nucleic acid amplification using a plurality of non-16S rRNA gene primers or primer pairs, each containing, or consisting essentially of, a sequence or sequences of a primer pair in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, at least one primer or one primer pair in the combination of primer pairs includes a modification that facilitates nucleic acid manipulation, amplification, ligation and/or sequencing of amplification products and/or reduction or elimination of primer dimers. In particular examples, a modification is one that facilitates multiplex nucleic acid amplification, ligation and/or sequencing of products of multiplex amplification.

In some examples of the methods provided herein for detection, diagnosis, prevention and/or treatment, reduction in symptoms, and/or prevention of microorganism (e.g., bacteria) imbalances and/or dysbiosis in a subject as well as of conditions, disorders and diseases associated therewith, the method is designed to focus on the make-up or composition of the population of microorganisms in the sample particularly with respect to certain groups of microorganisms, and, in some examples, the proportionate presence of the group and/or group members in the population. In such examples, the combination of primers and/or primer pairs includes a selected group or sub-group of microorganism-specific nucleic acids and includes kingdom-encompassing nucleic acids (e.g., 16S rRNA gene primers and primer pairs), and enables a focus on one or more particular microorganisms of interest that may be significant in certain states of health and disease or microbiota imbalance. In some examples, combinations of nucleic acids include microorganism-specific nucleic acids, and/or primer pairs, that specifically amplify a nucleic acid sequence contained in the genome of one or more microorganisms (e.g., bacteria) implicated in one or more conditions, disorders and/or diseases. In particular examples, the combination of nucleic acid primers and/or primer pairs includes a nucleic acid and/or a primer pair that specifically amplifies a target nucleic acid sequence contained within a genome of a microorganism selected from the microorganisms in Table 1. In some examples, the combination includes a plurality of nucleic acids and/or primer pairs that include at least one nucleic acid primer pair that specifically amplifies a target nucleic acid in each of the microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the plurality of primer pairs includes primer pairs that specifically amplify genomic target nucleic acids contained within at least 5, 10, 15, 20, 25, 30, 35, 40, 45, 50, 55, 60 or 70 of the microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In particular examples, the target nucleic acid sequences contained in the genome of the different microorganisms are unique to each of the microorganisms. In some examples, a combination of nucleic acids and/or nucleic acid primer pairs includes two or more nucleic acids and/or nucleic acid primer pairs that specifically amplify a unique nucleic acid sequence contained in the genome of one or more of the Group A microorganisms, the Group B microorganisms, the Group C microorganisms, the Group C microorganisms excluding *Helicobacter salomonis* (Subgroup 1 of Group C), the Group D microorganisms or the Group E microorganisms (see Table 2). In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group A, Group B, Group C, Subgroup 1 of Group C (the Group C microorganisms excluding *Helicobacter salomonis*), Group D or Group E.

In some examples of the methods provided herein for detection, diagnosis, prevention and/or treatment, reduction in symptoms, and/or prevention of microorganism (e.g., bacteria) imbalances and/or dysbiosis in a subject as well as of conditions, disorders and diseases associated therewith, the method involves utilization of nucleotide sequence information of amplification products. Such examples include obtaining sequence information from nucleic acid products amplified by the combination of primer pairs employed in the method. Examples of sequence information are provided herein and include, but are not limited to, the identities of the nucleotides and the order thereof in a contiguous polynucleotide sequence (the nucleotide sequence determination) of an amplification product (which can include, for example, barcode sequence, e.g., corresponding to amplicon library source, primers used, etc.), alignment and/or mapping of nucleotide sequence to a reference sequence (and identity of the genus or species of the reference sequence), the number of sequence reads that map to a reference sequence and/or portions thereof (e.g., hypervariable regions of a 16S rRNA gene), the number of sequence reads that map uniquely to a reference sequence, and the number of regions (e.g., target sequences) of a reference sequence to which sequence reads map. Exemplary methods of sequencing nucleic acids and of nucleotide sequence analysis workflows for aligning and/or mapping sequence reads of amplification products are provided herein. In some examples, sequence information provides the identities (e.g., genus, species) of microorganisms and/or the number of different microorganisms in a population which is used to determine the microorganism composition of the sample. In some examples, as described herein, sequence information provides a measure of the levels (e.g., relative and/or absolute) of microorganisms in a population (abundance and proportionate contribution or presence in a population), which can also be used in determining the microorganism composition of the sample.

Also described herein are methods for treating a subject with an immunotherapy. The composition of the gut microbiome has been implicated as a biomarker for cancer immunotherapies, including, for example, immune checkpoint inhibitors and CpG-oligonucleotide (CpG-ODN) immunotherapy. CpG-oligonucleotides are short single-stranded DNA molecules containing unmethylated cytosine-guanine motifs that serve as vaccine adjuvants in promoting antigen-specific immune responses, such as tumor antigen-specific cytotoxic T lymphocyte activation and accumulation. Immune checkpoint inhibitors are cancer therapeutics that target and inhibit checkpoint pathways of immune cells (e.g., T cells) involved in immunosuppression and particularly suppression of antitumor immune responses. Examples of checkpoint pathway proteins include, but are not limited to, PD-1, PD-L1 and CTLA-4. Checkpoint inhibitors include therapeutics that bind to these proteins, such as monoclonal antibodies directed to the proteins, and disrupt or prevent interaction of the proteins with other proteins. The composition of the gut microbiome has been shown to correlate with response to immune checkpoint inhibitors (see, e.g, Gong et al (2019) *Clin Trans Med 8: 9;* https://doi.org/10.1186/s40169-019-0225-x) and CpG-oligonucleotide (CpG-ODN) immunotherapy (see, e.g., Lida et al (2013) Science 342(6161):967-970) and thus is a potential predictor of response to such immunotherapies. Particular species associated with checkpoint inhibitor response include, for example, *Alistipes indistinctus, Anaerococcus vaginalis, Akkermansia muciniphila, Atopobium parvulum, Bacteroides caccae, Bacterioides fragilis, Bacteroides nordii, Bacteroides thetaiotamicron, Bacteroides vulgatus. Bifidobacterium adolescentis, Bifidobacterium breve, Bifidobacterium longum, Blautia obeum, Burkholderia cepacia, Cloacibacillus porcorum, Collinsella aerofaciens, Collinsella stercoris, Desulfovibrio alaskensis, Dorea formicigenerans, Enterococcus faecium, Enterococcus hirae, Eubacterium* spp., *Faecalibacterium prausnitzii, Gardnerella vaginalis, Gemmiger formicilis, Holdemania filiformis, Klebsiella pneumoniae, Lactobacillus* spp., *Parabacteroides merdae, Parabacteroides distasonis, Phascolarctobacterium faecium, Prevotella histicola, Roseburia intestinalis, Ruminococcus bromii, Slackia exigua Streptococcus infantarius Streptococcus parasanguinis and Veillonella parvula.* For example, specific microbes that have been positively correlated with response to checkpoint inhibition by inhibitors of CTLA-4 include *Bacteroides* spp. and *Burkholderia* spp. In another example, specific microbes that have been positively correlated with response to checkpoint inhibition by inhibitors of interaction of PD-L1 and PD-1 include *Bifidobacterium* spp., *Faecalibacterium* spp., and *Ruminococcaceae* family (particularly for inhibitors targeting PD-L1), and *Akkermansia muciniphila, Alistipes indistinctus* and *Enterococcus hirae* (particularly for inhibitors targeting PD-1). Microbes that have been negatively associated with response to checkpoint inhibition by inhibitors of PD-1 and/or CTLA-4 include Bacteroidales order (including *Bacteroides* ssp., e.g., *Bacteroides thetaiotamicron*), *Escherichia coli, Anaerotruncus colihominis* and *Roseburia intestinalis.*

In some examples, methods for treating a subject with an immunotherapy described herein include amplifying nucleic acids in or from a sample from the subject, obtaining sequence information of the nucleic acid amplification products, identifying genera of microorganisms in the sample and species of one or more of the microorganisms in the sample, and treating the subject with an immunotherapy or a composition that increases or decreases levels of one or more microorganisms in the sample and an immunotherapy. In some examples, the subject is treated with an immune checkpoint inhibition-based immunotherapy if the sample includes one or more microorganisms positively associated with response to immune checkpoint inhibition-based immunotherapy and/or excludes or has sufficiently low levels of one or more microorganisms negatively associated with response to immune checkpoint inhibition-based immunotherapy. A sufficiently low level of a microorganism negatively associated with response to immune checkpoint inhibition-based immunotherapy is a level that does not substantially or significantly interfere with or reduce a response to the immune checkpoint inhibition-based immunotherapy. In some examples, the subject is treated with a composition that increases levels of one or more microorganisms positively associated with response to immune checkpoint inhibition-based immunotherapy if the sample lacks one or more such microorganisms or sufficient levels thereof and/or a composition that eliminates or reduces levels of one or more microorganisms negatively associated with response to immune checkpoint inhibition-based immunotherapy if the sample contains one or more such microorganisms or prohibitively high levels thereof and is subsequently or simultaneously treated with an immune checkpoint inhibition-based immunotherapy. A less than sufficient level of a microorganism positively associated with response to immune checkpoint inhibition-based immunotherapy is a level that is insufficient to provide for a response to the immunotherapy. A prohibitively high level of a microorganism negatively associated with response to immune checkpoint inhibition-based immunotherapy is a level that substantially or significantly interferes with or reduces a response to the immune checkpoint inhibition-based immunotherapy. In some examples, the immune checkpoint inhibition-based immunotherapy is a composition that disrupts or prevents interaction of a checkpoint inhibitor pathway protein, including, for example, but are not limited to, PD-1, PD-L1 and/or CTLA-4. In some examples, an immune checkpoint inhibition-based immunotherapy includes an antibody, such as a monoclonal antibody, directed to a checkpoint inhibitor pathway protein. In some examples of the method, the sample from the subject is a sample from the alimentary canal of the subject, e.g., a fecal sample. In some examples, the method further comprises determining the relative level of one or more microorganisms in a sample from a subject by counting the number of sequence reads for nucleic acid products amplified from nucleic acids in the sample and normalizing the sequence read counts as described herein.

In some examples of the methods described herein for treating a subject with an immunotherapy, the step of amplifying nucleic acids in or from a sample from the subject includes (a) subjecting nucleic acids in or from a sample from the subject to nucleic acid amplification using a combination of primer pairs comprising (i) one or more primer pairs capable of amplifying nucleic acids containing sequences of one or more hypervariable regions of a prokaryotic 16S rRNA gene (referred to as the "16S rRNA gene primers or primer pairs") and (ii) one or more primer pairs capable of amplifying a target nucleic acid sequence contained within the genome of a microorganism that is not contained within a hypervariable region of a prokaryotic 16S rRNA gene, wherein the microorganism is one that is positively or negatively associated with response to immune checkpoint inhibition-based immunotherapy (referred to as the "non-16S rRNA gene primers or primer pairs"). In some examples, obtaining sequence information from amplified nucleic acid products in the method comprises obtaining sequence information from nucleic acid products amplified by the combination of primer pairs of (i) and (ii), and optionally determining the levels of nucleic acid products amplified by the one or more primer pairs of (i). In some examples, the method includes determining levels, e.g., relative and/or absolute levels, of nucleic acid products amplified by one or more primer pairs of (i), i.e., the 16S rRNA gene primer pairs, and/or (ii), i.e., the non-16S rRNA gene primer pairs, or sequence reads thereof. In some examples, the step of amplifying nucleic acids in or from a sample from the subject includes (a) subjecting the nucleic acids to two or more separate nucleic acid amplification reactions using a first set of primer pairs for one nucleic acid amplification reaction and a second set of primer pairs for the other nucleic acid amplification reaction, wherein (i) the first set of primer pairs comprises one or more primer pairs that amplifies a nucleic acid containing a sequence of one or more hypervariable regions of a prokaryotic 16S rRNA gene (referred to as the "16S rRNA gene primers or primer pairs") and (ii) the second set of primer pairs comprises one or more primer pairs that amplify a target nucleic acid sequence contained within the genome of a microorganism that is not contained within a hypervariable region of a prokaryotic 16S rRNA gene, wherein the microorganism is one that is positively or negatively associated with response to immune checkpoint inhibition-based immunotherapy (referred to as the "non-16S rRNA gene primers or primer pairs"), and obtaining sequence information comprises obtaining sequence information from nucleic acid products amplified by primer pairs of (i) and (ii). In some examples, the method includes determining levels, e.g. relative and/or absolute levels, of nucleic acid products amplified by one or more primer pairs of (i) and/or (ii) or sequence reads thereof.

In any examples of the methods described herein for treating a subject with an immunotherapy, the methods can include any examples of the methods for detecting and/or measuring the presence or absence of one or more microorganisms in a sample as described herein. For example, in some examples, the microorganism(s) is/are bacteria. In some examples, the prokaryotic 16S rRNA gene is a bacterial gene and/or the microorganism is a bacterium. In some examples the target nucleic acid sequence contained within a genome of a microorganism, e.g., bacteria, is unique to the microorganism. In some examples, the one or more 16S rRNA gene primer pairs amplify a nucleic acid sequence in a plurality of microorganisms, e.g., bacteria, from different genera. In some examples, the sample is a sample of contents of the alimentary tract of an animal. In some examples, the sample is a fecal sample.

In any examples of the methods described herein for treating a subject with an immunotherapy, a nucleic acid amplification can be performed according to any of the examples described herein for such amplification. For example, in some examples, the one or more primer pairs that amplifies a nucleic acid containing a sequence of a hypervariable region of a prokaryotic 16S rRNA gene separately amplify nucleic acids containing sequences of different hypervariable regions. In some examples, the primers of the one or more 16S rRNA gene primer pairs are directed to, or bind to, or hybridize to nucleic acid sequences contained in conserved regions of a prokaryotic 16S rRNA gene. In some examples, the one or more 16S rRNA gene primer pairs and/or non-16S rRNA gene primer pairs comprise a plurality of primer pairs. For example, the one or more 16S rRNA gene primer pairs of can comprise a plurality of primer pairs that amplify nucleic acid sequences of multiple hypervariable regions of a prokaryotic 16S rRNA gene and/or the one or more non-16S rRNA gene primer pairs can comprise a plurality of primer pairs that amplify different target nucleic acid sequences contained in the genomes of a plurality of different microorganisms. In some examples, the amplification, or two or more separate nucleic acid amplification reactions, is/are multiplex amplification conducted in a single reaction mixture. In some examples, each primer of the one or more 16S rRNA gene primer pairs contains less than 10, less than 9, less than 8, less than 7, less than 6, less than 5, less than 4, less than 3, or less than 2 contiguous nucleotides of sequence identical to a sequence of contiguous nucleotides of another primer in the combination of primer pairs. In some examples, the nucleic acid sequences being amplified by the one or more 16S rRNA gene primer pairs are less than about 300 bp, less than about 250 bp, less than about 200 bp, less than about 175 bp, less than about 150 bp, or less than about 125 bp in length. In some examples, the 16S rRNA gene primer pairs separately amplify nucleic acids separately containing sequences of 3 or more, 4 or more, 5 or more, 6 or more, 7 or more, 8 or more or 9 different hypervariable regions of a prokaryotic 16S rRNA gene thereby producing amplified copies of the nucleic acids containing sequences of the 3 or more, 4 or more, 5 or more, 6 or more, 7 or more, 8 or more or 9 different hypervariable regions of the 16S rRNA gene of one or more microorganisms, wherein the amplified copies of different hypervariable regions are separate amplicons. In some examples, the 16S rRNA gene primer pairs separately amplify nucleic acids separately containing sequences of 8 different hypervariable regions of a prokaryotic 16S rRNA gene. In some examples, the 8 different hypervariable regions are V2-V9. In some examples, the 16S rRNA gene primer pairs separately amplify nucleic acids containing sequences of 3 or more different hypervariable regions of a prokaryotic 16S rRNA gene wherein one of the 3 or more regions is a V5 region thereby producing amplified copies of the nucleic acids containing sequences of the 3 or more hypervariable regions of the 16S rRNA gene of one or more microorganisms. In some examples, the combination of primer pairs includes degenerate sequences of one or more primers in one or more primer pairs. In some examples, the 16S rRNA gene primer pair(s) comprise primers and/or primer pairs containing, or consisting essentially of, a sequence or sequences of a primer or primer pair in Table 15, or SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base.

In some examples of the methods described herein for treating a subject with an immunotherapy, the one or more non-16S rRNA gene primer pairs specifically amplifies a target nucleic acid sequence contained within a genome of a microorganism selected from genera and/or species of the microorganisms of Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis.* In some examples, the target nucleic acid is unique to the microorganism. In some such examples, amplified copies of a plurality of different microorganisms in Table 1, or Table 1, except for, or excluding, *Actinomyces viscosus* and/or *Blautia coccoides,* or Table 1, except for, or excluding, *Actinomyces viscosus, Blautia coccoides* and/or *Helicobacter salomonis,* is produced. In some examples, at least one, or one or more, target nucleic acid sequence(s) comprises or consists essentially of a nucleotide sequence selected from the nucleotide sequences in Table 17, or Table 17A and 17B, corresponding to the particular microorganisms species associated with checkpoint inhibitor response, or the complement thereof. In some examples, at least one, or one or more, target nucleic acid sequences comprises a nucleotide sequence selected from the sequences in Table 17, or Table 17A and 17B, corresponding to the particular microorganisms species associated with checkpoint inhibitor response ,or the complement thereof, and is less than about 500, less than about 475, less than about 450, less than about 400, less than about 375, less than about 350, less than about 300, less than about 275, less than about 250, less than about 200, less than about 175, less than about 150, or less than about 100 nucleotides in length. In some examples, the at least one non-16S rRNA gene primer pair does not detectably amplify a nucleic acid sequence contained within any genus other than the genus of the microorganism containing the target nucleic acid sequence. In some examples, the at least one non-16S rRNA gene primer pair does not detectably amplify a nucleic acid sequence contained within any species other than the species of the microorganism containing the target nucleic acid sequence. In some examples, at least one primer of the non-16S rRNA gene primer pair, or at least one non-16S rRNA gene primer pair, contains, or consists essentially of, the sequence or sequences of a primer or primer pair corresponding to the particular microorganisms species associated with checkpoint inhibitor response in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the nucleic acids are subjected to nucleic acid amplification using a plurality of non-16S rRNA gene primers or primer pairs, each containing, or consisting essentially of, a sequence or sequences of a primer pair corresponding to the particular microorganisms species associated with checkpoint inhibitor response in Table 16, or SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, in which one or more thymine bases is substituted with a uracil base. In some examples, at least one primer or one primer pair in the combination of primer pairs includes a modification that facilitates nucleic acid manipulation, amplification, ligation and/or sequencing of amplification products and/or reduction or elimination of primer dimers. In particular examples, a modification is one that facilitates multiplex nucleic acid amplification, ligation and/or sequencing of products of multiplex amplification.

**In** some examples of the methods described herein for treating a subject with an immunotherapy, the method is designed to focus on the make-up or composition of the population of microorganisms in the sample particularly with respect to certain groups of microorganisms, and, in some examples, the proportionate presence of the group and/or group members in the population. In such examples, the combination of primers and/or primer pairs includes a selected group or subgroup of microorganism-specific nucleic acids and includes kingdom-encompassing nucleic acids (e.g., 16S rRNA gene primers and primer pairs), and enables a focus on one or more particular microorganisms of interest that may be particularly significant in response to immunotherapies. In some examples, combinations of nucleic acids include microorganism-specific nucleic acids, and/or primer pairs, that specifically amplify a nucleic acid sequence contained in the genome of one or more microorganisms (e.g., bacteria) implicated in one or more conditions, disorders and/or diseases. In particular examples, the target nucleic acid sequences contained in the genome of the different microorganisms are unique to each of the microorganisms. In some examples, a combination of nucleic acids and/or nucleic acid primer pairs includes two or more nucleic acids and/or nucleic acid primer pairs that specifically amplify a unique nucleic acid sequence contained in the genome of one or more of the Group A microorganisms and/or the Group B microorganisms (see Table 2B). In some examples, the combination of nucleic acids and/or nucleic acid primer pairs includes a set of nucleic acid primer pairs in which each different nucleic acid primer pair specifically amplifies a different unique nucleic acid sequence contained in a different one of each of the genomes of the different microorganisms in Group A or Group B.

In some examples of the methods described herein for treating a subject with an immunotherapy, the method involves utilization of nucleotide sequence information of amplification products. Such examples include obtaining sequence information from nucleic acid products amplified by the combination of primer pairs employed in the method. Examples of sequence information are provided herein and include, but are not limited to, the identities of the nucleotides and the order thereof in a contiguous polynucleotide sequence (the nucleotide sequence determination) of an amplification product (which can include, for example, barcode sequence, e.g., corresponding to amplicon library source, primers used, etc.), alignment and/or mapping of nucleotide sequence to a reference sequence (and identity of the genus or species of the reference sequence), the number of sequence reads that map to a reference sequence and/or portions thereof (e.g., hypervariable regions of a 16S rRNA gene), the number of sequence reads that map uniquely to a reference sequence, and the number of regions (e.g., target sequences) of a reference sequence to which sequence reads map. Exemplary methods of sequencing nucleic acids and of nucleotide sequence analysis workflows for aligning and/or mapping sequence reads of amplification products are provided herein. In some examples, sequence information provides the identities (e.g., genus, species) of microorganisms and/or the number of different microorganisms in a population which is used in determining the treatment of the subject. In some examples, as described herein, sequence information provides a measure of the levels (e.g., relative and/or absolute) of microorganisms in a population (abundance and proportionate contribution or presence in a population), which can also be used in determining the treatment of the subject.

### Kits

In some examples, a kit is described for performing nucleic acid amplification comprising any one or more nucleic acid primers and/or primer pairs provided herein that comprise or consist essentially of a sequence or sequences selected from the sequences in Table 15 and Table 16. In some examples, the primers are less than about 100 nucleotides, less than about 90 nucleotides, less than about 80 nucleotides, less than about 70 nucleotides, less than about 60 nucleotides, less than about 50 nucleotides, less than about 45 nucleotides, less than about 44 nucleotides, less than about 43 nucleotides, less than about 42 nucleotides, less than about 41 nucleotides, less than about 40 nucleotides, less than about 38 nucleotides, less than about 35 nucleotides, or less than about 30 nucleotides in length. In some examples, the one or more nucleic acid primers and/or primer pairs that comprise or consist essentially of a sequence selected from the sequences in Table 15 are selected from SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base. In some examples, the one or more nucleic acid primers and/or primer pairs that comprise or consist essentially of a sequence selected from the sequences in Table 16 are selected from SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the kit contains one or more nucleic acid primer pairs comprising or consisting essentially of sequences of primer pairs selected from the sequences in Table 15 and Table 16. In some examples, the one or more nucleic acid primer pairs that comprise or consist essentially of sequences of primer pairs selected from the sequences in Table 15 are selected from SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base. In some examples, the one or more nucleic acid primer pairs that comprise or consist essentially of sequences of primer pairs selected from the sequences in Table 16 are selected from SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the kit is for performing multiplex nucleic acid amplification and comprises a plurality of primers and/or primer pairs comprising or consisting essentially of sequences selected from the sequences in Table 15 and Table 16. In some examples, the plurality of primers and/or primer pairs comprising or consisting essentially of sequences selected from Table 15 are selected from SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base. In some examples, the plurality of primers and/or primer pairs comprising or consisting essentially of sequences selected from Table 16 are selected from SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the kit comprises a composition containing a mixture of primers and/or primer pairs comprising or consisting essentially of sequences selected from the sequences in Table 15 and a separate composition containing a mixture of primers and/or primer pairs comprising or consisting essentially of sequences selected from the sequences in Table 16. In some examples, the composition containing a mixture of primers and/or primer pairs comprising or consisting essentially of sequences selected from the sequences in Table 15 are selected from SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base. In some examples, the composition containing a mixture of primers and/or primer pairs comprising or consisting essentially of sequences selected from the sequences in Table 16 are selected from SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In any of these examples, the kit further includes one or more of a DNA polymerase, an adapter, dATP, dCTP, dGTP and dTTP. The kit can further include one or more antibodies, nucleic acid barcodes, purification solutions or columns.

In some examples, a kit is provided for detecting or measuring one or more microorganisms, or for assessing, profiling, or characterizing a mixture or population of microorganisms, e.g., bacteria and includes (1) one or more kingdom-encompassing nucleic acid primer pairs capable of amplifying a sequence in a homologous gene or genomic region common to multiple, most, a majority, substantially all, or all microorganisms in a kingdom (e.g., bacteria), but that varies between different microorganisms in the kingdom, and (2) microorganism-specific nucleic acids and/or nucleic acid primer pairs that amplify a specific nucleic acid sequence unique to a particular microorganism (e.g., a species, subspecies or strain of microorganism, such as bacteria). In some examples, the kingdom-encompassing nucleic acid primer pairs and microorganism-specific nucleic acid primer pairs comprise or consist essentially of sequences selected from the sequences in Table 15 and Table 16, respectively. In some examples, the kingdom-encompassing nucleic acid primer pairs comprise or consist essentially of sequences selected from SEQ ID NOS: 1-24 in Table 15 and/or SEQ ID NOS: 25-48 in Table 15, or SEQ ID NOS: 11-16, 23 and 24 in Table 15 and/or SEQ ID NOS: 35-40, 47 and 48 in Table 15, or substantially identical or similar sequences, and optionally wherein one or more thymine bases is substituted with a uracil base. In some examples, the microorganism-specific nucleic acid primer pairs comprise or consist essentially of sequences selected from SEQ ID NOS: 49-520 of Table 16, or SEQ ID NOS: 49-452, 457-472 and 481-520 of Table 16, or SEQ ID NOS: 49-492 of Table 16, or SEQ ID NOS: 49-452, 457-472 and 481-492 of Table 16, or SEQ ID NOS: 49-480 of Table 16A, or SEQ ID NOS: 49-452 and 457-472 of Table 16A, or SEQ ID NOS: 521-826 of Table 16C, or SEQ ID NOS: 521-820 of Table 16C, or SEQ ID NOS: 827-1298 of Table 16, or SEQ ID NOS: 827-1230, 1235-1250 and 1259-1298 of Table 16, or SEQ ID NOS: 827-1270 of Table 16, or SEQ ID NOS: 827-1230, 1235-1250 and 1259-1270 of Table 16, or SEQ ID NOS: 827-1258 of Table 16D, or SEQ ID NOS: 827-1230 and 1235-1250 of Table 16D, or SEQ ID NOS: 1299-1604 of Table 16F, or SEQ ID NOS: 1299-1598 of Table 16F, or a substantially identical or similar sequence(s), or any of the aforementioned nucleotide sequences of nucleic acids or primer pairs in which one or more thymine bases is substituted with a uracil base. In some examples, the kit comprises a composition containing one or more kingdom-encompassing nucleic acid primer pairs and a separate composition containing one or more species-specific primer pairs. In some examples, the microorganism-specific nucleic acid primer pairs are primers that specifically amplify a sequence comprising or consisting essentially of one or more sequences in Table 17. In some examples, the microorganism-specific nucleic acid primer pairs are primers that specifically amplify a sequence comprising or consisting essentially of SEQ ID NOS: 1605-1979 in Table 17, or SEQ ID NOS: 1605-1806, 1809-1816, 1821-1970, 1972-1974 and 1977-1979 of Table 17, or SEQ ID NOS: 1605-1826 in Table 17, or SEQ ID NOS: 1605-1806, 1809-1816 and 1821-1826 in Table 17, or SEQ ID NOS: 1605-1820 in Table 17A, or SEQ ID NOS: 1605-1806 and 1809-1816 in Table 17A, or SEQ ID NOS: 1827-1979 in Table 17C, or SEQ ID NOS: 1827-1976 in Table 17C, or a substantially identical or similar sequence, or the complement thereof. In some examples, the kit further includes one or more of a DNA polymerase, an adapter, dATP, dCTP, dGTP and dTTP. The kit can further include one or more antibodies, nucleic acid barcodes, purification solutions or columns. In some examples, one or more of the primers in a primer pair have a cleavable group. In some examples, the cleavable group can be a uracil nucleotide. In some examples in which the one or more of the primers in a primer pair have a cleavable group, the kit can further include at least one cleaving reagent. In one example, the cleavable group can be 8-oxo-deoxyguanosine, deoxyuridine or bromodeoxyuridine. In some examples, the at least one cleaving reagent includes RNaseH, uracil DNA glycosylase, Fpg or alkali. In one example, the cleaving reagent can be uracil DNA glycosylase. In some examples, a kit is provided for amplifying multiple microbial sequences from a population of nucleic acid molecules in a single reaction. In some examples, the kit is provided to perform multiplex nucleic acid amplification in a single reaction chamber or vessel. In some examples, the kit includes at least one DNA polymerase, which can be a thermostable DNA polymerase. In some examples, the concentration of the one or more DNA polymerases is present in a 3-fold excess as compared to a single amplification reaction. In some examples, the final concentration of each primer pair is present at about 25 nM to about 50 nM. In one example, the final concentration of each primer pair can be present at a concentration that is 50% lower than conventional single-plex PCR reactions. In some examples, the kit provides amplification of at least 100, 150, 200, 250, 300, 350, 398, or more, microbial sequences from a population of nucleic acid molecules in a single reaction chamber. In particular examples, a provided kit of the invention is a test kit. In some examples, the kit further comprises one or more adapters, barcodes, and/or antibodies.

### Methods for Compressing Reference Databases and Analyzing Sequence Data for Profiling Microbial Populations

The methods claimed herein are used to compress reference nucleic acid sequences and analyze sequence reads generated through sequencing of nucleic acids of portions of the genomes of microbes in accordance with the claims. In some embodiments, the organisms are related, for example, as belonging to a common taxonomic group (e.g., kingdom, phylum, class, order, family, genus and/or species). The organisms are microorganisms, such as, for example, prokaryotes including bacteria and archaea. In some embodiments, the nucleic acid is obtained using oligonucleotides, such as primers, to amplify portions of the nucleic acids of a microbe. In some embodiments, the oligonucleotides are contained in a collection, referred to as gene panels, designed to profile the composition of a sample or environment, such as, for example, a microbial environment. The microbes to be profiled may include bacteria. The genes targeted by the panel include, but are not limited to, the 16S rRNA geneThe 16S rRNA gene contains hypervariable segments that can vary in sequence in different organisms or microbes but that are separated by conservative segments of similar or nearly identical sequence in different organisms or microbes. Various embodiments of the method described herein may be used to profile microbiomes in the following applications: gut microbiome, skin microbiome, oral microbiome, respiratory tract microbiome, sepsis, infectious disease, women's health, viral type, fungal sample, metagenomics - analysis of soil and water samples, food pathogens.

In some embodiments, the disclosure provides for amplification of multiple target-specific sequences from a population of target nucleic acid molecules. In some embodiments, the method comprises hybridizing one or more target-specific primer pairs to the target sequence, extending a first primer of the primer pair, denaturing the extended first primer product from the population of nucleic acid molecules, hybridizing to the extended first primer product the second primer of the primer pair, extending the second primer to form a double stranded product, and digesting the target-specific primer pair away from the double stranded product to generate a plurality of amplified target sequences. In some embodiments, the digesting includes partial digesting of one or more of the target-specific primers from the amplified target sequence. In some embodiments, the amplified target sequences can be ligated to one or more adapters. In some embodiments, adapters can include one or more DNA barcodes or tagging sequences. In some embodiments, amplified target sequences once ligated to an adapter can undergo a nick translation reaction and/or further amplification to generate a library of adapter-ligated amplified target sequences.

In some embodiments, the methods of the disclosure include selectively amplifying target sequences in a sample containing a plurality of nucleic acid molecules and ligating the amplified target sequences to at least one adapter and/or barcode. Adapters and barcodes for use in molecular biology library preparation techniques are well known to those of skill in the art. The definitions of adapters and barcodes as used herein are consistent with the terms used in the art. For example, the use of barcodes allows for the detection and analysis of multiple samples, sources, tissues or populations of nucleic acid molecules per multiplex reaction. A barcoded and amplified target sequence contains a unique nucleic acid sequence, typically a short 6-15 nucleotide sequence, that identifies and distinguishes one amplified nucleic acid molecule from another amplified nucleic acid molecule, even when both nucleic acid molecules minus the barcode contain the same nucleic acid sequence. The use of adapters allows for the amplification of each amplified nucleic acid molecule in a uniformed manner and helps reduce strand bias. Adapters can include universal adapters or propriety adapters both of which can be used downstream to perform one or more distinct functions. For example, amplified target sequences prepared by the methods disclosed herein can be ligated to an adapter that may be used downstream as a platform for clonal amplification. The adapter can function as a template strand for subsequent amplification using a second set of primers and therefore allows universal amplification of the adapter-ligated amplified target sequence. In some embodiments, selective amplification of target nucleic acids to generate a pool of amplicons can further comprise ligating one or more barcodes and/or adapters to an amplified target sequence. The ability to incorporate barcodes enhances sample throughput and allows for analysis of multiple samples or sources of material concurrently.

Conserved sequences of nucleic acids can be found in the genomes of different organisms or microbes. Such sequences can be identical or share substantial similarity in the different genomes (see, e.g., Isenbarger et al. (2008) *Orig Life Evol Biosph* doi:10.1007/s11084-008-9148-z). In many instances, conserved sequences are located in essential genes, e.g., housekeeping genes, that encode elements required across a category or group of organisms or microbes for carrying out basic biochemical functions of survival. Such genes are referred to herein as "homologous" genes. However, through evolution and adaptation of organisms and microbes to diverse conditions, even homologous genes diverged and contain sequences that vary between different organisms and microbes and that may be so divergent as to be unique to specific organisms or microbes such that they can be used to identify an individual organism or microbe or a related group (e.g., species) of organisms or microbes. These features of homologous genes can be exploited in characterizing or profiling the nucleic acid composition of samples, such as, for example, biological or environmental samples. For example, in profiling the microbiota of a sample, the goal is not only to determine the presence of microorganisms in the sample, but to generate a comprehensive characterization of the total microorganism population, including the identities of the constituent microorganisms, e.g., genera, species, and relative levels of different microorganisms. Analysis of homologous genes containing sequences conserved (e.g., conserved regions) across substantially all of the targeted elements of a population being profiled in a sample (e.g., all bacteria) as well as sequences that vary (e.g., variable regions) and provide information specific to individuals or subgroups within the total population provides a method for efficiently profiling a population in a sample. Homologous genes that contain multiple variable regions interspersed between conserved regions are particularly useful in such methods because they provide multiple sequences that can be analyzed to more accurately and definitively identify individual constituents of a population of targeted elements. One example of such a gene is the prokaryotic 16S rRNA gene encoding ribosomal RNAs which are the main structural and catalytic components of ribosomes. The 16S rRNA gene is about 1500 nucleotides in length and contains nine hypervariable regions (V1-V9) interspersed between and flanked by conserved sequences of conserved regions (FIG. 1). Sequences of the hypervariable regions of 16S rRNA genes which differ in different microorganisms can be used to identify microorganisms in a sample. One method of obtaining the nucleic acids of the hypervariable regions of microorganisms in a sample in order to sequence the regions is to generate multiple copies of the regions through nucleic acid amplification (e.g., polymerase chain reaction or PCR) of all the nucleic acids extracted from a sample. Amplification can be accomplished by contacting the nucleic acids with oligonucleotides (i.e., primers) that hybridize to sequences on each end of a hypervariable region to be amplified (referred to as the template) and synthesizing a complement sequence of each strand of the template through nucleotide polymerization extension of the primers. Instead of specifically amplifying a hypervariable region of every possible microorganism that could be present in a sample by using many oligonucleotide primers, each specific to the hypervariable region of each organism, it is possible to utilize the conserved, highly similar or identical sequences flanking the hypervariable regions as primer-binding sequences to which one, or a small number of, primer pair(s) will bind and amplify a hypervariable region in substantially all of the microorganisms, e.g., bacteria, in a sample. This allows specific nucleic acids that can be used to identify a microorganism to be amplified from substantially all the microorganisms which can then be sequenced for efficient profiling of the population.

The sequences of amplified hypervariable region nucleic acids of all microorganisms present in a sample can be compared to reference sequences of particular microorganisms, or microbes, through computer-assisted sequence alignment and mapped to a gene of a known microorganism to identify the sample microorganism. Databases of 16S rRNA gene sequences from numerous microorganisms (e.g., bacteria) are publicly available (see, e.g., www.greengenes.lbl.gov and www.arb-silva.de). There can be over 100,000 sequences in a database. Furthermore, the more of the nine hypervariable regions amplified and sequenced, the more the number of alignments that have to be performed. Thus, an analysis of multiple amplified nucleic acid regions of multiple microorganisms in a sample can require extensive processor memory and time and potentially introduce errors and uncertainties into the analysis. Methods of performing such analyses that reduce computational requirements, reduce memory requirements and improve the quality of characterization of nucleic acids in a sample are provided herein and are in accordance with the claims.

Also described herein are methods for facilitating and improving the efficiency of mapping sample nucleic acids to non-conserved genome regions unique to a particular microorganism or microbe that enable accurate identification of the nucleic acids in a sample that may contain a mixture of nucleic acids from a plurality of different organisms and/or microbes.

In some embodiments, an unaligned BAM file including sequence read information may be provided to a processor for analyzing the sequence reads corresponding to marker regions. Reads obtained from sequencing of library DNA templates may be analyzed to identify, and determine the levels of, microbial constituents of the samples. Analysis may be conducted using a workflow incorporating Ion Torrent Suite^{™} Software (Thermo Fisher Scientific) with a run plan template designed to facilitate microbial DNA sequence read analysis, and an AmpliSeq microbiome analysis software plugin which generates counts for amplicons targeted in the assay. Reference genome files used for alignment in read mapping aspects of the analysis may be included in the plugin. Reference sequences derived from the GreenGenes bacterial 16S rRNA gene sequence public database (see, e.g., www.greengenes.lbl.gov) may be used for mapping of reads obtained from sequencing of amplicons generated using the 16S primer pool. In some embodiments, other databases containing 16S rRNA gene sequence information may be used, such as Ribosomal Database Project (RDP) (https://rdp.cme.msu.edu/), GRD (https://metasystems.riken.jp/grd/), SILVA, (https://www.arb-silva.de/), and ExBioCloud (https://help.ezbiocloud.net/ezbiocloud-16s-database/). Reference microbial genome sequences available in an NCBI public database (see www.ncbi.nlm.nih.gov/genome/microbes/) may be used for mapping reads obtained from sequencing of amplicons generated using the species primer pool.Compressed 16S reference sequences may be derived from the GreenGenes or other 16S rRNA gene sequence database. The compressed 16S reference sequences comprise a plurality of the hypervariable regions of the 16S rRNA gene. FIG. 1 illustrates an example of a 16S rRNA gene having hypervariable regions. A full length 16S rRNA gene can include 1000 to 1700 bp. In this example, the full length 16S rRNA gene 101 includes 1500 bp and 9 hypervariable regions, V1 - V9. A primer pool design targeting 8 of the 9 hypervariable regions for amplification, V2 - V9, results in the set of hypervariable segments 102. The set of hypervariable segments 102 may be generated by applying an in silico PCR simulation using the primer pairs targeting V2 - V9 to the full length 16S sequences contained in the database to extract expected target segments for V2 - V9. The in silico PCR simulation may use available computational tools for calculation theoretical PCR results using a given set of primers and a target DNA sequence input by the user. One such tool is Primer-BLAST (https://www.ncbi.nlm.nih.gov/tools/primer-blast/index.cgi?LINK_LOC=BlastHome), described in Ye J, Coulouris G, Zaretskaya I, Cutcutache I, Rozen S, Madden TL. (2012) Primer-BLAST: a tool to design target-specific primers for polymerase chain reaction. BMC Bioinformatics. 13:134, and in NCBI Primer-BLAST An online tool for designing target-specific PCR primer pairs (with internal probes), NCBI Handout Series | Primer-BLAST | Last Update September 8, 2016 (https://ftp.ncbi.nih.gov/pub/factsheets/HowTo_PrimerBLAST.pdf). In some embodiments, a proprietary simulation tool for in silico PCR simulation may be used to determine the set of hypervariable segments 102.

For the example of FIG. 1, the set of hypervariable segments 102 derived from the in silico simulation provides a compressed reference containing the hypervariable region sequences of those full-length 16S rRNA sequences in the complete database that would be expected to be amplified by the primers. For this example, the number of base pairs is reduced from 1500 bp of the full length sequence 101 to 8 hypervariable segments 102 having a total of 1299 bp. The GreenGenes database contains about 150,000 16S rRNA gene sequences.

In some embodiments, more than one primer pair may target a given hypervariable region. The sequence of each primer of a primer pair for amplifying each region is directed to "conserved" sequence on each side of the particular V region so that the primer pair will theoretically amplify that variable region in the genome of every bacterium. However, some of the "conserved" regions on either side of each variable region, particularly the conserved regions on either side of V2 and V8, are not sufficiently conserved in order to amplify the V2 and V8 regions of all bacteria. Thus, for amplifying V2 and V8, the 3 primer pairs (instead of 1 primer pair) may be used with the sequences of each primer pair being almost identical but having one or two nucleotides different (referred to as "degenerate primers"). Using those 3 primer pairs is a means to amplify the V2 and V8 regions for all bacteria even though the conserved regions on either side of V2 and V8 may be slightly different for different bacteria.

A BAM file format structure is described in "Sequence Alignment/Map Format Specification," September 12, 2014 (https://github.com/samtools/hts-specs). As described herein, a "BAM file" refers to a file compatible with the BAM format. As described herein, an unaligned BAM file refers to a BAM file that does not contain aligned sequence read information and mapping quality parameters and an aligned BAM file refers to a BAM file that contains aligned sequence read information and mapping quality parameters.

Nucleic acid sequence data can be generated using various techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, electronic signature-based systems, etc.

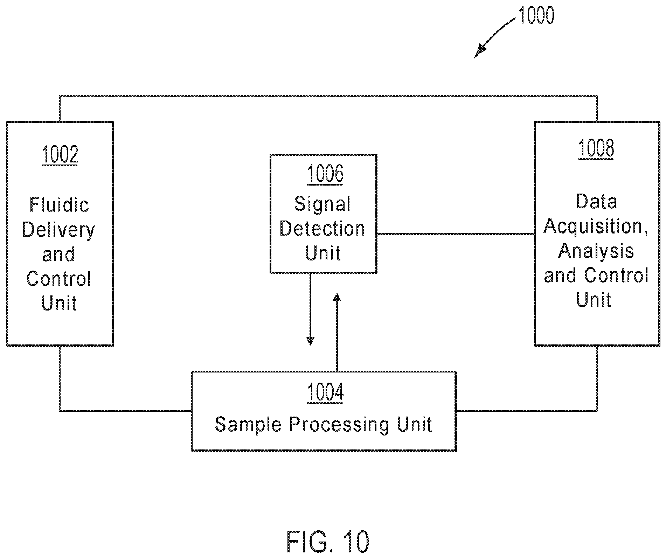

Various examples of nucleic acid sequencing platforms, such as a nucleic acid sequencer, can include components as displayed in the block diagram of FIG. 10. According to various examples, sequencing instrument 200 can include a fluidic delivery and control unit 202, a sample processing unit 204, a signal detection unit 206, and a data acquisition, analysis and control unit 208. Various examples of instrumentation, reagents, libraries and methods used for next generation sequencing are described in U.S. Patent Application Publication No. 2009/0127589 and No. 2009/0026082. Various examples of instrument 200 can provide for automated sequencing that can be used to gather sequence information from a plurality of sequences in parallel, such as substantially simultaneously.

In various examples, the fluidics delivery and control unit 202 can include reagent delivery system. The reagent delivery system can include a reagent reservoir for the storage of various reagents. The reagents can include RNA-based primers, forward/reverse DNA primers, oligonucleotide mixtures for ligation sequencing, nucleotide mixtures for sequencing-by-synthesis, optional ECC oligonucleotide mixtures, buffers, wash reagents, blocking reagent, stripping reagents, and the like. Additionally, the reagent delivery system can include a pipetting system or a continuous flow system which connects the sample processing unit with the reagent reservoir.

In various examples, the sample processing unit 204 can include a sample chamber, such as flow cell, a substrate, a micro-array, a multi-well tray, or the like. The sample processing unit 204 can include multiple lanes, multiple channels, multiple wells, or other means of processing multiple sample sets substantially simultaneously. Additionally, the sample processing unit can include multiple sample chambers to enable processing of multiple runs simultaneously. In particular embodiments, the system can perform signal detection on one sample chamber while substantially simultaneously processing another sample chamber. Additionally, the sample processing unit can include an automation system for moving or manipulating the sample chamber.

In various examples, the signal detection unit 206 can include an imaging or detection sensor. For example, the imaging or detection sensor can include a CCD, a CMOS, an ion or chemical sensor, such as an ion sensitive layer overlying a CMOS or FET, a current or voltage detector, or the like. The signal detection unit 206 can include an excitation system to cause a probe, such as a fluorescent dye, to emit a signal. The excitation system can include an illumination source, such as arc lamp, a laser, a light emitting diode (LED), or the like. In particular examples, the signal detection unit 206 can include optics for the transmission of light from an illumination source to the sample or from the sample to the imaging or detection sensor. Alternatively, the signal detection unit 206 may provide for electronic or non-photon based methods for detection and consequently not include an illumination source. In various examples, electronic-based signal detection may occur when a detectable signal or species is produced during a sequencing reaction. For example, a signal can be produced by the interaction of a released byproduct or moiety, such as a released ion, such as a hydrogen ion, interacting with an ion or chemical sensitive layer. In other examples a detectable signal may arise as a result of an enzymatic cascade such as used in pyrosequencing (see, for example, U.S. Patent Application Publication No. 2009/0325145) where pyrophosphate is generated through base incorporation by a polymerase which further reacts with ATP sulfurylase to generate ATP in the presence of adenosine 5' phosphosulfate wherein the ATP generated may be consumed in a luciferase mediated reaction to generate a chemiluminescent signal. In another example, changes in an electrical current can be detected as a nucleic acid passes through a nanopore without the need for an illumination source.

In various examples, a data acquisition analysis and control unit 208 can monitor various system parameters. The system parameters can include temperature of various portions of instrument 200, such as sample processing unit or reagent reservoirs, volumes of various reagents, the status of various system subcomponents, such as a manipulator, a stepper motor, a pump, or the like, or any combination thereof.

It will be appreciated by one skilled in the art that various examples of instrument 200 can be used to practice variety of sequencing methods including ligation-based methods, sequencing by synthesis, single molecule methods, nanopore sequencing, and other sequencing techniques.

In various eexamples, the sequencing instrument 200 can determine the sequence of a nucleic acid, such as a polynucleotide or an oligonucleotide. The nucleic acid can include DNA or RNA, and can be single stranded, such as ssDNA and RNA, or double stranded, such as dsDNA or a RNA/cDNA pair. In various examples, the nucleic acid can include or be derived from a fragment library, a mate pair library, a ChIP fragment, or the like. In particular embodiments, the sequencing instrument 200 can obtain the sequence information from a single nucleic acid molecule or from a group of substantially identical nucleic acid molecules.

In various examples, sequencing instrument 200 can output nucleic acid sequencing read data in a variety of different output data file types/formats, including, but not limited to: *.fasta, *.csfasta, *seq.txt, *qseq.txt, *.fastq, *.sff, *prb.txt, *.sms, *srs and/or *.qv.

According to various exemplary embodiments, one or more features of any one or more of the above-discussed teachings and/or exemplary embodiments may be performed or implemented using appropriately configured and/or programmed hardware and/or software elements. Determining whether an embodiment is implemented using hardware and/or software elements may be based on any number of factors, such as desired computational rate, power levels, heat tolerances, processing cycle budget, input data rates, output data rates, memory resources, data bus speeds, etc., and other design or performance constraints.

Examples of hardware elements may include processors, microprocessors, input(s) and/or output(s) (I/O) device(s) (or peripherals) that are communicatively coupled via a local interface circuit, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), logic gates, registers, semiconductor device, chips, microchips, chip sets, and so forth. The local interface may include, for example, one or more buses or other wired or wireless connections, controllers, buffers (caches), drivers, repeaters and receivers, etc., to allow appropriate communications between hardware components. A processor is a hardware device for executing software, particularly software stored in memory. The processor can be any custom made or commercially available processor, a central processing unit (CPU), an auxiliary processor among several processors associated with the computer, a semiconductor based microprocessor (e.g., in the form of a microchip or chip set), a macroprocessor, or generally any device for executing software instructions. A processor can also represent a distributed processing architecture. The I/O devices can include input devices, for example, a keyboard, a mouse, a scanner, a microphone, a touch screen, an interface for various medical devices and/or laboratory instruments, a bar code reader, a stylus, a laser reader, a radiofrequency device reader, etc. Furthermore, the I/O devices also can include output devices, for example, a printer, a bar code printer, a display, etc. Finally, the I/O devices further can include devices that communicate as both inputs and outputs, for example, a modulator/demodulator (modem; for accessing another device, system, or network), a radio frequency (RF) or other transceiver, a telephonic interface, a bridge, a router, etc.

Examples of software may include software components, programs, applications, computer programs, application programs, system programs, machine programs, operating system software, middleware, firmware, software modules, routines, subroutines, functions, methods, procedures, software interfaces, application program interfaces (API), instruction sets, computing code, computer code, code segments, computer code segments, words, values, symbols, or any combination thereof. A software in memory may include one or more separate programs, which may include ordered listings of executable instructions for implementing logical functions. The software in memory may include a system for identifying data streams in accordance with the present teachings and any suitable custom made or commercially available operating system (O/S), which may control the execution of other computer programs such as the system, and provides scheduling, input-output control, file and data management, memory management, communication control, etc.

According to various exemplary embodiments, one or more features of any one or more of the above-discussed teachings and/or exemplary embodiments may be performed or implemented using appropriately configured and/or programmed non-transitory machine-readable medium or article that may store an instruction or a set of instructions that, if executed by a machine, may cause the machine to perform a method and/or operations in accordance with the exemplary embodiments. Such a machine may include, for example, any suitable processing platform, computing platform, computing device, processing device, computing system, processing system, computer, processor, scientific or laboratory instrument, etc., and may be implemented using any suitable combination of hardware and/or software. The machine-readable medium or article may include, for example, any suitable type of memory unit, memory device, memory article, memory medium, storage device, storage article, storage medium and/or storage unit, for example, memory, removable or non-removable media, erasable or non-erasable media, writeable or re-writeable media, digital or analog media, hard disk, floppy disk, read-only memory compact disc (CD-ROM), recordable compact disc (CD-R), rewriteable compact disc (CD-RW), optical disk, magnetic media, magneto-optical media, removable memory cards or disks, various types of Digital Versatile Disc (DVD), a tape, a cassette, etc., including any medium suitable for use in a computer. Memory can include any one or a combination of volatile memory elements (e.g., random access memory (RAM, such as DRAM, SRAM, SDRAM, etc.)) and nonvolatile memory elements (e.g., ROM, EPROM, EEROM, Flash memory, hard drive, tape, CDROM, etc.). Moreover, memory can incorporate electronic, magnetic, optical, and/or other types of storage media. Memory can have a distributed architecture where various components are situated remote from one another, but are still accessed by the processor. The instructions may include any suitable type of code, such as source code, compiled code, interpreted code, executable code, static code, dynamic code, encrypted code, etc., implemented using any suitable high-level, low-level, object-oriented, visual, compiled and/or interpreted programming language.

According to various exemplary embodiments, one or more features of any one or more of the above-discussed teachings and/or exemplary embodiments may be performed or implemented at least partly using a distributed, clustered, remote, or cloud computing resource.

According to various exemplary embodiments, one or more features of any one or more of the above-discussed teachings and/or exemplary embodiments may be performed or implemented using a source program, executable program (object code), script, or any other entity comprising a set of instructions to be performed. When a source program, the program can be translated via a compiler, assembler, interpreter, etc., which may or may not be included within the memory, so as to operate properly in connection with the O/S. The instructions may be written using (a) an object oriented programming language, which has classes of data and methods, or (b) a procedural programming language, which has routines, subroutines, and/or functions, which may include, for example, C, C++, R, Pascal, Basic, Fortran, Cobol, Perl, Java, and Ada.

According to various exemplary embodiments, one or more of the above-discussed exemplary embodiments may include transmitting, displaying, storing, printing or outputting to a user interface device, a computer readable storage medium, a local computer system or a remote computer system, information related to any information, signal, data, and/or intermediate or final results that may have been generated, accessed, or used by such exemplary embodiments. Such transmitted, displayed, stored, printed or outputted information can take the form of searchable and/or filterable lists of runs and reports, pictures, tables, charts, graphs, spreadsheets, correlations, sequences, and combinations thereof, for example.

### EXAMPLES

### Example 1 - Assay Materials and Methods

Nucleic acid sequencing-based assays to identify and characterize the microbial composition of samples were conducted using DNA amplicon libraries generated from sample nucleic acids using two separate primer pools. One library was prepared using a primer pool for targeted amplification of microbial 16S rRNA DNA (the "16S primer pool") and the other library was prepared using a primer pool for targeted amplification of unique DNA sequences of different microbial species (the "species primer pool"). Primers used in the 16S primer pools included the primer pairs listed as SEQ ID NOS: 1-27 in Table 15 (see Example 5), and primers used in species primer pools the primer pairs listed as SEQ ID NOS: 49-520 in Table 16 (see Example 5) designed to amplify species-specific target sequences including sequences listed as SEQ ID NOS: 1605-1826 in Table 17 (see Example 5). After libraries were generated, templates prepared through amplification of library amplicons, e.g., using Ion Chef^{™} or Ion OneTouch^{™} 2 System, and templates were sequenced using next generation sequencing technology, e.g., an Ion S5^{™}, an Ion PGM^{™} System.

### Sample Processing and Nucleic Acid Extraction

When the sample was a biological specimen, such as human fecal stool specimen, total nucleic acids (including DNA and RNA) were extracted from the specimen (e.g., 50-250 mg) using the MagMAX^{™} Microbiome Ultra Nucleic Acid Isolation Kit (Thermo Fisher Scientific; catalog no. A42357 (with plate) or A42358 (with tubes)) and the Thermo Scientific^{™} Kingfisher^{™} Flex Magnetic Particle Processor with 96 deep well heads (Thermo Fisher Scientific; catalog no. 5400630) for automated lysate particle processing after an initial bead-beating lysis step using MagMax^{™} Microbiome kit reagents. Typical elution volume of extracted nucleic acids was 200 µl, and typical recovery volume was 180 µl. The extraction was conducted according to manufacturer's instructions except that RNaseA was added to the Wash-I Plate 1 and Wash-I-Plate 2 prior to purification to remove RNA from the extracted nucleic acids and obtain isolated DNA for use in the assays. Bacterial samples obtained from ATCC were of DNA that had already been extracted.

Extracted DNA was quantitated using the Qubit^{™} dsDNA BR Assay Kit (Thermo Fisher Scientific; catalog no. Q32853) or Qubit^{™} dsDNA HS Assay Kit (Thermo Fisher Scientific catalog no. Q32851) according to manufacturer's instructions. DNA concentrations of at least 1.67 ng/µl and preferably greater than 10ng/µl are preferable.

### DNA Amplicon Library Preparation

Two DNA amplicon libraries (a 16S rRNA gene segment DNA library and a targeted bacterial species DNA library) were prepared from each sample for each assay. The libraries were generated using reagents in the Ion AmpliSeq^{™} Library Kit Plus (Thermo Fisher Scientific; catalog no. A35907; see Table 3) for highly multiplexed PCR amplification of hundreds of target sequences.

| TABLE 3 - Ion AmpliSeq^{™} Library Kit Plus Components for Library Preparation | | | | |
|---|---|---|---|---|
| **Component** | **Cap Color** | **Quantity** | **Volume** | **Storage** |
| 5X Ion AmpliSeq^{™} HiFi Mix | Red | 1 tube | 480 µL | -30°C to -10°C |
| FuPa Reagent | Brown | 1 tube | 192 µL | -30°C to -10°C |
| Switch Solution | Yellow | 1 tube | 384 µL | -30°C to -10°C |
| DNA Ligase | Blue | 1 tube | 192 µL | -30°C to -10°C |
| 25X Library Amp Primers | Pink | 1 tube | 192 µL | -30°C to -10°C |
| 1X Library Amp Mix | Black | 1 tube | 4 x 1.2 mL | -30°C to -10°C |
| Low TE | White | 1 tube | 2 x 6 mL | 15°C to 30°C |

The 25X library amp primers and 1X library amp mix provided in the kit were not used. Instead, the following primers were used as shown in Table 3A.

| TABLE 3A - Ion AmpliSeq^{™} Microbiome Health Research Kit | | | | |
|---|---|---|---|---|
| **Component** | **Pool #** | **Concentration** | **Volume** | **Storage** |
| 16S rRNA Gene Primer Pool | 1 | 5X | 260 µL | -30°C to -10°C |
| Target Species Primer Pool | 2 | 5X | 260 µL | -30°C to -10°C |

The concentration of each of the individual primers in each primer pool is 1,000 nM (at the 5X concentration). The concentration of the individual primers in the amplification reaction is 200 nM (1X reaction concentration). Prior to conducting the amplification protocol, the Low TE bottle was removed from the library kit in cold storage, defrosted and stored in an ambient location. Ethanol (70%), used in library purification, was also prepared. To begin the amplification protocol, the HiFi Mix from the library kit was thawed and vortexed for 5 sec to mix. Primer pools (16S primer pool and species primer pool) were removed from cold storage, warmed to room temperature, vortexed for 5 sec to mix and quick spun to draw the liquid to the bottom of the tube. Next, for each separate primer pool reaction, the primer pool, Hifi Mix, sample DNA to be amplified and nuclease free water (e.g., Invitrogen^{™} Nuclease Free Water, not DEPC treated; Thermo Fisher Scientific; catalog no. AM9937) were combined in a 96-well reaction plate as set out in Tables 4 and 5 (two separate amplification reactions were conducted for each sample: one for each of the two primer pools).

| TABLE 4 - Amplification Reaction Mix Setup for Primer Pool 1 (16S Primer Pool) | | | |
|---|---|---|---|
| **Order of Addition** | **Component** | **Concentration** | **Volume uL** |
| 1 | Ion AmpliSeq^{™} HiFi Mix (red cap) | 5X | 4 |
| 2 | Ion AmpliSeq^{™} Primer Pool 1 - 16S | 5X | 4 |
| 3 | DNA Sample | 0.167ng/uL | 6 |
| 4 | Nuclease Free Water | n/a | 6 |
| | Total | | 20 |

| TABLE 5 - Amplification Reaction Mix Setup for Primer Pool 2 (Species Primer Pool) | | | |
|---|---|---|---|
| **Order of Addition** | **Component** | **Concentration** | **Volume uL** |
| 1 | Ion AmpliSeq^{™} HiFi Mix (red cap) | 5X | 4 |
| 2 | Ion AmpliSeq^{™} Primer Pool 2 -Species | 5X | 4 |
| 3 | DNA Sample | 1.67ng/uL | 6 |
| 4 | Nuclease Free Water | n/a | 6 |
| | Total | | 20 |

The 96-well reaction plate was sealed with a MicroAmp^{™} Clear Adhesive film (Thermo Fisher Scientific; catalog no. 4306311) and vortexed for 5 sec to thoroughly mix the components. The plate was quick spun to draw the liquid to the bottom of the plate and a MicroAmp^{™} Compression Pad was placed on the plate which was then placed in a thermal cycler. The amplification reaction was performed using the cycling parameters listed in Table 6.

| TABLE 6 - Amplification, Digestion and Ligation Reaction Thermal Cycling Parameters | | | | |
|---|---|---|---|---|
| **Stage** | **Step** | **Temperature** | **Time** | **Cycles** |
| AMPLIFICATION REACTION PARAMETERS | | | | |
| 1: Hold | 1 | 99°C | 2 min | n/a |
| 2: Cycling | 1 | 99°C | 15 sec | 20 |
| 2: Cycling | 2 | 60°C | 4 min | 20 |
| 3: Hold | 1 | 10°C | ∞ | n/a |

| DIGESTION REACTION PARAMETERS | | | | |
|---|---|---|---|---|
| 1: Hold | 1 | 50°C | 10 min | n/a |
| 2: Hold | 1 | 55°C | 10 min | n/a |
| 3: Hold | 1 | 60°C | 20 min | n/a |
| 4: Hold | 1 | 10°C | ∞ (1 hour max) | n/a |

| LIGATION REACTION PARAMETERS | | | | |
|---|---|---|---|---|
| 1: Hold | 1 | 22°C | 30 min | n/a |
| 2: Hold | 1 | 68°C | 5 min | n/a |
| 3: Hold | 1 | 72°C | 5 min | n/a |
| 4: Hold | 1 | 10°C | ∞ | n/a |

To trim the ends of the amplicons, the primer ends were partially digested with FuPa reagent from the library kit. The plate was removed from the thermal cycler and quick spun to draw the liquid to the bottom of the plate and then unsealed. FuPa reagent (2 µl) was added to 20 µl of amplified DNA sample. The plate was sealed with MicroAmp^{™} Clear Adhesive film, vortexed for 5 sec to thoroughly mix the components and quick spun. A MicroAmp^{™} Compression Pad was placed on the plate which was then placed in a thermal cycler. The digestion reaction was performed using the cycling parameters listed in Table 6.

The trimmed amplicons from each reaction were then ligated with IONCode^{™} Barcode Adapters 1-384 (Thermo Fisher Scientific; catalog no. A29751). Different adapter pairs were ligated to separate amplicon libraries. Each adapter pair contains a barcode adapter and an ION P1 adapter in order to enable unique identification of different libraries and sequencing in the Ion GeneStudio^{™} S5 sequencing system. In preparing the ligation reaction, the Switch Solution from the library kit and the Barcode Adapters were separately warmed to room temperature, vortexed for 5 sec and quick spun. The library plate from the digestion reaction was removed from the thermal cycler, quick spun and the seal film was removed from the plate. The components of the ligation reaction were then added to the wells of the plate as shown in Table 7.

| TABLE 7 - Ligation Reaction Mix Setup | | | |
|---|---|---|---|
| **Order of Addition** | **Component** | **Concentration** | **Volume** |
| n/a | Digested DNA Sample | n/a | 22 uL |
| 1 | Switch Solution (yellow cap) | n/a | 4 uL |
| 2 | Barcode Adapters | n/a | 2 uL |
| 3 | DNA Ligase (blue cap) | n/a | 2 uL |
| | Total | | 30 uL |

The reaction plate was sealed with a MicroAmp^{™} Clear Adhesive film, vortexed for 5 sec to thoroughly mix the components, quick spun and a MicroAmp^{™} Compression Pad was placed on the plate which was then placed in a thermal cycler. The ligation reaction was performed using the thermal parameters listed in Table 6.

The libraries were purified using the Agencourt AMPure XP Reagent (Fisher Scientific; catalog no. NC9959336). First, 70% ethanol was prepared by combining 100% ethanol with nuclease-free water (300 µl per library plus dead volume is required: 210 µl 100% ethanol + 90 µl nuclease-free water). The Agencourt AMPure XP reagent was allowed to warm to room temperature and vortexed for 30 sec immediately prior to use. The library plate was removed from the thermal cycler, quick spun and the seal film was removed from the plate. Agencourt AMPure XP Reagent (45 µl) was added to each library and the contents of each library were pipetted up and down 5 times to mix the components. The mix was incubated for 5 min at room temperature and then the plate was placed in a DynaMag-96 Side Magnet plate holder and incubated for 2 min at room temperature or until the mix cleared. A first wash step was performed by removing supernatant without disturbing the pellet, adding 150 µl of freshly prepared 70% ethanol and moving the plate from side-to-side 3 times in the DynaMag plate holder to wash the beads. The wash step was repeated once and after the wash, all the supernatant was removed and the plate was air dried for 5 min and then removed from the DynaMag plate holder. To elute the library DNA from the AMPure XP beads, 50 µl of Low TE from the library kit was added to each library and the plate was sealed with MicroAmp^{™} Clear Adhesive film, vortexed for 5 sec, quick spun and incubated for 2 min at room temperature. The plate was placed in a DynaMag-96 Side Magnet plate holder and incubated for 2 min at room temperature or until the mix cleared. The seal was then removed from the plate and the supernatant containing the eluted library was transferred to a new 96-well plate or tubes, without disturbing the pellet. The plates, or tubes, were labeled with sample information and stored at +4°C short term or -20°C long term.

Each eluted library was quantitated using real time PCR and reagents provided in the Ion Library TaqMan^{™} Quantification Kit (Thermo Fisher Scientific; catalog no. 4468802) alongside PCR reactions of a control library serial diluted to create a standard curve and a no template control. Two replicate quantitation reactions were performed for each eluted library, control library and no template control, and the mean value was used to calculate the final library concentration. Forty-four eluted libraries were able to be quantitated on a single reaction plate. The eluted library was prediluted (e.g., 1:500) in Low TE prior to measuring the concentration. An example of a plate layout used is shown in Table 8.

**TABLE 8 - Library Quantitation Template Plate Layout for 44 Libraries, 3 Control Libraries and 1 No-Template Control**

| Library Quantitation Template Plate Layout | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | S01-r1 | S01-r2 | S09-r1 | S09-r2 | S17-r1 | S17-r2 | S25-r1 | S25-r2 | S33-r1 | S33-r2 | S41-r1 | S41-r2 |
| B | S02-r1 | S02-r2 | S10-r1 | S10-r2 | S18-r1 | S18-r2 | S26-r1 | S26-r2 | S34-r1 | S34-r2 | S42-r1 | S42-r2 |
| C | S03-r1 | S03-r2 | S11-r1 | S11-r2 | S19-r1 | S19-r2 | S27-r1 | S27-r2 | S35-r1 | S35-r2 | S43-r1 | S43-r2 |
| D | S04-r1 | S04-r2 | S12-r1 | S12-r2 | S20-r1 | S20-r2 | S28-r1 | S28-r2 | S36-r1 | S36-r2 | S44-r1 | S44-r2 |
| E | S05-r1 | S05-r2 | S13-r1 | S13-r2 | S21-r1 | S21-r2 | S29-r1 | S29-r2 | S37-r1 | S37-r2 | STD01-r1 | STD01-r2 |
| F | S06-r1 | S06-r2 | S14-r1 | S14-r2 | S22-r1 | S22-r2 | S30-r1 | S30-r2 | S38r1 | S38r2 | STD02-r1 | STD02-r2 |
| G | S07-r1 | S07-r2 | S15-r1 | S15-r2 | S23-r1 | S23-r2 | S31-r1 | S31-r2 | S39-r1 | S39-r2 | STD03-r1 | STD03-r2 |
| H | S08-r1 | S08-r2 | S16-r1 | S16-r2 | S24-r1 | S24-r2 | S32-r1 | S32-r2 | S40-r1 | S40-r2 | NTC-r.1 | NTC-r.2 |

The TaqMan PCR Master Mix, TaqMan Quantitation Assay and control ibrary tubes from the Quantification Kit were warmed to room temperature, vortexed for 5 sec to mix and quick spun. Three 1:10 serial dilutions from the stock concentration (68 pM) of the control library were prepared for use in generating a standard curve as follows. Low TE (45 µl) was added to each of 4 microcentrifuge tubes labeled as STD01, STD02, STD03 and NTC. Stock concentration control library (5 µl) was added to a tube labeled STD01, which was then capped, vortexed for 5 sec and quick spun. Five microliters of diluted library from tube STD01 were added to tube STD02, which was then capped, vortexed for 5 sec and quick spun, followed by addition of 5 µl of diluted library from tube STD02 to tube STD03, which was also capped, vortexed for 5 sec and quick spun. This generated 3 control library serial dilutions for use as standards, with the following concentrations: 6.8 pm (STD01), 0.68 pM (STD02) and 0.068 pM (STD03). The no-template control (NTC) tube did not contain a library. Amplification reactions were then prepared. Each library had two duplicated reactions, the results of which were averaged. Each reaction included aliquots from the Master Mix, Assay and library (eluted sample, STD or NTC) tubes as listed in Table 9.

| TABLE 9 - Library Quantitation Reaction Mix Setup | | | |
|---|---|---|---|
| **Order of Addition** | **Component** | **Concentration** | **Volume uL** |
| 1 | TaqMan qPCR Master Mix | 2X | 10 |
| 2 | TaqMan Quantitation Assay | 20X | 1 |
| 3 | Sample (Diluted Library, STD Library, NTC) | n/a | 9 |
| | Total | | 20 |

The reaction plate was sealed with the adhesive film, vortexed for 5 sec, quick spun and placed in a real time PCR instrument. The library quantitation reactions were performed using the thermal cycling parameters and plate run setup settings listed in Tables 10 and 11.

| TABLE 10 - Library Quantitation Reaction Thermal Cycling Parameters | | | | |
|---|---|---|---|---|
| **Stage** | **Step** | **Temperature** | **Time** | **Cycles** |
| 1: Hold | 1 | 50°C | 2 min | n/a |
| 2: Hold | 1 | 95°C | 20 sec | n/a |
| 3: Cycling | 1 | 95°C | 1 sec | 40 |
| 3: Cycling | 2 | 60°C | 20 sec | 40 |

| TABLE 11 - Library Quantitation Plate Run Setup Settings | | |
|---|---|---|
| 1. Generate definitions: | | |
| | Target Assay Name | Define as LibQuant |
| | Reporter Dye | Define as FAM |
| | Quencher | Define as NFQ-MGB |
| | Passive Reference Dye | Define as ROX |
| | Sample Names | Define for each eluted library, standard library and the NTC |
| 2. Assign the target assay and samples to the appropriate plate locations | | |
| 3. Assign tasks: | | |
| | For each eluted library dilution | Assign the task as U (unknown) |
| | For each control library dilution | Assign the task as S (standard) and enter appropriate concentration |
| | For the NTC | Assign the task as N |
| 4. Enter reaction volume of 20 µl | | |
| 5. Set analysis settings: | | |
| | Threshold | Set Threshold to 0.2 |
| | Automatic baseline | Check the box net to Automatic Baseline |
| | Note: do not use default settings or automatic threshold | |
| 6. Export results | | |
| Note: when the quantitation run is complete, export the results which contain the mean calculated quantity of each of the eluted library dilutions (pM units). Refer to the generic Ion Library TaqMan Quantitation Kit user guide publication MAN0015802. | | |
| 7. Calculate final library concentration | | |
| | Calculate the stock concentration of the eluted library | |
| | Multiply the mean concentration of the eluted library dilution exported from the quantitation run by the dilution factor used (e.g., 500) | |

Each library was normalized to a concentration of 50 pM. The results of the library quantitation were used to calculate the dilution factor required to reach 50 pM concentration. If the library concentration was at or below 50 pM, then no dilution was required. To normalize the library, the following formula was used to calculate the dilution factor required to normalize the library to 50 pM: dilution factor = (eluted library concentration pM)/(50 pM). Eluted libraries were warmed to room temperature, vortexed for 5 sec and quick spun. The eluted library was combined with diluent (Low TE buffer) in a microcentrifuge tube or plate using the calculated dilution factor. A minimum of 5 µl of eluted library was used to create the normalized library. The diluted library was vortexed for 5 sec, quick spun and stored at +4°C short term or -20°C long term.

For conducting sequencing of the libraries, all the normalized libraries created from the 16S and species primer pools were combined at equimolar concentrations to formulate a pool that was 50 pM concentration. The library pool was created by combining equal volumes of each normalized library. A minimum of 5 µl of each elute library was typically used to create the library pool. If an eluted library concentration was at or below 50 pM, an equal volume of the eluted library was added into the pool. Libraries with a concentration of less than 50 M may not generate sufficient usable reads for analysis. The combined library pool was vortexed for 5 sec, quick spun and stored at +4°C short term or -20°C long term.

### Preparing the Library for Sequencing

An aliquot of the final library was used in templating of the library amplicons onto bead supports (e.g., Ion Sphere Particles) using an Ion Chef^{™} instrument and Ion 540^{™} Kit-Chef and Ion 540^{™} Chip Kit according to the manufacturer's instructions.

### Example 2 - Sequencing of Library Template DNAs

Semiconductor chips containing the library DNA-templated beads were loaded into an Ion S5 Sequencer (Thermo Fisher Scientific) and sequencing of the DNA templates was conducted according to manufacturer's instructions.

### Example 3 - Data Analysis

Reads obtained from sequencing of library DNA templates were analyzed to identify, and determine the levels of, microbial constituents of the samples. Analysis was conducted using a workflow incorporating Ion Torrent Suite^{™} Software (Thermo Fisher Scientific) with a run plan template designed to facilitate microbial DNA sequence read analysis. The analysis program, which includes computational methods described herein, is referred to as an AmpliSeq microbiome analysis software plugin which generates counts for amplicons targeted in the assay. Reference sequences derived from the GreenGenes bacterial 16S rRNA gene sequence public database (see, e.g., www.greengenes.lbl.gov) were used for mapping of reads obtained from sequencing of amplicons generated using the 16S primer pool. Reference microbial genome sequences available in an NCBI public database (see www.ncbi.nlm.nih.gov/genome/microbes/) were used for mapping reads obtained from sequencing of amplicons generated using the species primer pool.

As shown in FIG. 2, which is a block diagram of a method for processing the sequence reads to determine microbial composition, the reads from the two amplicon libraries (16S primer pool library and species primer pool library) were separately analyzed. The barcode/sample name parser separates the sequence reads into a set corresponding to reads of amplicons generated using the 16S primer pool (the 16S amplicons) and a set corresponding to reads of amplicons generated using the species primer pool (the species amplicons). In this process, the unaligned sequence reads from BAM files are first trimmed (quality and length trimming performed using BaseCaller software) and then filtered to remove short (e.g., <60 bp) reads likely to have not originated from the amplified DNA product. The BAM file reads are then mapped to reference sequences.

FIG. 3 is a block diagram of the amplicon processing pipeline used in analysis of the amplicon library generated using the 16S primer pool. The 16S amplicon reads were subjected to two alignment/mapping steps. In the first alignment step, the reads were aligned and mapped, with multi-mapping and end-to-end mapping enabled, to segments of bacterial 16S rRNA reference gene sequences obtained by in silico PCR of a set of full-length bacterial 16S rRNA reference genes (e.g., the GreenGenes database) to generate amplicon sequences expected to be amplified using the 16S primers (i.e., expected hypervariable region amplicons). Expected signature patterns of hypervariable region amplicons expected for each microorganism identified by in silico PCR as containing sequences of expected hypervariable region amplicons were generated based on which of each of the 16S primer pairs would be expected to amplify a sequence in the microorganism and which of the 16S primer pairs would not be expected to amplify a sequence in the microorganism. For example, the amplicons for each of 8 hypervariable regions that could be amplified by the 16S primer pairs per microorganism 16S rRNA reference gene sequence were assigned a binary notation based on whether or not an amplicon was expected to be amplified for the microorganism to yield an expected signature pattem of ones (indicating a amplicon was expected to be generated) and zeros (indicating an amplicon was not expected to be generated). Thus, for example, for purposes of illustration, a particular species of microorganism could have a signature pattern of expected hypervariable region amplicons of: V2 (1), V2 (0), V2 (1), V3 (1), V4 (0), V5 (1), V6 (1), V7 (0), V8 (1), V8 (0), V8 (1), V9 (0). In this illustration, the 16S primer pool used in the in silico PCR of a set of full-length bacterial 16S rRNA reference genes would have included 3 versions of primers (i.e., degenerate primers) for amplifying the V2 region, 3 versions of primers (i.e., degenerate primers) for amplifying the V8 region, and only one primer pair each for amplifying each of the other hypervariable regions (i.e., V3, V4, V5, V6, V7 and V9). In a first mapping step, the reads are aligned to the reference hypervariable segments of the 16S reference set, with multi-mapping and end-to-end mapping enabled. The mapping steps determine aligned sequence reads and associated mapping quality parameters. The mapped reads are filtered based on alignment quality. After the first alignment step, the sequence reads were separated and assigned to a hypervariable region according to the different expected hypervariable region amplicons produced by the separate 16S primer pairs based on the alignments to generate a matrix of observed read counts for each of the targeted hypervariable regions for each species and strain. The number of sequence reads assigned to each expected hypervariable region for each microorganism were counted to obtain a total sequence read count for each microorganism and a series of computational steps as described herein were performed applying read thresholds (including read count thresholds per hypervariable region, as well as total read counts per 16S rRNA gene sequence) to reduce the number of reference sequences that would be used in a second alignment and mapping. The decision as to whether to include a 16S rRNA gene reference sequence in the second alignment step was thus determined by using read count thresholds per hypervariable region, as well as total read counts per 16S rRNA gene sequence. Only those 16S rRNA gene reference sequences that satisfied the criteria of at least a threshold number of total read counts per sequence, at least a threshold number of read counts per hypervariable region, and for which there was an observed pattern of reads that had at least a threshold level of similarity to the expected signature pattern were not excluded from the reference sequences used in the second mapping step. This group of microorganisms was used as a processed, reduced, filtered, high-confidence group of microorganism full-length 16S rRNA reference gene sequences (compared to the original complete set of 16S rRNA reference gene sequences contained in the GreenGenes database) to which all of the sequence reads were aligned in a second alignment step. **In** processing the original database of 16S rRNA gene sequences in this method, the number of reference sequences used in the second, and final, alignment step was reduced on the order of typically at least 50-fold, 75-fold or more, depending on the number of microorganisms in a sample. Furthermore, the quality of the reference sequences used in the second alignment step was greatly improved relative to the original database reference sequences as unannotated and incorrectly classified reference sequences were identified and reannotated and corrected (based on a sequence similarity metric (levenshtein distance)) during the processing of the database. Following the second alignment step, the total number of sequence reads aligning to each reference 16S rRNA gene sequence of the filtered group of microorganism sequences was determined as a sequence read count for each of the reference sequences. Only sequence reads aligning to the expected amplicon sequences were included in the read count (i.e., reads aligning to unexpected sequences in a 16S rRNA gene based on the primers used in the amplification were excluded from the count). Each read count was normalized by dividing it by the number of expected hypervariable region amplicons for the microorganism (e.g., the number of "ones" in the expected signature pattern). In a second normalizing step, the first normalized counts were divided by an average copy number of the 16S gene for the species to form second normalized counts. The copy numbers for the species may be obtained from a 16S copy number database, such as rrnDB (https://rrndb.umms.med.umich.edu/; Stoddard S.F, Smith B.J., Hein R., Roller B.R.K. and Schmidt T.M. (2015) rrnDB: improved tools for interpreting rRNA gene abundance in bacteria and archaea and a new foundation for future development. Nucleic Acids Research 2014; doi: 10.1093/nar/gku1201). For a given species, the copy numbers for the 16S gene given in the database records may be averaged to form the average copy number used for the second normalizing step. The normalized read counts for each reference sequence were then used to determine aggregate read counts by summing the read counts per species, genus and family. The second normalized counts are aggregated, or added, for the species level, genus level and family level. The percentage of aggregated counts to the total number of mapped reads was calculated and thresholds were applied for species detection, genus detection and family detection to give relative abundances if the threshold criteria are met. The species, genus and/or family may be reported as present if the percentage value is greater than the respective threshold. The threshold for detection may is also referred to as a noise threshold.

Based on the aggregated read counts, a determination was made as to whether a species was present in the sample (a species aggregated read count had to meet a threshold of being greater than 0.1% of the total normalized read count), a genus was present in the sample (a genus aggregated read count had to meet a threshold of being greater than 0.5% of the total normalized read count) and a family was present in the sample (a family aggregated read count had to meet a threshold of being greater than 1% of the total normalized read count). Relative abundance was reported as species-specific, genus-specific and family-specific normalized read count each divided by the total normalized read counts.

The reads from the amplicons generated using the species primer pool were separately analyzed (FIG. 4) using microbial genome sequences as a reference which was not limited to a subset of genes as was the reference used for mapping of reads from the amplicons generated using the 16S primer pool. Prior to mapping, the microbial genome database was pre-processed to provide for more efficient and accurate alignment of amplicon reads to the reference sequences. In this pre-processing, the microbial genome sequences in the database were subjected to an in silico PCR analysis conducted using primers of the species primer pool to generate expected amplicon (primers + inserts) sequences from the whole genomes of all microbial strains in the database. The in silico PCR results identify genomes in the database that contain sequences that will be amplified by the primers in the species primer pool. Any genomes that do not contain sequence that would be amplified by the species primers were eliminated from the database. Any genomes that contain sequence that would be amplified using the species primers but would not be expected to contain such sequence were evaluated to determine the average nucleotide identity (ANI) between the genome and a genome that was expected to be amplified by the primers to assess possible misclassification and reannotation of the genome, and retainment of the genome in the database. A genome was reclassified only if it had greater than 95% identity to the genome to which it was being reclassified. Following pre-processing of the reference database, the sequence reads were aligned and subjected to alignment quality filtering. Only those reads that uniquely mapped to a single species (either uniquely to one reference sequence or to multiple reference sequences of the same species) were included in a read count. The number of reads mapping to a species was calculated and an aggregate read count per species was determined, normalized by dividing the aggregate number for a species by the number of total amplicons for the species (i.e., the total number of amplicons for the species for which there was a minimum threshold number (e.g., greater than 10) of aligning sequence reads) and reported at the species level. Based on the aggregated read counts, a determination was made as to whether a species was present in the sample (a species aggregated read count had to meet a threshold of being greater than 0.1% of the total normalized read count). Relative abundance was reported as species-specific normalized read count divided by the total normalized read counts.

### Example 4 - Assay Results

Sample mixtures containing DNA from microbial species as shown in Table 12 were prepared. The samples are mixtures of known microbial DNA, at different limits of detection. Sample nos. 1-15 contain microbial DNAs at or above 5% LOD. Sample no. 16 was used as a negative control.

| TABLE 12 - Microbial DNA Sample Mixtures | | | | |
|---|---|---|---|---|
| **Sample #** | **Sample Type** | **Sample ID** | **Sample Composition Genus/Species (ATCC Accession No.)** | |
| 1 | Microbial DNA mixture | MSA100 2 | 20 Species *@* 5% each (18 Genus): | *Lactobacillus gasseri* (33323) |
| | | | *Acinetobacter baumannii* (17978) | *Neisseria meningitidis* (BAA-335) |
| | | | *Actinomyces odontolyticus* (17982) | |
| | | | | *Porphyromonas gingivalis* (33277) |
| | | | *Bacillus cereus* (10987) | |
| | | | *Bacteroides vulgatus* (8482) | *Pseudomonas aeruginosa* (9027) |
| | | | *Bifidobacterium adolescentis* (15703) | |
| | | | | *Rhodobacter sphaeroides* (17029) |
| | | | *Clostridium beijerinckii* (35702) | |
| | | | *Cutibacterium acnes* (11828) | *Staphylococcus aureus* (BAA-1556) |
| | | | *Deinococcus radiodurans* (BAA-816) | |
| | | | | *Staphylococcus epidermidis* (12228) |
| | | | *Enterococcus faecalis* (47077) | |
| | | | *Escherichia coli* (700926) | *Streptococcus agalactiae* (BAA-611) |
| | | | *Helicobacter pylori* (700392) | |
| | | | | *Streptococcus mutans* (700610) |
| 2 | Microbial DNA mixture | MSA100 6 | 12 Species *@* 8.3% each (11 Genus): | *Enterococcus faecalis* (700802) |
| | | | | |
| | | | *Bacteroides fragilis* (25285) | *Escherichia coli* (700926) |
| | | | *Bacteroides vulgatus* (8482) | *Fusobacterium nucleatum subsp. nucleatum (25586)* |
| | | | *Bifidobacterium adolescentis* (15703) | |
| | | | | *Helicobacter pylori* (700392) |
| | | | *Clostridioides difficile* (9689) | *Lactobacillus plantarum* (BAA-793) |
| | | | *Enterobacter cloacae* (13047) | |
| | | | | *Salmonella enterica subsp enterica* (9150) |
| | | | | *Yersinia enterocolitica* (27729) |
| 3 | Microbial DNA mixture | MIX05 | 20 Species *@* 5% each (13 genus) | |
| | | | | *Citrobacter rodentium* (51638) |
| | | | *Actinomyces viscosus* (27045) | *Collinsella aerofaciens* (25986) |
| | | | *Atopobium parvulum* (33793) | *Escherichia coli* (10798D-5) |
| | | | *Bacteroides fragilis* (25285D-5) | *Gardnerella vaginalis* (14019D-5) |
| | | | *Bacteroides vulgatus* (8482D-5) | |
| | | | *Bifidobacterium adolescentis* (15703D-5) | *Helicobacter pylori* (43504D-5) |
| | | | | *Klebsiella pneumoniae* (700721D-5) |
| | | | *Bifidobacterium longum* (15697D-5) | |
| | | | | *Parabacteroides distasonis* (8503D-5) |
| | | | *Campylobacter concisus* (BAA-1457D-5) | |
| | | | | *Parabacteroides merdae* (43184) |
| | | | *Campylobacter curvus* (BAA-1459D-5) | *Porphyromonas gingivalis* (BAA-308D-5) |
| | | | *Campylobacter jejuni* (700819D-5) | |
| | | | *Campylobacter rectus* (33238D-5) | |
| | | | *Bacteroides thetaiotaomicron (Bacillus thetaiotaomicron)* (29148D-5) | |
| 4 | Microbial DNA mixture | MIX06 | 20 Species *@* 5% each (13 genus) | |
| | | | *Akkermansia muciniphila* (BAA-835D-5) | *Lactobacillus acidophilus* (4357D-5) |
| | | | | *Lactobacillus delbrueckii* (9649D-5) |
| | | | *Anaerococcus vaginalis* (51170) | |
| | | | *Borreliella burgdorferi* (35210D-5) | *Lactobacillus murinus* (35020) |
| | | | | *Lactobacillus reuteri* (23272D-5) |
| | | | *Desulfovibrio alaskensis* (14563) | *Lactobacillus rhamnosus* (21052D-5) |
| | | | *Dorea formicigenerans* (27755) | *Peptostreptococcus anaerobius* (49031D-5) |
| | | | *Enterococcus faecium* (BAA-472D-5) | |
| | | | | *Streptococcus gallolyticus* (9809D-5) |
| | | | *Enterococcus gallinarum* (49573) | |
| | | | | *Streptococcus infantarius* (BAA-102) |
| | | | *Enterococcus hirae* (10541D-5) | |
| | | | *Faecalibacterium prausnitzii* (27766) | *Veillonella parvula* (17745D-5) |
| | | | *Fusobacterium nucleatum* (25586D-5) | |
| | | | *Helicobacter bilis* (51631) | |
| 5 | Microbial DNA mixture | MIX07 | 40 Species @ 2.5% each (25 genus) | |
| | | | *Actinomyces viscosus* (27045) | *Enterococcus hirae* (10541D-5) |
| | | | | *Escherichia coli* (10798D-5) |
| | | | *Akkermansia muciniphila* (BAA-835D-5) | *Faecalibacterium prausnitzii* (27766) |
| | | | *Anaerococcus vaginalis* (51170) | *Fusobacterium nucleatum* (25586D-5) |
| | | | *Atopobium parvulum* (33793) | |
| | | | *Bacteroides fragilis* (25285D-5) | *Gardnerella vaginalis* (14019D-5) |
| | | | *Bacteroides thetaiotaomicron* (29148D-5) | |
| | | | | *Helicobacter bilis (51631)* |
| | | | *Bacteroides vulgatus* (8482D-5) | *Helicobacter pylori* (43504D-5) |
| | | | *Bifidobacterium adolescentis* (15703D-5) | *Klebsiella pneumoniae* |
| | | | | (700721D-5) |
| | | | *Bifidobacterium longum* (15697D-5) | *Lactobacillus acidophilus* |
| | | | | (4357D-5) |
| | | | *Borreliella burgdorferi* (35210D-5) | *Lactobacillus delbrueckii* (9649D-5) |
| | | | *Campylobacter concisus* (BAA-1457D-5) | *Lactobacillus murinus* (35020) |
| | | | | *Lactobacillus reuteri* (23272D-5) |
| | | | *Campylobacter curvus* (BAA-1459D-5) | *Lactobacillus rhamnosus* (21052D-5) |
| | | | *Campylobacter jejuni* (700819D-5) | *Parabacteroides distasonis* (8503D-5) |
| | | | *Campylobacter rectus* (33238D-5) | *Parabacteroides merdae* (43184) |
| | | | *Citrobacter rodentium* (51638) | *Peptostreptococcus anaerobius* (49031D-5) |
| | | | *Collinsella aerofaciens* (25986) | |
| | | | *Desulfovibrio alaskensis* (14563) | *Porphyromonas gingivalis* (BAA-308D-5) |
| | | | *Dorea formicigenerans* (27755) | *Streptococcus gallolyticus* (9809D-5) |
| | | | *Enterococcus faecium* (BAA-472D-5) | |
| | | | *Enterococcus gallinarum* (49573) | *Streptococcus infantarius* (BAA-102) |
| | | | | *Veillonella parvula* (17745D-5) |
| 6 | Microbial DNA mixture | MIX09 | 22 Species *@* 4.6% each (16 genus) | |
| | | | *Bifidobacterium animalis* (27536) | *Helicobacter bizzozeronii* (700031) |
| | | | *Bifidobacterium bifidum* (29521) | *Helicobacter hepaticus* (51448) |
| | | | | *Holdemania filiformis* (51649) |
| | | | *Blautia*/*Ruminococcus gnavus* (29149) | *Lactobacillus johnsonii* (33200) |
| | | | | *Lactococcus lactis* (19435D-5) |
| | | | *Campylobacter gracilis* (33236D-5) | *Mycoplasma fermentans* (19989D-5) |
| | | | *Campylobacter hominis* (BAA-381D-5) | *Mycoplasma penetrans* (55252) |
| | | | | *Parvimonas micra (33270)* |
| | | | *Chlamydia pneumoniae* (VR-1360D-5) | *Proteus mirabilis (29906)* |
| | | | | *Pseudomonas aeruginosa* (47085D-5) |
| | | | *Chlamydia trachomatis* (VR-885D-5) | |
| | | | | *Ruminococcus bromii* (27255) |
| | | | *Clostridioides difficile* (9689D-5) | |
| | | | *Enterobacter cloacae* (13047D-5) | |
| | | | *Enterococcus faecalis* (47077D-5) | |
| | | | *Eubacterium rectale* (33656) | |
| 7 | Microbial DNA mixture | MIX11 | 20 Species @ 5% each | |
| | | | *Akkermansia muciniphila,Anaerococcus vaginalis,Atopobium parvulum, Bacteroides fragilis, Bifidobacterium animalis,Borreliella burgdorferi, Campylobacter concisus, Citrobacter rodentium, Clostridioides difficile,Desulfovibrio alaskensis,* | *Dorea formicigenerans,Enterococcus faecium,Eubacterium rectale,Faecalibacterium prausnitzii,Fusobacterium nucleatum,Helicobacter bizzozeronii,Holdemania filiformis,Lactobacillus acidophilus,Mycoplasma penetrans,Parabacteroides merdae* |
| 8 | Microbial DNA mixture | MIX12 | 20 Species @ 5% each | |
| | | | *Akkermansia muciniphila,Anaerococcus vaginalis,Atopobium parvulum,Bacteroides thetaiotaomicron,Bifidobacterium bifidum,Borreliella burgdorferi, Campylobacter curvus, Citrobacter rodentium, Desulfovibrio alaskensis,* | *Dorea formicigenerans,Enterococcus gallinarum,Eubacterium rectale,Helicobacter hepaticus,Lactobacillus delbrueckii,Parvimonas micra,Peptostreptococcus anaerobius,Proteus mirabilis,Ruminococcus bromii, Streptococcus infantarius,Veillonella parvula* |
| 9 | Microbial DNA mixture | MIX13 | 20 Species @ 5% each | |
| | | | *Bifidobacterium longum,Borreliella burgdorferi, Campylobacter hominis,Desulfovibrio alaskensis,Dorea formicigenerans,Enterococcus gallinarum,Eubacterium rectale,Faecalibacterium prausnitzii, Fusobacterium nucleatum,Helicobacter pylori,Holdemania filiformis,* | *Lactobacillus johnsonii,Mycoplasma penetrans,Parabacteroides merdae,Parvimonas micra,Peptostreptococcus anaerobius,Proteus mirabilis,Ruminococcus bromii, Streptococcus infantarius, Veillonella parvula* |
| 10 | Microbial DNA mixture | MIX14 | 20 Species @ 5% each | |
| | | | *Akkermansia muciniphila,Anaerococcus vaginalis,Atopobium parvulum,Bacteroides fragilis, Bifidobacterium animalis, Campylobacter jejuni,Desulfovibrio alaskensis,Dorea formicigenerans,Enterococcus faecium,Faecalibacterium prausnitzii,* | *Fusobacterium nucleatum,Helicobacter pylori,Holdemania filiformis,Lactobacillus murinus,Mycoplasma penetrans,Parabacteroides merdae,Parvimonas micra,Peptostreptococcus anaerobius,Proteus mirabilis,Ruminococcus bromii* |
| 11 | Microbial DNA mixture | MIX15 | 20 Species @ 5% each | |
| | | | *Akkermansia muciniphila,Anaerococcus vaginalis,Atopobium parvulum, Bacteroides thetaiotaomicron,Bifidobacterium bifidum,Borreliella burgdorferi,Campylobacter rectus, Citrobacter rodentium,Enterococcus gallinarum,* | *Fusobacterium nucleatum,Helicobacter hepaticus,Lactobacillus reuteri,Mycoplasma penetrans,Parabacteroides merdae,Parvimonas micra,Peptostreptococcus anaerobius,Proteus mirabilis,Ruminococcus bromii, Streptococcus infantarius,Veillonella parvula* |
| 12 | Microbial DNA mixture | MIX16 | 20 Species @ 5% each | |
| | | | *Bacteroides fragilis,Bacteroides thetaiotaomicron,Bifidobacterium animalis, Bifidobacterium bifidum,Bifidobacterium longum, Campylobacter concisus, Campylobacter curvus, Campylobacter hominis, Campylobacter jejuni, Campylobacter rectus,* | *Enterococcus faecium,Enterococcus gallinarum,Helicobacter bizzozeronii,Helicobacter hepaticus,Helicobacter pylori,Lactobacillus acidophilus,Lactobacillus delbrueckii,Lactobacillus johnsonii,Lactobacillus murinus,Lactobacillus reuteri* |
| 13 | Microbial DNA mixture | MIX17 | 20 Species @ 5% each | |
| | | | *Enterococcus faecium,Enterococcus gallinarum, Fusobacterium nucleatum,Helicobacter bizzozeronii,Helicobacter hepaticus,Helicobacter pylori,Holdemania filiformis,Lactobacillus acidophilus,Lactobacillus delbrueckii,Lactobacillus iohnsonii,* | *Lactobacillus murinus, Lactobacillus reuteri,Mycoplasma penetrans,Parabacteroides merdae,Parvimonas micra,Peptostreptococcus anaerobius,Proteus mirabilis,Ruminococcus bromii, Streptococcus infantarius, Veillonella parvula* |
| 14 | Microbial DNA mixture | MIX18 | 20 Species @ 5% each | |
| | | | *Akkermansia muciniphila,Anaerococcus vaginalis,Atopobium parvulum,Bacteroides fragilis, Bacteroides thetaiotaomicron,Bifidobacterium animalis, Bifidobacterium bifidum,Bifidobacterium longum,Borreliella burgdorferi, Campylobacter concisus,* | *Campylobacter curvus, Campylobacter hominis, Campylobacter jejuni, Campylobacter rectus, Citrobacter rodentium, Clostridioides difficile, Desulfovibrio alaskensis, Dorea formicigenerans,Eubacterium rectale,Faecalibacterium prausnitzii* |
| 15 | Microbial DNA mixture | MIX19 | 40 Species @ 2.5% each | |
| | | | *Akkermansia muciniphila,Anaerococcus vaginalis,Atopobium parvulum,Bacteroides fragilis, Bacteroides thetaiotaomicron,Bifidobacterium animalis, Bifidobacterium bifidum, Bifidobacterium longum,Borreliella burgdorferi, Campylobacter concisus, Campylobacter curvus, Campylobacter hominis, Campylobacter jejuni, Campylobacter rectus, Citrobacter rodentium, Clostridioides difficile, Desulfovibrio alaskensis,Dorea formicigenerans,Enterococcus faecium,Enterococcus gallinarum,* | *Eubacterium rectale,Faecalibacterium prausnitzii,Fusobacterium nucleatum,Helicobacter bizzozeronii,Helicobacter hepaticus,Helicobacter pylori,Holdemania filiformis,Lactobacillus acidophilus,Lactobacillus delbrueckii,Lactobacillus johnsonii,Lactobacillus murinus,Lactobacillus reuteri,Mycoplasma penetrans,Parabacteroides merdae,Parvimonas micra,Peptostreptococcus anaerobius,Proteus mirabilis,Ruminococcus bromii, Streptococcus infantarius,Veillonella parvula* |
| 16 | No Template Control | Water | n/a | |

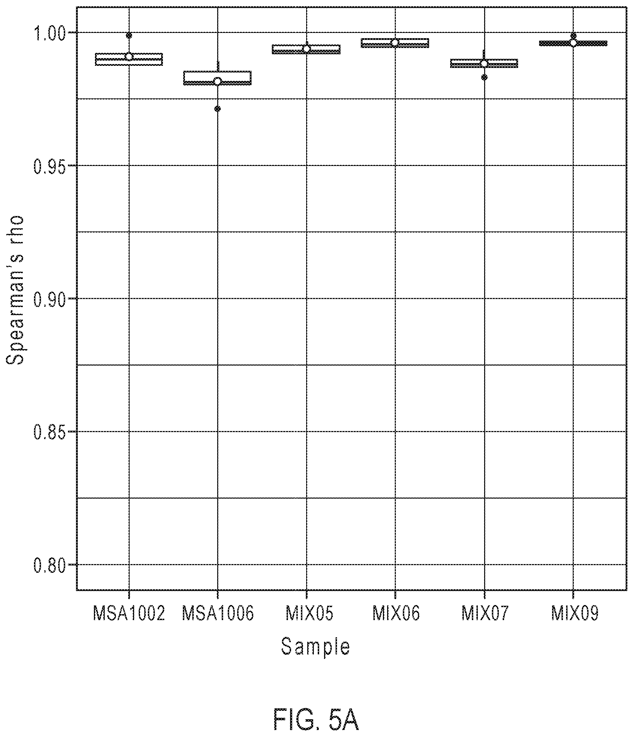

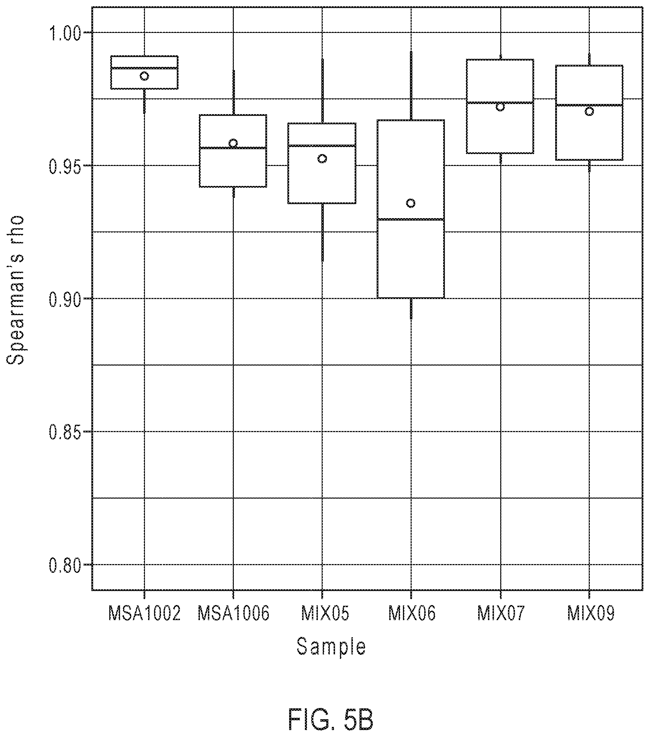

Two libraries were prepared for each sample as described in Example 1: one library generated using a 16S primer pool containing 12 primer pairs (SEQ ID NOs: 1-24; see Table 15) and 1 library using a species primer pool containing 236 primer pairs (SEQ ID NOs: 49-520; see Table 16). Four replicate aliquots of each library were included on a semiconductor sequencing chip and sequenced as described in Example 2. The ability to replicate bacteria detection results for a sample was evaluated using Spearman's RHO, which is a non-parametric test used to measure the strength of association between two variables (rank order correlation) where r=1 means a perfect positive correlation. This test is based on detection of a monotonic trend between two variables (i.e., replicates), as opposed to just a linear trend. This analysis is better suited for read counts as it makes no assumptions of a normal distribution. FIG. 5A is a plot of Spearman's RHO for replicate sequencing of libraries generated using a 16S Primer Pool. FIG. 5B is a plot of Spearman's RHO for replicate sequencing of libraries generated using a species primer pool. As shown in FIG. 5A and 5B, which depict the comparison of the results of sequencing of four replicate aliquots of libraries generated from six of the samples, the assay was very reproducible across samples.

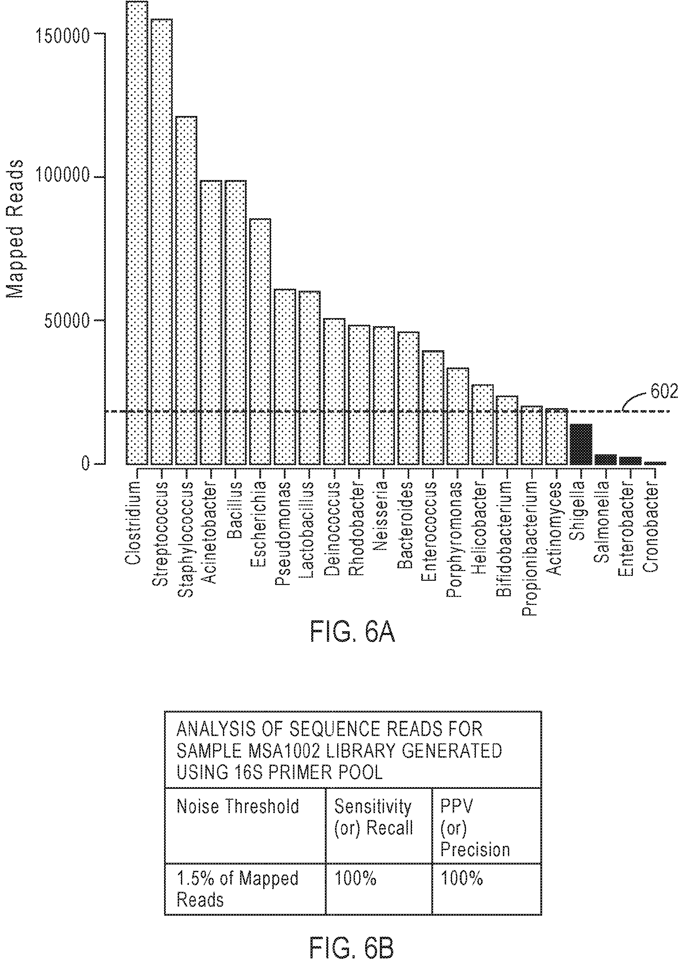

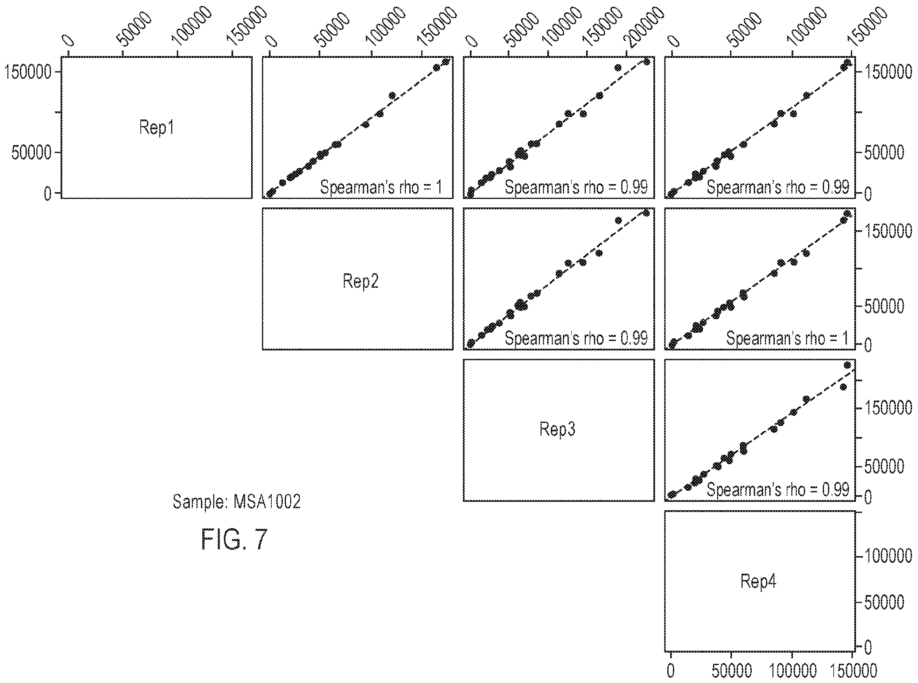

An example of results of analysis of 16S sequence reads from sequencing of a library generated from Sample no 1 using the pool of 16S primers is shown in FIGS. 6A and 6B (*Propionibacterium* shown in FIG. 6A is the scientific name for *Cutibacterium*). FIG. 6A shows that all 18 of the 18 bacterial genera present in Sample no. 1 (MSA1002) were detected in the sequencing assay. FIG. 6A shows results where the second mapping step 312 was applied using the first reduced set of full-length 16S rRNA gene sequences (without reannotation) and without the first and second normalizing steps 316 (refer to block diagram in FIG. 3). The dashed line 602 in FIG. 6A is at 1.5% of the total number of mapped reads on the y-axis which represents the threshold in this analysis for the number of mapped reads that were considered as "noise" or background. This threshold, which can be varied for any given analysis, is the number of mapped reads out of the total number of mapped reads that can be considered as being attributable to non-specific sequences, such as, for example, primer dimers, erroneous amplification products and truncated reads. The number of mapped reads for each genus or species in excess of the noise threshold are those reads considered as specific, relevant and different from the reads at or below the threshold number of reads. FIG. 6B gives a table of the noise threshold, sensitivity and PPV for the example of Sample no. 1 (MSA1002) library generated using a 16S primer pool. The assay was highly reproducible as shown in an analysis of a comparison of the results of sequencing of four replicate aliquots of a library generated from Sample no. 1 (MSA1002) using a 16S primer pool (see FIG. 7).

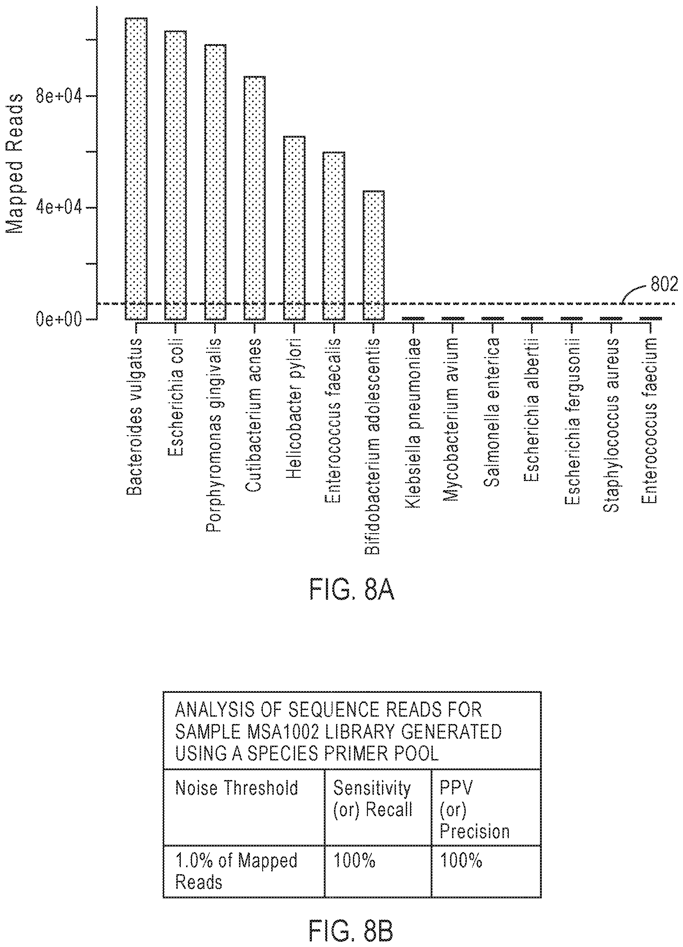

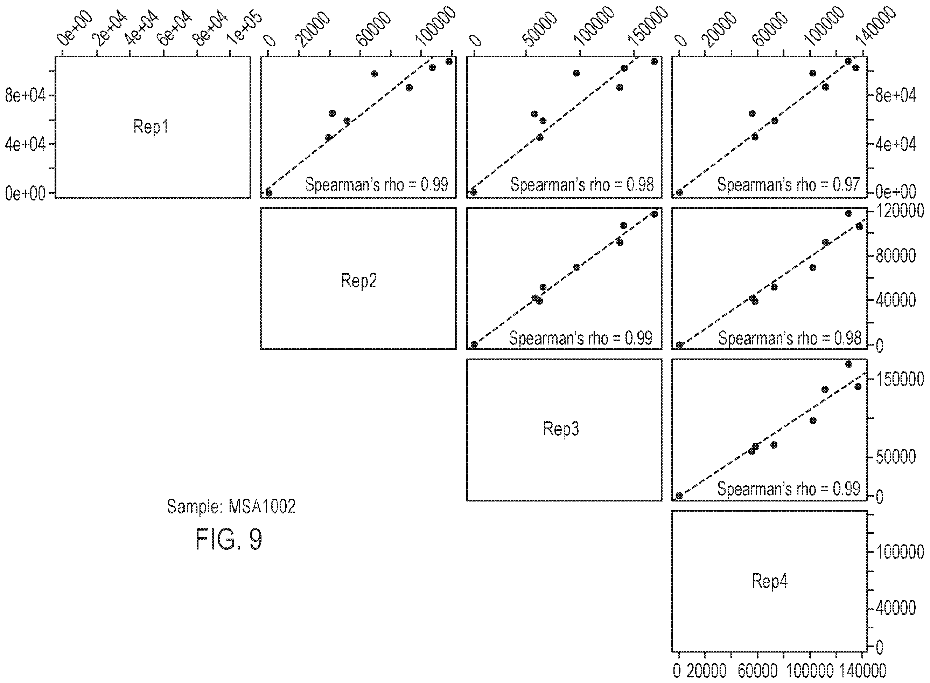

An example of results of analysis of targeted species sequence reads from sequencing of a library generated from Sample no. 1 using the species primer pool is shown in FIGS. 8A and 8B. Sample no. 1 (MSA1002) contains 20 different bacterial species, 7 of which were targeted by the library preparation amplification (as described in Example 1) using a pool of species primers. As shown in FIG. 8A, all 7 of the bacterial species (*Bacteroides vulgatus, Escherichia coli, Porphyromonas gingivalis, Cutibacterium acnes, Helicobacter pylori, Enterococcus faecalis and Bifidobacterium adolescentis* ) that were targeted by species primers in generating the library from Sample no. 1 were detected and correctly identified, whereas other, non-targeted species were not detected at a noise threshold of 1% of mapped reads. The dashed line 802 in FIG. 8A is at 1.0% of the total number of mapped reads on the y-axis which represents the threshold above which the species may be detected as present in the sample. This threshold can be set by the user for any given analysis. The resolution of the assay using the species primer pool to generate library amplicons was greater than that of the assay using the 16S primer pool. For example, the only *Bifidobacterium* species detected in a library generated from Sample no. 1 (MSA1002) using the species primer pool was *B. adolescentis,* which is the only *Bifidobacterium* species contained in Sample no. 1. However, reads of sequences from a library generated from Sample no. 1 using the 16S primer pool mapped to four additional *Bifidobacterium* species, as well as to *B. adolescentis,* which did have the greatest number read counts of the 5 *Bifidobacterium* species to which reads mapped. FIG. 8B gives a table of the noise threshold, sensitivity and PPV for the example of Sample no. 1 (MSA1002) library generated using a species primer pool. The assay was highly reproducible as shown in an analysis of a comparison of the results of sequencing of four replicate aliquots of a library generated from Sample no. 1 (MSA1002) using a species primer pool (see FIG. 9).

Performance metrics evaluated for the analysis conducted of the sample sequencing results included calculation of precision (or positive predictive value; PPV) and sensitivity (or recall) and generating precision recall (PR) curves. PR evaluation is a useful measure of success of prediction, particularly for unequal class distributions. Precision is a measure of result relevancy whereas recall is a measure of the quantity of relevant results returned in an analysis. High precision correlates with a low false positive rate and high recall correlates with low false negative rate. Precision is calculated as the number of results identified as positive in a test that are positive (i.e., true positives) divided by the total number of results identified as positive in the test (i.e., true positives + false positives). Recall is calculated as the number of results identified as positive in a test that are positive (i.e., true positives) divided by the number of true positives plus then number of false negatives. A PR curve is a plot of precision vs. recall for different thresholds. A high area under a PR curve (AUC) reflects high recall and high precision and many correctly identified results. Performance metrics determined for the analysis of sequence reads obtained from sequencing of one chip are shown in Tables 13 and 14. Table 13 shows examples of results for 16S sequence reads, where the noise threshold was set for genus level detection. Table 14 shows examples of results for targeted species sequence reads where the noise threshold was set for species level detection.

| TABLE 13 - Performance Metrics for Sequencing of Sample DNA Amplicons Generated Using 16S Primers | | |
|---|---|---|
| Sample ID | Area Under PR Curve | Precision, Recall (Noise Threshold) ^{a} |
| MSA1002 | 1.00 | 1.00, 1.00 (0.5%) |
| MSA1006 | 0.97 | 0.91, 1.00 (0.5%)^{b} |
| MIX05 | 0.98 | 0.92, 1.00 (0.5%) |
| MIX06 | 1.00 | 0.92, 1.00 (0.5%) |
| MIX07 | 0.99 | 1.00, 0.96 (0.5%) |
| MIX09 | 0.96 | 0.85, 1.00 (0.5%) ^{b} |
| MIX11 | 0.95 | 0.95, 0.95 (0.5%) ^{b} |
| MIX12 | 0.95 | 0.95, 0.95 (0.5%) ^{b} |
| MIX13 | 1.00 | 1.00, 1.00 (0.5%) |
| MIX14 | 1.00 | 1.00, 1.00 (0.5%) |
| MIX15 | 0.95 | 0.97, 0.90 (0.5%) ^{b} |
| MIX16 | 1.00 | 1.00, 1.00 (0.5%) |
| MIX17 | 1.00 | 1.00, 1.00 (0.5%) |
| MIX18 | 0.95 | 0.92, 0.95 (0.5%) ^{b} |
| MIX19 | 0.95 | 0.92, 0.95 (0.5%) ^{b} |

| TABLE 14 - Performance Metrics for Sequencing of Sample DNA Amplicons Generated Using Species Primers | | |
|---|---|---|
| Sample ID | Area Under PR Curve | Precision, Recall |
| MSA1002 | 1.00 | 1.00, 1.00 (0.1%) |
| MSA1006 | 1.00 | 1.00, 1.00 (0.1%) |
| MIX05 | 1.00 | 1.00, 1.00 (0.1%) |
| MIX06 | 1.00 | 1.00, 1.00 (0.1%) |
| MIX07 | 1.00 | 1.00, 1.00 (0.1%) |
| MIX09 | 1.00 | 1.00, 1.00 (0.1%) |
| MIX11 - MIX19 | 1.00 | 1.00, 1.00 (0.1%) |

| | | |
|---|---|---|
| ^{a} Noise threshold as a percentage of mapped reads ^{b} *Enterobacteriaceae* family is poorly resolved by 16S rRNA gene hypervariable region analysis (see, e.g., Chakravorty et al. (2007) J Microbiol Methods 69(2):330-339). | | |

### Example 5 - Primer and Amplicon Sequences

This example provides primer sequences that can be included in pools used to amplify microbial 16S rRNA (Table 15) and microbial species-specific DNA sequences (Table 16) in assays to identify microbes and/or characterize microbial populations in samples. Table 17 provides microbial sequences, some or all of which can be targeted by primers in a species primer pool used in such assays.

| **TABLE 15 -16S rRNA GENE PRIMER SEQUENCES** | | | | |
|---|---|---|---|---|
| HYPERVARIABLE REGION | SEQ ID NO: | PRIMER 1 | SEQ ID NO: | PRIMER 2 |
| V2 | 1 | GGCGGACGGGUGAGUAA | 2 | AGTCUGGACCGTGTCUCA |
| V2 | 3 | GGCGCACGGGUGAGUAA | 4 | AGTCUGGACCGTGTCUCA |
| V2 | 5 | GGCGAACGGGUGAGUAA | 6 | AGTCUGGACCGTGTCUCA |
| V3 | 7 | ACUCCUACGGGAGGCAGCAG | 8 | ACGGAGTUAGCCGGTGCUT |
| V4 | 9 | CAGCAGCCGCGGUAAUAC | 10 | CGCAT TUCAC CGCUACAC |
| V5 | 11 | GGGAGCAAACAGGAUTAGAUACCC | 12 | CCCCCGTCAAUTCATTTGAGTUT |
| V6 | 13 | ATGTGGUTTAATTCGAUGCAACGC | 14 | TUCACAACACGAGCUGACGAC |
| V7 | 15 | TGGGUTAAGUCCCGCAACG | 16 | AAGGGCCAUGATGACTUGACG |
| V8 | 17 | GGGCUACACACGCGCUAC | 18 | CCCGGGAACGUATUCACC |
| V8 | 19 | GGGCUACACACGUGCAAC | 20 | CCCGGGAACGUATUCACC |
| V8 | 21 | GGGCUACACACGTGCUAC | 22 | CCCGGGAACGUATUCACC |
| V9 | 23 | TTCCCGGGCCUTGUACAC | 24 | CUTGTTACGACTUCACCCCAGT |
| V2 | 25 | GGCGGACGGGTGAGTAA | 26 | AGTCTGGACCGTGTCTCA |
| V2 | 27 | GGCGCACGGGTGAGTAA | 28 | AGTCTGGACCGTGTCTCA |
| V2 | 29 | GGCGAACGGGTGAGTAA | 30 | AGTCTGGACCGTGTCTCA |
| V3 | 31 | ACTCCTACGGGAGGCAGCAG | 32 | ACGGAGTTAGCCGGTGCTT |
| V4 | 33 | CAGCAGCCGCGGTAATAC | 34 | CGCATTTCACCGCTACAC |
| V5 | 35 | GGGAGCAAACAGGATTAGATACCC | 36 | CCCCCGTCAATTCATTTGAGTTT |
| V6 | 37 | ATGTGGTTTAATTCGATGCAACGC | 38 | TTCACAACACGAGCTGACGAC |
| V7 | 39 | TGGGTTAAGTCCCGCAACG | 40 | AAGGGCCATGATGACTTGACG |
| V8 | 41 | GGGCTACACACGCGCTAC | 42 | CCCGGGAACGTATTCACC |
| V8 | 43 | GGGCTACACACGTGCAAC | 44 | CCCGGGAACGTATTCACC |
| V8 | 45 | GGGCTACACACGTGCTAC | 46 | CCCGGGAACGTATTCACC |
| V9 | 47 | TTCCCGGGCCTTGTACAC | 48 | CTTGTTACGACTTCACCCCAGT |

| **TABLE 16 - SPECIES PRIMER AND PROBE SEQUENCES** | | | | |
|---|---|---|---|---|
| GENUS AND SPECIES | PRIMER 1 | SEQ ID NO: | PRIMER 2 | SEQ ID NO: |
| **TABLE 16A (PRIMERS/PROBES SEQ ID NOS: 49-480)** | | | | |
| *Bifidobacterium longum* | ACCAAGGUTCUAGCCGGT | 49 | GGCTTGGUGGCAGTAAGUG | 50 |
| *Bifidobacterium longum* | ACCAUCTGGATUGCCGCA | 51 | AGTGAAACAACAGUATTGA UGCCG | 52 |
| *Clostridioides difficile QCD-66c26* | ACATTTGCTGAAUCTTTTGC TCTTTTUACT | 53 | TCAAGATAAAGGACAUCAA GTGTUAGGT | 54 |
| *Clostridioides difficile QCD-66c26* | CATCTACTGAAGCUGCTTCA AATUAGT | 55 | TTTGCTCTTTGAUATTTTT GCCAUACAGAT | 56 |
| *Clostridioides difficile QCD-66c26* | ATCTTGAATAGUAACTTTTA AACTTUGCCCT | 57 | GATTCTGCTAAACUAATCG AAGAGGTUAGA | 58 |
| *Lactococcus lactis subsp. lactis Il1403* | CAGCGAATAAUAATTCCCCT UGACAG | 59 | GGATGACTTTCUATCGGCA CTUCA | 60 |
| *Lactococcus lactis subsp. lactis Il1403* | GCAACAGCACUTCGUAACGA T | 61 | GGAGAACCAAAUTCAACAC GAGTUT | 62 |
| *Chlamydia pneumoniae TW-183* | AATTCACAGCTUGAGGAAAA GGUGT | 63 | TGGCAACAUCTGUCAGGA C | 64 |
| *Chlamydia pneumoniae TW-183* | TGCGTTGCUCGCTCUCT | 65 | TGCACTCTTUCAGAAAGAA GGTCUT | 66 |
| *Chlamydia pneumoniae TW-183* | ACGAAGAAGCUGUGGAGAAG T | 67 | CCUTGAGACUACCAGGGAG C | 68 |
| *Chlamydia pneumoniae TW-183* | AAAAGTAAACAAUAAGAAAG AGGTTCAATAUGC | 69 | CGCGCAACAUAGACUCCC | 70 |
| *Fusobacterium nucleatum subsp. nucleatum ATCC 25586* | AATTGTTCCTCAUCAACTAT TTTAATTCCTUG | 71 | GTAGCGAGGAGGAUTATAG UGAAAGA | 72 |
| *Porphyromonas gingivalis W83* | GTGGCTTTCTTAUGTGCATG GATTUG | 73 | TATTCGTAATTAGAGUAGG AGGAGAAGCTTUT | 74 |
| *Porphyromonas gingivalis W83* | TGTGGCACAUGACAGTCGTU G | 75 | CATAAGGUCTTTGCGCUGG T | 76 |
| *Helicobacter hepaticus ATCC 51449* | GTGGCAATUACTTGCGTATT UGG | 77 | CCTGCUCAACCCCTATCUG G | 78 |
| *Helicobacter hepaticus ATCC 51449* | AGACAAAGTAUCAACATTGC TCAUACCT | 79 | CGAAAGCGGGAAUGCUCCA A | 80 |
| *Lactobacillus johnsonii NCC 533* | AAATGAATGGUAGAAGCTG GUGT | 81 | TTAAGATAACTAGGUCGCC GACUAC | 82 |
| *Lactobacillus johnsonii NCC 533* | TTCAGCTTCAUTAGAAGACC UCGG | 83 | CGTCAATTGGACTTTACT GATUGGA | 84 |
| *Lactobacillus johnsonii NCC 533* | TCACCATCAAGUAGAACTGT ATTTTGUGT | 85 | CCAGAAGAAUTGCTUCCCC AT | 86 |
| *Lactobacillus johnsonii NCC 533* | ACAATATTGGTCTUTTATTT TTAGCAACTUGT | 87 | AGCTTATATUGAGGATTGT GGCUACAC | 88 |
| *Cutibacterium acnes KPA171202* | TCGGTGUCATTGGGAUCGAC | 89 | CUGGGCGACGACGCTUT | 90 |
| *Cutibacterium acnes KPA171202* | GUGCCGTCATUGACCAGCAT | 91 | CGGAGGGCUAUCGCGGA | 92 |
| *Helicobacter pylori 26695* | GTGCCUAAAAGCACAAGCAA TUG | 93 | AGGGAGTTTAAAAAUGAAA CGCTTUCAA | 94 |
| *Helicobacter pylori 26695* | AAAGGTGAGAGGAUTTAGGA CTTTTTACUAAA | 95 | CTAGAGAGATAGCACCUAC TATAACAGATTUC | 96 |
| *Borreliella burgdorferi B31* | AGAGAAACCAGUTGGCCTTT UGG | 97 | AACAAATCCUCGATTTATT TCAUGGCAG | 98 |
| *Borreliella burgdorferi B31* | AATGGATTTATTTTGAUTCC GAATATGCTTUT | 99 | ATTGCCAATATTCAAUCTT CTAAATTCAUCAAT | 100 |
| *Borreliella burgdorferi B31* | TTGGCAATGTGAUCTTTATT GCAATTTAAUT | 101 | AGAAATGAGATAGCUTTTA ATAATCACUGCA | 102 |
| *Chlamydia trachomatis D*/*UW-3*/*CX* | GCTGCAGGGAUTATTCTTTC UCCA | 103 | AGGGCTCUATCTATCAGAA UCGGAA | 104 |
| *Chlamydia trachomatis D*/*UW-3*/*CX* | AGAGCCCUTCTCGAATAUGG GA | 105 | AAATCGGGUGCACCTTCTG UAA | 106 |
| *Chlamydia trachomatis D*/*UW-3*/*CX* | AGCAAAAGCUTGCATATUGG CA | 107 | ACCTCTATAGGUGTCCGTT ATTTTGAUG | 108 |
| *Campylobacter jejuni subsp. Jejuni* | GCGTTCTCCAUCTTTTATAG CAGAAAUACG | 109 | TTATTTTAGTGGGTUCTGC AATGACAAGAUA | 110 |
| *Campylobacter jejuni subsp. Jejuni* | AACAATTCTTTUAGCCTAAC AGUGCCA | 111 | GCGAAAGTTACUTAGGTGG TCTUGC | 112 |
| *Campylobacter jejuni subsp. Jejuni* | GTTATGAAGCTTATUAATGG TAGTGGTGAUGA | 113 | CCTCAAATTGATCUTCTGC TGAAGTATUA | 114 |
| *Bacteroides fragilis YCH46* | TUGGCGGAUACAGCCCT | 115 | ATCCAGACUCTCCTGATTG UCCA | 116 |
| *Bacteroides fragilis YCH46* | GATCTGCCAUAGAATCTCGU CG | 117 | CGGCUGAAGAAGAGUGGGA A | 118 |
| *Bacteroides fragilis YCH46* | TCCGGGCAGCGAGUCUG | 119 | GGCAGAUCGATUGCAGGGT | 120 |
| *Lactobacillus reuteri JCM 1112* | AAAAACGGAGGAGACUAATT AATAUGGCAA | 121 | TGCTTTTGCTTCUTGTAAT TACGAATUAACT | 122 |
| *Lactobacillus reuteri JCM 1112* | CCGGTUGACCGTATACUACG CT | 123 | CACAATCGTTTTUAGCTAG AATCACTGUT | 124 |
| *Bifidobacterium adolescentis ATCC 15703* | GGAACAGCCGUCTGAUCAC | 125 | AAAAACACTCATUGTTTTC ATCGTTTTUCA | 126 |
| *Bifidobacterium adolescentis ATCC 15703* | CCAAAGACTUCGAGTAGGGC TUG | 127 | GATTGTTCATAUGGGCTCT CCTAUCC | 128 |
| *Bifidobacterium adolescentis ATCC 15703* | CGCCGAATGAUGTTCGAAAT AUGGT | 129 | CCGACAATCUCAAGAAAAC GCUGAT | 130 |
| *Lactobacillus rhamnosus* GG | ACGGGTCTUAGCATTGGCUT | 131 | GCACGCGUCAATUAAGCCC | 132 |
| *Lactobacillus rhamnosus* GG | TCAATGGTUAAGTTGGCCGU AG | 133 | ACGATCACUCAAAATGGUG CG | 134 |
| *Bacteroides thetaiotaomicron VPI-5482* | CCAAAGCATUGGCATATGCA GAUA | 135 | AAGCCCAATCGUCATCTTT GTAGUT | 136 |
| *Bacteroides thetaiotaomicron VPI-5482* | ACTAATAATAAGGGAUTTTC TGAATTTGGUGAT | 137 | AACTTTTTAGTATCCTTAG CGAAGTUGAC | 138 |
| *Bacteroides thetaiotaomicron VPI-5482* | TGCTCAAAGUGAGAACTTTT CAAATCGUAA | 139 | TCTGTTTGTGAAUAACTAC CGTUAGGAC | 140 |
| *Mycoplasma penetrans HF-2* | AGCATTACTACAAAAAGAAU CAAGCAATAAUAA | 141 | ATTTAGGGTGUAACAAAGA TGAAAAACATUAAT | 142 |
| *Mycoplasma penetrans HF-2* | GCACCTGCTUTTATAACATC ATTUCCA | 143 | ACAGAAGAAAATAUGTCTG CTACAAAUAGAT | 144 |
| *Mycoplasma penetrans HF-2* | GTAATCCUACTTTCATCATA UGAAGAAGAACT | 145 | GGTGCAACAUGAAAUCAAG GUGA | 146 |
| *Mycoplasma penetrans HF-2* | GAAAUUGCUACAGAGAUAGU CCCACC | 147 | GTAATGCTTTUAAAAATCA TTCTAAUGACCCA | 148 |
| *Lactobacillus acidophilus NCFM* | ACTGGCAATTCAUCAGAAAA TACATCUAC | 149 | CCGTAGTTUTTCCTTGCUG ACC | 150 |
| *Lactobacillus acidophilus NCFM* | GGACAGCUACCCTTGTUGCA | 151 | AAAGCACGAUTAATAGTTA AATUACCAAAAACA | 152 |
| *Lactobacillus acidophilus NCFM* | CGCTTCAACTGAUCATGTAG AAAAAGUG | 153 | CAGCATGACTGUTATCAGT GTTTGUT | 154 |
| *Lactobacillus acidophilus NCFM* | GGTGTTAAGGUGAATTGGAC UCAAAC | 155 | CCTGTGCCCAAUTCATTAT TAGTATUCAT | 156 |
| *Desulfovibrio alaskensis G20* | AAACCTTUGCCGGGCGUC | 157 | CGCAUCAGGCUCCCGCA | 158 |
| *Desulfovibrio alaskensis G20* | GCGGAUAUCACGGACGC | 159 | GGCTGCGGUTGTGGUCG | 160 |
| *Desulfovibrio alaskensis G20* | AGGUACCGGCCTGCUGCAT | 161 | TUCGCUGCCCGAAGCCG | 162 |
| *Desulfovibrio alaskensis G20* | AGCAGAAAGACAGGCAUGAU G | 163 | AGCACCUACTGCAUCGCC | 164 |
| *Bacteroides vulgatus ATCC* 8482 | AUGCAGCCACAACCAAUCG | 165 | TTCGGCCACAUTCCATCCU AA | 166 |
| *Bacteroides vulgatus ATCC* 8482 | TUGCUGACCAAAACCACCAC | 167 | TTTTTATGGAAUGTTTTTC TGUCGGG | 168 |
| *Bacteroides vulgatus ATCC* 8482 | GTTCCTATTCCUATCTCTTC CGGUGG | 169 | CCGCCUTTGATAGAUCCGC T | 170 |
| *Parabacteroides distasonis ATCC 8503* | AGUCCCAACGCCATTGUGC | 171 | CAAGGAUGUUUAUGAACGG CAAAACA | 172 |
| *Parabacteroides distasonis ATCC 8503* | GAATATGAGCCAUGAGATAC GUACGC | 173 | AGAAAGACATGCUACCGGA TTCTAUG | 174 |
| *Lactobacillus delbrueckii subsp.bulgaricus ATCC BAA-365* | GAAGCTGGAUTTGCCGACCU A | 175 | GCGGGCACAAAACUCTUCA | 176 |
| *Lactobacillus delbrueckii subsp.bulgaricus ATCC BAA-365* | ACTCAGGCGACUCAGTCTUG | 177 | GGCGGUTCTGGUCAAGC | 178 |
| *Lactobacillus delbrueckii subsp.bulgaricus ATCC BAA-365* | TTCUGACGCCTAUGGGACA | 179 | GGTUGCGGACCTGCAUC | 180 |
| *Campylobacter curvus 525.92* | CCCACGAAUGCGAUCACG | 181 | CAGCAAGGCCGAUGAGAUA AG | 182 |
| *Campylobacter curvus 525.92* | GTGACATCUGAGGTAGATGA TAUGGC | 183 | ACUCGGCACAGAUACAAGC A | 184 |
| *Campylobacter curvus 525.92* | AGCCAGAUCTCCACGCUC | 185 | TAGGGCATATCGAUAAAAG CTGTAAUAAAAA | 186 |
| *Campylobacter curvus 525.92* | ATGCCCUAAAAUCGCAAGC T | 187 | GAUATGGCUGCAAACGCGA | 188 |
| *Campylobacter hominis ATCC BAA-381* | GCCGGAGTAUCAAGATTTAA ACCAUAAG | 189 | AGATTGTTTTATTUATTTG CAAAGAGAUGACG | 190 |
| *Campylobacter hominis ATCC BAA-381* | CTTTGCAAAAUTTTGCATAT UCACCGA | 191 | GATTGATGUGGCTATTAAA AGTAUCGGC | 192 |
| *Campylobacter hominis ATCC BAA-381* | GCTGACGCUCTCAUAAACGG A | 193 | TTGCAAAGAATTUTGCGCC ATTAUT | 194 |
| *Campylobacter hominis ATCC BAA-381* | AGGTTTAAAGTATUTTCTAC AAAAACTUCAACA | 195 | ACUCCGGCAGAAAGGGAUT | 196 |
| *Campylobacter concisus 13826* | CATCGATAAGCUCATCATCA UGCCAA | 197 | TAAATTTATCTCAUAGTCT GAGATAUCGACCT | 198 |
| *Campylobacter concisus 13826* | ATAAUACGAGCAGCACACCU ACCG | 199 | AAATGAACCGGAUCAAAGC UCCC | 200 |
| *Campylobacter concisus 13826* | AGAGGAGTCUTTTAAAAAGA CUGAAGAAGAT | 201 | TTGCGUCAGTGATCUCAGA AACAT | 202 |
| *Akkermansia muciniphila ATCC BAA-835* | GGCAUTCTGAGGUACCGGAA | 203 | TTTTCGCCTCUCACATTGG AAATTAUT | 204 |
| *Akkermansia muciniphila ATCC BAA-835* | TGGGCAUGAUCGGAGAAAGA AG | 205 | TTGCCAUGGTATTCCTUGG CG | 206 |
| *Akkermansia muciniphila ATCC BAA-835* | CCAATTGAACUACTGACCTG TUGGAG | 207 | CACCGUGGGTGCTGGUCG | 208 |
| *Bifidobacterium animalis subsp. lactis AD011* | CGCAGTACAUGGATCACCTG TUC | 209 | CGTATGCGAUGCGTUCGC | 210 |
| *Bifidobacterium animalis subsp. lactis AD011* | CGCAUACGUGCAGCGGT | 211 | GGACAGGUGCCCGGUGG | 212 |
| *Bifidobacterium animalis subsp. lactis AD011* | CTGTTCUGCTGGTTCUGCGA | 213 | GCCGTAGUAACAGCCUCGA | 214 |
| *Bifidobacterium animalis subsp. lactis AD011* | ACTACGGCAUCATCGTTGUC T | 215 | GTUACGCGCAUCGAGCC | 216 |
| *Atopobium parvulum DSM 20469* | GCAGCCAGCCCUTCTUG | 217 | GGCAGAAGAUUGAUGCUC CAT | 218 |
| *Atopobium parvulum DSM 20469* | ACAGCCGCTUGATTATATTT AAACUGCC | 219 | AGAGGTATTCCAAAUGCAG CTTATUG | 220 |
| *Atopobium parvulum DSM 20469* | ACGATACCAGTAAUACTTAT TAAACTCAUCAAA | 221 | TGGCUGCTUGGAAACGAG | 222 |
| *Veillonella parvula DSM* 2008 | GCTGGTATTGGUATGATTCC AGAUGG | 223 | AAACCAAACCGUTGCCCCA UA | 224 |
| *Veillonella parvula DSM* 2008 | TCGACTGATATATCAAGAGA AAGAAAGTGUA | 225 | CATCAGCCAUGTGUACAAA ACCT | 226 |
| *Veillonella parvula DSM 2008* | AGAAACGGCUATACCAATTC AUGAAGAG | 227 | CTTCGTTCGTAAUAGATGG CTCTACAAUAAG | 228 |
| *Citrobacter* rodentium *ICC168* | GCGGAAUGGCGTTUACAGT | 229 | TTTAGCTTATCAAUAGCAC AATTTUAGAAAACA | 230 |
| *Citrobacter rodentium ICC168* | GCCACCCAGCCAUGAUG | 231 | GCGCGGUGGAGGTGTCUA | 232 |
| *Citrobacter rodentium ICC168* | ACTATGAATAAAAUTTATTT CTCUCAAGACCCG | 233 | TGGGUGGCGGAGCAUCA | 234 |
| *Citrobacter rodentium ICC168* | CTGGAUACGCAGACCGAUGT | 235 | CATTCCGCUGTTTCATCUG CA | 236 |
| *Streptococcus gallolyticus UCN34* | ATGTTGTTCAAGGUGACGGT ACUG | 237 | CAAGGTTTCAAGGAACAUT GAAGTGAUAA | 238 |
| *Streptococcus gallolyticus UCN34* | CAAAACAGGAGAUAAGATTT TTGUCACAGGA | 239 | AAACAGTUCAGCACGTTCC UGA | 240 |
| *Streptococcus gallolyticus UCN34* | CGGTGACACCUAAAGAACTG ATGATATUCT | 241 | TGACGATATCCTUTTTATT CAAGTCTCUAAGG | 242 |
| *Enterococcus faecium TX0133a04* | TAATGAAATCCAAAUATTCT CTTTCTTTAUGGC | 243 | AACGAGCUAGCGAUCGCA | 244 |
| *Enterococcus faecium TX0133a04* | TCCUGCAAUCACCGGCA | 245 | TCACGCCGAUGAAUGAAGA G | 246 |
| *Enterococcus faecium TX0133a04* | ATTCTACCCATGUCTCTGGG ATTTUGA | 247 | AGAAAAACCAAAAGCAACU GGUACG | 248 |
| *Peptostreptococcus stomatis DSM 17678* | GGAUTCATGGAUAGGAGAAA GGCT | 249 | TGCCGCCUACCTACCAGTA UG | 250 |
| *Peptostreptococcus stomatis DSM 17678* | GTATCCTAGATATGUCATTT AGGTCTTCUACA | 251 | AGAGATTGATGACCUGACT ATAGAGUCT | 252 |
| *Peptostreptococcus stomatis DSM 17678* | TTGAACTTGAAUCGACCCTA UGCA | 253 | ATGAATCCAAAUAGGATT CTGACTAUGT | 254 |
| *Peptostreptococcus stomatis DSM* 17678 | ATCTCTATAUCAAAGCTCCU GGACACA | 255 | AGGTTTAGGAAGGAAUTTA CAACTGAAAAUA | 256 |
| *Mycoplasma fermentans JER* | TCCTTGCGACUTTTGCAAAT AATATUGA | 257 | AAAGATCTTGATTAUGAAA TTCAAGAGCAAUT | 258 |
| *Mycoplasma fermentans JER* | TTTTTCAGCTUGCAAACGCT TTATTAAAUT | 259 | TGAATTGCCTATTUATACA CGCAATAAATUT | 260 |
| *Mycoplasma fermentans JER* | TCGGTTAATTTACUGAATGC AAAAAGUAAAAA | 261 | AATACAAATAATCTAUCGC TTTTTGGGUGT | 262 |
| *Mycoplasma fermentans JER* | TTTTACATTCTGTTUACCAG GATCAATUACA | 263 | GCCTTCTTCAAAUTCTTTA TAGCTTTTUGC | 264 |
| *Eubacterium limosum KIST612* | TTUCGCGGTGUAGAGCCG | 265 | CUGCAGAGCCGGCCCUC | 266 |
| *Eubacterium limosum KIST612* | GCUGAGCCGGTCAAUGC | 267 | AGTGUGGCACCAAUGAACC | 268 |
| *Eubacterium limosum KIST612* | GTTCCGGUAAAAGCAGGUGT | 269 | ACCCGCUGGTCAATTTCUC T | 270 |
| *Eubacterium limosum KIST612* | CACCTTACATGUAAAAATTC TTGCGATTUC | 271 | CCGGAACCCCAUCCCUGT | 272 |
| *Blautia obeum ATCC 29174* | CTTCTGCAUCCCGAACCUCC | 273 | TATUTCGTTGGCAAUAGAA GAGCCA | 274 |
| *Parabacteroides merdae ATCC43184* | CACTTTTAUACTGTACCUCG ACCACA | 275 | GGGCGUAGTCGGUGAGT | 276 |
| *Parabacteroides merdae ATCC43184* | CGACCCUGACACTTTTTGCA UT | 277 | TCATGATGAGAACUTGGAG AUAAAGCCT | 278 |
| *Parabacteroides merdae ATCC43184* | CTACGCCCACUTTAAACTGU GG | 279 | CAGGGTCGATAUCGATATC GATAAUGT | 280 |
| *Parabacteroides merdae ATCC43184* | TCCTUGCAGGCATUCAGGT | 281 | ACTGACTATAAATUGATAT TGTGTGAUGACAG | 282 |
| *Faecalibacterium prausnitzii M21*/*2* | AAGCCGAAAUCTGAAUGACC GA | 283 | TCGAAGAAGCACUGCATCA TGUC | 284 |
| *Faecalibacterium prausnitzii M21*/*2* | GTGCAGGCGAUCTACAACAT UC | 285 | AATAAUTATCAGTTGCUCG CAGCCT | 286 |
| *Parvimonas micra ATCC 33270* | CTAAAGCTTUGTCTATCTTA UCAACAGCT | 287 | GGTAACUCAGACGAGTTCT CGUG | 288 |
| *Parvimonas micra ATCC 33270* | AGATGGATTGTTUATCCAGT TTTCTGUG | 289 | GGAACTACACTUTCTTTTA ATGCTTTUAAAGAT | 290 |
| *Parvimonas micra ATCC 33270* | GCGAATAAATATUCTACTGA CGCTUCAT | 291 | TCTTGTUGCCTTCAGTUCC AACT | 292 |
| *Parvimonas micra ATCC 33270* | CCATTGTTGAGUCGTCAGCT TCATTUAT | 293 | AGCTTUAGCAAGAGCTAUA AACCAAGT | 294 |
| *Streptococcus infantarius subsp. infantarius ATCC BAA-102* | GCTGAGACAAUTCTTTTTCG AACUCA | 295 | GCCAGAAGCGACAGUAGCT UA | 296 |
| *Streptococcus infantarius subsp. infantarius ATCC BAA-102* | TGATATCATCAACAUTAAAC ATCTCATAGUCC | 297 | ACCAAGCTTTTAUAAGAGA GTTGCUCT | 298 |
| *Streptococcus infantarius subsp. infantarius ATCC BAA-102* | AGCTTGGTAATCAGACAAA TCAATUCG | 299 | GTCTCAGCAUGATTATTTC CATUCACG | 300 |
| *Bifidobacterium bifidum NCIMB 41171* | CGUCGCCAAGCCTUCGA | 301 | TGGTTCUGGTCGACCUGT | 302 |
| *Bifidobacterium bifidum NCIMB 41171* | GACCUCGCTUACCCGGAA | 303 | ACCTCCUGAATCTTAUCCG CGA | 304 |
| *Bifidobacterium bifidum NCIMB 41171* | CACGGUGGCCGCTTTAAUG | 305 | TGGCGACGGUACTUGGC | 306 |
| *Bifidobacterium bifidum NCIMB 41171* | CATCAGCGUCAAATCAGUCA ACCG | 307 | GGUACGCTGTUCGCCGT | 308 |
| *Collinsella stercoris DSM 13279* | AGGAGTAGACAUCCATGAAU CCG | 309 | TTCGCGUCATGGCATAUGC T | 310 |
| *Collinsella stercoris DSM 13279* | GGAACTGGAUGTATCGCGAU GA | 311 | GUCGCCAAAUGGGCGAT | 312 |
| *Collinsella stercoris DSM 13279* | TGUAAAACCGGCGAGGUGG | 313 | CGCTCAAAUGTCCUCGCT | 314 |
| *Collinsella stercoris DSM 13279* | TTUGAGCGCACAAGUAGGGT | 315 | CCAGTUCCCAGTCCAUGCA | 316 |
| *Roseburia intestinalis L1-82* | CCGGTTUCCCTGGTUCG | 317 | CTGAATTUACGCGTGAGGU GA | 318 |
| *Roseburia intestinalis L1-82* | CGATCACTCCAAAUCCGGAG CAUA | 319 | AACCGGGUGGCAGCCGUA | 320 |
| *Roseburia intestinalis L1-82* | CGGCACCUTTCUGGCAC | 321 | GACTGUGGCTTGCUGCA | 322 |
| *Roseburia intestinalis L1-82* | CTGCCCGGUATTTCGCAUT | 323 | ACGGGCACAGAUTATCGUG T | 324 |
| *Enterococcus gallinarum EG2* | TTTGGAGCAATGAUTATCGG TCCATUAA | 325 | CTCCAATTAAGCCUGCAGA AAAATUACG | 326 |
| *Enterococcus gallinarum EG2* | ATTACGGUACCTGGAAAUGA AGGCT | 327 | GATAGCACGACCGAUCAAA TAAAAATACTAUT | 328 |
| *Enterococcus gallinarum EG2* | ATGGTTGGTAUGGCAGTTAT UGGC | 329 | TTGATAATGCCUTGTAAGA AUGCCC | 330 |
| *Prevotella copri DSM 18205* | CCACACCAUTTTTGCCCTTU CAC | 331 | CGGCTUCACCCAGTUCG | 332 |
| *Prevotella copri DSM 18205* | TGAAGCCGGAUGGCTUGA | 333 | TCTTCAAATTTTAAUTCTT AGATGTTGAUCCAC | 334 |
| *Holdemania filiformis DSM* 12042 | CGUCCCAGCUGACGCAA | 335 | TCGGTAUGGGATTATCCGU CCT | 336 |
| *Holdemania filiformis DSM* 12042 | CTTTAAAATCAGAUCCAGAT TTTCATGTUCCA | 337 | TGAAGAAAATUCCGCCGCU GA | 338 |
| *Holdemania filiformis DSM* 12042 | GCCATAGACCGCUCTGACTU CC | 339 | CGCAGCUCAGACCATTCAT UGG | 340 |
| *Holdemania filiformis DSM* 12042 | TTGGAAGACGUCATCCTCGA TATAAUGA | 341 | TCAAUGCAACCCTTUCCCA G | 342 |
| *Helicobacter bilis ATCC 43879* | AGAGTGAGACAAUTACGCTA CCTUG | 343 | TTGATATTTCATTTCAAG GTGTTTAAAGUGAG | 344 |
| *Helicobacter bilis ATCC 43879* | AGATTCTAAAGAAGUGCTAG ATTTAAGUGCG | 345 | TTGATGACATTTGAGAGA ATGTCTTGCAUA | 346 |
| *Slackia exigua ATCC 700122* | GGAAUGTGCGUCGAACGG | 347 | CCAGCUGCGGTTGCGAUT | 348 |
| *Slackia exigua ATCC 700122* | CCGTACCGGAUTCCAGCGUA T | 349 | GTCTGGAATGUAGAACTAT GCGATGATAUAT | 350 |
| *Slackia exigua ATCC 700122* | CTCTUGGCGCGAAUGGAC | 351 | TGGGCGGCUATCTGGAUG | 352 |
| *Anaerococcus vaginalis ATCC 51170* | AAGGACTTAUGCCTCAATTA ATUCAACC | 353 | TCTACCGCAGAUAAAACTC CCACUA | 354 |
| *Anaerococcus vaginalis ATCC 51170* | ACCTATAGTCAUATCAACTG GAATUGCG | 355 | AAGTCCTUGCATCCACTTU GG | 356 |
| *Anaerococcus vaginalis ATCC 51170* | TACTGGAGATGTAUTAGTGG GAGAAGUT | 357 | TTGCATAATAATTTGUAAG GTTTTTCATCCUC | 358 |
| *Collinsella aerofaciens ATCC 25986* | CGUTCCAUCCCACCCCT | 359 | GCATCCAGAAUGCTTTTCT UACCG | 360 |
| *Collinsella aerofaciens ATCC 25986* | TCCCCAAUCTTCCGTAUAGC G | 361 | ATCAGCGAAAUGCCGTUCA AA | 362 |
| *Collinsella aerofaciens ATCC 25986* | AAAGACCGCCGUTGCGGTTT UA | 363 | AUGGAACGGCCCAUGCA | 364 |
| *Dorea formicigenerans ATCC 27755* | ATGCATCUGTTTCCUGGCCA T | 365 | TTTTGCAATCUGAATGTGA TCUGGG | 366 |
| *Dorea formicigenerans ATCC 27755* | AAACAGATCACGUCCAAGGT CAUC | 367 | GGGCCGAUGCAUGGAGA | 368 |
| *Dorea formicigenerans ATCC 27755* | AUCGGCCCAGTAUCCGAT | 369 | ATCCGGGUTGATUAGGAGG AAGA | 370 |
| *Dorea formicigenerans ATCC 27755* | TTGCAAAATAACATUTGTAA TCCCAATTUCC | 371 | AAGAGGGCAGAGUAUGCCG | 372 |
| *Ruminococcus gnavus ATCC 29149* | ATGCCCTGGAUTATCCCAAU GAA | 373 | TTCAATGCCTCAUAATGCA TCTGAUC | 374 |
| *Ruminococcus gnavus ATCC 29149* | TCAACAGCTUGAGTAGTCTC GUC | 375 | TTCTGCAGUAACTGCAGGG UAC | 376 |
| *Ruminococcus gnavus ATCC* 29149 | ACGGAATGTTTUCCGCAATC GUT | 377 | CAGGGCAUAAGAGGCAUAA GC | 378 |
| *Ruminococcus gnavus ATCC 29149* | TGCAGCAUCACCTGCUGA | 379 | GCTGTUGAAGGGCUCGG | 380 |
| *Campylobacter rectus RM3267* | GCTTATTACGCACAAUAGCG AATUAAAACA | 381 | AATAGTTTTGUAATAACAA GAUGCAACCAG | 382 |
| *Campylobacter rectus RM3267* | AACCGAAGAAGGAGAGUTAA AGACUT | 383 | CGGTAGTGGUGGTGTTATC GTUAAAT | 384 |
| *Campylobacter rectus RM3267* | CAGGTTGAGGGCCAUCTAAA TAATUCA | 385 | ATTGACAAAATCAUAGTTA AAAACTCCTTUGAA | 386 |
| *Campylobacter gracilis RM3268* | TTTACTACCAUCGCGCCGAT ATUT | 387 | ATCGCCGCGUTTUGCGT | 388 |
| *Campylobacter gracilis RM3268* | AAACGGCUCATCTGCGUCA | 389 | GUTGCACCGUAAAAGAGAG GACT | 390 |
| *Peptostreptococcus anaerobius 653-L* | AACCTAGCCATACUAGTATA GTCCCUT | 391 | GAGTTGGUATCAGGAGAUG AAGAAGC | 392 |
| *Peptostreptococcus anaerobius 653-L* | CTGCAAACACAUCAAAAUAA AAGGCAG | 393 | TGCCAAAAATAAGAUACAC CTTCCTAUAAGA | 394 |
| *Peptostreptococcus anaerobius 653-L* | ACCAACTCTAUATCGGCAAA ATTUGT | 395 | ACCTGAGGGUGACGACTUG | 396 |
| *Peptostreptococcus anaerobius 653-L* | TGTCCCTCAACCUAATTTTT GGCUT | 397 | GTTTGCAGAUAGGTGTUCA AGCA | 398 |
| *Prevotella histicola F0411* | GUTTGGCUCAGGAAGAGAAA CCT | 399 | GATACUACCATCGCUAGAA ACACAGAA | 400 |
| *Prevotella histicola F0411* | GCAAAGGCAGAGGUGGACAT UAC | 401 | TCAAACGAACAGCCUGTUC C | 402 |
| *Prevotella histicola F0411* | TCGTTUGACGAATAACAUGC CG | 403 | AGAGCCTATCAUAGAAGAC ATCAAUAGC | 404 |
| *Prevotella histicola F0411* | AGCACCTACCUTCTGGATGA UC | 405 | AGCAGCACAGGUCCTGUT | 406 |
| *Helicobacter bizzozeronii CIII-1* | GGATAGCATGGUGCATGTTA CAGAUAT | 407 | GTUCCACAAGAGAGAUGGG CA | 408 |
| *Helicobacter bizzozeronii CIII-1* | TTTGGGCAGUAACCTCUAGG G | 409 | TGCCCUAGAAGCCATTTAU GACAAA | 410 |
| *Helicobacter bizzozeronii CIII-1* | ACTGATAUGCACGCCATAGA UCAC | 411 | CCAAAGCATUTTAACCGAA AAUGGT | 412 |
| *Enterococcus hirae ATCC 9790* | GGCGUTGAUACCCCAGC | 413 | TTGTCAGTCTATAUTGTGA GATGTTTCUCAAA | 414 |
| *Enterococcus hirae ATCC* 9790 | TGGTCCAACAGCUGTTTCTA CUT | 415 | ATGAAGCAAAAGAAAAAUT ATUAGCACAACAA | 416 |
| *Enterococcus hirae ATCC* 9790 | TTTTTGAGGCUAACTTTGCC ATTUCT | 417 | TCAACGCCUTCTGGTATUC CC | 418 |
| *Enterococcus hirae ATCC* 9790 | AGATTCGGACCAAGUTTAAC TCTUCAA | 419 | ACCTTTAGGGAAGUACGGT ATUGAA | 420 |
| *Bacteroides nordii CL02T12C05* | ACCAAGACTGCUGACAGCAT AUG | 421 | TGCAGGCACGUATATUGGC | 422 |
| *Bacteroides nordii CL02T12C05* | TGCCUGCATTGTGAUGGAG | 423 | AGACGACGUGTCCAACTAU CAG | 424 |
| *Barnesiella intestinihominis YIT 11860* | CGAAGCAATTCAAUAAAACA CGAAAGUG | 425 | AGTTGCGUATTATCCAGTU GCGA | 426 |
| *Barnesiella intestinihominis YIT 11860* | CGATGAATACUAAGCTCATA CTCTUCGG | 427 | TTGCTTCGAAGUAAGCGAT ATATTGTTTUT | 428 |
| *Barnesiella intestinihominis YIT 11860* | AAAAUTGCGACCUCCCGAAA AAT | 429 | AATTTTCTCACGGAUACTC ACATTAATTUCGT | 430 |
| *Barnesiella intestinihominis YIT 11860* | ACCGATAATUACACCAAACA ACAUGG | 431 | CGTCGAUCAACAGTGCGUT | 432 |
| *Lactobacillus murinus ASF361* | CCGATCACAUAAGCCACACC UAAC | 433 | GTGAGTCAAATAUCATTGA TGTGAUCGT | 434 |
| *Lactobacillus murinus ASF361* | TCATCUGGAGCGACGUGA | 435 | GAACUGACCAACAAAGAUC AAUGGA | 436 |
| *Lactobacillus murinus ASF361* | ATCCGTGCCUTAAGTAGTTU GCT | 437 | CAAGGAAGGTAUAAATGAT ACACATTAUCCCA | 438 |
| *Lactobacillus murinus ASF361* | CCTTGATGCUTGGCTTGATG UT | 439 | GGCAAAATAAGCUCCTAAA ACAUCG | 440 |
| *Eubacterium rectale CAG: 36* | TCCTACCGUAAAGCTCTGTG TUAC | 441 | TTTATTAGGTTTGAUTTTT CAGACCUGCCT | 442 |
| *Eubacterium rectale CAG: 36* | AGGTATTTTCTCTAUCCTCT TCCCTTUAAAACC | 443 | GCAGGCACTUTTAATATTC AATGTUCCG | 444 |
| *Cloacibacillus porcorum* | GAAAGGGUCAACATUGCCGT | 445 | GCGAUCGCCGTCGUGAC | 446 |
| *Cloacibacillus porcorum* | GAAGGTGCCGAUCGAGAAGU G | 447 | ACCCTUTCAGGATUGGCAC A | 448 |
| *Cloacibacillus porcorum* | ATAACCGGCGCGGUCUT | 449 | ACCUCCGUGACAGAGGGA | 450 |
| *Cloacibacillus porcorum* | CGATCATCACGTTGAGGCT TUG | 451 | TATGAATCTUAGCGCACGC AAUC | 452 |
| *Blautia coccoides* | GACTCAGATTTUCAACCCCT GTCUG | 453 | TGCTTTATACGCAUAAAAA TAAGCTTAATUCA | 454 |
| *Blautia coccoides* | ATACUCCAGGGCACTUGCCG | 455 | TTTACCCTTGGGCAUTACC GTATATACUA | 456 |
| *Ruminococcus bromii* | AUTAAGGTTGTUGAAGAAAG CAGAAGAA | 457 | AATACCGCCUCACTTACTA UAGCC | 458 |
| *Ruminococcus bromii* | TTGTCGGGACUTCTTGATTA UGCA | 459 | TCGGTAUCGCAGCTGAATT TAUAGT | 460 |
| *Phascolarctobacterium faecium DSM 14760* | CUGACAGGGACAGAAAGUAA CG | 461 | TGCAACGGCUTTGTACUCA CT | 462 |
| *Phascolarctobacterium faecium DSM 14760* | CCGATCGUTCCGCTTUCA | 463 | GTAACTAUCAGCGGCGGUA CT | 464 |
| *Phascolarctobacterium faecium DSM 14760* | ACATCGATGTTTTUGATGGC TTTAATATUGC | 465 | ACGAUCGGCGGCGAUAT | 466 |
| *Gemmiger formicilis* | GAAAAGCCATTTTAUATTCT CCTGTTCTTTUT | 467 | CTGAAAAAGATTGGUGACA TCACAGAUAT | 468 |
| *Gemmiger formicilis* | GCACUGCGCCAGATAGGUA | 469 | GGCUCGGTTUCCGCGAT | 470 |
| *Gemmiger formicilis* | TGTAAGACCUGCGCGTTGUG | 471 | GCGATAGCCUGACCCAGUT | 472 |
| *Helicobacter salomonis* | GCAAAACCCUCTCTTGCTUG T | 473 | TGGTGGCCUTGATAAGAGT TUGA | 474 |
| *Helicobacter salomonis* | GCTGCTCTTCCUGTCAGGTA TTTUAG | 475 | GGTTTUGCAACAAGGGCTU C | 476 |
| *Helicobacter salomonis* | GCAGGGCUGGCGAUCAA | 477 | ATGGGTTTUAAACGCTTGA AAAAUGC | 478 |
| *Helicobacter salomonis* | GCCAAGGCCUCTCTTCUCA | 479 | AGCCCUGCCCCTAATUGG | 480 |

| **TABLE 16B (PRIMERS/PROBES SEQ ID NOS: 481-520)** | | | | |
|---|---|---|---|---|
| *Gardnerella vaginalis* | AGCAGGCCUTTTCUCAGGA | 481 | GGAGCAACUTGTTAGCAGA UGG | 482 |
| *Gardnerella vaginalis* | AGTUGCAGGTTTUGCGAGT | 483 | TGCCAAAAAGCCUTGAGAA TATTCUG | 484 |
| *Gardnerella vaginalis* | TTGACGAATCUATTTAAACC TUACCGC | 485 | GGCCTGCUACTAATTCACT TATUGC | 486 |
| *Klebsiella pneumoniae* | ACGGTGGUCGCTGTACUG | 487 | GCAGGGUGCUGACCGAG | 488 |
| *Klebsiella pneumoniae* | TCAGCGCGCAGAGAAUACUG | 489 | ACCACCGUAACCGGCUC | 490 |
| *Klebsiella pneumoniae* | CACCCUGCGGGCTGUCT | 491 | GGATTACGCAUCGGAUCGG G | 492 |
| *Escherichia coli* | TGCAATCUTGTGAGUGGCAG A | 493 | CUCGACCACCACGAAUCGC | 494 |
| *Escherichia coli* | CAATCTTCGGCGUTTTGCTG UAT | 495 | GTTGAAGAUGACAUGAGCG TUGAC | 496 |
| *Escherichia coli* | CGCCAGCGAAGGCUATUT | 497 | GTGGTGGAUGTTCCTCTGG UG | 498 |
| *Enterococcus faecalis* | CGTAGCCAAAACUAATCCGG ATUG | 499 | CATTTGGACTUAAGAGGTA TTGCGATUT | 500 |
| *Enterococcus faecalis* | GCATTAAGAGCAAAUCACTG GGAAUT | 501 | ACGATTAUTTTAAAAGCGT UAGAAGAAGCC | 502 |
| *Enterococcus faecalis* | GTTGTTGTAAAUGCCATGGG TUCC | 503 | TCTGAAGTACUAGTTGCAG TGATUCAAC | 504 |
| *Proteus mirabilis* | CTTAAAGAAAGTCAUAATCC TCACCTUCCC | 505 | GCGTTTGGGUTTATGAGCT UGAAA | 506 |
| *Proteus mirabilis* | ATAAAGAAGCATATGGUGAA AAATAAAACTCUG | 507 | GGCATTUGCGCCCATACUG | 508 |
| *Proteus mirabilis* | GTCGAGUACGACTUGCGAGA A | 509 | GAGTCACCTATAUAAGCAT CACTCTAUAAGAT | 510 |
| *Escherichia coli* | TCGTCGGUTCTGGCCUACT | 511 | GAGAAGCGACGACAUGATT AACUCT | 512 |
| *Escherichia coli* | CGCCACGGCAAUGGTTUC | 513 | GCGCAAACGUGGTTAATGG UA | 514 |
| *Escherichia coli* | CGUTATGUCGGGCGAACCA | 515 | GCAATCAUGGAAAACATCA ACGUCAT | 516 |
| *Escherichia coli* | CTTCACCGCCAUTTCCGUAA C | 517 | CCACACCGUTAGCAGCAAU CA | 518 |
| *Escherichia coli* | GACCCAUCCGGCTGAUACC | 519 | CCGTGCUCGGCAATTTUAC AT | 520 |

| **TABLE 16C (PRIMERS/PROBES SEQ ID NOS: 521-826)** | | | | |
|---|---|---|---|---|
| *Escherichia coli* | CGCATUGGTGAGCUGGC | 521 | GACAGCAACUCGCGGAUC | 522 |
| *Escherichia coli* | TCCGUATCGATCCUGAACAC CA | 523 | GCCACUCGCCCCTTGUT | 524 |
| *Bifidobacterium longum* | GUACCCAACGGGCCGTUT | 525 | CAGAUGGUGCCCAGACG | 526 |
| *Bifidobacterium longum* | GCCUCGCGCGAGGGAUT | 527 | ACCUTGGUCGAGGCCGCT | 528 |
| *Clostridioides difficile QCD-66c26* | ACAAGAAAGGAGCGAUAACT TTGGUT | 529 | CTCATCAATATTUAAAGCT CTTTGTUCAGCT | 530 |
| *Clostridioides difficile QCD-66c26* | TGGATATTAAAAGUAAAACT AGCTGATGUGG | 531 | ATCATGTTATCCCUCCCAA TTTGTUCT | 532 |
| *Clostridioides difficile QCD-66c26* | TTGATGAGATATCAACGGAA ATAACTAGTATG | 533 | AATTTCTCACCCUAGTAAA TACTGTTTCUC | 534 |
| *Lactococcus lactis subsp. lactis Il1403* | GCTCCTTGAGUATAACCATT GGUC | 535 | TCAGAUGAAACAAAAGCGG CTTUC | 536 |
| *Lactococcus lactis subsp. lactis Il1403* | GAAATTCACTTCAUCAATTA TACCAUAAACCAT | 537 | GCTGTTGCAACUGCTTTGU CA | 538 |
| *Lactococcus lactis subsp. lactis Il1403* | TCTAAGGCAAUTGCTTTTAT CATUGGG | 539 | TTTCTGAAATTCUCTTTTA TGTCATTTUAGGAC | 540 |
| *Lactococcus lactis subsp. lactis Il1403* | GAATTCTACAACCAUCTTCA CCACTUCA | 541 | ATTCGCTGATUTTTCAGGT ATTUGCT | 542 |
| *Chlamydia pneumoniae TW-183* | CGCCUATGTUCAGGCAGC | 543 | CGTCACUCGATUCCCCGT | 544 |
| *Chlamydia pneumoniae TW-183* | GAGUGACGGAATCTTTUACC CC | 545 | GAGCCTCUGGGTTCTGCUG | 546 |
| *Fusobacterium nucleatum subsp. nucleatum ATCC 25586* | CAAAACCAATTAAAGAGUTA GAGCAACATAUG | 547 | AAATGTGGATTAAUTTGAC TGTAAAGUGCAT | 548 |
| *Fusobacterium nucleatum subsp. nucleatum ATCC 25586* | TCCTATCAAGAATACUCATT GGACATTGAUT | 549 | TGGTTTTGTAATUCTTCTC AATACACUGAT | 550 |
| *Porphyromonas gingivalis W83* | CAACCTTUAGCCTCGCCAUA GAA | 551 | ACCTTTGAAAAUACAACAG AGGTGAUAAA | 552 |
| *Porphyromonas gingivalis W83* | GAGUCGACAACCGTCUGC | 553 | CGAGTATCTGCUGAAATGA GTGATAUAAC | 554 |
| *Helicobacter hepaticus ATCC 51449* | AGCUAAGGCGCCTUGCAA | 555 | GTCTTCACCUTTAGAATCC AUCGCT | 556 |
| *Helicobacter hepaticus ATCC 51449* | TGCAAAGCTAAGCAAUTTAG TCAAGCTTTUA | 557 | GCAATGTTGAUACTTTGTC TUCACCT | 558 |
| *Lactobacillus johnsonii NCC 533* | TTCAAAAACATATCUTCTAG ATCTTCTUGGT | 559 | ATGGCCACCAUAATTTTGC TTTUAAAGG | 560 |
| *Cutibacterium acnes KPA171202* | GCCCUCCGCATCGCUGT | 561 | ACGTAUACCAGGCUCAAGG CT | 562 |
| *Cutibacterium acnes KPA171202* | TCGCCCAGGUGCTCUCC | 563 | GGGUTGGUGGAACGCGA | 564 |
| *Cutibacterium acnes KPA171202* | GCCGCAGCCGAACUGGUT | 565 | ACCGCGAACUCGGGUGG | 566 |
| *Cutibacterium acnes KPA171202* | TUCGCGGUCGACACCAA | 567 | GAGTTGAGGUGCTGAUCAA CG | 568 |
| *Chlamydia trachomatis D*/*UW-3*/*CX* | GTGAGCGAAUCAAGAAAGTU CGT | 569 | AGAAGCTAGTGUATACACT GCTTGUC | 570 |
| *Campylobacter jejuni subsp. jejuni NCTC 11168 ATCC 700819* | TATAGTTGGCGUGGAGCAAA AATUGA | 571 | ATTTTAATATTTUCCCCAG TATCTTTAGUGCA | 572 |
| *Campylobacter jejuni subsp. jejuni NCTC 11168 ATCC 700819* | AAGGTCTAAATTTUGTCCAT CTAGCAUG | 573 | TGCTTTCUCAAAAAGGATC UCAAGGT | 574 |
| *Campylobacter jejuni subsp. jejuni NCTC 11168 ATCC 700819* | AAAACGAAAACGAAAAAGAU GAAGGTUT | 575 | TCTCTCAUAAAAACGCATA CCACUAAGT | 576 |
| *Campylobacter jejuni subsp. jejuni NCTC 11168 ATCC 700819* | AGAAAGCAUCAAAACCAATA AAGGAUCAG | 577 | TGCTATAAAAGAUGGAGAA CGCTAUAGT | 578 |
| *Campylobacter jejuni subsp. jejuni NCTC 11168 ATCC 700819* | AATAAGGTTTGAUTGCAAA ATTCTTUAGGAA | 579 | CCACTTTAGACAUAGGTGG UGGT | 580 |
| *Bacteroides fragilis YCH46* | AGCCGCAAAUGAAUACGGC | 581 | CCATGGAGCUGGTTGGTUG | 582 |
| *Bacteroides fragilis YCH46* | GGTATAAATGGAUCGTACGT TUCGA | 583 | TCCGCCAACAAAACCUATG UCT | 584 |
| *Bacteroides fragilis YCH46* | GGCAACCACUTCCGGAATUT | 585 | TTGCGGCUGGAUGAGGT | 586 |
| *Lactobacillus reuteri JCM 1112* | GGGATGGACAAUTATTTTAT GGATTCUGA | 587 | CUGGCCAGUAACGGCGA | 588 |
| *Lactobacillus reuteri JCM 1112* | AGTATTTTGGCUCACCAAGC AUCA | 589 | TGCCATTGAUCCACCTCAC UT | 590 |
| *Lactobacillus reuteri JCM 1112* | ATTATGATTATTGGUGGAGG ATGGTATACUGT | 591 | AATUACGCCAACGUACCCA CC | 592 |
| *Lactobacillus reuteri JCM 1112* | CTGGCCAGUATTTUGGCGGT | 593 | ACAGTTGAGGCUGAGAGAA AACTTUG | 594 |
| *Bifidobacterium adolescentis ATCC 15703* | GCCAAUCCCCGTCAUAGC | 595 | GATCCGGCGGCUGATATUC | 596 |
| *Lactobacillus rhamnosus* GG | CCGGTTTTUGCGCGCTUC | 597 | GCGAUGGCAGAAGCGUT | 598 |
| *Lactobacillus rhamnosus* GG | AAACCTTGAUGATTGCTTTU GGCAA | 599 | AGACCCGUAAUGCCGCCT | 600 |
| *Lactobacillus rhamnosus* GG | GCCAUCGCTCTUGGCGT | 601 | ACTTTGGTTTGAATCAAGA CTTGATCAC | 602 |
| *Lactobacillus rhamnosus* GG | GTGATCGUCATGTGCGAUCC | 603 | AAGGATGGAUCAACCGTTA TCCTTAAUAAA | 604 |
| *Bacteroides thetaiotaomicron VPI-5482* | GCTGGTUCAGGTATTACAAC UGC | 605 | GCGATTACGATTUGAAAAG TTCTCACTUT | 606 |
| *Bacteroides thetaiotaomicron VPI-5482* | TTATTGCAGGGUATGGUAGC CAG | 607 | CTCCACCUAGTCCCTGUCC G | 608 |
| *Bacteroides thetaiotaomicron VPI-5482* | AAATCACCCUGGUGGAGCGA | 609 | CUACTGCCTTCTCCGGGA AAAT | 610 |
| *Lactobacillus acidophilus NCFM* | TAATGACGAGAUGCGTTUGG ACAG | 611 | CTGAATTAAATTUAGTGCA TTTTCUAGCAAAGC | 612 |
| *Lactobacillus acidophilus NCFM* | CGGTTTAAACGAUGCTACTC UCGA | 613 | TTTCAGCAUACCAAAGTGG ATATTUCCAT | 614 |
| *Desulfovibrio alaskensis G20* | GCAGCGAAAGUCCGUCG | 615 | ATATCCGCGCUGCGCUG | 616 |
| *Desulfovibrio alaskensis G20* | CTGATGCGUGCCGUGCC | 617 | AGAACAGCGCAUGCGCUC | 618 |
| *Bacteroides vulgatus ATCC* 8482 | CAAACCACTTGUTCAACTTC CCUG | 619 | GTCAGCAAUGTAACCGUCA GG | 620 |
| *Bacteroides vulgatus ATCC* 8482 | AGGCAUGAGCAUGAAACGC | 621 | GGTGGAACUGACCGUAGGC | 622 |
| *Bacteroides vulgatus ATCC* 8482 | TTUGGCCACAGCAUGGGA | 623 | AAAATGCTTCTUGTTCCAG TTCAUCC | 624 |
| *Parabacteroides distasonis ATCC 8503* | CCCGGUCGTGTTTAUGGG | 625 | TCTGCGCAUTCATGUCCG | 626 |
| *Parabacteroides distasonis ATCC 8503* | CCGGAGGGAGUGGAGUT | 627 | CCCTUACCCGTATCTTUCA CGG | 628 |
| *Parabacteroides distasonis ATCC 8503* | GAGGAAAAGGCGGAGUUAUAT AGAUCUG | 629 | CCCTCCGGCAUCATCAATU G | 630 |
| *Parabacteroides distasonis ATCC 8503* | TGCGCAGATUCAGGATATTT GUGC | 631 | CCAGATACGCUTTATTATA ATAATTCUCGCC | 632 |
| *Lactobacillus delbrueckii subsp. bulgaricus ATCC BAA-365* | CCGCAACCUGGTCTUAAAGA G | 633 | TTGGCGCUTCAGCCAGUAT | 634 |
| *Lactobacillus delbrueckii subsp. bulgaricus ATCC BAA-365* | GUGCCCGCUGAAACGGA | 635 | CCGGUCAAGTUCCGGGCA | 636 |
| *Lactobacillus delbrueckii subsp. bulgaricus ATCC BAA-365* | CCTTUAAGAGCAGCCGGGAU T | 637 | GCCUGAGTCAAUCCCGACC | 638 |
| *Campylobacter curvus* 525. 92 | GCGACGGAUGACGUCCT | 639 | GGATGTUGCTAGCUAGCGG | 640 |
| *Campylobacter hominis ATCC BAA-381* | CGCGCGAAAUGCUGAGA | 641 | GGUGAAGCGAUGGCGAA | 642 |
| *Campylobacter hominis ATCC BAA-381* | GCTTCACCGAAUCCGUCG | 643 | TTTAGCATCTATUGAAAAT AGTAGGATTUCACC | 644 |
| *Campylobacter concisus 13826* | GGTTTGAUGGCAAAAATTTG TGUGGT | 645 | AGTTAATTGTGGCUTTAGC TAGGATAAAUT | 646 |
| *Akkermansia muciniphila ATCC BAA-835* | TGTAAGCGGCGUTGTATTTG UCC | 647 | CCAATACCAGUCCAGUGCA GC | 648 |
| *Akkermansia muciniphila ATCC BAA-835* | CATTATCAACGGUTTTCAGC GTGUAG | 649 | AAGAAAGUAAACCTTACTA UCACGGC | 650 |
| *Atopobium parvulum DSM 20469* | TAGGTCCTATAUTCCCCAGA CUCAAA | 651 | AGTAGTTTTGTCUACTCTT GGAGTAGUG | 652 |
| *Atopobium parvulum DSM 20469* | TAUTGATTCAAGTTTTGUGA AGAGAGAAAAAC | 653 | AGGACCTAUACTTGCAATT UAAACGAC | 654 |
| *Veillonella parvula DSM 2008* | GGAACUCACGAACUGACCAA AGA | 655 | CAGATTTAATAAAAGCAUC CCCATTTTUAGCC | 656 |
| *Veillonella parvula DSM 2008* | GCGCCCATUCCAACTAATAC ATTATCTUC | 657 | CCAATGTACAGGAUAACTC TGTATUACACG | 658 |
| *Veillonella parvula DSM 2008* | AAAAGAAGCGGAUAGTTGAG TTAAUCAGC | 659 | CACTTGTGGACUGTAGAAT AUGGCA | 660 |
| *Citrobacter rodentium ICC168* | GCTGGAUGGCGGTAUCACT | 661 | GAGCAUCAATCCATGUCGG AT | 662 |
| *Citrobacter rodentium ICC168* | TGAUGCUCCGCCACCCA | 663 | AGGTGTCUACGGCACUCAC | 664 |
| *Streptococcus gallolyticus UCN34* | AACTTGAAAAAGCAAAAGAU ACAAGAGTTAAUG | 665 | AGCTTATACUAACGATAAT AAAAATUAACCCGA | 666 |
| *Streptococcus gallolyticus UCN34* | AGAAAGCCCAACGGUATAAA CATUACAA | 667 | CAATCGCTGTCTCUTACTT CATTTATTTTAUGA | 668 |
| *Enterococcus faecium TX0133a04* | CGGCGUGAUCAGCGCCA | 669 | GGTTGCTGUGCCTCTTATG UGG | 670 |
| *Enterococcus faecium TX0133a04* | AGCTTTATACAAAAGCAUAT CTGCTCCUT | 671 | CTTTAACGAACGUGTTCGC UAAAAA | 672 |
| *Enterococcus faecium TX0133a04* | AGCTCGUTCTCATUCAGCAG A | 673 | ACUCAGGAAGCTTUGGCAG A | 674 |
| *Peptostrepto-coccus stomatis DSM 17678* | CCTCCATATACCAACUTAAA TACTAAACAUGT | 675 | CCCAAGAATAUTTTGCCAA GGUCA | 676 |
| *Peptostrepto-coccus stomatis DSM 17678* | TCTTGGGCUATACCCAUAGA CCT | 677 | GAGTCGATAAUAAAGAGGC TTTTAAGUGAT | 678 |
| *Mycoplasma fermentans JER* | AGCCACTTTTUGTTCGTCTT AGUACT | 679 | ATTCAGCATATTUACCACT TGCAATGUT | 680 |
| *Mycoplasma fermentans JER* | TGGATGGATTUTATGATGCT TAUCCACA | 681 | AAGTGGCTTTUTAGTTCCT TCUGC | 682 |
| *Eubacterium limosum KIST612* | CACAAGGGUCGCCGCGUC | 683 | CCGCGAAAUACGGCGAACU G | 684 |
| *Eubacterium limosum KIST612* | AAACATAAACGAUGGAAAAC AGATTAUGGAAAA | 685 | TAAGAAATUAACGGAAGGA GAUGAAACAC | 686 |
| *Parabacteroides merdae ATCC 43184* | TCAATGUACCGGUGGGCAA | 687 | ACAGACAGCCUAATTAACG TAGUCC | 688 |
| *Parabacteroides merdae ATCC 43184* | TUCGGAACCAUCCGGCA | 689 | CCAUGCAGAAAAACCGAUU CCG | 690 |
| *Faecalibacterium prausnitzii M21*/*2* | GCCATTGCGCAUCGTCAAAA AUA | 691 | CATUGCAGGCAAGGAAUGA AGAG | 692 |
| *Faecalibacterium prausnitzii M21*/*2* | TAAGCCGAAAUCTGAAUGAC CGA | 693 | CAGCTGAUGGATGAGATGA UCGAA | 694 |
| *Faecalibacterium prausnitzii M21*/*2* | CCTUAACGGCAACCACGAUG | 695 | GCGCTUCCAGCAUGCCA | 696 |
| *Faecalibacterium prausnitzii M21*/*2* | CCGGUAUGGAAUAGGAAAA AGC | 697 | AUGCCCCCGCGCAAAAUC | 698 |
| *Parvimonas micra ATCC 33270* | CCAGCACUCCGACTATAGAT TUAGT | 699 | AATACAAAAGUATTCTGAU GACGGAGAG | 700 |
| *Parvimonas micra ATCC 33270* | AGCTACTGAUCCCCAAAGTA AAATTCUC | 701 | CGGACAACGAAGUCCGTTG TUT | 702 |
| *Bifidobacterium bifidum NCIMB 41171* | AGCGUACCGGAAGCUCG | 703 | GUCGGCAAUGCCGGCAC | 704 |
| *Bifidobacterium bifidum NCIMB 41171* | GGCCGAATAUGTTTCGCGGA UA | 705 | GCGAGGUCAAGAUAUACGG C | 706 |
| *Collinsella stercoris DSM 13279* | CGCCACCCCACCUCAAUT | 707 | ACCCCCGUTGCGCACAUT | 708 |
| *Roseburia intestinalis L1-82* | GTCAGATTCUCTGCATAATT TTUCCG | 709 | AGCGTCAAUCAGGAUGAGG T | 710 |
| *Roseburia intestinalis L1-82* | TTGACGCUGTCATCCUGCT | 711 | AAAAATCGAGGAUCTGCUG CG | 712 |
| *Enterococcus gallinarum EG2* | GAGAAGATAAGUACCTAAAT CUGAAAGAAACGC | 713 | CAATGAAAACUGGATCACC CTTCUGAT | 714 |
| *Enterococcus gallinarum EG2* | AAGGATGTGUCCAACATGAA UCAGGA | 715 | TCAAGAAATACUGTCTTTC TTCUGACCG | 716 |
| *Enterococcus gallinarum EG2* | GAAGCCAATCCUGGTCCTGG TTUA | 717 | AAATGGGAAATAAACUTCA TGAATACCTCCUAT | 718 |
| *Prevotella copri DSM 18205* | AGTCATTAUGAAGGAGACCA ATTUCGAC | 719 | GGGCGGAUAGATTUCCGGC A | 720 |
| *Prevotella copri DSM 18205* | AUCCGCCCAGCTUAGCC | 721 | CTGATTTTGUAGAGAATCC CTCGTUGA | 722 |
| *Prevotella copri DSM 18205* | AACCUTGCACAUCGAAGAGG | 723 | ATTGCTTTATCGTUACTGA AATTCATAATCTUC | 724 |
| *Prevotella copri DSM 18205* | AGACAATTCTUGGCAAACAA TTCUGG | 725 | GCAAGGTUCCTACGAAAUC AAGC | 726 |
| *Holdemania filiformis DSM* 12042 | ACCCUCCTUACCCCACCA | 727 | AACAGGCGAAGGAAAAAUG TACTUAC | 728 |
| *Holdemania filiformis DSM* 12042 | AGCGUCGACGCTCTAUCCA | 729 | AGGAGGGUGAACGTTTUGG T | 730 |
| *Helicobacter bilis ATCC 43879* | AAACAGAAGAACAAAUTCAA ATGCGUAACAA | 731 | TTTGCATGGUATTCTAGCU CAGC | 732 |
| *Helicobacter bilis ATCC 43879* | GGATATGTATAATCUCAATC CACAAGATAUCAC | 733 | CGACAUGATATGCACUCCC AGAGA | 734 |
| *Anaerococcus vaginalis ATCC 51170* | TTATGCAACGAAUATCCTAA ATACAAUGGAT | 735 | AAACTCCATUAAGCATAGG TAATGAUGAGA | 736 |
| *Anaerococcus vaginalis ATCC 51170* | AGUCTAAATTCTAAATCUAG GGCAACGG | 737 | ATTGCCGAUTTTCAGGAUA AGCCA | 738 |
| *Anaerococcus vaginalis ATCC 51170* | TCGGCAATATCAUTTTGATT TCCTUCA | 739 | AATAGTTGCGGAUTATATA ATCAACAAUCCAA | 740 |
| *Collinsella aerofaciens ATCC 25986* | GAGCAGUCGGGTGTCUCC | 741 | GGGAAAUCAGCCCTTGAGA UC | 742 |
| *Dorea formicigenerans ATCC 27755* | AACCGGGAAUGACTAATCAA GUGT | 743 | CCGATTCAUCAAAGCAUAC CCC | 744 |
| *Dorea formicigenerans ATCC 27755* | ACAAAAGAAAUUAUUGGAAC CAUUGGCA | 745 | TCCCGGUTCUACCGAAACC | 746 |
| *Ruminococcus gnavus ATCC* 29149 | CCGGAGUTTGATACCAUGGG ACA | 747 | ATACCTGCUGCCCGGTUGA | 748 |
| *Ruminococcus gnavus ATCC* 29149 | GCCGCTTUTACTGGCATUGT | 749 | TCTCUTTTTCCTGUCCCGC A | 750 |
| *Campylobacter rectus RM3267* | TTGTTTGCGTCUTATACTCG TGUCT | 751 | CGATAATTTCTUAAAATTT AGATGTCUGACACA | 752 |
| *Campylobacter rectus RM3267* | AGTCATTTTGCUTGACTGTA TTTTUGGT | 753 | GCAAACAAACGGAUTTACG AAGCUA | 754 |
| *Campylobacter rectus RM3267* | AAAAACAAACAAAUTTGAGA GCAUAGAGGA | 755 | TTTTGTTTTACTTTAUCGT CCATATCGACUT | 756 |
| *Actinomyces viscosus C505* | GUACAGGCUCCCGGCGT | 757 | GCTTGCUGCAGCCCUCG | 758 |
| *Actinomyces viscosus C505* | CTCCACCGUCGGGTUGT | 759 | TTCCAACAUGTTGGCUCGC | 760 |
| *Actinomyces viscosus C505* | TGTTGGAAUGCCCGCTTAUC A | 761 | CUTACGCCGUGGCCGGT | 762 |
| *Campylobacter gracilis RM3268* | CGCGCCUCGTCGATCUT | 763 | CGCCGUGCTTTTUGACGA | 764 |
| *Campylobacter gracilis RM3268* | GCACGGCGUCAATGCUT | 765 | TGCCAACGGCUTTATATAT TTCTACAUC | 766 |
| *Campylobacter gracilis RM3268* | TCCTAGATTUGCGATCAGCG UAAG | 767 | AGCCGTTUTACGCGCUG | 768 |
| *Campylobacter gracilis RM3268* | GCGGCGAUTGAGCGAAAUT | 769 | TGGGCGAGAGUUAUCGUG C | 770 |
| *Peptostrepto-coccus anaerobius 653-L* | CCATAACGGUCTTACTGCTC TUGAA | 771 | AGTTACAGGTAGUCCCATC TCTAUACAG | 772 |
| *Peptostrepto-coccus anaerobius 653-L* | AGACTTGCAUGTTCTCCTGA UGA | 773 | GATCGTAAACGUAACCACA TGGUC | 774 |
| *Prevotella histicola F0411* | TTGATAATGTGUTTACCAAC AUCACCAC | 775 | CAGACGGTCUCAGTATTGT TCUGAT | 776 |
| *Prevotella histicola F0411* | ACCGCAACCCUTGUGAGGT | 777 | AGGUGCUAACGGCGAGA | 778 |
| *Helicobacter bizzozeronii CIII-1* | GATCCAAAGUGATGGGTCCA UAGAG | 779 | TGCCCAAAAUCTCCAAAAG ATUGT | 780 |
| *Helicobacter bizzozeronii CIII-1* | TGCTTUGGCTTCUCCCACT | 781 | CGATTTTATGGATUGCTTA AAAAGGGTUAAGA | 782 |
| *Helicobacter bizzozeronii CIII-1* | TGTGGAACAAAUGAGTATTC UAGCCAA | 783 | ATAAACATCGGUCGCACGA TUAGT | 784 |
| *Enterococcus hirae ATCC* 9790 | AAAACTTATGATUGACAATC GAGGCAUT | 785 | TGGCTGATGUTTGGTCTGU ACA | 786 |
| *Enterococcus hirae ATCC* 9790 | AAACGGAAGAAGGAGUCTAT CAUGA | 787 | ATCCTACACGACUAATCAT TAGAGAAAGUT | 788 |
| *Bacteroides nordii CL02T12C05* | CGUAGAGCCTUCCCGGT | 789 | GGGCACCGAUGAGAAAAGU T | 790 |
| *Bacteroides nordii CL02T12C05* | TGATCACUCCGGCUACAAAG GT | 791 | TCCGGATAUAGATACTATU GCACCG | 792 |
| *Bacteroides nordii CL02T12C05* | AAACGCCUTAAATTGATUCA AGCGA | 793 | GTGGUGAAAGTTTCTGUGC CC | 794 |
| *Bacteroides nordii CL02T12C05* | TCAAGTTTCCTUCTAAAAGT AGCUCGT | 795 | GGCGTTTTCUGGTGTTTAT GTUCT | 796 |
| *Barnesiella intestinihominis YIT 11860* | AATCTTTGATUGGAAGGTTA GAAGTAUAAAAGG | 797 | ACGCAAGAUUUCAUUCUCU GAAAGAGGAG | 798 |
| *Lactobacillus murinus ASF361* | CTTTGCGACCACACUTAGCU C | 799 | ATTCATAAGCGGUCGTGAC TTTTAACUT | 800 |
| *Lactobacillus murinus ASF361* | TGACTCACCUTCATATUCAA AGCC | 801 | ACGTTTUGAGCGATACGGU CC | 802 |
| *Eubacterium rectale CAG: 36* | AATTACTCCTCTCTUCTTTT AACCTTTGATCUG | 803 | TACCTTATTATGAUATCGT CATCAAAUCGCC | 804 |
| *Cloacibacillus porcorum* | TCTCTTGATGUACTTGTTAA TAAUGCCG | 805 | AGAGCACTAUTCGACGCUA CC | 806 |
| *Cloacibacillus porcorum* | AGUGCTCTUAGCGGACGC | 807 | TTCTAATAGACGUTCACGT GATATUGGT | 808 |
| *Blautia coccoides* | CTTCCATCCUCAGGTATACU CCAG | 809 | TGCTCTGTAAAUGGAAAAT AGTCCAUCAAAT | 810 |
| *Ruminococcus bromii* | GCGGUATTTAUGAAGAACAG CGT | 811 | CCCGACAAAATUTCTTCAA GAGTAUCC | 812 |
| *Phascolarcto-bacterium faecium DSM 14760* | CCGTUGCAAAGGCTTUACAC | 813 | CGGCCCAGUAACCAGAAGU A | 814 |
| *Phascolarcto-bacterium faecium DSM 14760* | GGUTCTGGTTTTUCGAAAGC GAG | 815 | CCTGUCAGCAATAGTUCAG CACT | 816 |
| *Helicobacter salomonis* | CCUCCACAAATTUGAGGGCT | 817 | ACAAGGACUATATGAAGTA TAUGCAAGCG | 818 |
| *Helicobacter salomonis* | TCACTAATCTTTUACTTGCC ATCTCUCC | 819 | TGUGGAGGCGTUGGCAT | 820 |
| *Gardnerella vaginalis* | TTGCCGCTAUAGGAGCAGUA A | 821 | ATTCTGCTTTAAUTGAACG CAAUCG | 822 |
| *Gardnerella vaginalis* | AGCAGCAGUCGTGTTUGG | 823 | AGCGGCAACAACUGAGAUG A | 824 |
| *Gardnerella vaginalis* | TTTTGGCAACUTGGGCUAGG | 825 | ACCCAAGUGACATUGCGCT | 826 |

| **TABLE 16D (PRIMERS/PROBES SEQ ID NOS: 827-1258)** | | | | |
|---|---|---|---|---|
| *Bifidobacterium longum* | ACCAAGGTTCTAGCCGGT | 827 | GGCTTGGTGGCAGTAAGTG | 828 |
| *Bifidobacterium longum* | ACCATCTGGATTGCCGCA | 829 | AGTGAAACAACAGTATTGA TGCCG | 830 |
| *Clostridioides difficile QCD-66c26* | ACATTTGCTGAATCTTTTGC TCTTTTTACT | 831 | TCAAGATAAAGGACATCAA GTGTTAGGT | 832 |
| *Clostridioides difficile QCD-66c26* | CATCTACTGAAGCTGCTTCA AATTAGT | 833 | TTTGCTCTTTGATATTTTT GCCATACAGAT | 834 |
| *Clostridioides difficile QCD-66c26* | ATCTTGAATAGTAACTTTTA AACTTTGCCCT | 835 | GATTCTGCTAAACTAATCG AAGAGGTTAGA | 836 |
| *Lactococcus lactis subsp. lactis Il1403* | CAGCGAATAATAATTCCCCT TGACAG | 837 | GGATGACTTTCTATCGGCA CTTCA | 838 |
| *Lactococcus lactis subsp. lactis Il1403* | GCAACAGCACTTCGTAACGA T | 839 | GGAGAACCAAATTCAACAC GAGTTT | 840 |
| *Chlamydia pneumoniae TW-183* | AATTCACAGCTTGAGGAAAA GGTGT | 841 | TGGCAACATCTGTTCAGGA C | 842 |
| *Chlamydia pneumoniae TW-183* | TGCGTTGCTCGCTCTCT | 843 | TGCACTCTTTCAGAAAGAA GGTCTT | 844 |
| *Chlamydia pneumoniae TW-183* | ACGAAGAAGCTGTGGAGAAG T | 845 | CCTTGAGACTACCAGGGAG C | 846 |
| *Chlamydia pneumoniae TW-183* | AAAAGTAAACAATAAGAAAG AGGTTCAATATGC | 847 | CGCGCAACATAGACTCCC | 848 |
| *Fusobacterium nucleatum subsp. nucleatum ATCC 25586* | AATTGTTCCTCATCAACTAT TTTAATTCCTTG | 849 | GTAGCGAGGAGGATTATAG TGAAAGA | 850 |
| *Porphyromonas gingivalis W83* | GTGGCTTTCTTATGTGCATG GATTTG | 851 | TATTCGTAATTAGAGTAGG AGGAGAAGCTTTT | 852 |
| *Porphyromonas gingivalis W83* | TGTGGCACATGACAGTCGTT G | 853 | CATAAGGTCTTTGCGCTGG T | 854 |
| *Helicobacter hepaticus ATCC 51449* | GTGGCAATTACTTGCGTATT TGG | 855 | CCTGCTCAACCCCTATCTG G | 856 |
| *Helicobacter hepaticus ATCC 51449* | AGACAAAGTATCAACATTGC TCATACCT | 857 | CGAAAGCGGGAATGCTCCA A | 858 |
| *Lactobacillus johnsonii NCC 533* | AAATGAATGGGTAGAAGCTG GTGT | 859 | TTAAGATAACTAGGTCGCC GACTAC | 860 |
| *Lactobacillus johnsonii NCC 533* | TTCAGCTTCATTAGAAGACC TCGG | 861 | CGTCAATTTGGACTTTACT GATTGGA | 862 |
| *Lactobacillus johnsonii NCC 533* | TCACCATCAAGTAGAACTGT ATTTTGTGT | 863 | CCAGAAGAATTGCTTCCCC AT | 864 |
| *Lactobacillus johnsonii NCC 533* | ACAATATTGGTCTTTTATTT TTAGCAACTTGT | 865 | AGCTTATATTGAGGATTGT GGCTACAC | 866 |
| *Cutibacterium acnes KPA171202* | TCGGTGTCATTGGGATCGAC | 867 | CTGGGCGACGACGCTTT | 868 |
| *Cutibacterium acnes KPA171202* | GTGCCGTCATTGACCAGCAT | 869 | CGGAGGGCTATCGCGGA | 870 |
| *Helicobacter pylori 26695* | GTGCCTAAAAGCACAAGCAA TTG | 871 | AGGGAGTTTAAAAATGAAA CGCTTTCAA | 872 |
| *Helicobacter pylori 26695* | AAAGGTGAGAGGATTTAGGA CTTTTTACTAAA | 873 | CTAGAGAGATAGCACCTAC TATAACAGATTTC | 874 |
| *Borreliella burgdorferi B31* | AGAGAAACCAGTTGGCCTTT TGG | 875 | AACAAATCCTCGATTTATT TCATGGCAG | 876 |
| *Borreliella burgdorferi B31* | AATGGATTTATTTTGATTCC GAATATGCTTTT | 877 | ATTGCCAATATTCAATCTT CTAAATTCATCAAT | 878 |
| *Borreliella burgdorferi B31* | TTGGCAATGTGATCTTTATT GCAATTTAATT | 879 | AGAAATGAGATAGCTTTTA ATAATCACTGCA | 880 |
| *Chlamydia trachomatis D*/*UW-3*/*CX* | GCTGCAGGGATTATTCTTTC TCCA | 881 | AGGGCTCTATCTATCAGAA TCGGAA | 882 |
| *Chlamydia trachomatis D*/*UW-3*/*CX* | AGAGCCCTTCTCGAATATGG GA | 883 | AAATCGGGTGCACCTTCTG TAA | 884 |
| *Chlamydia trachomatis D*/*UW-3*/*CX* | AGCAAAAGCTTGCATATTGG CA | 885 | ACCTCTATAGGTGTCCGTT ATTTTGATG | 886 |
| *Campylobacter jejuni subsp. Jejuni* | GCGTTCTCCATCTTTTATAG CAGAAATACG | 887 | TTATTTTAGTGGGTTCTGC AATGACAAGATA | 888 |
| *Campylobacter jejuni subsp. Jejuni* | AACAATTCTTTTAGCCTAAC AGTGCCA | 889 | GCGAAAGTTACTTAGGTGG TCTTGC | 890 |
| *Campylobacter jejuni subsp. Jejuni* | GTTATGAAGCTTATTAATGG TAGTGGTGATGA | 891 | CCTCAAATTGATCTTCTGC TGAAGTATTA | 892 |
| *Bacteroides fragilis YCH46* | TTGGCGGATACAGCCCT | 893 | ATCCAGACTCTCCTGATTG TCCA | 894 |
| *Bacteroides fragilis YCH46* | GATCTGCCATAGAATCTCGT CG | 895 | CGGCTGAAGAAGAGTGGGA A | 896 |
| *Bacteroides fragilis YCH46* | TCCGGGCAGCGAGTCTG | 897 | GGCAGATCGATTGCAGGGT | 898 |
| *Lactobacillus reuteri JCM 1112* | AAAAACGGAGGAGACTAATT AATATGGCAA | 899 | TGCTTTTGCTTCTTGTAAT TACGAATTAACT | 900 |
| *Lactobacillus reuteri JCM 1112* | CCGGTTGACCGTATACTACG CT | 901 | CACAATCGTTTTTAGCTAG AATCACTGTT | 902 |
| *Bifidobacterium adolescentis ATCC 15703* | GGAACAGCCGTCTGATCAC | 903 | AAAAACACTCATTGTTTTC ATCGTTTTTCA | 904 |
| *Bifidobacterium adolescentis ATCC 15703* | CCAAAGACTTCGAGTAGGGC TTG | 905 | GATTGTTCATATGGGCTCT CCTATCC | 906 |
| *Bifidobacterium adolescentis ATCC 15703* | CGCCGAATGATGTTCGAAAT ATGGT | 907 | CCGACAATCTCAAGAAAAC GCTGAT | 908 |
| *Lactobacillus rhamnosus* GG | ACGGGTCTTAGCATTGGCTT | 909 | GCACGCGTCAATTAAGCCC | 910 |
| *Lactobacillus rhamnosus* GG | TCAATGGTTAAGTTGGCCGT AG | 911 | ACGATCACTCAAAATGGTG CG | 912 |
| *Bacteroides thetaiotaomicron VPI-5482* | CCAAAGCATTGGCATATGCA GATA | 913 | AAGCCCAATCGTCATCTTT GTAGTT | 914 |
| *Bacteroides thetaiotaomicron VPI-5482* | ACTAATAATAAGGGATTTTC TGAATTTGGTGAT | 915 | AACTTTTTAGTATCCTTAG CGAAGTTGAC | 916 |
| *Bacteroides thetaiotaomicron VPI-5482* | TGCTCAAAGTGAGAACTTTT CAAATCGTAA | 917 | TCTGTTTGTGAATAACTAC CGTTAGGAC | 918 |
| *Mycoplasma penetrans HF-2* | AGCATTACTACAAAAAGAAT CAAGCAATAATAA | 919 | ATTTAGGGTGTAACAAAGA TGAAAAACATTAAT | 920 |
| *Mycoplasma penetrans HF-2* | GCACCTGCTTTTATAACATC ATTTCCA | 921 | ACAGAAGAAAATATGTCTG CTACAAATAGAT | 922 |
| *Mycoplasma penetrans HF-2* | GTAATCCTACTTTCATCATA TGAAGAAGAACT | 923 | GGTGCAACATGAAATCAAG GTGA | 924 |
| *Mycoplasma penetrans HF-2* | GAAATTGCTACAGAGATAGT CCCACC | 925 | GTAATGCTTTTAAAAATCA TTCTAATGACCCA | 926 |
| *Lactobacillus acidophilus NCFM* | ACTGGCAATTCATCAGAAAA TACATCTAC | 927 | CCGTAGTTTTTCCTTGCTG ACC | 928 |
| *Lactobacillus acidophilus NCFM* | GGACAGCTACCCTTGTTGCA | 929 | AAAGCACGATTAATAGTTA AATTACCAAAAACA | 930 |
| *Lactobacillus acidophilus NCFM* | CGCTTCAACTGATCATGTAG AAAAAGTG | 931 | CAGCATGACTGTTATCAGT GTTTGTT | 932 |
| *Lactobacillus acidophilus NCFM* | GGTGTTAAGGTGAATTGGAC TCAAAC | 933 | CCTGTGCCCAATTCATTAT TAGTATTCAT | 934 |
| *Desulfovibrio alaskensis G20* | AAACCTTTGCCGGGCGTC | 935 | CGCATCAGGCTCCCGCA | 936 |
| *Desulfovibrio alaskensis G20* | GCGGATATCACGGACGC | 937 | GGCTGCGGTTGTGGTCG | 938 |
| *Desulfovibrio alaskensis G20* | AGGTACCGGCCTGCTGCAT | 939 | TTCGCTGCCCGAAGCCG | 940 |
| *Desulfovibrio alaskensis G20* | AGCAGAAAGACAGGCATGAT G | 941 | AGCACCTACTGCATCGCC | 942 |
| *Bacteroides vulgatus ATCC* 8482 | AT GCAGCCACAACCAATCG | 943 | TTCGGCCACATTCCATCCT AA | 944 |
| *Bacteroides vulgatus ATCC* 8482 | TTGCTGACCAAAACCACCAC | 945 | TTTTTATGGAATGTTTTTC TGTCGGG | 946 |
| *Bacteroides vulgatus ATCC* 8482 | GTTCCTATTCCTATCTCTTC CGGTGG | 947 | CCGCCTTTGATAGATCCGC T | 948 |
| *Parabacteroides distasonis ATCC 8503* | AGTCCCAACGCCATTGTGC | 949 | CAAGGATGTTTATGAACGG CAAAACA | 950 |
| *Parabacteroides distasonis ATCC 8503* | GAATATGAGCCATGAGATAC GTACGC | 951 | AGAAAGACATGCTACCGGA TTCTATG | 952 |
| *Lactobacillus delbrueckii subsp.bulgaricus ATCC BAA-365* | GAAGCTGGATTTGCCGACCT A | 953 | GCGGGCACAAAACTCTTCA | 954 |
| *Lactobacillus delbrueckii subsp.bulgaricus ATCC BAA-365* | ACTCAGGCGACTCAGTCTTG | 955 | GGCGGTTCTGGTCAAGC | 956 |
| *Lactobacillus delbrueckii subsp.bulgaricus ATCC BAA-365* | TTCTGACGCCTATGGGACA | 957 | GGTTGCGGACCTGCATC | 958 |
| *Campylobacter curvus 525.92* | CCCACGAATGCGATCACG | 959 | CAGCAAGGCCGATGAGATA AG | 960 |
| *Campylobacter curvus 525.92* | GTGACATCTGAGGTAGATGA TATGGC | 961 | ACTCGGCACAGATACAAGC A | 962 |
| *Campylobacter curvus 525.92* | AGCCAGATCTCCACGCTC | 963 | TAGGGCATATCGATAAAAG CTGTAATAAAAA | 964 |
| *Campylobacter curvus 525.92* | ATGCCCTAAAAATCGCAAGC T | 965 | GATATGGCTGCAAACGCGA | 966 |
| *Campylobacter hominis ATCC BAA-381* | GCCGGAGTATCAAGATTTAA ACCATAAG | 967 | AGATTGTTTTATTTATTTG CAAAGAGATGACG | 968 |
| *Campylobacter hominis ATCC BAA-381* | CTTTGCAAAATTTTGCATAT TCACCGA | 969 | GATTGATGTGGCTATTAAA AGTATCGGC | 970 |
| *Campylobacter hominis ATCC BAA-381* | GCTGACGCTCTCATAAACGG A | 971 | TTGCAAAGAATTTTGCGCC ATTATT | 972 |
| *Campylobacter hominis ATCC BAA-381* | AGGTTTAAAGTATTTTCTAC AAAAACTTCAACA | 973 | ACTCCGGCAGAAAGGGATT | 974 |
| *Campylobacter concisus 13826* | CATCGATAAGCTCATCATCA TGCCAA | 975 | TAAATTTATCTCATAGTCT GAGATATCGACCT | 976 |
| *Campylobacter concisus 13826* | ATAATACGAGCAGCACACCT ACCG | 977 | AAATGAACCGGATCAAAGC TCCC | 978 |
| *Campylobacter concisus 13826* | AGAGGAGTCTTTTAAAAAGA CTGAAGAAGAT | 979 | TTGCGTCAGTGATCTCAGA AACAT | 980 |
| *Akkermansia muciniphila ATCC BAA-835* | GGCATTCTGAGGTACCGGAA | 981 | TTTTCGCCTCTCACATTGG AAATTATT | 982 |
| *Akkermansia muciniphila ATCC BAA-835* | TGGGCATGATCGGAGAAAGA AG | 983 | TTGCCATGGTATTCCTTGG CG | 984 |
| *Akkermansia muciniphila ATCC BAA-835* | CCAATTGAACTACTGACCTG TTGGAG | 985 | CACCGTGGGTGCTGGTCG | 986 |
| *Bifidobacterium animalis subsp. lactis AD011* | CGCAGTACATGGATCACCTG TTC | 987 | CGTATGCGATGCGTTCGC | 988 |
| *Bifidobacterium animalis subsp. lactis AD011* | CGCATACGTGCAGCGGT | 989 | GGACAGGTGCCCGGTGG | 990 |
| *Bifidobacterium animalis subsp. lactis AD011* | CTGTTCTGCTGGTTCTGCGA | 991 | GCCGTAGTAACAGCCTCGA | 992 |
| *Bifidobacterium animalis subsp. lactis AD011* | ACTACGGCATCATCGTTGTC T | 993 | GTTACGCGCATCGAGCC | 994 |
| *Atopobium parvulum DSM 20469* | GCAGCCAGCCCTTCTTG | 995 | GGCAGAAGATTTGATGCTC CAT | 996 |
| *Atopobium parvulum DSM 20469* | ACAGCCGCTTGATTATATTT AAACTGCC | 997 | AGAGGTATTCCAAATGCAG CTTATTG | 998 |
| *Atopobium parvulum DSM 20469* | ACGATACCAGTAATACTTAT TAAACTCATCAAA | 999 | TGGCTGCTTGGAAACGAG | 1000 |
| *Veillonella parvula DSM 2008* | GCTGGTATTGGTATGATTCC AGATGG | 1001 | AAACCAAACCGTTGCCCCA TA | 1002 |
| *Veillonella parvula DSM 2008* | TCGACTGATATATCAAGAGA AAGAAAGTGTA | 1003 | CATCAGCCATGTGTACAAA ACCT | 1004 |
| *Veillonella parvula DSM 2008* | AGAAACGGCTATACCAATTC ATGAAGAG | 1005 | CTTCGTTCGTAATAGATGG CTCTACAATAAG | 1006 |
| *Citrobacter rodentium ICC168* | GCGGAATGGCGTTTACAGT | 1007 | TTTAGCTTATCAATAGCAC AATTTTAGAAAACA | 1008 |
| *Citrobacter rodentium ICC168* | GCCACCCAGCCATGATG | 1009 | GCGCGGTGGAGGTGTCTA | 1010 |
| *Citrobacter rodentium ICC168* | ACTATGAATAAAATTTATTT CTCTCAAGACCCG | 1011 | TGGGTGGCGGAGCATCA | 1012 |
| *Citrobacter rodentium ICC168* | CTGGATACGCAGACCGATGT | 1013 | CATTCCGCTGTTTCATCTG CA | 1014 |
| *Streptococcus gallolyticus UCN34* | ATGTTGTTCAAGGTGACGGT ACTG | 1015 | CAAGGTTTCAAGGAACATT GAAGTGATAA | 1016 |
| *Streptococcus gallolyticus UCN34* | CAAAACAGGAGATAAGATTT TTGTCACAGGA | 1017 | AAACAGTTCAGCACGTTCC TGA | 1018 |
| *Streptococcus gallolyticus UCN34* | CGGTGACACCTAAAGAACTG ATGATATTCT | 1019 | TGACGATATCCTTTTTATT CAAGTCTCTAAGG | 1020 |
| *Enterococcus faecium TX0133a04* | TAATGAAATCCAAATATTCT CTTTCTTTATGGC | 1021 | AACGAGCTAGCGATCGCA | 1022 |
| *Enterococcus faecium TX0133a04* | TCCTGCAATCACCGGCA | 1023 | TCACGCCGATGAATGAAGA G | 1024 |
| *Enterococcus faecium TX0133a04* | ATTCTACCCATGTCTCTGGG ATTTTGA | 1025 | AGAAAAACCAAAAGCAACT GGTACG | 1026 |
| *Peptostrepto-coccus stomatis DSM 17678* | GGATTCATGGATAGGAGAAA GGCT | 1027 | TGCCGCCTACCTACCAGTA TG | 1028 |
| *Peptostrepto-coccus stomatis DSM 17678* | GTATCCTAGATATGTCATTT AGGTCTTCTACA | 1029 | AGAGATTGATGACCTGACT ATAGAGTCT | 1030 |
| *Peptostrepto-coccus stomatis DSM 17678* | TTGAACTTGAATCGACCCTA TGCA | 1031 | ATGAATCCAAATAGGGATT CTGACTATGT | 1032 |
| *Peptostrepto-coccus stomatis DSM 17678* | ATCTCTATATCAAAGCTCCT GGACACA | 1033 | AGGTTTAGGAAGGAATTTA CAACTGAAAATA | 1034 |
| *Mycoplasma fermentans JER* | TCCTTGCGACTTTTGCAAAT AATATTGA | 1035 | AAAGATCTTGATTATGAAA TTCAAGAGCAATT | 1036 |
| *Mycoplasma fermentans JER* | TTTTTCAGCTTGCAAACGCT TTATTAAATT | 1037 | TGAATTGCCTATTTATACA CGCAATAAATTT | 1038 |
| *Mycoplasma fermentans JER* | TCGGTTAATTTACTGAATGC AAAAAGTAAAAA | 1039 | AATACAAATAATCTATCGC TTTTTGGGTGT | 1040 |
| *Mycoplasma fermentans JER* | TTTTACATTCTGTTTACCAG GATCAATTACA | 1041 | GCCTTCTTCAAATTCTTTA TAGCTTTTTGC | 1042 |
| *Eubacterium limosum KIST612* | TTTCGCGGTGTAGAGCCG | 1043 | CTGCAGAGCCGGCCCTC | 1044 |
| *Eubacterium limosum KIST612* | GCTGAGCCGGTCAATGC | 1045 | AGTGTGGCACCAATGAACC | 1046 |
| *Eubacterium limosum KIST612* | GTTCCGGTAAAAGCAGGTGT | 1047 | ACCCGCTGGTCAATTTCTC T | 1048 |
| *Eubacterium limosum KIST612* | CACCTTACATGTAAAAATTC TTGCGATTTC | 1049 | CCGGAACCCCATCCCTGT | 1050 |
| *Blautia obeum ATCC 29174* | CTTCTGCATCCCGAACCTCC | 1051 | TATTTCGTTGGCAATAGAA GAGCCA | 1052 |
| *Parabacteroides merdae ATCC43184* | CACTTTTATACTGTACCTCG ACCACA | 1053 | GGGCGTAGTCGGTGAGT | 1054 |
| *Parabacteroides merdae ATCC43184* | CGACCCTGACACTTTTTGCA TT | 1055 | TCATGATGAGAACTTGGAG ATAAAGCCT | 1056 |
| *Parabacteroides merdae ATCC43184* | CTACGCCCACTTTAAACTGT GG | 1057 | CAGGGTCGATATCGATATC GATAATGT | 1058 |
| *Parabacteroides merdae ATCC43184* | TCCTTGCAGGCATTCAGGT | 1059 | ACTGACTATAAATTGATAT TGTGTGATGACAG | 1060 |
| *Faecalibacterium prausnitzii M21*/*2* | AAGCCGAAATCTGAATGACC GA | 1061 | TCGAAGAAGCACTGCATCA TGTC | 1062 |
| *Faecalibacterium prausnitzii M21*/*2* | GTGCAGGCGATCTACAACAT TC | 1063 | AATAATTATCAGTTGCTCG CAGCCT | 1064 |
| *Parvimonas micra ATCC 33270* | CTAAAGCTTTGTCTATCTTA TCAACAGCT | 1065 | GGTAACTCAGACGAGTTCT CGTG | 1066 |
| *Parvimonas micra ATCC 33270* | AGATGGATTGTTTATCCAGT TTTCTGTG | 1067 | GGAACTACACTTTCTTTTA ATGCTTTTAAAGAT | 1068 |
| *Parvimonas micra ATCC 33270* | GCGAATAAATATTCTACTGA CGCTTCAT | 1069 | TCTTGTTGCCTTCAGTTCC AACT | 1070 |
| *Parvimonas micra ATCC 33270* | CCATTGTTGAGTCGTCAGCT TCATTTAT | 1071 | AGCTTTAGCAAGAGCTATA AACCAAGT | 1072 |
| *Streptococcus infantarius subsp. infantarius ATCC BAA-102* | GCTGAGACAATTCTTTTTCG AACTCA | 1073 | GCCAGAAGCGACAGTAGCT TA | 1074 |
| *Streptococcus infantarius subsp. infantarius ATCC BAA-102* | TGATATCATCAACATTAAAC ATCTCATAGTCC | 1075 | ACCAAGCTTTTATAAGAGA GTTGCTCT | 1076 |
| *Streptococcus infantarius subsp. infantarius ATCC BAA-102* | AGCTTGGTAATTCAGACAAA TCAATTCG | 1077 | GTCTCAGCATGATTATTTC CATTCACG | 1078 |
| *Bifidobacterium bifidum NCIMB 41171* | CGTCGCCAAGCCTTCGA | 1079 | TGGTTCTGGTCGACCTGT | 1080 |
| *Bifidobacterium bifidum NCIMB 41171* | GACCTCGCTTACCCGGAA | 1081 | ACCTCCTGAATCTTATCCG CGA | 1082 |
| *Bifidobacterium bifidum NCIMB 41171* | CACGGTGGCCGCTTTAATG | 1083 | TGGCGACGGTACTTGGC | 1084 |
| *Bifidobacterium bifidum NCIMB 41171* | CATCAGCGTCAAATCAGTCA ACCG | 1085 | GGTACGCTGTTCGCCGT | 1086 |
| *Collinsella stercoris DSM 13279* | AGGAGTAGACATCCATGAAT CCG | 1087 | TTCGCGTCATGGCATATGC T | 1088 |
| *Collinsella stercoris DSM 13279* | GGAACTGGATGTATCGCGAT GA | 1089 | GTCGCCAAATGGGCGAT | 1090 |
| *Collinsella stercoris DSM 13279* | TGTAAAACCGGCGAGGTGG | 1091 | CGCTCAAATGTCCTCGCT | 1092 |
| *Collinsella stercoris DSM 13279* | TTTGAGCGCACAAGTAGGGT | 1093 | CCAGTTCCCAGTCCATGCA | 1094 |
| *Roseburia intestinalis L1-82* | CCGGTTTCCCTGGTTCG | 1095 | CTGAATTTACGCGTGAGGT GA | 1096 |
| *Roseburia intestinalis L1-82* | CGATCACTCCAAATCCGGAG CATA | 1097 | AACCGGGTGGCAGCCGTA | 1098 |
| *Roseburia intestinalis L1-82* | CGGCACCTTTCTGGCAC | 1099 | GACTGTGGCTTGCTGCA | 1100 |
| *Roseburia intestinalis L1-82* | CTGCCCGGTATTTCGCATT | 1101 | ACGGGCACAGATTATCGTG T | 1102 |
| *Enterococcus gallinarum EG2* | TTTGGAGCAATGATTATCGG TCCATTAA | 1103 | CTCCAATTAAGCCTGCAGA AAAATTACG | 1104 |
| *Enterococcus gallinarum EG2* | ATTACGGTACCTGGAAATGA AGGCT | 1105 | GATAGCACGACCGATCAAA TAAAAATACTATT | 1106 |
| *Enterococcus gallinarum EG2* | ATGGTTGGTATGGCAGTTAT TGGC | 1107 | TTGATAATGCCTTGTAAGA ATGCCC | 1108 |
| *Prevotella copri DSM 18205* | CCACACCATTTTTGCCCTTT CAC | 1109 | CGGCTTCACCCAGTTCG | 1110 |
| *Prevotella copri DSM 18205* | TGAAGCCGGATGGCTTGA | 1111 | TCTTCAAATTTTAATTCTT AGATGTTGATCCAC | 1112 |
| *Holdemania filiformis DSM* 12042 | CGTCCCAGCTGACGCAA | 1113 | TCGGTATGGATTATCCGT CCT | 1114 |
| *Holdemania filiformis DSM* 12042 | CTTTAAAATCAGATCCAGAT TTTCATGTTCCA | 1115 | TGAAGAAAATTCCGCCGCT GA | 1116 |
| *Holdemania filiformis DSM* 12042 | GCCATAGACCGCTCTGACTT CC | 1117 | CGCAGCTCAGACCATTCAT TGG | 1118 |
| *Holdemania filiformis DSM* 12042 | TTGGAAGACGTCATCCTCGA TATAATGA | 1119 | TCAATGCAACCCTTTCCCA G | 1120 |
| *Helicobacter bilis ATCC 43879* | AGAGTGAGACAATTACGCTA CCTTG | 1121 | TTGATATTTCATTTTCAAG GTGTTTAAAGTGAG | 1122 |
| *Helicobacter bilis ATCC 43879* | AGATTCTAAAGAAGTGCTAG ATTTAAGTGCG | 1123 | TTGATGACATTTTGAGAGA ATGTCTTGCATA | 1124 |
| *Slackia exigua ATCC 700122* | GGAATGTGCGTCGAACGG | 1125 | CCAGCTGCGGTTGCGATT | 1126 |
| *Slackia exigua ATCC 700122* | CCGTACCGGATTCCAGCGTA T | 1127 | GTCTGGAATGTAGAACTAT GCGATGATATAT | 1128 |
| *Slackia exigua ATCC 700122* | CTCTTGGCGCGAATGGAC | 1129 | TGGGCGGCTATCTGGATG | 1130 |
| *Anaerococcus vaginalis ATCC 51170* | AAGGACTTATGCCTCAATTA ATTCAACC | 1131 | TCTACCGCAGATAAAACTC CCACTA | 1132 |
| *Anaerococcus vaginalis ATCC 51170* | ACCTATAGTCATATCAACTG GAATTGCG | 1133 | AAGTCCTTGCATCCACTTT GG | 1134 |
| *Anaerococcus vaginalis ATCC 51170* | TACTGGAGATGTATTAGTGG GAGAAGTT | 1135 | TTGCATAATAATTTGTAAG GTTTTTCATCCTC | 1136 |
| *Collinsella aerofaciens ATCC 25986* | CGTTCCATCCCACCCCT | 1137 | GCATCCAGAATGCTTTTCT TACCG | 1138 |
| *Collinsella aerofaciens ATCC 25986* | TCCCCAATCTTCCGTATAGC G | 1139 | ATCAGCGAAATGCCGTTCA AA | 1140 |
| *Collinsella aerofaciens ATCC 25986* | AAAGACCGCCGTTGCGGTTT TA | 1141 | ATGGAACGGCCCATGCA | 1142 |
| *Dorea formicigenerans ATCC 27755* | ATGCATCTGTTTCCTGGCCA T | 1143 | TTTTGCAATCTGAATGTGA TCTGGG | 1144 |
| *Dorea formicigenerans ATCC 27755* | AAACAGATCACGTCCAAGGT CATC | 1145 | GGGCCGATGCATGGAGA | 1146 |
| *Dorea formicigenerans ATCC 27755* | ATCGGCCCAGTATCCGAT | 1147 | ATCCGGGTTGATTAGGAGG AAGA | 1148 |
| *Dorea formicigenerans ATCC 27755* | TTGCAAAATAACATTTGTAA TCCCAATTTCC | 1149 | AAGAGGGCAGAGTATGCCG | 1150 |
| *Ruminococcus gnavus ATCC* 29149 | ATGCCCTGGATTATCCCAAT GAA | 1151 | TTCAATGCCTCATAATGCA TCTGATC | 1152 |
| *Ruminococcus gnavus ATCC* 29149 | TCAACAGCTTGAGTAGTCTC GTC | 1153 | TTCTGCAGTAACTGCAGGG TAC | 1154 |
| *Ruminococcus gnavus ATCC* 29149 | ACGGAATGTTTTCCGCAATC GTT | 1155 | CAGGGCATAAGAGGCATAA GC | 1156 |
| *Ruminococcus gnavus ATCC* 29149 | TGCAGCATCACCTGCTGA | 1157 | GCTGTTGAAGGGCTCGG | 1158 |
| *Campylobacter rectus RM3267* | GCTTATTACGCACAATAGCG AATTAAAACA | 1159 | AATAGTTTTGTAATAACAA GATGCAACCAG | 1160 |
| *Campylobacter rectus RM3267* | AACCGAAGAAGGAGAGTTAA AGACTT | 1161 | CGGTAGTGGTGGTGTTATC GTTAAAT | 1162 |
| *Campylobacter rectus RM3267* | CAGGTTGAGGGCCATCTAAA TAATTCA | 1163 | ATTGACAAAATCATAGTTA AAAACTCCTTTGAA | 1164 |
| *Campylobacter gracilis RM32 68* | TTTACTACCATCGCGCCGAT ATTT | 1165 | ATCGCCGCGTTTTGCGT | 1166 |
| *Campylobacter gracilis RM32 68* | AAACGGCTCATCTGCGTCA | 1167 | GTTGCACCGTAAAAGAGAG GACT | 1168 |
| *Peptostreptococcus anaerobius 653-L* | AACCTAGCCATACTAGTATA GTCCCTT | 1169 | GAGTTGGTATCAGGAGATG AAGAAGC | 1170 |
| *Peptostreptococcus anaerobius 653-L* | CTGCAAACACATCAAAATAA AAGGCAG | 1171 | TGCCAAAAATAAGATACAC CTTCCTATAAGA | 1172 |
| *Peptostreptococcus anaerobius 653-L* | ACCAACTCTATATCGGCAAA ATTTGT | 1173 | ACCTGAGGGTGACGACTTG | 1174 |
| *Peptostreptococcus anaerobius 653-L* | TGTCCCTCAACCTAATTTTT GGCTT | 1175 | GTTTGCAGATAGGTGTTCA AGCA | 1176 |
| *Prevotella histicola F0411* | GTTTGGCTCAGGAAGAGAAA CCT | 1177 | GATACTACCATCGCTAGAA ACACAGAA | 1178 |
| *Prevotella histicola F0411* | GCAAAGGCAGAGGTGGACAT TAC | 1179 | TCAAACGAACAGCCTGTTC C | 1180 |
| *Prevotella histicola F0411* | TCGTTTGACGAATAACATGC CG | 1181 | AGAGCCTATCATAGAAGAC ATCAATAGC | 1182 |
| *Prevotella histicola F0411* | AGCACCTACCTTCTGGATGA TC | 1183 | AGCAGCACAGGTCCTGTT | 1184 |
| *Helicobacter bizzozeronii CIII-1* | GGATAGCATGGTGCATGTTA CAGATAT | 1185 | GTTCCACAAGAGAGATGGG CA | 1186 |
| *Helicobacter bizzozeronii CIII-1* | TTTGGGCAGTAACCTCTAGG G | 1187 | TGCCCTAGAAGCCATTTAT GACAAA | 1188 |
| *Helicobacter bizzozeronii CIII-1* | ACTGATATGCACGCCATAGA TCAC | 1189 | CCAAAGCATTTTAACCGAA AATGGT | 1190 |
| *Enterococcus hirae ATCC* 9790 | GGCGTTGATACCCCAGC | 1191 | TTGTCAGTCTATATTGTGA GATGTTTCTCAAA | 1192 |
| *Enterococcus hirae ATCC* 9790 | TGGTCCAACAGCTGTTTCTA CTT | 1193 | ATGAAGCAAAAGAAAAATT ATTAGCACAACAA | 1194 |
| *Enterococcus hirae ATCC* 9790 | TTTTTGAGGCTAACTTTGCC ATTTCT | 1195 | TCAACGCCTTCTGGTATTC CC | 1196 |
| *Enterococcus hirae ATCC* 9790 | AGATTCGGACCAAGTTTAAC TCTTCAA | 1197 | ACCTTTAGGGAAGTACGGT ATTGAA | 1198 |
| *Bacteroides nordii CL02T12C05* | ACCAAGACTGCTGACAGCAT ATG | 1199 | TGCAGGCACGTATATTGGC | 1200 |
| *Bacteroides nordii CL02T12C05* | TGCCTGCATTGTGATGGAG | 1201 | AGACGACGTGTCCAACTAT CAG | 1202 |
| *Barnesiella intestinihominis YIT 11860* | CGAAGCAATTCAATAAAACA CGAAAGTG | 1203 | AGTTGCGTATTATCCAGTT GCGA | 1204 |
| *Barnesiella intestinihominis YIT 11860* | CGATGAATACTAAGCTCATA CTCTTCGG | 1205 | TTGCTTCGAAGTAAGCGAT ATATTGTTTTT | 1206 |
| *Barnesiella intestinihominis YIT 11860* | AAAATTGCGACCTCCCGAAA AAT | 1207 | AATTTTCTCACGGATACTC ACATTAATTTCGT | 1208 |
| *Barnesiella intestinihominis YIT 11860* | ACCGATAATTACACCAAACA ACATGG | 1209 | CGTCGATCAACAGTGCGTT | 1210 |
| *Lactobacillus murinus ASF361* | CCGATCACATAAGCCACACC TAAC | 1211 | GTGAGTCAAATATCATTGA TGTGATCGT | 1212 |
| *Lactobacillus murinus ASF361* | TCATCTGGAGCGACGTGA | 1213 | GAACTGACCAACAAAGATC AATGGA | 1214 |
| *Lactobacillus murinus ASF361* | ATCCGTGCCTTAAGTAGTTT GCT | 1215 | CAAGGAAGGTATAAATGAT ACACATTATCCCA | 1216 |
| *Lactobacillus murinus ASF361* | CCTTGATGCTTGGCTTGATG TT | 1217 | GGCAAAATAAGCTCCTAAA ACATCG | 1218 |
| *Eubacterium rectale CAG: 36* | TCCTACCGTAAAGCTCTGTG TTAC | 1219 | TTTATTAGGTTTGATTTTT CAGACCTGCCT | 1220 |
| *Eubacterium rectale CAG: 36* | AGGTATTTTCTCTATCCTCT TCCCTTTAAAACC | 1221 | GCAGGCACTTTTAATATTC AATGTTCCG | 1222 |
| *Cloacibacillus porcorum* | GAAAGGGTCAACATTGCCGT | 1223 | GCGATCGCCGTCGTGAC | 1224 |
| *Cloacibacillus porcorum* | GAAGGTGCCGATCGAGAAGT G | 1225 | ACCCTTTCAGGATTGGCAC A | 1226 |
| *Cloacibacillus porcorum* | ATAACCGGCGCGGTCTT | 1227 | ACCTCCGTGACAGAGGGA | 1228 |
| *Cloacibacillus porcorum* | CGATCATCACGTTTGAGGCT TTG | 1229 | TATGAATCTTAGCGCACGC AATC | 1230 |
| *Blautia coccoides* | GACTCAGATTTTCAACCCCT GTCTG | 1231 | TGCTTTATACGCATAAAAA TAAGCTTAATTCA | 1232 |
| *Blautia coccoides* | ATACTCCAGGGCACTTGCCG | 1233 | TTTACCCTTGGGCATTACC GTATATACTA | 1234 |
| *Ruminococcus bromii* | ATTAAGGTTGTTGAAGAAAG CAGAAGAA | 1235 | AATACCGCCTCACTTACTA TAGCC | 1236 |
| *Ruminococcus bromii* | TTGTCGGGACTTCTTGATTA TGCA | 1237 | TCGGTATCGCAGCTGAATT TATAGT | 1238 |
| *Phascolarcto-bacterium faecium DSM 14760* | CTGACAGGGACAGAAAGTAA CG | 1239 | TGCAACGGCTTTGTACTCA CT | 1240 |
| *Phascolarcto-bacterium faecium DSM 14760* | CCGATCGTTCCGCTTTCA | 1241 | GTAACTATCAGCGGCGGTA CT | 1242 |
| *Phascolarcto-bacterium faecium DSM 14760* | ACATCGATGTTTTTGATGGC TTTAATATTGC | 1243 | ACGATCGGCGGCGATAT | 1244 |
| *Gemmiger formicilis* | GAAAAGCCATTTTATATTCT CCTGTTCTTTTT | 1245 | CTGAAAAAGATTGGTGACA TCACAGATAT | 1246 |
| *Gemmiger formicilis* | GCACTGCGCCAGATAGGTA | 1247 | GGCTCGGTTTCCGCGAT | 1248 |
| *Gemmiger formicilis* | TGTAAGACCTGCGCGTTGTG | 1249 | GCGATAGCCTGACCCAGTT | 1250 |
| *Helicobacter salomonis* | GCAAAACCCTCTCTTGCTTG T | 1251 | TGGTGGCCTTGATAAGAGT TTGA | 1252 |
| *Helicobacter salomonis* | GCTGCTCTTCCTGTCAGGTA TTTTAG | 1253 | GGTTTTGCAACAAGGGCTT C | 1254 |
| *Helicobacter salomonis* | GCAGGGCTGGCGATCAA | 1255 | ATGGGTTTTAAACGCTTGA AAAATGC | 1256 |
| *Helicobacter salomonis* | GCCAAGGCCTCTCTTCTCA | 1257 | AGCCCTGCCCCTAATTGG | 1258 |

| **TABLE 16E (PRIMERS/PROBES SEQ ID NOS: 1259-1298)** | | | | |
|---|---|---|---|---|
| *Gardnerella vaginalis* | AGCAGGCCTTTTCTCAGGA | 1259 | GGAGCAACTTGTTAGCAGA TGG | 1260 |
| *Gardnerella vaginalis* | AGTTGCAGGTTTTGCGAGT | 1261 | TGCCAAAAAGCCTTGAGAA TATTCTG | 1262 |
| *Gardnerella vaginalis* | TTGACGAATCTATTTAAACC TTACCGC | 1263 | GGCCTGCTACTAATTCACT TATTGC | 1264 |
| *Klebsiella pneumoniae* | ACGGTGGTCGCTGTACTG | 1265 | GCAGGGTGCTGACCGAG | 1266 |
| *Klebsiella pneumoniae* | TCAGCGCGCAGAGAATACTG | 1267 | ACCACCGTAACCGGCTC | 1268 |
| *Klebsiella pneumoniae* | CACCCTGCGGGCTGTCT | 1269 | GGATTACGCATCGGATCGG G | 1270 |
| *Escherichia coli* | TGCAATCTTGTGAGTGGCAG A | 1271 | CTCGACCACCACGAATCGC | 1272 |
| *Escherichia coli* | CAATCTTCGGCGTTTTGCTG TAT | 1273 | GTTGAAGATGACATGAGCG TTGAC | 1274 |
| *Escherichia coli* | CGCCAGCGAAGGCTATTT | 1275 | GTGGTGGATGTTCCTCTGG TG | 1276 |
| *Enterococcus faecalis* | CGTAGCCAAAACTAATCCGG ATTG | 1277 | CATTTGGACTTAAGAGGTA TTGCGATTT | 1278 |
| *Enterococcus faecalis* | GCATTAAGAGCAAATCACTG GGAATT | 1279 | ACGATTATTTTAAAAGCGT TAGAAGAAGCC | 1280 |
| *Enterococcus faecalis* | GTTGTTGTAAATGCCATGGG TTCC | 1281 | TCTGAAGTACTAGTTGCAG TGATTCAAC | 1282 |
| *Proteus mirabilis* | CTTAAAGAAAGTCATAATCC TCACCTTCCC | 1283 | GCGTTTGGGTTTATGAGCT TGAAA | 1284 |
| *Proteus mirabilis* | ATAAAGAAGCATATGGTGAA AAATAAAACTCTG | 1285 | GGCATTTGCGCCCATACTG | 1286 |
| *Proteus mirabilis* | GTCGAGTACGACTTGCGAGA A | 1287 | GAGTCACCTATATAAGCAT CACTCTATAAGAT | 1288 |
| *Escherichia coli* | TCGTCGGTTCTGGCCTACT | 1289 | GAGAAGCGACGACATGATT AACTCT | 1290 |
| *Escherichia coli* | CGCCACGGCAATGGTTTC | 1291 | GCGCAAACGTGGTTAATGG TA | 1292 |
| *Escherichia coli* | CGTTATGTCGGGCGAACCA | 1293 | GCAATCATGGAAAACATCA ACGTCAT | 1294 |
| *Escherichia coli* | CTTCACCGCCATTTCCGTAA C | 1295 | CCACACCGTTAGCAGCAAT CA | 1296 |
| *Escherichia coli* | GACCCATCCGGCTGATACC | 1297 | CCGTGCTCGGCAATTTTAC AT | 1298 |

| **TABLE 16F (PRIMERS/PROBES SEQ ID NOS: 1299-1604)** | | | | |
|---|---|---|---|---|
| *Escherichia coli* | CGCATTGGTGAGCTGGC | 1299 | GACAGCAACTCGCGGATC | 1300 |
| *Escherichia coli* | TCCGTATCGATCCTGAACAC CA | 1301 | GCCACTCGCCCCTTGTT | 1302 |
| *Bifidobacterium longum* | GTACCCAACGGGCCGTTT | 1303 | CAGATGGTGCCCAGACG | 1304 |
| *Bifidobacterium longum* | GCCTCGCGCGAGGGATT | 1305 | ACCTTGGTCGAGGCCGCT | 1306 |
| *Clostridioides difficile QCD-66c26* | ACAAGAAAGGAGCGATAACT TTGGTT | 1307 | CTCATCAATATTTAAAGCT CTTTGTTCAGCT | 1308 |
| *Clostridioides difficile QCD-66c26* | TGGATATTAAAAGTAAAACT AGCTGATGTGG | 1309 | ATCATGTTATCCCTCCCAA TTTGTTCT | 1310 |
| *Clostridioides difficile QCD-66c26* | TTGATGAGATATCAACGGAA ATAACTAGTATG | 1311 | AATTTCTCACCCTAGTAAA TACTGTTTCTC | 1312 |
| *Lactococcus lactis subsp. lactis Il1403* | GCTCCTTGAGTATAACCATT GGTC | 1313 | TCAGATGAAACAAAAGCGG CTTTC | 1314 |
| *Lactococcus lactis subsp. lactis Il1403* | GAAATTCACTTCATCAATTA TACCATAAACCAT | 1315 | GCTGTTGCAACTGCTTTGT CA | 1316 |
| *Lactococcus lactis subsp. lactis Il1403* | TCTAAGGCAATTGCTTTTAT CATTGGG | 1317 | TTTCTGAAATTCTCTTTTA TGTCATTTTAGGAC | 1318 |
| *Lactococcus lactis subsp. lactis Il1403* | GAATTCTACAACCATCTTCA CCACTTCA | 1319 | ATTCGCTGATTTTTCAGGT ATTTGCT | 1320 |
| *Chlamydia pneumoniae TW-183* | CGCCTATGTTCAGGCAGC | 1321 | CGTCACTCGATTCCCCGT | 1322 |
| *Chlamydia pneumoniae TW-183* | GAGTGACGGAATCTTTTACC CC | 1323 | GAGCCTCTGGGTTCTGCTG | 1324 |
| *Fusobacterium nucleatum subsp. nucleatum ATCC 25586* | CAAAACCAATTAAAGAGTTA GAGCAACATATG | 1325 | AAATGTGGATTAATTTGAC TGTAAAGTGCAT | 1326 |
| *Fusobacterium nucleatum subsp. nucleatum ATCC 25586* | TCCTATCAAGAATACTCATT GGACATTGATT | 1327 | TGGTTTTGTAATTCTTCTC AATACACTGAT | 1328 |
| *Porphyromonas gingivalis W83* | CAACCTTTAGCCTCGCCATA GAA | 1329 | ACCTTTGAAAATACAACAG AGGTGATAAA | 1330 |
| *Porphyromonas gingivalis W83* | GAGTCGACAACCGTCTGC | 1331 | CGAGTATCTGCTGAAATGA GTGATATAAC | 1332 |
| *Helicobacter hepaticus ATCC 51449* | AGCTAAGGCGCCTTGCAA | 1333 | GTCTTCACCTTTAGAATCC ATCGCT | 1334 |
| *Helicobacter hepaticus ATCC 51449* | TGCAAAGCTAAGCAATTTAG TCAAGCTTTTA | 1335 | GCAATGTTGATACTTTGTC TTCACCT | 1336 |
| *Lactobacillus johnsonii NCC 533* | TTCAAAAACATATCTTCTAG ATCTTCTTGGT | 1337 | ATGGCCACCATAATTTTGC TTTTAAAGG | 1338 |
| *Cutibacterium acnes KPA171202* | GCCCTCCGCATCGCTGT | 1339 | ACGTATACCAGGCTCAAGG CT | 1340 |
| *Cutibacterium acnes KPA171202* | TCGCCCAGGTGCTCTCC | 1341 | GGGTTGGTGGAACGCGA | 1342 |
| *Cutibacterium acnes KPA171202* | GCCGCAGCCGAACTGGTT | 1343 | ACCGCGAACTCGGGTGG | 1344 |
| *Cutibacterium acnes KPA171202* | TTCGCGGTCGACACCAA | 1345 | GAGTTGAGGTGCTGATCAA CG | 1346 |
| *Chlamydia trachomatis D*/*UW-3*/*CX* | GTGAGCGAATCAAGAAAGTT CGT | 1347 | AGAAGCTAGTGTATACACT GCTTGTC | 1348 |
| *Campylobacter jejuni subsp. jejuni NCTC 11168 ATCC 700819* | TATAGTTGGCGTGGAGCAAA AATTGA | 1349 | ATTTTAATATTTTCCCCAG TATCTTTAGTGCA | 1350 |
| *Campylobacter jejuni subsp. jejuni NCTC 11168 ATCC 700819* | AAGGTCTAAATTTTGTCCAT CTAGCATG | 1351 | TGCTTTCTCAAAAAGGATC TCAAGGT | 1352 |
| *Campylobacter jejuni subsp. jejuni NCTC 11168 ATCC 700819* | AAAACGAAAACGAAAAAGAT GAAGGTTT | 1353 | TCTCTCATAAAAACGCATA CCACTAAGT | 1354 |
| *Campylobacter jejuni subsp. jejuni NCTC 11168 ATCC 700819* | AGAAAGCATCAAAACCAATA AAGGATCAG | 1355 | TGCTATAAAAGATGGAGAA CGCTATAGT | 1356 |
| *Campylobacter jejuni subsp. jejuni NCTC 11168 ATCC 700819* | AATAAGGTTTTGATTGCAAA ATTCTTTAGGAA | 1357 | CCACTTTAGACATAGGTGG TGGT | 1358 |
| *Bacteroides fragilis YCH46* | AGCCGCAAATGAATACGGC | 1359 | CCATGGAGCTGGTTGGTTG | 1360 |
| *Bacteroides fragilis YCH46* | GGTATAAATGGATCGTACGT TTCGA | 1361 | TCCGCCAACAAAACCTATG TCT | 1362 |
| *Bacteroides fragilis YCH46* | GGCAACCACTTCCGGAATTT | 1363 | TTGCGGCTGGATGAGGT | 1364 |
| *Lactobacillus reuteri JCM 1112* | GGGATGGACAATTATTTTAT GGATTCTGA | 1365 | CTGGCCAGTAACGGCGA | 1366 |
| *Lactobacillus reuteri JCM 1112* | AGTATTTTGGCTCACCAAGC ATCA | 1367 | TGCCATTGATCCACCTCAC TT | 1368 |
| *Lactobacillus reuteri JCM 1112* | ATTATGATTATTGGTGGAGG ATGGTATACTGT | 1369 | AATTACGCCAACGTACCCA CC | 1370 |
| *Lactobacillus reuteri JCM 1112* | CTGGCCAGTATTTTGGCGGT | 1371 | ACAGTTGAGGCTGAGAGAA AACTTTG | 1372 |
| *Bifidobacterium adolescentis ATCC 15703* | GCCAATCCCCGTCATAGC | 1373 | GATCCGGCGGCTGATATTC | 1374 |
| *Lactobacillus rhamnosus* GG | CCGGTTTTTGCGCGCTTC | 1375 | GCGATGGCAGAAGCGTT | 1376 |
| *Lactobacillus rhamnosus* GG | AAACCTTGATGATTGCTTTT GGCAA | 1377 | AGACCCGTAATGCCGCCT | 1378 |
| *Lactobacillus rhamnosus* GG | GCCATCGCTCTTGGCGT | 1379 | ACTTTGGTTTGAATCAAGA CTTGATCAC | 1380 |
| *Lactobacillus rhamnosus* GG | GTGATCGTCATGTGCGATCC | 1381 | AAGGATGGATCAACCGTTA TCCTTAATAAA | 1382 |
| *Bacteroides thetaiotaomicron VPI-5482* | GCTGGTTCAGGTATTACAAC TGC | 1383 | GCGATTACGATTTGAAAAG TTCTCACTTT | 1384 |
| *Bacteroides thetaiotaomicron VPI-5482* | TTATTGCAGGGTATGGTAGC CAG | 1385 | CTCCACCTAGTCCCTGTCC G | 1386 |
| *Bacteroides thetaiotaomicron VPI-5482* | AAATCACCCTGGTGGAGCGA | 1387 | CTACTGCCTTCTTCCGGGA AAAT | 1388 |
| *Lactobacillus acidophilus NCFM* | TAATGACGAGATGCGTTTGG ACAG | 1389 | CTGAATTAAATTTAGTGCA TTTTCTAGCAAAGC | 1390 |
| *Lactobacillus acidophilus NCFM* | CGGTTTAAACGATGCTACTC TCGA | 1391 | TTTCAGCATACCAAAGTGG ATATTTCCAT | 1392 |
| *Desulfovibrio alaskensis G20* | GCAGCGAAAGTCCGTCG | 1393 | ATATCCGCGCTGCGCTG | 1394 |
| *Desulfovibrio alaskensis G20* | CTGATGCGTGCCGTGCC | 1395 | AGAACAGCGCATGCGCTC | 1396 |
| *Bacteroides vulgatus ATCC* 8482 | CAAACCACTTGTTCAACTTC CCTG | 1397 | GTCAGCAATGTAACCGTCA GG | 1398 |
| *Bacteroides vulgatus ATCC* 8482 | AGGCATGAGCATGAAACGC | 1399 | GGTGGAACTGACCGTAGGC | 1400 |
| *Bacteroides vulgatus ATCC* 8482 | TTTGGCCACAGCATGGGA | 1401 | AAAATGCTTCTTGTTCCAG TTCATCC | 1402 |
| *Parabacteroides distasonis ATCC 8503* | CCCGGTCGTGTTTATGGG | 1403 | TCTGCGCATTCATGTCCG | 1404 |
| *Parabacteroides distasonis ATCC 8503* | CCGGAGGGAGTGGAGTT | 1405 | CCCTTACCCGTATCTTTCA CGG | 1406 |
| *Parabacteroides distasonis ATCC 8503* | GAGGAAAAGGCGGAGTTTAT AGATCTG | 1407 | CCCTCCGGCATCATCAATT G | 1408 |
| *Parabacteroides distasonis ATCC 8503* | TGCGCAGATTCAGGATATTT GTGC | 1409 | CCAGATACGCTTTATTATA ATAATTCTCGCC | 1410 |
| *Lactobacillus delbrueckii subsp. bulgaricus ATCC BAA-365* | CCGCAACCTGGTCTTAAAGA G | 1411 | TTGGCGCTTCAGCCAGTAT | 1412 |
| *Lactobacillus delbrueckii subsp. bulgaricus ATCC BAA-365* | GTGCCCGCTGAAACGGA | 1413 | CCGGTCAAGTTCCGGGCA | 1414 |
| *Lactobacillus delbrueckii subsp. bulgaricus ATCC BAA-365* | CCTTTAAGAGCAGCCGGGAT T | 1415 | GCCTGAGTCAATCCCGACC | 1416 |
| *Campylobacter curvus 525.92* | GCGACGGATGACGTCCT | 1417 | GGATGTTGCTAGCTAGCGG | 1418 |
| *Campylobacter hominis ATCC BAA-381* | CGCGCGAAATGCTGAGA | 1419 | GGTGAAGCGATGGCGAA | 1420 |
| *Campylobacter hominis ATCC BAA-381* | GCTTCACCGAATCCGTCG | 1421 | TTTAGCATCTATTGAAAAT AGTAGGATTTCACC | 1422 |
| *Campylobacter concisus 13826* | GGTTTGATGGCAAAAATTTG TGTGGT | 1423 | AGTTAATTGTGGCTTTAGC TAGGATAAATT | 1424 |
| *Akkermansia muciniphila ATCC BAA-835* | TGTAAGCGGCGTTGTATTTG TCC | 1425 | CCAATACCAGTCCAGTGCA GC | 1426 |
| *Akkermansia muciniphila ATCC BAA-835* | CATTATCAACGGTTTTCAGC GTGTAG | 1427 | AAGAAAGTAAACCTTACTA TCACGGC | 1428 |
| *Atopobium parvulum DSM 20469* | TAGGTCCTATATTCCCCAGA CTCAAA | 1429 | AGTAGTTTTGTCTACTCTT GGAGTAGTG | 1430 |
| *Atopobium parvulum DSM 20469* | TATTGATTCAAGTTTTGTGA AGAGAGAAAAAC | 1431 | AGGACCTATACTTGCAATT TAAACGAC | 1432 |
| *Veillonella parvula DSM 2008* | GGAACTCACGAACTGACCAA AGA | 1433 | CAGATTTAATAAAAGCATC CCCATTTTTAGCC | 1434 |
| *Veillonella parvula DSM 2008* | GCGCCCATTCCAACTAATAC ATTATCTTC | 1435 | CCAATGTACAGGATAACTC TGTATTACACG | 1436 |
| *Veillonella parvula DSM 2008* | AAAAGAAGCGGATAGTTGAG TTAATCAGC | 1437 | CACTTGTGGACTGTAGAAT ATGGCA | 1438 |
| *Citrobacter rodentium ICC168* | GCTGGATGGCGGTATCACT | 1439 | GAGCATCAATCCATGTCGG AT | 1440 |
| *Citrobacter rodentium ICC168* | TGATGCTCCGCCACCCA | 1441 | AGGTGTCTACGGCACTCAC | 1442 |
| *Streptococcus gallolyticus UCN34* | AACTTGAAAAAGCAAAAGAT ACAAGAGTTAATG | 1443 | AGCTTATACTAACGATAAT AAAAATTAACCCGA | 1444 |
| *Streptococcus gallolyticus UCN34* | AGAAAGCCCAACGGTATAAA CATTACAA | 1445 | CAATCGCTGTCTCTTACTT CATTTATTTTATGA | 1446 |
| *Enterococcus faecium TX0133a04* | CGGCGTGATCAGCGCCA | 1447 | GGTTGCTGTGCCTCTTATG TGG | 1448 |
| *Enterococcus faecium TX0133a04* | AGCTTTATACAAAAGCATAT CTGCTCCTT | 1449 | CTTTAACGAACGTGTTCGC TAAAAA | 1450 |
| *Enterococcus faecium TX0133a04* | AGCTCGTTCTCATTCAGCAG A | 1451 | ACTCAGGAAGCTTTGGCAG A | 1452 |
| *Peptostrepto-coccus stomatis DSM 17678* | CCTCCATATACCAACTTAAA TACTAAACATGT | 1453 | CCCAAGAATATTTTGCCAA GGTCA | 1454 |
| *Peptostrepto-coccus stomatis DSM 17678* | TCTTGGGCTATACCCATAGA CCT | 1455 | GAGTCGATAATAAAGAGGC TTTTAAGTGAT | 1456 |
| *Mycoplasma fermentans JER* | AGCCACTTTTTGTTCGTCTT AGTACT | 1457 | ATTCAGCATATTTACCACT TGCAATGTT | 1458 |
| *Mycoplasma fermentans JER* | TGGATGGATTTTATGATGCT TATCCACA | 1459 | AAGTGGCTTTTTAGTTCCT TCTGC | 1460 |
| *Eubacterium limosum KIST612* | CACAAGGGTCGCCGCGTC | 1461 | CCGCGAAATACGGCGAACT G | 1462 |
| *Eubacterium limosum KIST612* | AAACATAAACGATGGAAAAC AGATTATGGAAAA | 1463 | TAAGAAATTAACGGAAGGA GATGAAACAC | 1464 |
| *Parabacteroides merdae ATCC 43184* | TCAATGTACCGGTGGGCAA | 1465 | ACAGACAGCCTAATTAACG TAGTCC | 1466 |
| *Parabacteroides merdae ATCC 43184* | TTCGGAACCATCCGGCA | 1467 | CCATGCAGAAAAACCGATT CCG | 1468 |
| *Faecalibacterium prausnitzii M21*/*2* | GCCATTGCGCATCGTCAAAA ATA | 1469 | CATTGCAGGCAAGGAATGA AGAG | 1470 |
| *Faecalibacterium prausnitzii M21*/*2* | TAAGCCGAAATCTGAATGAC CGA | 1471 | CAGCTGATGGATGAGATGA TCGAA | 1472 |
| *Faecalibacterium prausnitzii M21*/*2* | CCTTAACGGCAACCACGATG | 1473 | GCGCTTCCAGCATGCCA | 1474 |
| *Faecalibacterium prausnitzii M21*/*2* | CCGGTATGGGAATAGGAAAA AGC | 1475 | AT GCCCCCGCGCAAAATC | 1476 |
| *Parvimonas micra ATCC 33270* | CCAGCACTCCGACTATAGAT TTAGT | 1477 | AATACAAAAGTATTCTGAT GACGGAGAG | 1478 |
| *Parvimonas micra ATCC 33270* | AGCTACTGATCCCCAAAGTA AAATTCTC | 1479 | CGGACAACGAAGTCCGTTG TTT | 1480 |
| *Bifidobacterium bifidum NCIMB 41171* | AGCGTACCGGAAGCTCG | 1481 | GTCGGCAATGCCGGCAC | 1482 |
| *Bifidobacterium bifidum NCIMB 41171* | GGCCGAATATGTTTCGCGGA TA | 1483 | GCGAGGTCAAGATATACGG C | 1484 |
| *Collinsella stercoris DSM 13279* | CGCCACCCCACCTCAATT | 1485 | ACCCCCGTTGCGCACATT | 1486 |
| *Roseburia intestinalis L1-82* | GTCAGATTCTCTGCATAATT TTTCCG | 1487 | AGCGTCAATCAGGATGAGG T | 1488 |
| *Roseburia intestinalis L1-82* | TTGACGCTGTCATCCTGCT | 1489 | AAAAATCGAGGATCTGCTG CG | 1490 |
| *Enterococcus gallinarum EG2* | GAGAAGATAAGTACCTAAAT CTGAAAGAAACGC | 1491 | CAATGAAAACTGGATCACC CTTCTGAT | 1492 |
| *Enterococcus gallinarum EG2* | AAGGATGTGTCCAACATGAA TCAGGA | 1493 | TCAAGAAATACTGTCTTTC TTCTGACCG | 1494 |
| *Enterococcus gallinarum EG2* | GAAGCCAATCCTGGTCCTGG TTTA | 1495 | AAATGGGAAATAAACTTCA TGAATACCTCCTAT | 1496 |
| *Prevotella copri DSM 18205* | AGTCATTATGAAGGAGACCA ATTTCGAC | 1497 | GGGCGGATAGATTTCCGGC A | 1498 |
| *Prevotella copri DSM 18205* | ATCCGCCCAGCTTAGCC | 1499 | CTGATTTTGTAGAGAATCC CTCGTTGA | 1500 |
| *Prevotella copri DSM 18205* | AACCTTGCACATCGAAGAGG | 1501 | ATTGCTTTATCGTTACTGA AATTCATAATCTTC | 1502 |
| *Prevotella copri DSM 18205* | AGACAATTCTTGGCAAACAA TTCTGG | 1503 | GCAAGGTTCCTACGAAATC AAGC | 1504 |
| *Holdemania filiformis DSM* 12042 | ACCCTCCTTACCCCACCA | 1505 | AACAGGCGAAGGAAAAATG TACTTAC | 1506 |
| *Holdemania filiformis DSM* 12042 | AGCGTCGACGCTCTATCCA | 1507 | AGGAGGGTGAACGTTTTGG T | 1508 |
| *Helicobacter bilis ATCC 43879* | AAACAGAAGAACAAATTCAA ATGCGTAACAA | 1509 | TTTGCATGGTATTCTAGCT CAGC | 1510 |
| *Helicobacter bilis ATCC 43879* | GGATATGTATAATCTCAATC CACAAGATATCAC | 1511 | CGACATGATATGCACTCCC AGAGA | 1512 |
| *Anaerococcus vaginalis ATCC 51170* | TTATGCAACGAATATCCTAA ATACAATGGAT | 1513 | AAACTCCATTAAGCATAGG TAATGATGAGA | 1514 |
| *Anaerococcus vaginalis ATCC 51170* | AGTCTAAATTCTAAATCTAG GGCAACGG | 1515 | ATTGCCGATTTTCAGGATA AGCCA | 1516 |
| *Anaerococcus vaginalis ATCC 51170* | TCGGCAATATCATTTTGATT TCCTTCA | 1517 | AATAGTTGCGGATTATATA ATCAACAATCCAA | 1518 |
| *Collinsella aerofaciens ATCC 25986* | GAGCAGTCGGGTGTCTCC | 1519 | GGGAAATCAGCCCTTGAGA TC | 1520 |
| *Dorea formicigenerans ATCC 27755* | AACCGGGAATGACTAATCAA GTGT | 1521 | CCGATTCATCAAAGCATAC CCC | 1522 |
| *Dorea formicigenerans ATCC 27755* | ACAAAAGAAATTATTGGAAC CATTGGCA | 1523 | TCCCGGTTCTACCGAAACC | 1524 |
| *Ruminococcus gnavus ATCC* 29149 | CCGGAGTTTGATACCATGGG ACA | 1525 | ATACCTGCTGCCCGGTTGA | 1526 |
| *Ruminococcus gnavus ATCC* 29149 | GCCGCTTTTACTGGCATTGT | 1527 | TCTCTTTTTCCTGTCCCGC A | 1528 |
| *Campylobacter rectus RM3267* | TTGTTTGCGTCTTATACTCG TGTCT | 1529 | CGATAATTTCTTAAAATTT AGATGTCTGACACA | 1530 |
| *Campylobacter rectus RM3267* | AGTCATTTTGCTTGACTGTA TTTTTGGT | 1531 | GCAAACAAACGGATTTACG AAGCTA | 1532 |
| *Campylobacter rectus RM3267* | AAAAACAAACAAATTTGAGA GCATAGAGGA | 1533 | TTTTGTTTTACTTTATCGT CCATATCGACTT | 1534 |
| *Actinomyces viscosus C505* | GTACAGGCTCCCGGCGT | 1535 | GCTTGCTGCAGCCCTCG | 1536 |
| *Actinomyces viscosus C505* | CTCCACCGTCGGGTTGT | 1537 | TTCCAACATGTTGGCTCGC | 1538 |
| *Actinomyces viscosus C505* | TGTTGGAATGCCCGCTTATC A | 1539 | CTTACGCCGTGGCCGGT | 1540 |
| *Campylobacter gracilis RM3268* | CGCGCCTCGTCGATCTT | 1541 | CGCCGTGCTTTTTGACGA | 1542 |
| *Campylobacter gracilis RM3268* | GCACGGCGTCAATGCTT | 1543 | TGCCAACGGCTTTATATAT TTCTACATC | 1544 |
| *Campylobacter gracilis RM3268* | TCCTAGATTTGCGATCAGCG TAAG | 1545 | AGCCGTTTTACGCGCTG | 1546 |
| *Campylobacter gracilis RM3268* | GCGGCGATTGAGCGAAATT | 1547 | TGGGCGAGAGTTTATCGTG C | 1548 |
| *Peptostrepto-coccus anaerobius 653-L* | CCATAACGGTCTTACTGCTC TTGAA | 1549 | AGTTACAGGTAGTCCCATC TCTATACAG | 1550 |
| *Peptostrepto-coccus anaerobius 653-L* | AGACTTGCATGTTCTCCTGA TGA | 1551 | GATCGTAAACGTAACCACA TGGTC | 1552 |
| *Prevotella histicola F0411* | TTGATAATGTGTTTACCAAC ATCACCAC | 1553 | CAGACGGTCTCAGTATTGT TCTGAT | 1554 |
| *Prevotella histicola F0411* | ACCGCAACCCTTGTGAGGT | 1555 | AGGTGCTAACGGCGAGA | 1556 |
| *Helicobacter bizzozeronii CIII-1* | GATCCAAAGTGATGGGTCCA TAGAG | 1557 | TGCCCAAAATCTCCAAAAG ATTGT | 1558 |
| *Helicobacter bizzozeronii CIII-1* | TGCTTTGGCTTCTCCCACT | 1559 | CGATTTTATGGATTGCTTA AAAAGGGTTAAGA | 1560 |
| *Helicobacter bizzozeronii CIII-1* | TGTGGAACAAATGAGTATTC TAGCCAA | 1561 | ATAAACATCGGTCGCACGA TTAGT | 1562 |
| *Enterococcus hirae ATCC* 9790 | AAAACTTATGATTGACAATC GAGGCATT | 1563 | TGGCTGATGTTTGGTCTGT ACA | 1564 |
| *Enterococcus hirae ATCC* 9790 | AAACGGAAGAAGGAGTCTAT CATGA | 1565 | ATCCTACACGACTAATCAT TAGAGAAAGTT | 1566 |
| *Bacteroides nordii CL02T12C05* | CGTAGAGCCTTCCCGGT | 1567 | GGGCACCGATGAGAAAAGT T | 1568 |
| *Bacteroides nordii CL02T12C05* | TGATCACTCCGGCTACAAAG GT | 1569 | TCCGGATATAGATACTATT GCACCG | 1570 |
| *Bacteroides nordii CL02T12C05* | AAACGCCTTAAATTGATTCA AGCGA | 1571 | GTGGTGAAAGTTTCTGTGC CC | 1572 |
| *Bacteroides nordii CL02T12C05* | TCAAGTTTCCTTCTAAAAGT AGCTCGT | 1573 | GGCGTTTTCTGGTGTTTAT GTTCT | 1574 |
| *Barnesiella intestinihominis YIT 11860* | AATCTTTGATTGGAAGGTTA GAAGTATAAAAGG | 1575 | ACGCAAGATTTTCATTCTT GAAAGAGGAG | 1576 |
| *Lactobacillus murinus ASF361* | CTTTGCGACCACACTTAGCT C | 1577 | ATTCATAAGCGGTCGTGAC TTTTAACTT | 1578 |
| *Lactobacillus murinus ASF361* | TGACTCACCTTCATATTCAA AGCC | 1579 | ACGTTTTGAGCGATACGGT CC | 1580 |
| *Eubacterium rectale CAG: 36* | AATTACTCCTCTCTTCTTTT AACCTTTGATCTG | 1581 | TACCTTATTATGATATCGT CATCAAATCGCC | 1582 |
| *Cloacibacillus porcorum* | TCTCTTGATGTACTTGTTAA TAATGCCG | 1583 | AGAGCACTATTCGACGCTA CC | 1584 |
| *Cloacibacillus porcorum* | AGTGCTCTTAGCGGACGC | 1585 | TTCTAATAGACGTTCACGT GATATTGGT | 1586 |
| *Blautia coccoides* | CTTCCATCCTCAGGTATACT CCAG | 1587 | TGCTCTGTAAATGGAAAAT AGTCCATCAAAT | 1588 |
| *Ruminococcus bromii* | GCGGTATTTATGAAGAACAG CGT | 1589 | CCCGACAAAATTTCTTCAA GAGTATCC | 1590 |
| *Phascolarcto-bacterium faecium DSM 14760* | CCGTT GCAAAGGCTT TACAC | 1591 | CGGCCCAGTAACCAGAAGT A | 1592 |
| *Phascolarcto-bacterium faecium DSM 14760* | GGTTCTGGTTTTTCGAAAGC GAG | 1593 | CCTGTCAGCAATAGTTCAG CACT | 1594 |
| *Helicobacter salomonis* | CCTCCACAAATTTGAGGGCT | 1595 | ACAAGGACTATATGAAGTA TATGCAAGCG | 1596 |
| *Helicobacter salomonis* | TCACTAATCTTTTACTTGCC ATCTCTCC | 1597 | TGTGGAGGCGTTGGCAT | 1598 |
| *Gardnerella vaginalis* | TTGCCGCTATAGGAGCAGTA A | 1599 | ATTCTGCTTTAATTGAACG CAATCG | 1600 |
| *Gardnerella vaginalis* | AGCAGCAGTCGTGTTTGG | 1601 | AGCGGCAACAACTGAGATG A | 1602 |
| *Gardnerella vaginalis* | TTTTGGCAACTTGGGCTAGG | 1603 | ACCCAAGT GACATT GCGCT | 1604 |

| **TABLE 17- SPECIES NUCLEIC ACID SEQUENCES** | | |
|---|---|---|
| GENUS AND SPECIES | SEQ ID NO: | NUCLEOTIDE SEQUENCE |
| **TABLE 17A - SEQ ID NOS: 1605-1820** | | |
| *Bifidobacterium longum* | 1605 | GATATCAGGGATAGGCCGGAGGCCTCGTAATGTGTCTTCGGATTGTTCAT ATCGGGCATATAGACATGTCGTAAGCGCTGATGGCATTGACGAGATCCAT GATCGGAAGTCACGATGGTTATGCAGTCATCATGCAAATGCCATTGATGT TCGAATCCATAGG |
| *Bifidobacterium longum* | 1606 | GTATGCATAGTGATGGGGCTGGTGGTCATCATTCTCGGATTCAGAGGACG GCGTACCGGTGGTCTGATTCCTCTCGGACTGGTTGCCGGTGGATGCGCGC TCTGCATGACCATCGTTTCAGGCACGTATGGCGTGTACTACCGTGATCTT GGTGCCAG |
| *Clostridioides difficile QCD-66c26* | 1607 | TCAACTGTATTTGTAGATTTTATAGTTGCTGTTAACTTGTTATCAGAATC TTCTGCTAAAGTTGCATTGTAAGCATTAACTGCATCTACTATTTCTTGAG CAGTTTTGTAGTTTTCAATTTTATAGTCAACTACTTTTCC |
| *Clostridioides difficile QCD-66c26* | 1608 | AAGAAAGTTATGTGGGAGATGAAATTATGAGTTTAGATTTTAGTTTTTTA AGTAGATTTGGGACTTCTTTTTTGGAAGGAACAGGTGTGACAGTATCAAT TTCTCTTGTGGCATTATGCTTTGGATTTATAATAGGTATAATT |
| *Clostridioides difficile QCD-66c26* | 1609 | TCTGTTAAAGAGTTTATTTTATTTTGTAATTGAGTTGCATTAGTTAAGTT TACATTGTCTACTTTTATATCGTAAACTCTATCATTTACATTTGCTCCTG CTTTTGTATCTTCAAACTTAACTTCTAATGCTTT |
| *Lactococcus lactis subsp. lactis Il1403* | 1610 | CTTTTTCTATTTCTTCTTCTTGTTCAACAAATAGTCCCACCAACTTCTCA CTTAAAATAAGTGAGTTAATACTTTCTAGCTCCAAAAGTTCTTTAAGAAA TTTATCATAGATTAGCTCAAAATACTCTAATACGACTTCTTCCTTATTTT |
| *Lactococcus lactis subsp. lactis Il1403* | 1611 | GCTGAACTCGTTATCGCTAACGTCATTGACCTTCGTGCCTTCCAATCTAT CTCAGCCTATGATTCAGTTGTTGCTGATGATACACACAAAGGTGCTGAAA ACCTCATTAATGACTTTGCTGACGAAGCAAAAAAAGCTGGCGTTAAAAAA GTCA |
| *Chlamydia pneumoniae TW-183* | 1612 | GGGAATTTCAATACCCACAGCAGCTTTCCCCGGAATCGGAGCAATAATCC GTATGCTCGAAGCTTGGAGTTTTAAAGCTATATCATTTTCTAAAGATTTG ATTTTCTGAACCTTAACTCCAGAATGAGGTAACACTTCAAAAGCTGCTAA TGTCG |
| *Chlamydia pneumoniae TW-183* | 1613 | CTCAACTTGTAGGCGAAGAAGACGCCCAGTCCCAAAAGGAAATCGACTTT CTCTCGCAGTGTGACAAGCTCTCTTGGCGTGCGTTCCTCAAAAATAGCTA CGAGATCATCCCAACATTTAAAGAGATGG |
| *Chlamydia pneumoniae TW-183* | 1614 | ATCTTAAGTTGCGACAGCGAGCCCCTTTGAGACTTGCCTCAAAGTTGTTC CGCTTTTTAGATGTTCCCTCGATTCGATTTAGTAGCTAAGCTATCGGGAA GATTCTCCTGCAACACTCCTAGGAGATGGTGTATAAGAA |
| *Chlamydia pneumoniae TW-183* | 1615 | GAAGTTTAGGTTGAAGGTTTTAGAGTCAGATTTAGAAGGGATTCTAGCTC AGACTGAGAGTGCTGAGAGTCTGTTAACTCAAGAAGAACTTCCGATTCTT GCAACTCGGGGAGCCTTAGAGAAAGCTGTTTTCAAA |
| *Fusobacterium nucleatum subsp. nucleatum ATCC 25586* | 1616 | TTCAAGCATTTCATTAATAAGTTGTTCTATGTTTTCAGCTGGAAAATCAT CACCTAATTCTTCATTAATTTCTTCATAAGTTATAATCCCTTCTTCTACT GCTTTTTTTATTAAAGCTCTAGCTTTTTCATTTTTTATTAGC |
| *Porphyromonas gingivalis W83* | 1617 | GAGCGCTAATGCTCAGACAATGGCTCCAAATTACTTCCATGCCGATCCGC AGCAATTCAAACACAGGATTGTAAAAGAA |
| *Porphyromonas gingivalis W83* | 1618 | CAGGTGGCACGCATCGCGTGGGAACTATGTTGCCAAGTGGCATAGTAAGG CCCCATTACATGGCAATTGATACAAACTCGTGGATCGTCACTTAAGTAGC TATAAGCTTTGGACATATATACAAGGTATGCGCCTAATCCAACGAATACA CCAACTAGT |
| *Helicobacter hepaticus ATCC 51449* | 1619 | CACAAGACTCAATAGCTCTGCAAAGGTATCTTGTGGGATAAAGAGCATAC TTGCTCCACTAAAAATCGCTTTTTTAAGCTCATCTTTATTTTGCACCCCA ATAAATGGCTCAAAGCTAAGGCGCCTTGCAAAGCTAAGCAATTTAGTCAA GCTTTTA |
| *Helicobacter hepaticus ATCC 51449* | 1620 | TCTGACAAATCAAGCATATAGGCTTCAAAATGTCCAAGCGTTTCCATTTG CTCCTCAAGTGCATAGGCTCTATGAATATAAGAGAGAGAATCTGCACAAA AAGTAGCAGATTCTATAAATAATCTTTGGGCAATTT |
| *Lactobacillus johnsonii NCC 533* | 1621 | AATTTTCTTCATTGTGATAATCAACTCTATTATTGGTATTATCCAAGAAA AGAAAGCCCAAGCTTCTCTTGCCGCTCTAAAAACAATGAGTGCTCCAACT GCAACAGTAATTAGAAATGGATCTGAAAAAATTGTTTCAGCTAGTGAATT A |
| *Lactobacillus johnsonii NCC 533* | 1622 | TTCTAAAATATAATCTATACTATCTCTAAAAAATATAGAAATCAAGAGAG GATAAAGATCTATGTATTTAGATGATTTCTTAATTTTAATTTCTGATCTC CATCCTAATTTTCAATTTTTTTATCAAAATAAGAAAAATGGCGTAATTGA |
| *Lactobacillus johnsonii NCC 533* | 1623 | GATTAGTTTAGTGATTTGACTATCTGTAAGATTGCGCAGCAGTTGACCAG CGATTGCAATTGTTTGATCTTCGATTTCAAAATTATCTTGAATAGGCAAA ATAGTTGTTGCAAAGCTAAAACTGGCTAAAAGACTAGCCCAGTTTTTTTC |
| *Lactobacillus johnsonii NCC 533* | 1624 | TTTACGCTACTAGCATCTTTTAAAAAGTAACCTGCTCTAATTCTAGTTAT TCCACTTAAAACTTCAACTAAAGGAAAACGGGGAAAAGCCGGAAAATGAT ATAAGTCTTCTGCTTCGTGTTCTATCTCATCCTTAACATTA |
| *Cutibacterium acnes KPA171202* | 1625 | GAACGGACAAGCTCGAAGTATCAAAGCGGTTGGATTCGTCGGATGGGGCT CGGCGGAGAAACCGAAAGCCGTGAGGAACACGCGGATTTTCGTCATATGA TCGGCCAGGCTGAATTTGTCACTGGGGGAGTGCTCCGTGTGATCCGGGAC GTTGGCCATCGCT |
| *Cutibacterium acnes KPA171202* | 1626 | CGTCACCGACCTGGCAGCGATGTCGTCAGCAACCTTGCGGCCAGATCGTA CCTCACGATCGGCCGCCGCGAGGTCAACCGGCCTGCCCGTAATGCAGCGA CGCCGCTTCCAAGAGAGGATGCGCAGCACCGATTCGTCGAGACGCCGCTC GCTAACCCGACCG |
| *Helicobacter pylori 26695* | 1627 | GATTAAAATAAGCGGGAGTCTAAAGACCTTAAATTGCTCATAGATTTCAG AATTTAAATTGACTTCTGGCTGAATTTCATCGTCTTTTTTGATTTTAAAA AATTTCAACTTTTCAAACAAAACCAATTCCTAGATCAAAATAAATTCTT |
| *Helicobacter pylori 26695* | 1628 | AAGTTCCTAAAATTGATTTTTGTTTCAATTTATTCTCATTCAATCGCTAT ATTTAATCAAAAAGAAAGCAATTTTATAGTAGAATGTAGCATTTAGAACT CAAGTAGAGAAAATGTAGAAGGAAGGAATACATGAA |
| *Borreliella burgdorferi B31* | 1629 | CAGTTGTAGTTCTAAGTGATAGAAATCTAAATTCAAATCCAAATCTTAAG TCTTATTTATAAAGAAATAAAAGCTAAGACAGGAGCTGCCTTAAGTAAGG CTAGATCATGTATTGGTAAATACTGTTCTTTTAAATAGTTTAGAAAG |
| *Borreliella burgdorferi B31* | 1630 | TGATCATATCGGAAAAACTTTCTCTCTAGTATAAATAAGTCAGTAGTTTT TAGATTAAAAATAAGATTTTCAGCAATGCATAAATAATTAGAATATTTTT TCTTATAAGCTTTGATGAAGATATTGTATTAAATT |
| *Borreliella burgdorferi B31* | 1631 | CATTAATGGTTATTTTCCATAAGGAATATAAAAGTATTATTTTGTGAGAC AGGGCATAAGCTCATCATTTGTGTCTATGGTTAGTAACTAGTACTCGGGG GGGGGGGATAATTAACTAAATATA |
| *Chlamydia trachomatis D*/*UW-3*/*CX* | 1632 | GTCATTTTTCACCTTCCACTGGAAGCCTCTACTCTATTGTCTATAATAGT ATCGGGTATTGCTTTTATTATTTTATCTATAGGACGCCTATAGTCTAACC TTTGGGAGAAGTATTTGCTTCGTTATCTAATTTCTTGTCGTGATCTCGTT G |
| *Chlamydia trachomatis D*/*UW-3*/*CX* | 1633 | AAAAGCGGTTTCTATAGTTTCATCAAAAGGTGTTTTCGTAAGCTGGATTT TTTTAGTTAACAAAGAAGGTTTTGCTTGTGTCAATACAGCAAATTTAAAT CTTTTAGAAAACCCTGAAGGGAGAATCTCGCTTTGAATAAAGTTCACGAG ACCTT |
| *Chlamydia trachomatis D*/*UW-3*/*CX* | 1634 | TGAAGAAGAGGCTTATTGAGTTGACACGTTAAACACCACTTTGCAGTGAA ATTTACAAAAACTGGAATCCCTTTTTCGCGTAAATCAGCTAGCTTTTCGG GAGAAAAAGATTGCCAATCAGAGCTATGTGCAGGAGGGACGTTCT |
| *Campylobacter jejuni subsp. Jejuni* | 1635 | CAAAGAAGTCTTAGAACTTTCTATAAGTGAATTTTGAGTATGCTCTCTAA TAGGTAAAATACCTTCAAAACCAGCA |
| *Campylobacter jejuni subsp. Jejuni* | 1636 | ATATCAAGAGATATGCAAGAAATAATTCTATTATTTTTTATTAAACACAA TTCACTAGAACCACCACCTATGTCTAAAGTGGTTCCATCCTTAAAAGGAC TTAATAAATTTAGAGCT |
| *Campylobacter jejuni subsp. Jejuni* | 1637 | AAATACGCAAAATCAAAGTGTATTGCCAAGTGAACCTATAGCAACTCAAG ACAATAACAATGATACTTCTTTTGAAAGTATGCCAATTACAGA |
| *Bacteroides fragilis YCH46* | 1638 | TGGTTTTCTTCCCTGCCTTTTCTTAATATCAATAGTATGCTAAATTTTAA AAATCTGTTTCTGGTAAGTGTTGCTCTGTGGTCGGCAGTAGGAATGGTTC GTGCCCAGGAGTTCGATCCGAAGCAAAGCTACGAGATCCATACCCAGAAC GGACTTGTCC |
| *Bacteroides fragilis YCH46* | 1639 | GCATCCCCTTCCACAAAGCCCTCCTGCAACAGATACTCAATTCCGACACC TACTCCGCACAAACCGTCACCATAAGTCACCGGAAGTTCCAAAGAACAAT TCTCCATCACCTCATCCAGCAACACTTCTGCTTTCTC |
| *Bacteroides fragilis YCH46* | 1640 | CTATCCAGTCAATCAGGTAAATTAAATGCTCTTTCATTCGTAGCACCTTG ATGTCTTCCTCACCTTTCCTCCGGGACAACCGGTAATACAGATAATAAGC GAGACCACAAATACCTTTCCCGATGCCTAAAGTTCCGATCGCCCTACTAT TAATCGTGTTAAAT |
| *Lactobacillus reuteri JCM 1112* | 1641 | AGAGCAAGAAAAAAGACATTGTTATTTCTAACAGCGCCGATTTCGATCAA CAAGAATATGACACCGCAGTTGGTA |
| *Lactobacillus reuteri JCM 1112* | 1642 | ATGTTCTCCCATTAAAATGATTTTCGCATGGCTAGTACCAATCCCTTGTT GTTTCACCATCGTAACCTACTTTCTACATAAAAATTCAAACTTAATCATA GCATAA |
| *Bifidobacterium adolescentis ATCC 15703* | 1643 | AAAAACGATGGAAAGCGGTGAGACAACCAGAAGCATTTGTCTCACCGCTT TTTCTATCGGATTTTAGGTATGCGCTACTTGACTTCGACTAACCGTAAAG GGTATGCGAGAATGCTGTTTCATCGTTTTTCAGAAAAAGAATGGAACTTT C |
| *Bifidobacterium adolescentis ATCC 15703* | 1644 | CGTGTTCCGGGCTGATTCGGAACGATGAGCATTCCGCAGAGTGCTGTGAT TTGACTTCAAGCGGCTTCAAGGTTGTATGGTGCTTTCTGAACCGATGTTC AGAATGAT |
| *Bifidobacterium adolescentis ATCC 15703* | 1645 | GGGGGCCGGTGACATCCGAGTGATGCCATCGGCCCCCACCATATCCGGAA GAATCCCGGAAGAATCCCGGAAGAATCATAGGCCCGCATCCGCCAGCGAA ATGTAGCCGGGTTCA |
| *Lactobacillus rhamnosus GG* | 1646 | ATTGATGATGCCTTTTGTCGGTGTTGATCGCCAAGGAATGGAGAATCTTT ACACCATGTTACCGGTCCGCCGTACTACCATTGTCGCCGGCCATTATCTG TTCGGTTTGATGACAGT |
| *Lactobacillus rhamnosus GG* | 1647 | CATCCTGTGCAAAACTCAGTTGACCGTCATTTGCTACATTAATTGCATTG ACACTCTTTGTCCCTGTACCCGTTACATTAAGATCCAAAGTGCTGCCACT TGCAACGAAGAAGCCTCCGGTACCTTTAATATTGATAAAGCCATAGCCAA GTTTAGG |
| *Bacteroides thetaiotaomicron VPI-*5482 | 1648 | CCGATAGTAGCCGCTGGTTTGTATCTGATATAAAGTCGGAAGTACGGCCG ACATACGCATTGGCAAGCCTCGTTCAATTAGCT |
| *Bacteroides thetaiotaomicron VPI-*5482 | 1649 | GCCGGACAGGGACTAGGTGGAGGTAATGCTGGTTCAGGTATTACAACTGC ACAATCGTTGGGA |
| *Bacteroides thetaiotaomicron VPI-*5482 | 1650 | TCGCCATGATTTTCGCGTTGATTTCCGTTTGGAGTGGAGACCTGACACAT TGACTACGATTATTTTCC |
| *Mycoplasma penetrans HF-2* | 1651 | TACAGAACTGAATAATAATTATTATTCACAAAATAAAGACTTACTATAGC AGTTACCGGAATCAAGAATAAGAATGAAATAACAAATCAAAACACATAGG ATTTTTTTAACTGTTGGAAATATAGCTTTGTTAC |
| *Mycoplasma penetrans HF-2* | 1652 | ACATAAAGTTCATTGTAATCTTTTGGGGTATTTGTTGAATACATTAATTT AGAAGGATTTTGAACATATGCATCAAACTTAGTTTGATCAAATACATTGT TAGCAATTAATTTATTTAATAATCCAATTGCTAATAAACCAT |
| *Mycoplasma penetrans HF-2* | 1653 | ATTACTTCCATTATTAGTTGTTAAACTAACACCCATCGCTCTTAATGTGT CACTAGAGAATGCCTTTTGAATTCTTGTGTCATTTTCTAAAGAAGATCCA TTTGATGTTGATGAATTAGGTAAAGTTTTTACTTCATATTTATTC |
| *Mycoplasma penetrans HF-2* | 1654 | AACTAATGTATTAAATAGAATAGTTACAAATTGAATAAAGGATATTACTT GTACTTTTGATCAGAAAATTTTATTTCTAGATACTGATTGAGTAATTAAA TCAAAATCAATTGAATTTCTAATATTGTTTGTGGATATTATA |
| *Lactobacillus acidophilus NCFM* | 1655 | GAATGTAACCACTCAAAGCCCTGTAGATAATAGTACTAATAATGAT GTTAATGTAAATAATTCTAATTTAGCTGATACACAAGCAGAATTAA TTGATTCAAATACACAGTTTTATGAAAGTTCGCCTTTAATTGATCA AATT |
| *Lactobacillus acidophilus NCFM* | 1656 | GCGAGTCCAAATGCAGATAATTACACTACTGTTAATAACTATAATG ATCTTCAAAGAGCTGTTAGCAATTATAGTGTAAGCGGAGTAAATAT CGATGGTGATATTTA |
| *Lactobacillus acidophilus NCFM* | 1657 | ATTGTTGAACAAAATCAATCATCAAGTGAAGGTGCTCAACAAGATA TTAATGCAGCAAATGATGTATCTGCACAAAATGATCAAAAAAGTGT TAATAAAATAAATGATGAAATTATAAAAAATGAAAATGTAGACGCT GATATTAA |
| *Lactobacillus acidophilus NCFM* | 1658 | TAAAGGATATAACATTCAATCAAGTACTGTTAATGTCGATGATAAT GCTTCACTAACAATTAATCGCTCATCTGTTGGCGATGGTATCCATT TGTTAAGTAATGGTATTGTTAATGTTGGTAATTATAGCCAATTAAC TATTAAT |
| *Desulfovibrio alaskensis G20* | 1659 | CCGAGCGCTGCATGTACTCAGACGCGGCATGATGCAGGGCACCGGT CAGCGTTGCTGCGTGATGCGGCAGGCTGCGGCGCGGTGCTTTCCAC AGCGCCGAAACCGGCGGATGCGGGTTGCGGGCGGAGCGGGTTGTCA TGGGCTGTTCTCCG |
| *Desulfovibrio alaskensis G20* | 1660 | CCGCACGGCTTAACTGTTTCCAGCGTATCATGGTCAGCCTGTCATG GTCGTAGTTGCGCGCGGGCATGTGCCAGCGGCCTGAGGTGTCTATG AACATGACGCGGCAGTCGCCTATCTGCGCCCATTGTGCGCTGTTTT CTGTCAGACGCAGAGCTGCCGCACTGGC |
| *Desulfovibrio alaskensis G20* | 1661 | GGTCAGCGCCACGGCGGCCACGTCGTCATGGCATTTGAAGCGCGGA AACATGCGGCAGTCGGGGTCCTGACTCTGCAGCGCGTGTATATGCC GGTGCAGTCCCTCAAGGCCTCTGGTCAGGTAGGCGCGGGCCAGATC GCCGAACGATGTTCCGTACTCCGGTG |
| *Desulfovibrio alaskensis G20* | 1662 | GTGTAAAAAATTGTAAGCATGAGAGTGTCCGGTTGATGAATGGCAG CCGGACGGCATGACCGCCCGGCAAAGAAGGACGATCAGCATACCCC CTCTTGGCAGGGCTTTCAATACGCCGGAGTATGTAAAATGGAACTG TCAGGAATCGTTGTGTTTTGCAT |
| *Bacteroides vulgatus ATCC 8482* | 1663 | CAGCAACGAAGGCAATTCATGGAGAAGTGTCCTTACCTCTATTCTC GGACGCGTATTCTATTCCTACCAAAATAAATACTTATTCACCGCCA CTATCCGCCGGGACGGTTCCTCCAAATTCGGTAAGAACAATCGATA TGGTTACTTCCCCTCTTTTTCA |
| *Bacteroides vulgatus ATCC 8482* | 1664 | AACCATCTTGCCGCCAAAACCAACAACAGCGACTGGTGGTATTATT ATGAAATTCCAATGATAAGGAAGACAAGAACATGGATGAACTCTCA GACCG |
| *Bacteroides vulgatus ATCC 8482* | 1665 | TGCCAATGACCCTATACGCAATGCGGGCAAGATACGTAACAATGGC TTTGAATTCAATTTAGGATGGATGGACCAACCCAATCCGGATATTT CGTATGGCATCAACTTAATTGGGTCTTTCAATAAAAACAAAGTAAT AGCCATGGGAAGTGAA |
| *Parabacteroides distasonis ATCC 8503* | 1666 | CTGCCGGAGCAGTTTTAATACGTTTTTCATTTGAGATTCCGTAGCGCCTT TGTCCCAAGCGAATTTCGCTTCTATATGGGCTTTTGTCAATTCTTCCTCT ACACGCATGCGTATGGCGTAGACTT |
| *Parabacteroides distasonis ATCC 8503* | 1667 | CTCTGAATGCGATCGTAGGCTTCTCCCAATTGCTCAATTCGGATATGCCT TTGGAACCGGAGGAAAAGGCGGAGTTTATAGATCTGATTACCAAAAACAG TGACCTGTTGCTTAAGCTGATCAACGATATCTTGGATCTATCACG |
| *Lactobacillus delbrueckii subsp.bulgaricus ATCC BAA-365* | 1668 | CCTGGCCTTTGCCAGAAGCCTTTTGCTGGATCCCCTGGCTAACTACGCCT TGCGCCTGGCGGTGTGCGAGGACCTGGTCAAGCTGGGGGTTAAAGAAGAG ATGCAGGTTATGATCCTGGGGGACT |
| *Lactobacillus delbrueckii subsp.bulgaricus ATCC BAA-365* | 1669 | ATTAAGCCGCTTTTGACCATGAACAATGACCAAATTCAAGTCCTGCGGGC AGAAGCTGGCAAGATAGCGGACAAATTGCAGCTGGTCGGCTTTTTAAGCG TCCACTTCGCCATCAGCCACCGGGGTACGGAAATGGTCTACAAGCTCTTG GCCGTTAAGCCAC |
| *Lactobacillus delbrueckii subsp.bulgaricus ATCC BAA-365* | 1670 | GCCGTCTTGCTCTTGAAGATCATCGACAAGCTGGCTTCTTTGCCCCAGGC AACTTTGAATGTTCTGGGCTCTTTGGCCAGTGGCCTTATCCGGGACACGG GGGACGTGATCAAGGTGATTGCTGACCAGTCCCGGCAAAGCAGGCGCAAA CTGCCCAAAGACAA |
| *Campylobacter curvus 525.92* | 1671 | AAATAGGGGATGTAGTCCCGAAAATACGGGGCGAAACGCCTCAAAATATC TCTTAGTTTCAGCTCTTTTTTACTCATTCGTATCCCAAATTTTAAATTTT CTCCCACATCGTGCCTTGGGCGGTATCCATTATGGCGATATCCATCGCAT TGAGTTTTTTC |
| *Campylobacter curvus 525.92* | 1672 | TAAGCTCATAGTCACTGATGTGAGGAAGAAGAAATTTTAAGGATTGCTCC TTACTCAAATGCTGATCGCATCCTCCATATTCTCGCAAGCAAATTGCAGG AAAAATGCGAACAGCGTTTGAGTAGTGTAACGAAGCAAAATTCCCTTAAA ATTT |
| *Campylobacter curvus 525.92* | 1673 | TTTAACTTCTAAAATATATGATCTTTCACTCATAAAATTTAGCCAAGTTA TATATATCAATTGATAAAATCAACTTTA |
| *Campylobacter curvus 525.92* | 1674 | AAAGAGTAATATTTCATTATAATTTTTAAATAAATTAAAGATTACTTTAA TATTATCGAGTTACAATAACGCCCAAATAATACGTAAATTTATAGTAAAG GAGCTTTTATGAGATCCATAACCAACAAAATAGCACTCATGCTATTGATT GCGTTGTTTA |
| *Campylobacter hominis ATCC BAA-381* | 1675 | TAGACAACATATGATCAAAAGTTTCTTCTTCAAGTCTATTTCCGATTAAT TCACTAAATTTATCGTGTATATACTTGAGATTGTATCTTATTTGTCTTGA TTGTAAATATTCACCATTTTGATAAACAACCAAAAATG |
| *Campylobacter hominis ATCC BAA-381* | 1676 | GTTTTTTAGCAATTTTTCCATCGTCTGTTTCATCCGTTTTAGTTAAAATT AGAATATGTGGCGGTCTTTTTGCGATTTTTAAAAATTCTTCATAATCTCT TGTATCATCATGAATGCTTGCTAAAAACAACACCAAATCAGCATC |
| *Campylobacter hominis ATCC BAA-381* | 1677 | TTCTAAAATAAAATCTCTGTATATATCGCGTAAATTTGTGTTACTGATGA TTTGCGGATCAAAGAAATACTCATGCTCCGGCAATAATTTAGCAATCTCT CTTAAAATTTCAGCACGATAAACTTTTTTTTTAATATTTACAGG |
| *Campylobacter hominis ATCC BAA-381* | 1678 | CCAGGAATTTCGCCAACATCAGTTCCAAAATATATATTTTGAATCTTGAT GTTATAAATTTTATTCAAAGATGTAATTATGTCATTAAAATAATAAAATA TCTCATCAAAAATTTGGATTATAT |
| *Campylobacter concisus 13826* | 1679 | CTTTTATAAGTCCTTTTAAGAGCGAATTTTCAGCTCCGCCAGAGCTTCGC CTAAAGTGGATAAGAGAAATTTGGGGCGGCCTAGAGA |
| *Campylobacter concisus 13826* | 1680 | ATACGCCCAAAGCCGTTTATTGCTACTTTAACTGACATCTTAGCTCCTTT TGATATAATTACGCCTAATTCTACAAAAAAGAATTTTAAAACAAATATAA ACAAGGCACTTTAATAGATGAAGTTAGCACTTTTTGGC |
| *Campylobacter concisus 13826* | 1681 | AGTGACGCAAAGATCGTCAGTATCAATGGTGATGAGGTTTTAATCGACGT TGGCAAGAAGTCAGAAGGCATTTTAA |
| *Akkermansia muciniphila ATCC BAA-835* | 1682 | TCCTAACGAACCCCAAGTCAAACCGGACCCCCGCGGCGGTTTTTCAGGAA GCCGCCGCTGAGAGACCGCACAATTCCGGGTGAAGCCGCTTTACACACTT GCCAATAGTGGGAAGCGTGCTA |
| *Akkermansia muciniphila ATCC BAA-835* | 1683 | TGGGGAGAGTAAAACTAGATTGCCAACTGGATGAGATAGTTGACCACGCT GTGAAGAAAGATGTCGAACGATGCAACAAACG |
| *Akkermansia muciniphila ATCC BAA-835* | 1684 | TCTCTTCTTTCGTGGGAAATGAGGGGGCCTGCGGGGAGGCCCCCTCATTA AACCTGATGTAGATTCCTCTACAAGTTCCTGAGGAACTTAGTCAAGGATT TCGCTGATA |
| *Bifidobacterium animalis subsp. lactis AD011* | 1685 | CTGTGCGAAGTCGCGCCGGGCGGCAACGGCGAACCGGTCGTCGGCGATGA CGAAAGCATGCGCGTCGGGTGGTTCGCGCTCGACGATCTGCCCGAACCGC TCAGCGACAGCACAC |
| *Bifidobacterium animalis subsp. lactis AD011* | 1686 | ATCTCAGGCACCGTGCGGAAGGAAGCGCACGGGCACAGTTCGCGTTCGAC GGGGTCATCGAGCATCTGTAGGCCGCATACAGCGGCATATGAATCAATGG ATGCTGCCGAACT |
| *Bifidobacterium animalis subsp. lactis AD011* | 1687 | GGACTGGGAGCCGCTGTTGCTGTCTCCATTGCCGGAGTTGCCGTTGCTCC CGCTGCTGCCGTTGCTATTGCTCGGTGAGGGGCTGGGGCTTGGGCTTTCC TTGGGCTCAGTGGTGAGTGTCACCTCGGTACCTGGATTGACCTGAGACCC TGCGCTCGGAT |
| *Bifidobacterium animalis subsp. lactis AD011* | 1688 | TTGCTATTGGCTGCCACCTTCCACTTGAGGTTGAGAGCCGAATCGGTGAG TGCGGCCTTGGCTTCCCCGAGTGTACGGCCTACAACGTTCGGCACTGCTA CCTTGCCGTTCGACACCCAGATGGTCACGGAGGCGCCTCGCTCCACCGAT GTGCCCTCGTTC |
| *Atopobium parvulum DSM 20469* | 1689 | GTAGGCCTATATGAAGCCTTAATCTCAGAATCTGCTGCATCAACGCATTC TAGTAACTCATCTTGTACACAGGCATCAAACACTTCTTGTAAAGTTAATG CACCAAATACCTGCTCAGACTCATCCAAAAGCGCAGGCGCAATTAGCTCT GTGAGGCGTGC |
| *Atopobium parvulum DSM 20469* | 1690 | AGGAATTCATAATGAATCACTAGCTTAAAGACAGCTTGTGTTCAGATGCT TCTTGTTTGCCCACAAGTTGTGTAACTGTTTCTTTAATTTCTAATGCAAG ATCAATAAAATCTTGAGCAGTAACACATCTCACTGACTTGCCACGC |
| *Atopobium parvulum DSM 20469* | 1691 | AGATGTAATATCATCAAAGCTAGGTCTTTTTTCTGCCATTCTTCAATCCT TTCTTTATTATTCATTTTATTTTGTAACATCAATTAAATACTAACTGCAT CAAATAAATTTTTCTAACATTATCTTAACTCCCAAAAACGGCCATAAAG |
| *Veillonella parvula DSM 2008* | 1692 | AATAGATATTGGTCCACATCGCGTAAAAGCAGGGCTAGATATCATTTTGT CAGGTGCTATTGGAGATCACTCCATTGCCGT |
| *Veillonella parvula DSM 2008* | 1693 | CGTCTCAGTGAGAATATGTGGGGAACTCACGAACTGACCAAAGAAGCTAT GGAACGCTCTTTGCGTGCTCTAAA |
| *Veillonella parvula DSM 2008* | 1694 | GTACAGTCAGTATGTAATATATTAGGGTATGACCCTTTATATTTAGCGAA TGAAGGGAAAGTGGTT |
| *Citrobacter rodentium ICC168* | 1695 | TTTCATCATTTGTCATACTTAAGCATATTTTTTATCAATCATTATAACAA AAATTGTACAGAGCAGAGATGAAATATATCTTGTATCTCTATGATTTTAA TGTATTTATAACGCGTATGAATTATTTTA |
| *Citrobacter rodentium ICC168* | 1696 | AATTGATTGCACAAGCGGAAGCCGAAAAACAACGGTTGATTGATGAGACC AACGTCTGGATAAACGGGCAGCAATGGCCGTCTAAATTAGCGCTGGGCCG CCTCTCTGAGGATGAAAAAGCGCAGTTTAACGAATGGCTGGACTATCTGG ACGCGGTGAGTGCCG |
| *Citrobacter rodentium ICC168* | 1697 | GTGGGTTTTTATATCGAAGGTGTGTCTGCGGTTCCCTCCAATGCTATTGA AGTTAGCGCGGATATTTATAATGAGTTTGCCGGAGTGGCGTGGCCTGATG GGAAAGTACTAGGTGCTGATGATTCAGGATATCCGACATGGAT |
| *Citrobacter rodentium ICC168* | 1698 | CAACCCCATCCGCAAAAAACAGCGCGCCCGAGGGAAGTAAATGCGTCAGT GACTTTAGCTAATTGTGCTGAAATTTACCCGTAAT |
| *Streptococcus gallolyticus UCN34* | 1699 | CCAAAAGTGTGTTATTGGAAGAAAGCGTTGAAAACTTTGATGCTGTTGCT ACCTTGACAGGAGTTGACGAAGAAAATA |
| *Streptococcus gallolyticus UCN34* | 1700 | AATCGTGTGGAAATGATTCTCTTCCACAACTTTGTCAAAACCAAAATCAT TAAAAATCTGATGATTATCGGCGCAGGACGAATCGCATACTATCTCCTCA ACATCTTAAAACATACAAGAATCAATCTTAAAGTGATTGAAAACAA |
| *Streptococcus gallolyticus UCN34* | 1701 | CTTTCAATATCTTTTAATTGATGATAAATGTGATATTCCTCATCAGCGGT TACGACCTTGACCATATGAATTTTTT |
| *Enterococcus faecium TX0133a04* | 1702 | CTTTCTGGTTTCTTTAATAAGCGGACCTTTCGTACTCCAGAAATTTTTTC TAGCTGTTGTTTCACTTGATTGAGCTGTTCAAAATCAGTACTGTCTATGT CAATCAATATGTAGACATGCTCATTCTTATGGTTACGCGATATATC |
| *Enterococcus faecium TX0133a04* | 1703 | CACGGCCAGCCACTTTCTTTACTGTCAGTTGAAGGATCTGCTCCTCCTCA GATTGTGTGAGCGTAGAAGATTCTCCTGTTGTACCTAAAACTAATAATCC GTCTGTTTGTTCATCTAAATGGAATTGGATCAATTTTTCCAAACCAGCAT AATCTACTGATCC |
| *Enterococcus faecium TX0133a04* | 1704 | ATGTATTCACTCGGCTGTCATATTCGTGATTGTTATCAGCAATATTGAAC CAGAATTTATACATGTCGTTTTTGTATTCCCAATAAGTAGTTTTCGTCAC TTTTTCAGCATCGCCGTCTGTCTTTTCTACTTTATTTGTTTCGTTATC |
| *Peptostreptococcus stomatis DSM 17678* | 1705 | ATTAGTATACCAAATAGCTCAACTATAGCTGATATAAATATAAGTCTGCC CATATTTATAGGTACACTTCCCATAGTCTCCTTGGTTCTGGCATTTACTG CAACATAGTGGAGGATATTTTCACTACCCTTTTTCTCCATATAGGAGTAT AGC |
| *Peptostreptococcus stomatis DSM 17678* | 1706 | TTGGTATCCCTCTTCTCAGATGTGTAGCCTCTTAGGTAGTTTGAATTATA CTTGACCACATTATCTGTATCAAAAGGCATTATGGAATTTATAATGTTGT TGGTCTTGCGCCTGTTAGATTGGTCTAGCTTGTCTGAGCT |
| *Peptostreptococcus stomatis DSM 17678* | 1707 | TAGTCCTAACCTTCTGGTCGCTCGTCTCCATATTTTTTATATTGTATTGG GTTTCTCGCTCATAGGTATGTCTTGCATTTGAATTTCTATACCTCAAATA CTTGATTATAAAGAATATAAATCCTAATAGGTAGAATACCCAA |
| *Peptostreptococcus stomatis DSM 17678* | 1708 | TTATATACATCTACATCATATACTACCTTCTTGTTGTCGTCAGACCCAAT TGTATATTCACATATAGTCTCTTCACCAACGCCCCTTAGGTCCACATGGG CCTTCATGTCAACTATCATATAAGGTAAGTATACACCACTTA |
| *Mycoplasma fermentans JER* | 1709 | CTGTTTAAAATAAAAATAGGATTATTTGTATTATCTGCAATTACATAATT AACAAGATTTTTAATCTTGCCTGAAGTTAAATTGATTTTATCTTCGCAAT GAGCAAAAATAAATTTTCTAATT |
| *Mycoplasma fermentans JER* | 1710 | CTTTTTCTAATCATTCTAATTCTTTGTTTTTTTCATTAACTTCATCAATA ATTTTTTGTTTTTCTTCGTTAGTTAATTTCTTTAGTTTTAATCT |
| *Mycoplasma fermentans JER* | 1711 | AAGAGATGAAAAAATATTTGGTTTTTTAAATGAAAATGAAACTAAAAAAC TAGTCAATAAATTGCACAAATATAACAAAAAATATTTACTAAAATCAATT AAATACTTTAGAATTGGTAAAATTGTAGAAAGAAAAAA |
| *Mycoplasma fermentans JER* | 1712 | GCTAATGGATTAGAAATTTTAGATGGTAAAATTATGAATGTAGAAAGTGA TGGAATGCTTTGCTCAGCAGAATCTTTAGGTTTAGAA |
| *Eubacterium limosum KIST612* | 1713 | TTTCAGCACAGTGGTCAGCAGCATGTAGGCCGCCACGACAGCCAGCAGAA AGCCAAAGTAGCGCGGCGGGAGGATGGTCAGCCCCAGCACGCTGAACAGC GGGGTAAAGGAAAGCCCGGTGAACAGCAGGATACCGGCAACGGTGATGAG CATGACCGGGGCA |
| *Eubacterium limosum KIST612* | 1714 | GTATTTTGCTTTTTTCACGTTCGGACATTCTCTGCCAGACGTTGAGCCAG CCAGGGGTCAGCCCGGCGAAAAAGGCGGCTCCGGCCGCCAGAAAGATCAG CCCGAAAATAATGCAGTATA |
| *Eubacterium limosum KIST612* | 1715 | ATTCTGCGATGATCTCGATGCGGCCGCCGGGCAGGTCGTTGCTGTCCAGG CTTACCAGGTACATTTTCCGGTTCCGCACCGCGTTGATGAGGTAGCTGCG CAGGCGGCCCTCATACCGCTCGTCGCACACAACGGATACGGTGTAGCGGT TGTCCGTCTC |
| *Eubacterium limosum KIST612* | 1716 | TTGCGATTAAAAAAGCCGGACATAAGCCCGGCTACTGAACCTGCTCCCAC TTGGCGGAAACCTTGTCCTCATTTTTCAGGCAGCTCGTGGTGATCTGCTC AATG |
| *Blautia obeum ATCC* 29174 | 1717 | TTAATAAATACATCTTTTGTCATAACTTTGTCTTCTCCTTTTGTCCAGCC GGATTACTACTCATGTTTACTTTTACACTTATTAGATGCATCGGAAAGGA AATCGGTTCTCATAAAAGTATTTTTTTCCGT |
| *Parabacteroides merdae ATCC43184* | 1718 | TCATCCAAACGGCGTTCCGTCTGCAACTCCGTCGGTATATCTAAAATATA AGACAACCAATAATTCACCTGAGCAGATAAAGTATAAGAGGAATCATCTT TAATCAATTCCGGCATTAAAGGATTAGATTTTTCTTTTTCAAATGAACCT ACTATAT |
| *Parabacteroides merdae ATCC43184* | 1719 | ATAATACTTATTTCTAATCGTATCGAATGTCAGATTCACAATAGTATCCA GTAAAATTTGTCCATTTTTTCCTATCCTGCGAACAGGAAGTAATACCGAC TGTATCAAACTTGACTTACCTGCCGAGTTAACACCCGTTACAATTGTAAA |
| *Parabacteroides merdae ATCC43184* | 1720 | CGAAATTTTGGAATGCAACTCCGCCCCGATTCTATTGGCAGACAAATAGT ATAAATTTTGCTCTATGTCGATCGGCATGCTGGCATCCTCTTCCGAAGGA AGAAAAACCAAAGAGAGTTCATTTGTTCCGTGATAGGAAATATGC |
| *Parabacteroides merdae ATCC43184* | 1721 | ATTGATCGTACAGGCATATTTTTTTCGCATCCTGGCATAGTGCTCTTATG TGCCTGATCGCCTTGTCTCTATGGGCATTTCTATAAAAACAACCGGTGAT ATGGCTACTGATCTGATCGGACATGATATTGACATACGGATAATCCGTT |
| *Faecalibacterium prausnitzii M21*/*2* | 1722 | GCCCACGGGGGTGTATTTGATGGCGTTATCCACCAGATTGACAACGACCT GCATGATCAGCCGTGCATCCACATTGACCAGGAGGATCTCGTCCCCATAC TGTGTTGTGATGGTGTGTTCGCAGCTTTTCCGGTT |
| *Faecalibacterium prausnitzii M21*/*2* | 1723 | CCGCCGCGCCACGCCGATCGTCAGGCTAGCGCTTTGGTAGCTTCATGAGT TCCTGCCGGTCCGCAAAGCTTAGGTCCGTTCAGGTGCTGTGTCTAAAGGA CGCCCCGACCCCACACGACACAAGAGTGGGCGGAACGCGCAGCACTC |
| *Parvimonas micra ATCC 33270* | 1724 | GATTTCTTTTCTTCAGTTGTAAGAGTGTTGTCTGAATTAACTTTATCCTT AATACTATTTGCATAAGCAGTTAATTCTTTTTTTGCAGCTTCTTTTGCTT TTAAAGCTTTTTGATCATCATTATCTGATGCTTTTAATACATTTGTTG |
| *Parvimonas micra ATCC 33270* | 1725 | CGATTTGTATTGGTATATTTTTGTTTTTTAAAAATACTATGAATTTAAAT TTCCTACCTTGGAGAAATGCATATTTGATTGTTACATTCTATGTTTCAGC TGTTTGTACATTAATGGCATTTATAGCTATTCCTAAAAC |
| *Parvimonas micra ATCC 33270* | 1726 | ATAAGTTTATAAATGGAATTTTGGGAACTATCGTTAGAGCAAAAAAATAA AATATATTTTTAGGAGGTAGTTATAATGTTACTTAATACTTTATTGTTAG TTGTGTTCGTTGGTATTGTTTTTTCAGGCATAGCTGTGTCAACTTTTTT |
| *Parvimonas micra ATCC 33270* | 1727 | AATTTTAAATTGCTTCTTTAACTCCTCATCAATAACTTTTTTAGATATAT CCTTAATTTTATTTTTAACAACTGAAGCCTCACCAATCTTTTGTTTCATC TCTCTTTTTATTAAATCAACATACTTATGAGTATGTAAGATATTA |
| *Streptococcus infantarius subsp. infantarius ATCC BAA-102* | 1728 | TTATGGCGTTCTAACAATATACGAAGTATCTCTTTATAATTAGCATTAGG ATACTTATCAAGATATGCCAACTGAGTTTCTAAAAGTTTATCAAAACGTT TATCAAGTGCTTCTTTACTCTTAGGGCGAGAATCTGAACCTTGGTTAACA CGA |
| *Streptococcus infantarius subsp. infantarius ATCC BAA-102* | 1729 | AATGTAAATGATTTTGAGCGTAATATTGAAATATCATATACTAGTTGAGG ATCGTTATCTTTAAACATTACGTAATTACCAATGATGTCACCATTAGTAT AACTTGGTAAAATTTCGGTGTTAGATTCATTATTAACT |
| *Streptococcus infantarius subsp. infantarius ATCC BAA-102* | 1730 | TAAACTAGTAGCTTCTTCTGGAACACTAAAAAAGACATTGTCTTTGTATT GTAGTTGAAATTCCAGTGCTTTTTCTTGAGAAAATCCTTCTTCTGGTGTT GCATAATAAATTGTTACTTTTTGAGAACTTACT |
| *Bifidobacterium bifidum NCIMB 41171* | 1731 | GTATTTCCTGCTCGTCGTTCTTGCTGACCAGTCCTTGGGCGCCGGCCTGC GCCACGTCGAATCCGAACGTCTCCTTGGCGAACGAGGTCATGGCCAGAAG CTTCACCATGCTGTCGCGTCGGCGGATACGACGGCATACCATGACGCCCG ACATGGATTCCATCG |
| *Bifidobacterium bifidum NCIMB 41171* | 1732 | TACAAGATCTGCATTGAGTTCGACGGAGGGCATCACGCCGGTCAATGGCT GGAAGATGCACGCCGGCGGCAGGCAATCGAAGACGAGAAGTGGCGGTATA TCCAAGTCACCAAGCTTGACCTCGGTGATGAATGGAGTGAGGAAGCTTTG GCCAGACGGA |
| *Bifidobacterium bifidum NCIMB 41171* | 1733 | CCGAGCCGTTTCGCTATCGATAAGTCAGTCAGCCCACGCGACATAAGATC GAGCACCTGCACTTCGCGGTTGGAGACCAGGTCAGCCCGTTTCGGCGTCT CATGCGCGAGCTGGTCGAATGAGGCACGGGCGTCAACGAACCCGAAATCA CCGTAAACGCCTCC |
| *Bifidobacterium bifidum NCIMB 41171* | 1734 | CTTCCCCTTTCCTGAATATGGATAACCGTAGTACCCATATGGTTAGTGGT CATATTGTAGGCGCATAATGTGGACAGCCGACGCTAGGCTAGAGTTAGTG GAATGGCGGTCGCGAGGCTGCCGCGGATTGCGATTTGTGGAGTGTGATGA CGATGGGCG |
| *Collinsella stercoris DSM 13279* | 1735 | CCGACCAACGCCAAGATGATGCCGAGCTCGATCGACTCGGAAACCTGCTT TGCGCGTTGCATGCACCCTCCCTCTCGAATATGCCGTCAGTGCAACTCCC TAC |
| *Collinsella stercoris DSM 13279* | 1736 | CATGCACAATCGAGCGAGGTGCGATCGATGCCATCTCGGTGCCGTAGTCC CGATGATGAGCCGGAATTTATAATCAAGGCGTTGAGATGGGAGGTTGCTT GTATGATTTGGTTCACATCCGACACGCACTTCGGCCATGCCAACGTGCTG CATTTCACCG |
| *Collinsella stercoris DSM 13279* | 1737 | TCAAAATACCCTACTTGTGCGCCGAGCCACGTGAGGGAGATCGGCAAAAC CGCAGGATTTGTTGGCGTGGATGCGCTCGGGTGCAAGGTCGGTGGGCTCC AGGCATCATTTGCAACGGCAAAACCGCAGGATTGCTGCGCACGAATGAAT ATACATGACGGCA |
| *Collinsella stercoris DSM 13279* | 1738 | ATTTTGACCACTATCGCAAAAACGTATCAACCGTCGAGGGCGAGGGCCTC TGCGCCCGCTGAGATGCGAGTCTGAAAGTCGGGTTCTGCGCGAAGTGATC GTCAACTTGACACATATGCCGAGCACGACGGCGGGTTTATCATGGGCGCC C |
| *Roseburia intestinalis L1-82* | 1739 | AAACGGCAAAAAAAGTCCCCACCAGTAATACGATTGGAAATATATATGAA ATAATTCCAAATAAACCAAAAAAGAAGCGGCTGACTGTACCGCCGATCGT ACCGCCAAATCCAAAGTTACTGATAAACAGCAAAAGTGAAACAGCAACCA CGATCCATAAAA |
| *Roseburia intestinalis L1-82* | 1740 | ACATATGGGAAAGTCCGCCTCCGATCAGCCCGCCTCCACTTTTTGCATCA AAACTGTACCGATAAGCATCCATTGCACTTTTTGCATCAGTTCCATCGGA AATGAGTTCAAAAAACATACATAAAAATGCCACAAACAAAATGACCGCCA CTAATTT |
| *Roseburia intestinalis L1-82* | 1741 | CGTTTTTTGTCCATGACGCTATGGTAAACCCAATACTCACAAACGTCAAC ATTCCTATTGTCTTTTAAGAAACATTTTAGATTTTGGTTATATTTCCACA TATGGATATCTTCTTATCTATTGATATCTGATTTAAGCAGGACGATAAGC CGGGTTATGTCTAAAC |
| *Roseburia intestinalis L1-82* | 1742 | TAAATATCTCTGGTTCTCTTCGTAACGCTTTGACACATTCCGTGAGAGTA TTCTGAAATAGGAATAAATTAACAATGCGAGGCCAAGCCAGTATAAAATA TTTACCCGGAAAAATGCAGATAAAAATGTGGATACAAGTAACAGAACCAT CG |
| *Enterococcus gallinarum EG2* | 1743 | TGGGTGGCATGCTTCAATTTTTTGATCATATCTTTTCTCGAAAAGTAAAG ACTGGCTATGAGATGCTCTTT |
| *Enterococcus gallinarum EG2* | 1744 | GGGTCGTCTTGAAGGTGGCTGAGGTCCCGATTGGCTTCGGCAAACAAGTG CAAGGTGTGGTGAAAAACTTCTTCCCGAAAGGATTGCGGTTCACTCTGGA ATAAACGA |
| *Enterococcus gallinarum EG2* | 1745 | GGTAGTGCAGGATTCATCGCATTGGCGATAACGATTGCTTTTTATAATTT TGGAAATCCATTAGCAGGGGAAGCCATCTATAAAGTTTGGACTCAAGAAT CATTTCCAACAGAATCTC |
| *Prevotella copri DSM 18205* | 1746 | CAATAGACAATTCTTGGCAAACAATTCTGGCAGTCTACTCCTTCCTGATA TTTGCCCATTCCTTCGCAACCCTGGGAGGAGCCTTCTACCGTAAGTTTCC TGTGCTTCTGACAGCATGTACGGGATTGGCACTTTGTCTGATTTTGGGTT ATATTATCAA |
| *Prevotella copri DSM 18205* | 1747 | TTTCGTAGGAACCTTGCACATCGAAGAGGGTTCTTCTGCTCAATATTGTG CTGTATTCACCTCGTCTGCAGTATTTCTTGCTTTGGCTGCCTTCAACTAT TGGGCATCCTACAAGCTCTTCACCCGTATGCAGGTTATCTGCAACAA |
| *Holdemania filiformis DSM 12042* | 1748 | CAGACGACCTTGCGGACTCTGTCTGGAAACACGCTGTCCGCGTGCCATCA GAAGCTTTTTCACTCGATAATAATCCAGCGTTAAACGA |
| *Holdemania filiformis DSM 12042* | 1749 | ATAATTCCGGTGCCAGAACAATCGCGGGATTAAGAAATCCGATCGCCATC GGCGTAGGTGCTGACCGGCTGGCAA |
| *Holdemania filiformis DSM 12042* | 1750 | TTCGACTGCCCTTTTAATAATCGCTGATCACAGGCCATCTCGATATCATT TTCCGCCTGATTGTTCATCAGCCAGACGAACGGATTAAA |
| *Holdemania filiformis DSM 12042* | 1751 | TAGAGGGTCTTTTCAGTGAGCTTGATTTCAGGATGGCTTTGTAGAATGGC ATAGACCGACTGGCCCTGCGCTAACAGAGGCTTGATTAGAAGACCGAGCT GCTTGAT |
| *Helicobacter bilis ATCC 43879* | 1752 | CCTTTTAGAGCAAGATGAAGCAGGGCTATTATATGAGTATCTGGGCTTTA TTAAAAGCCGTGATGACA |
| *Helicobacter bilis ATCC 43879* | 1753 | TTATGGCAAGAATACTTTGCAAAAACGCATACGCATGTATCACAAGAAGC AATCATTAGTAGTGGTAAAAA |
| *Slackia exigua ATCC 700122* | 1754 | GGATATGCATCGGCGGTGTAGAAACGGCGTGCATGCGACATCGCACTTAG ACGGGGACATGCGAAGCGCATCGCAGCGTCTGCGTCTAGACAACGGGTGC GCGGCGACGCAGCGTCCATGCCTGGATAGTAGGCGCGATGTACCGTACCT CGAATGTTGTCTGC |
| *Slackia exigua ATCC 700122* | 1755 | CGAGCGCGCCGTCGCGATACATCAGCAGCGCTGCCGTTTTAGAGAGGCGC TCTTCTTCGTGGGCAAGCTTCACGTCGTCCATTATGCCCAATCTGTTTTC GAGGGTCATCCAACCACCTGCCTGCCGGTACCGAATTCGG |
| *Slackia exigua ATCC 700122* | 1756 | GCCGAGCTTCTGATAGATGTGCTTGATATGGGTCTTGACAGTGTTGTAGG AAAGCACAAGCTCCTGTTCAATGAACTTTCCGTCGCGCCCGCGCGCAAGC TGGCCCAGAATCTCGAGCTCGCGCGTCGTGAGCCCATATGCTCGAGCAAG CACTTCGCACCTCT |
| *Anaerococcus vaginalis ATCC 51170* | 1757 | TTTAATAGTAAGTTTGATTATAGCTATTTTGATGCAATATATAATTGGAG TTCCTATAATTTGGTTGACAGAAAGTGTAAATTCACTTCTTAAAAGTTTA AGCTCTAAGCCAGAATATTCTATGATTTTTGGAGTTG |
| *Anaerococcus vaginalis ATCC 51170* | 1758 | TATTCTATAGCGGACAAAGCTGGAATAGCACCAGGAATTATTTTGGGTGT TTTATGTAAGACAAATGGTTATGGATTTTTAGGTGGTATAGTAGTAGGAT TTTTAGCTGGATATTTAACAAAAATAGTTTTAAGTAATTTAAAACTT |
| *Anaerococcus vaginalis ATCC 51170* | 1759 | TCTGAAGTTAATGATGATGATAATGACAATAGGTTTTATGAACAAGTAGG AAGATTTCCTGAAATAATA |
| *Collinsella aerofaciens ATCC 25986* | 1760 | GCAGCAACTGCCTCACGGGCTCATTGAAAACCGCATAGCACCCCAACGTC AGCACATCACCCACGGCAAGCACATCACTCACGACAACCAACACATCAAC AAACAGCACGAACATCAACCGATTGGAGAAGCTATGACAAACCACCGCGC TGAAGACGG |
| *Collinsella aerofaciens ATCC 25986* | 1761 | TGGATGAGATTTACAAGATTAAGACGAGCGGAAAATACCATTCCTTCGTC CCTGTTTATCAAGATCGCGGAAATCATCGTTATGCTATTTCCCGATCGGC GCCAAAACAGGACTTTTCTATCCTTCTATGCGAGGACAGTAAGTCTGGTT TCCAGTTC |
| *Collinsella aerofaciens ATCC 25986* | 1762 | AGTAGATCGAGCATATGAAGTTTGAGTTTTAGCGTGTAAGAGAAGGAAGA TCGGCAAAGTACAACCCCAAACGCAATGACAACTTAATGAGGTAACTGCC ACATGTGAGGCACCCAGGGCATTGCTGTCTCGATTTTGAGCACGATGCCT TCCGCCT |
| *Dorea formicigenerans ATCC 27755* | 1763 | TCTCAATCTTTCTTCCTTTTACAATTAATGAAAATGTCCCCGTCATTACA TTAATTCCTGCAAATACACCATCTACATCGGTTACAAGGATACCATGACA CATTTTTTTCTTCAAGTCCAAAATTGAGGATTCTGTTTTCTCA |
| *Dorea formicigenerans ATCC 27755* | 1764 | TGTTCCGAACCAATGTGCTGCACTTGGTCCCTGATTGATTGCACCCGAAT CAATTTTCTCATATCCATACCCACTGATGGCCGGTCCGAATATAATGAAA AATATAAGAACAAGAAGGATAATACCTGCCAGAAGCGCTACTTTATTTTC TTTAAATCT |
| *Dorea formicigenerans ATCC 27755* | 1765 | TCTCGGACGAGCAGGAACCTCTTCATCTTTCTCAAGTCCGATGATTTCAA ACATTTCATCTGGGATATTTTCTGTCATATCAAATGTATTCTGGTTTTCC ATCATGTA |
| *Dorea formicigenerans ATCC 27755* | 1766 | ATGATGTA ACATCAGATTTTGGCGATTGTTTAAAACCATTTCCTGTAGGTTCGATTTT TAATTTTTTCGCAAGTTCTTTATTTACAAGTTGTGTTTTAAATGTTCCTT CTTTTATCAGGTATTTTTCTTTCACAGGTATTCCTTCATCATCAATTCTA |
| *Ruminococcus gnavus ATCC 29149* | 1767 | TTGAAATTTGTAGCAGTAGCGGAACCAAGAGCGGATCGACGGGAAGAGTT TGCAAAGCTTCATGATATTGCACCTCAAAACGCTGTGGAATCGGACATGG AGCTTTTGAACCGTCCGAAAATGGCGGACTGTGTTCTGATCTGTACACAG |
| *Ruminococcus gnavus ATCC 29149* | 1768 | TTAATCATATGTTTCTTTCCTTCCCATTCTCTTTTTTTCTTTTGCAGAAG TGATCACGTGCCGGATTGCCATGATTGGATGATAAAATAACATTCTCGGT CCGGCAAACCGCATCACATTTCGAATTTCTTCTCTCATCTGCGGCTTGTA ACAAT |
| *Ruminococcus gnavus ATCC 29149* | 1769 | GTAATTCTGCTTCCGCCGGTGATCTTGTTCTGCTGTTTCAGCAAGTACTT TATTGAAGCACTGTCTGGTGGGGCAGTAAAAGGCTAAAAGGAGAGAAAAA ACATGAAACAAGTAACAGCCATTCTTTTGGGAGCCGGACAGAGAGGGGCA GAG |
| *Ruminococcus gnavus ATCC 29149* | 1770 | TCCAATGTTTTTTCAAACAGCTGCTGGATCATATTTCCTGCCGCAAACAC TGCTGCGATCTGAAACAGCAGTGCGATCCACTGCCAGAAAATATTAGCTC CGATATATTTTC |
| *Campylobacter rectus RM3267* | 1771 | TTTTATGCTTTTTATTCCAGCAACCTTCATTAAGCTCTACTAATCCTATT ATGTTTTTAAAACTTAAAATTTGATAGTTCTAAATTTATTAAAGTGCTTA TTAATCGTATCGCAATTGATACTCGAAGGCTTATTTGTT |
| *Campylobacter rectus RM3267* | 1772 | TAAAAGAACATAACATAAAATCCATAGATTTACGTTATAAAGAAACACAG ATAGATAACAACGGCAATCTAATCAAACAAACCTCTACCGTTAC |
| *Campylobacter rectus RM3267* | 1773 | AATTTATCTTTTTGACAATCACAAAATTTAAATTTGACAATTAGCGGCCT TGTTGTTAGATTTGAGGATAAATTTTGGCTCAAGATAAGTTAAAAACT |
| *Campylobacter gracilis RM3268* | 1774 | CGCCAAAATTTAGCGATGTCAAGTCCGATTACACGAGAATAGTAGATGTT CATCACGCAGATATTTAGCAAGGTGATCGCAAAAGCCGTACCGCACGCAG CGCCCACGCCACCGTAAGCCTTAGCTAGCGGGATAGAAATAGCGATATTT ACGAGCGTG |
| *Campylobacter gracilis RM3268* | 1775 | AATTTTATCACGCAGACTTCATTCTCGCGGCAGAAAAAATTTGCCAGCAC GCTTAGCACGCGCTCGGCACCGCCCTTGCCTAAAGCAGAAATTACCATAG CTATTTTCATTTTATGAGCCTAAACTTATTTTTAAAAATTTCATCG |
| *Peptostreptococcus anaerobius 653-L* | 1776 | AACAGATTATTCCAATATGTTTTAGCCTTTTCATAATGTTTGTTATCTGG CAAAACTACACCTCCTCTACTTAGTTTTTGTTTAGATCAATAAATTCTGC TATCTTGTTGACAGACCTAGCTTGCTGTTTTTCAGGACTAATAATA |
| *Peptostreptococcus anaerobius 653-L* | 1777 | TATTTATCAATTTATCATTTAAACTATTTAGGTGGCCTTCTGAAGATATC ATATAGTAGTAGTATTCCATGGAATCACTTGTCTGTCTCATAATATCTCT TGCAAGGTCTGTAAACCTCATGTCCCTACTAGATGGATTGAC |
| *Peptostreptococcus anaerobius 653-L* | 1778 | GCTATTTCCACCCTCTATAGCATAGTATTTAGACTTGCTCGGTAGGTTTT TCTTGGCTTCCTTAATTCTAGTAAAATCAAGAAGTCCGTCATTTGTACCC CATATAGACAATACCGGCATATTTACTAGGTT |
| *Peptostreptococcus anaerobius 653-L* | 1779 | GGCTATTATTTGATTTATAATGTGCTTAAATAGGTATATTATACTCCTAG CAGTCACCTTGTCATAATAAGATGGCTTGTACATAAGCTTTATAGACATT CCCTTTGTAAGACTGACCTTCATATATAAATCAAGGTCTTTCTCTTTTAT CG |
| *Prevotella histicola F0411* | 1780 | ATAAAATATGAAACAAAAAACTTTTCCTTACCAGACAAGATGCCATTGTA CCCTGGAGGTGATGGAGCATTAAGAGCTTTCTTATCTTTGAACTTACATT ATCCTGAAAAGGCACAAGCTTTTGGTGTAGAAGGTAGAAGTCTCATGAAG |
| *Prevotella histicola F0411* | 1781 | GGTTGTCTTCAATGTTGAAATGGATGGAACAGTGACGGGAGCTCGTGTTG CAGATGTGAAGAATGCCCGTGGAACCAGCAAGCGTTTTATGAAGATGGAA CCAGCAAAACAACAACGGATTCTTAAAGAATGCGTTGATTACTACAA |
| *Prevotella histicola F0411* | 1782 | AAATGGACACCAGCTCAGGAGAACGGACGCCCCGTTCAGAGCAGGACTTC TCTGACAATTGCCTTTCGGGCCTGACCATTAAAGAAACTAACTCATGATA ATACGAAGAGAGCATATCGATAAGAAGACAAGCTATAG |
| *Prevotella histicola F0411* | 1783 | GTTGTCACGTCAACAGAAACAGGATCAGACTCTGCTCCGTCAGCATATAC GGCAGTTACGCTGTAGTTATGCTTTGTTCCGTCAACGGTAACATCACCAC TCTCCAGCTTGATGCTGTTAGATAGATATTCACCATCACGATAGATGTTG TATGA |
| *Helicobacter bizzozeronii CIII-1* | 1784 | TGTCAAATTCTTGCAAGAATCGATGAAAGTCGTCTATGATCACCACTTGG TCAAGGATTTCATTGAAAAGGTTTGGATTCTCAATACCTACCAAATCTCG CCCAAGATGTTGGAAATTCTCCAAGATGAGCTCATGCTAGACATTGTGCA AA |
| *Helicobacter bizzozeronii CIII-1* | 1785 | TGCTAATTTCTTTGGTGTTGCCCAACATCGATGCCGCCACCTCGTGCGCC ATGCGGGCGATCTGATTGACCTTATCAGCGATGTTCTCAATGCTTTGGTG CTGATCTTTGGAGTAAGACACGACATTTTTGGCATCTCTTAAGGAATGGG TGGC |
| *Helicobacter bizzozeronii CIII-1* | 1786 | ACCTCGCACATCTAACTCCATAAAAGTGGGGGCGAGCAAGGACACCCTAC CTACCAAACCGACAATCATGCTAAAACTTGCTGATGTGCTGGCGGCTACT GACCCTTACCAGCTCTCCATTCTCTTTATCTAGGGCAAAATCCGTCATTT C |
| *Enterococcus hirae ATCC 9790* | 1787 | GCCAGTTAGGAAAGTGATTTTTGAACTAGTGGCTATTGTTGCTACCGCTT CTTCAACGGTTACTTTTTTCATTTCCTTTCACCTCTCTCTTATTATACAT CTATCTTTCGTTTATTGATTGATCGTACTTTTTATTCAAACGATTTCATT T |
| *Enterococcus hirae ATCC 9790* | 1788 | TGACAGAGTATAAACTCAAATCTTTTGTTGTCTCTTTCTTTACGAGAATC AAACGAAAGGATGTATCAATCTGACAAGTTACTCGACTGTCGGCAAAAGG ACCATCTCCGTCTTCAATATCAAGTAATAACTGTTCATCTTCGTT |
| *Enterococcus hirae ATCC 9790* | 1789 | AAGTGAATAATATTTGGTCTTGCTGTTGGATGATAGATTTTTTTCACAAA TGAATAAAATTTTTGTGGCTCATTGTTCATTGCTTCATGACTTAGAAGGT ATTCGGGTTGTTCCAATCCATGATAGATTCCTTTTAACGATCGATAGTC |
| *Enterococcus hirae ATCC 9790* | 1790 | ATCCTAATTTTATCAATGCTTGACTCGCTTCTGGAACAAATCCTCTTCCC CAATAGTTCTTATTCAGTGCATATCCGATCTCTGCGTTATCTTCATTTTC TTGTACTCTTAAATCAATTGTCCCGATGAATTGTTGATTTTCTTTTAA |
| *Bacteroides nordii CL02T12C05* | 1791 | CACGGATATAGTATGTATTTCCCGGCTTCAAGCCACCGATTGTTCCATAA ATCGTTGTATCGGGCACTAATGAAATCTCTTTCAGATTGGCAATGGTAGG TTCTTGTACCTCCGAACTATAACAGAATCCTTTCTCTGTCACCAAAGTAC TATTG |
| *Bacteroides nordii CL02T12C05* | 1792 | ACATTGGTAGAGTCTTGCGGAGCACAGGAAGAAACAACGGGAAGATCAGT CGTCTTTGTTGTTACATAAGTTGTTGTACCATACCCACGCCCGTTGGAAT TGATAGCATACGCACGCACTGCGTAGAGCCTTCCCGGTTTCAAGTCATTG ATACGTAC |
| *Barnesiella intestinihominis YIT 11860* | 1793 | TTACAATATGATATGGAAGGAACTTATGTATTACCACGTTTTTCCCTAAC CTTATTTCAACATAACCATAACAACTTGAAGAATTCCTAAAACTACTGTT CCAAGAACAAATTTTTTCTATCCATTCTGTTTTA |
| *Barnesiella intestinihominis YIT 11860* | 1794 | CTTTCCCTTCGACAATTCTCCATAAATTCAATCGTGAAGAATTAGGAGAA TCAATATGATCTAATTTTTCTCCATTCCAATAATTCTTAATACTTATATC ATTTAAAATAAATTCTTTTAACTCTAAATTTTCTATAATAAG |
| *Barnesiella intestinihominis YIT 11860* | 1795 | CGACACCTTGATTCATTCCACGCGTGAGGAACGAGATGACAAATACAGCA ACGATAGCTAATATGCATATATAACCCGTCTTGCGCAAGCCC |
| *Barnesiella intestinihominis YIT 11860* | 1796 | CGAATGCGAAACTGCGAACCGATTCAGCTCCCAACAAGAGAATACACAAC AACACGATAATAGTACTTTGCCCCGTGTTGATCGTACGAGGTAACGTACT GTTC |
| *Lactobacillus murinus ASF361* | 1797 | ATCAAAGCAAAAAATATCACGATAAACACACTGTACCAAAGGGTGACTCT ATAAACGATACTCCCCTTATGCTTCTTTGATAACATAACCTAAACCTCGT TTTGTCTTGATCAAAGCTGGATCTTTTGTCTTTTTACGGATATTTTTG |
| *Lactobacillus murinus ASF361* | 1798 | TCCAAGCCTTCGTTTTGATCGGTGGGGCTTTGCTCATCATCTTTATCGGG GTCTTCTCGATCGATGGTGGCTTTGCGACTGTTGCCCATACCGCCGCTAG CAACCACAAGATCCTCTCCAGTGCTGACTTTAAGATCAATGATCTAGCAG CTTTTGT |
| *Lactobacillus murinus ASF361* | 1799 | ATTCATTATTTATCATATCGGACGGGTCGCGGTGGTAACTTACCTACCTG TATTAGCGGTCGCTTCAGTGACTGATATCGATCCTTTACTCGTTGCAGCC TGTGT |
| *Lactobacillus murinus ASF361* | 1800 | ATTGTTCTTTGTTCTTGCTCTTAGCTTAGGTAAATGGTTCGTTCCTAAGT TTTTAGCTGTTTTTAGTCGGTTAAATGCAAGTGAAAATGAAACGACCGCA GCTTTGGTCCTTTGTTTTGGTTTTGCTTTTTTAGCAGTCAGTCTGGGGAT GAG |
| *Eubacterium rectale CAG: 36* | 1801 | TGTACAGTGAAGCAGGTTCTTGATAAACTCTACAATCACTCCTGCTAACG GTCCGTATGCAAATGCTCCGATAAGTGC |
| *Eubacterium rectale CAG: 36* | 1802 | GGTGACAGTCTCTTGTATATAAGCACTGTGATAAGTGTTGATATAAGTCC CTTTACGATGGTAAA |
| *Cloacibacillus porcorum* | 1803 | AAGCGGCGGCCGCGTGGCGGCGCTGACGGCGGAGGAACCTTCGGCGGCCA GCGTCATTGACGCCGCCGGACTCTGCGTCTCTCCGGGTTTTATCGACACA CATATGCACGATGAAGAGGCCGAGGACGGTGACACGGTCGAACAGGCGCT TTTGCGGCAGGGG |
| *Cloacibacillus porcorum* | 1804 | GTGGAAATACTTCGTTCCCTTTTTCCTCCTCCTCTTCGCCGTGCAGATGG CCTTCATATTTGTCGCGGTAATGATCAATTATCATTAAAGATTACAGTAA TGGAAAAATTTGAACTTCTTATCCGGGGAGGAGAGGTCATCCTCCCCGGA |
| *Cloacibacillus porcorum* | 1805 | CGCGTACGGTTACGCCTTCACACCGACCATGATATCGGAGGCTTTGATTA TCGCGCAGACCTCTTTACCCTCTTTAAGTCCGAGGCGTCCTGTGCTGGCC TTGGTGATGATGGAGGCGATCTTCTCGCCGCCGGCGAGGGTCACGAGGAT CTCGCAGTTTACCGC |
| *Cloacibacillus porcorum* | 1806 | ATCACCGCATAGGCCTCGGCGCCCTCTTTGAGGCCAAGGTTTTCGCAGCT GGTCTCGGTGATGATGGAGGTGAGCATCGTACCGTCCGCGAGGCGAAGCG ATACTTCGTCGTTGACGGCTCCCTTTTTAACTGTGGCAACAGTAGCTTTT AGCT |
| *Blautia coccoides* | 1807 | AAAACTTTTTATAAAAGAACAAGTCCATGTAAGCAGGGACTTATACTCTC TTACATGGACTTGTGAAA |
| *Blautia coccoides* | 1808 | CACTACAGGTGCCATAGCCTGCACTGTATATACTGCAATCCTTTCCTGTT CTGGCCGCCGGAGGATTTATGTTGGTTA |
| *Ruminococcus bromii* | 1809 | GCGGTGATCCTGCTGTATTGATTGCATCAAGCGATAAGGCGAAAACTGTA CTTGGCTGGAAACCTGAGTATGATGATCTGGGAACAATTATTAAAACAGC GTGGAAATGGCATTCAACACATCCCAAC |
| *Ruminococcus bromii* | 1810 | TACGAAAAAGGAATCATTCCCGAAAATACAGTAACATATCGCGACTTATT TGATGTGAAACTTATGGCTTCACTTGTTAAGCGACCGTCAGAAGTAATAA GAGAATTTTGGCAGAATTATAGCTGTTCTCCTAAGCTTGCAACCGATAGC T |
| *Phascolarctobacterium faecium DSM 14760* | 1811 | ACAGGGATTAAAAATAAATGTGCGATTTTCAACAAAGCTTTTAACAAAGC CGCTGTGTGTTAAAACGCTTGATACAGGCTGGGAATGGAATTTGTCAGGG AAAGTAGTACATGGTAATCAACAAAGGTTATACAT |
| *Phascolarctobacterium faecium DSM 14760* | 1812 | ATAAGTATGCTGTTACTGTTGGCGTTGCCCATACCAGTATAGCCGCCGTA GATATCGCTGCTGACAGTGCTGCCGTTAATGATCTTTACTGTGTTGCTGC TGGCTGAACCTGAACCAAAGGTTCCTGTTGAGGAATACCCTCCCATGACC TGGGATGCTAT |
| *Phascolarctobacterium faecium DSM 14760* | 1813 | CGCCCGTATTTTCAAAACGAAGCGTATTGCCTGTTTTCGCATTGCCACTG CTGGAGTCGCCGCCATAGATGGTTCTGCCAGACAGATCTACTGCGCCTCT GATCGTGATGCTGTTGTTATTGGCGGTTCCATAACCAGTATGGCCGCCGT AG |
| *Gemmiger formicilis* | 1814 | ATTTGTTCCTCGGCAGGAACCTGTTTGAACGTTCATTAAGTAAAGAGAAT AGGAAACACTTTTTGTAAGCCA |
| *Gemmiger formicilis* | 1815 | CGAAAGTTTCGGGCGGTGGTTTCGAGCGTGGAGACAATCTCAGCGTAGGC GATGTTCCGCTCATTCGTAGGGGCTTCGTACTGGAAAATCACGAAGAAGC GACGGCTAACAGC |
| *Gemmiger formicilis* | 1816 | GGGCAGGGTATCCGGGTCGTCATAGTAGACAACACGGTACCGCTTGCTGA TGAACCGCAGATGCCGATGTCGGTTGGCTTCGAGATTGCCCTGCGGTGTA GGCGTAACGATACGGCGGCGCTTCATAAAGCGGAACCAGTGTGCCACA |
| *Helicobacter salomonis* | 1817 | TTTGATTTTCATTATTATTGCCATCATTGGTGTGCGTATGATGAGCACAG AGGGCGGGTTTGGGGATCGTTTTCTCTCAACTAGTACTAAAAATGTTAGC TATCACGAGCTTAAACAGTTGATCGAAAACAAAGAAGTGGACAATGTAAG CATTGG |
| *Helicobacter salomonis* | 1818 | ACACCCAAGAGAATGAAGTATATACAATTTATGCAAAAGATTTTACTCTT CTGCGCAGAGACCAAGAAAATGAATGGGTGTGCTTGACTCTGTGCAGGCA TTTTAACTCTTAAGGATATCTATGGACAATCGATCGCAAAATCCTAACCC CAA |
| *Helicobacter salomonis* | 1819 | ATCCAGCGAACAAGCCCAATGCACGCGCATGCAAACCAAGTACTTTGCTA CACTTGACAACCACTACAGCACCCTACAACATGCCTATACAATATTGTTG CAAGACATTGTCGCTGCTTGCCACACCCGCGCTAGCAAACAGGCCGTATT GCAGAGC |
| *Helicobacter salomonis* | 1820 | AGACATCCAGGGCACACCTTTAGCTAATGGTGTAGAAATCCAGCGTAGCG ATCTGCTCGCAGAAATGCAAGACTCTCAAGAAACCCAAGCCCCGCTCCCC CCTCCAACCCAGCAATCTTTCCATGCCCTCTTGAATAATTGTGCTAGGAA CGATCTTTTTAA |

| **TABLE 17B - SEQ ID NOS: 1821-1826** | | |
|---|---|---|
| *Gardnerella vaginalis* | 1821 | GCACTAGCTGAGCATGTAGGAATTTTCAGGACAACAGTTGCTGCATGCTT AGCAAGAATGATTGCGGGATTATGTATTATCGCAATTGTATCGGTATCCT TCTCAACC |
| *Gardnerella vaginalis* | 1822 | AGTAAATATTTATACATTCCAATTCTATTTACAGAAACTATCGCATTAGC AGGAATAATAATTTTTATTTATTTTATTTACAAGCATGCAGTTAGAACCG GAATTTATACTTCAGACGTCGCAGATGAAAATC |
| *Gardnerella vaginalis* | 1823 | GGATATCTGCGTTTAGCAGCTAGTGATTTTCTTTCTAACTATGCAACTAC ACTAGCCTCAACTACTATTCAATTATGGCTATTGAACTCACTTTTTATCG GTTTTCAGCAGTCAAGCCATTATTTATCGCTTTACCTCACCCTCACT |
| *Klebsiella pneumoniae* | 1824 | TACCAGAATATTAATACCATTATGGGCCAGGGTGTCTGCGATGTCTCCCA GTTCCCCAGGACGTTCCTGAGGTAACTTTCGTATCAGAGGACGGCATACA TTGCTGACCGTAAAGCCGGC |
| *Klebsiella pneumoniae* | 1825 | ACCCTTGACGAGTCAGAGAGGGCTGAGGCCACCATCGCCATAGCGGTTTC CAGCGCCTCCTCTGGCTCACTGTCTTGCTGCGGTTTCAGGAGATTCATTC AGAAAGTATGGCCCATTTTTTGGTCACTTCCGCTGCCCGGGCGTCATCAT CGGTAAGGAGAAT |
| *Klebsiella pneumoniae* | 1826 | CACCGTCTTCCACCAGAAAATGCGCATGTCCGGCATCAGGCGTGGTGAAT ACGCCGCCGCCTTCGAGCCCCACGCCGTTGTTACCCAGCGCCATACCCAT TGCGCCAAGCGAGCCCGGCGAGTTGCTGAGAATCACGTGAATGTCATACA TCTGACTGTGCTC |

| **TABLE 17C - SEQ ID NOS: 1827-1979** | | |
|---|---|---|
| *Escherichia coli* | 1827 | AAAAATGGCGGAAGTAACACCCGACACGATTCGTTATTACGAAAAGCAGC AGATGATGGAGCATGAAGTGCGTACCGAAGGTGGGTTTCGCCTGTATACC GAAAGCGATCTCCAGCGATTGAAATTTATCCGCCATGCCAGACAACTAGG TTTCAGTCTGGAGTC |
| *Escherichia coli* | 1828 | TACCTGTCAGGAGTCAAAAGGCATTGTGCAGGAAAGATTGCAGGAAGTCG AAGCACGGATAGCCGAGTTGCAGAGTATGCAGCGTTCCTTGCAACGCCTT AACGATGCCTGTTGTGGGACCGCCCATAGCAGTGTTTATTGCTCGATTCT TGAAGCTCTTG |
| *Bifidobacterium longum* | 1829 | GCCCCCGACCGTATGAATGCGACGGCCTATGCCCCCACCGAGTCCAAGCG CCGCAAGCCGGCGGGCCCAATCGTGGTCTTGAGCATACTGGGCCTTACCT TCCTCTCCTTTGCCGGTCTGATGGGACTGATTTGGGTAAACGACATGGGC ATTATCGGCATTATG |
| *Bifidobacterium longum* | 1830 | CTCCACCATTCTCGCTCCTTTCGTTTGCATAGCGCAGCAGGCGGAATACT CGGGCATAATCGATTCTCCATTGAACGCAGTCGGCGGCTATCTCTTCCTT GGTTATTCCCTTGGCAAGGGCCGAGTCAAAGATCGGTAACGCAAACCGAA AATCAAGGTCTAT |
| *Clostridioides difficile QCD-66c26* | 1831 | GAGAAGGATAATATTTCTTCCATGAAATTTTTGAGGAATAATAATGTAAA CGATAGATTAATATATACGAATGACATTGTAGAAGTAGTGATAGATTCTG CAAAGCAAGAAGTGTTAAATAAAAAAATACTTAATGGTACTAA |
| *Clostridioides difficile QCD-66c26* | 1832 | CTGAACTTGATACGTTATTAAGTGATAAAGAATACGAGGCTGGATTAGAA TAATTAAAGATTAAGATATTATTATAATTGGGGAAAGTATAGTAAAATTT GAGTGCACGCAAATTTAGCTATACTTTCCTTTTATCATATAT |
| *Clostridioides difficile QCD-66c26* | 1833 | ATAATAAGTATAATGAAAGAGAAACTTTTGAAAGAATACATTCTAAAATG CTATGGGCGTACATATCCTGAAGATAAAAATAATATATACATATATTCAC TGAACATATTTGCTAAAAAAGAGATTTTTATGA |
| *Lactococcus lactis subsp. lactis Il1403* | 1834 | CCAGCTGATAAAAGTTGGAGCCATTTTAAATTTTTATATTTTGCTAATAA ATCAGGCTTTGGCAGACCAACTAATACTTCTGTTTCCAGAAAATCTGATT CTGTCAGCTCTTCTTCTTTTTTAAATTGAAACTGGTAAGCTTTATTTTTC TT |
| *Lactococcus lactis subsp. lactis Il1403* | 1835 | TTTTTAACAGTTTTCTTTTTACAAATTGACAAAATAAGAGTAAAATTAAA ATATAGATAAAAACTTATGAAGGAGTCGCCTATGTTAGATAACTATAAGA AAATCCTTGTTGGTATTGATGGTTCGGTAGAAGCTACTAAAGCTTT |
| *Lactococcus lactis subsp. lactis Il1403* | 1836 | ACTCCTTTGTATAAAATGAATTTTAAAGACTACTTAAAGAGAATAAAGTC AAGCCTTGTTTTCTTTATCTTCTTTCTTTGATTATAGCACTTTGCCTTAA TTTTATAAAAAATAGAAGTTTGACAATTCTGAAATTATCA |
| *Lactococcus lactis subsp. lactis Il1403* | 1837 | TCATAAGACTCTTGACAGCCATTTTTCACCCAATTTCCTAAAACATAAAA TAAACCAGCAGCCATAAAATCGTTCCAAAATTTTTGCTTTGTCCCTAGAT AATCAGCCCAAGTCGTTGTTTCATTATAAAAAATAGTCATCTTTTG |
| *Chlamydia pneumoniae TW-183* | 1838 | CCTCGATTCATCCATGCATACTCAGGATATCTATGTTCTAGGGCTCTCTC CGACTGTCTATATTAGAGGGAACTATCACGTACAGCACTACCGTGTTCGA GGATTTTGGCCCTCTTGCCTGGATTCTCTAGCGGCCTGTGCGGAAAATAC ATCAGTACTTCCCT |
| *Chlamydia pneumoniae TW-183* | 1839 | TCTCTATTCAGCCACACATTTGATAACGCGATACGGTATGGTGAGAGATG CCTGTTGGTTTGTTCTGAGGGCATGGGAATGCTTCCAGAAACGCAACAAC AAACATCTCCTTTAACTTCACTAGAAGGGGGACATGAGGTAGCTCTAGTT CTCAATCCC |
| *Fusobacterium nucleatum subsp. nucleatum ATCC 25586* | 1840 | AATAATTTTAATAATGATTTATCAAAAATAAATTTAAAAGGTGATGTAAG TATTGAAATTTTAAGTTTACAAAACCATTTTAATGAAATGATAGATAAGA TTAAATATTTAAGAGAATATGAAATTA |
| *Fusobacterium nucleatum subsp. nucleatum ATCC 25586* | 1841 | GAAATTTCATATATGCAAGAAATTGACAGCTTAAAAAATCATTTTTTTGA AATGATAGTTATAAGTTGTTTGGCTTCTCTTTTAATTACAGTTTTAATAA AT |
| *Porphyromonas gingivalis W83* | 1842 | TTAATCGATAAAAATCCACGCCTTTAGCACTGTCCTGTAGGTGCCTTTTT CCGTATCGGTATCTCAATGGACATCCCTAATGCTTTTGGGCGTTTTTTTG TCTTTTAGGCT |
| *Porphyromonas gingivalis W83* | 1843 | AAAGGATCTATTCGGTGGATGAGTCTTCGGGAGAGATCGAACACGAAAGA CGATTCTTTTTCAATGAAGGCGGATATATGATTCGTGAGGAGGAATACGA TGGAACCGTTCAGATACCTGTCAGAAAATGGGAATTTGTCCGCGATGACA AGG |
| *Helicobacter hepaticus ATCC 51449* | 1844 | AGCTAAGCAATTTAGTCAAGCTTTTACCAGATAGGGGTTGAGCAGGGATA AGAATCGTATCTGCGCCAAAAAGAGCAGATTCTAAAATCTGATATTCTTC TAAAAAAATATCGCTATGGATAATCGGAAGTGTGCTATGACGGCGCAAAA G |
| *Helicobacter hepaticus ATCC 51449* | 1845 | CCAGATAGGGGTTGAGCAGGGATAAGAATCGTATCTGCGCCAAAAAGAGC AGATTCTAAAATCTGATATTCTTCTAAAAAAATATCGCTATGGATAATCG GAAGTGTGCTATGACGGCGCAAAAGAGCGATGGATTCTAA |
| *Lactobacillus johnsonii NCC 533* | 1846 | AATCTTCAATTACTGTAGGTATATCATTTATAAACTCAATGACATTATCA CCAAATTCATCAAAAAGTTTTTCATTTATGTTATCAATTATAACGGCAGC CATTAAATGATGCTCTTTAAGATATTTGTTTAGGTCTTTTC |
| *Cutibacterium acnes KPA171202* | 1847 | GACGACGGCATCAGCGTCATGGGGGTTCAGCAAAATGTCGGATCCGGCTT CGAAAGCCTGCACCGCAACACGCCCCCGGATCGGTTTCATAAAGGCGTTG AACTCGGCACTCGCATGCCCCTCATCGGCCGCCGTGGGGTCATTGCCCTC GGCGTCGGCAGC |
| *Cutibacterium acnes KPA171202* | 1848 | CAGGCTTCTCGCGGCGGCGACGGGATCGTCGCTGGTGTCGCGAATCCACC GGATTGTCGACCGGCTCAGGTGCCGTAACAGATCGGCCGGTGAATTTGGT CCGGACAAGGTCCGGCTCGTGTATCCCCAGTATGGACGGCCCCGGCCTGC TGCTGGGAGTTTC |
| *Cutibacterium acnes KPA171202* | 1849 | GACTGCGAGGTCTACTTGCTCGTGGTGAGCTGCCAGGACAGTGGTGGTGG CGCTGGCGCAGAACACGTCGGGAGTGCCTTCTCCCACGTCCAAGCGGCTC GACAAATCGAGGGTGACGAAGTGATGATCGCTGGTGTGCATGGGGCGGTG CGTGCTCAGGCC |
| *Cutibacterium acnes KPA171202* | 1850 | GGACATCGCTGAGCTGGTAGACCGCTCCGCGGGCGGGGTGGGCGGTGGGG TCACCGACGCCGACCAGACCGCCTCCGGCTGCGACAAATCTGCGCACTGC CGAGGTGACACGCTCGTCGGCCCATTCGGATCCACCACTGAACGCAGTGC CGGCGGCACCGA |
| *Chlamydia trachomatis D*/*UW-3*/*CX* | 1851 | GAATTATTAAAAACCTATCGAGAAGGAGTTTTTTCAGCTTGGCTCTTACT CACCTATGGGAATCGGCAGACACCTTATAATTTTCTTGTTTATTACGAGC TATTCTCAGCTCTTCCAGACACTCTTAAACTCGAGTTAGAAAGACTGCCT C |
| *Campylobacter jejuni subsp. jejuni NCTC 11168 ATCC 700819* | 1852 | AAATATTTTAAATTTTCAAAATGATTTTAAAAATGTTAAACTTGTAAAAC TTGAACAAAACTATCGTTCAGTAGGGACTATTTTACAAGCAGCAAATAAT CTCATATCTCACAATGAGCAACGACTTGGAAAAACTTTAATC |
| *Campylobacter jejuni subsp. jejuni NCTC 11168 ATCC 700819* | 1853 | CTTAGAAAATGTAAGTTTTCACTTGAACTTAAAGTTAAATTTTCATCAAT TTTTACAAAAGGTGCTTTAACATTATCATTCATAAAAGCAAAGTTAACCC CTTCTATATTCTTAACTTCACCTTTTTCAAATTTAACATCAAC |
| *Campylobacter jejuni subsp. jejuni NCTC 11168 ATCC 700819* | 1854 | ATATATCGCTCAAGAAGTGAAAAAATTGCTAAATTCTGGAGTAGAAGCTA AAGAGATCGCCATTTTATTTCGAGTTAATGCACTATCAAGAGCAATAGAA GAAGCATTTATGAAGAAACAAATTTCTTATAAACT |
| *Campylobacter jejuni subsp. jejuni NCTC 11168 ATCC 700819* | 1855 | AATTATTATTTTCTTTATAAGTATAATGAGCATTTAAGATTAAATCTTTA TATTTTAATACAGCTTGATCATCACCTAAATTAAGTTTTAGTTTAAAAGA ATTAGCAAAAGGCAAATTTCCTATATAACGATCATTAACCGC |
| *Campylobacter jejuni subsp. jejuni NCTC 11168 ATCC 700819* | 1856 | TTTGTTCTAAAATTGGTTTTATAAATTCTTCTAAAGAATCCATTTTTCCA GTATCATAGAACAATTCTTTTAGCCTAACAGTGCCAATATCAAGAGATAT GCAAGAAATAATTCTATTATTTTTTATTAAACACAATTCACTAGA |
| *Bacteroides fragilis YCH46* | 1857 | TCTCACTCAAATTCCGCTCAAAACTGTTCGACAAAGCATTCTCATACTTA CCATCCCAATCATAAATGAAAAGATCCAACTGTAATCCACCGAACTGCAT CGGCTCGGCCGGAGTCTTTTTATCCGGAACATACCGGCTGTGCCGATCTC TCAACCGCGCCT |
| *Bacteroides fragilis YCH46* | 1858 | ACCGGTAAAAGCCAAACAGGTACGTCTTCGTATCCTCGACGGATTTGCCT GTCCTGCTATCCATACATTCGGAGTGTACAAACAATCGGCTTTATTTCAG TAAAAACAATAGGTAGTTGGGTTGAATATAGGTTTGGGCTGTATCACACA GGC |
| *Bacteroides fragilis YCH46* | 1859 | GCCTTTCCTCTTCCGGGGGAAACGTTCTATAATCTCCATAACAACGGGTC AGATAAGCATCGTATCCTGACGGAACCGGAAACTCCACTCCTTCAAATCG TGCAGTATCCAGATATTCC |
| *Lactobacillus reuteri JCM 1112* | 1860 | TAATCTTTAATATTGGATTTTATTGGCCAGCTTATCCTATTGCCTTATGG ATGATTCTAGCTCTGTTATTAATTGCTTTACAAATTATTCACAATCATGA ATTCATTTA |
| *Lactobacillus reuteri JCM 1112* | 1861 | TTAATTGGTAACTTGGATAACTTGATTGTAAAATAAGACGTTTTAATTGT ATTGCTGCTAACTTGTGGTATTATAGATACTAGTTAAATGTAAAAATAGG TGGAGGTCGCATTCCGTTAGGTTGCGACCTTTCATTTCGTTGCTGTTCGC TTACT |
| *Lactobacillus reuteri JCM 1112* | 1862 | TAACCACTTTACAAATTTAACTAGTAATAGTTCAAAAGTTGTTAAACCAA AATATGTTGAAAAGAAAAATGTAAAGCTTGTTGCACTAGGTGATTCCCTT ACTCACGGTCAAGGGGATGAAACTAATAAT |
| *Lactobacillus reuteri JCM 1112* | 1863 | ATTCGGCATATTATGCTTTAACTATATATATTGGTAGCTTAATTATTTCA CCATGGTTACCGTTGATATAAAAATATTAAGTGAATTGAGGCTGGGAGAA AA |
| *Bifidobacterium adolescentis ATCC 15703* | 1864 | ATCAAGCACCACATGCGCACCCCGCGCACACTCGACCGCAGCCTGCAGCC GCTCACCAGCCTCACCCGCGGAAAAACCAGACGTGCACCCAG |
| *Lactobacillus rhamnosus GG* | 1865 | CACTGGGGTCATCACCTCAACTATTGGTATTTGCGTAAAAAGATTGTAAC GACCCAATCTTTTGCGCAAGCGCCATCATTTTTGTGATAGTCTTAAATCA TCATGAAACCTTTCTGAAAGGAAGTCTCACAATTGAAAATGATCAATCTC GGGCGCTCCGGCTTA |
| *Lactobacillus rhamnosus GG* | 1866 | GGGGAGGCGTGCACATGCGTGATTTGGTCAAACTTGTTGGCTTGGACCTG GCTGTATTTCATACTAATAGTAAGAAGTATTTTTGGTTTGGTACTTTGAT CAGTATCGTTTTGTTGCTTCTCCCTTTCTTGAACGGTGACTTAGCAACCC CGCTTGC |
| *Lactobacillus rhamnosus GG* | 1867 | CATGCGCCTACCGAATCTTGATCGTCCGCGCGCAACGGAATTGCTCGATG CTGCTTACGATGTCGGAATTAACTTCTTTGATAACGCCGATATTTACAGT AATGGTAAGGCCGAACAGTTATTCAGTGACGCACTGAAAAAAGCGAGCTT CACCC |
| *Lactobacillus rhamnosus GG* | 1868 | TTCGTTGAGGTTGACAGTTCCGGTACCGTCAATTCTTACCGGGATGTTTC CAGTTGCACCGGTAATGGATACCGTTGCATTCTTATCAACGGTTAGATTA CCACCGTCTTCAATATAGATTGGTGCAAAGCCATCTTTCACAAGTGCATT |
| *Bacteroides thetaiotaomicron VPI-*5482 | 1869 | ACAATCGTTGGGAGTCAACTTCGCTAAGGATACTAAAAAGTTGCAGATCG GGGGGAATGTACAATATGGACATTCTGATAATGACGCTCGTCGCAAAACC TCTTCAGAAACATTTTTGGGGGAAACATCTTCTTTTGCTC |
| *Bacteroides thetaiotaomicron VPI-*5482 | 1870 | GATCGGTATGAAGGGGGAGTAATGATCAGCCGCTTTAAAGATGATGCGAG TCTTTCAATTATAGGTTCTGCAAATAATACTAATAATAAGGGATTTTCTG AATTTGGTGATGC |
| *Bacteroides thetaiotaomicron VPI-*5482 | 1871 | AGTATCTGATGCGGTAAAAGCAATTCCCCTATATCGCATGGCTGAGAAAG GATTAAGAGAGGATGGGTATCCGATGGGACCG |
| *Lactobacillus acidophilus NCFM* | 1872 | GGGTTCAATTCCCCTCATCTCCATAGATAAAAATAGAACCGCTCTAGTAA GTTGTCTAGAGCGGTTTTTTTGAT |
| *Lactobacillus acidophilus NCFM* | 1873 | TGGTATTACTTTTGGAAATGGTGAATCTCCTTTAAAAGAAACAAAGAATA TAAATTTAAAAAATTCAATTTTCAA |
| *Desulfovibrio alaskensis G20* | 1874 | GTGAACAGCAGAATATGGGCTATGCCGTCCAGCGACATTTCTCCGTACTG CAGAAACCTGCTCATTTCCGGCTCACCGTTCAGAACGCCGAAGGTGCGGT TCATGCTGCGGCGTACCGATACAATCTGGTCGTG |
| *Desulfovibrio alaskensis G20* | 1875 | GCTGATTCCAGTATGCGCGCCGATGTGCGGGCGCCGTCCAGATTGATGCC GGTTTTTTGCGGACGCCGCGCCAGCGCGGCCTGCATCAGCTCTGCCAGCT TGTGCGGTTCCAGATCGTCGGGCTGCAGGATGCGCAGGGCGCCCAGACGT TCGAGCCGCAGG |
| *Bacteroides vulgatus ATCC 8482* | 1876 | TCCGTCTGATTCTAGCCTTGCACAACTATGATATCACTATACATGAACAA GACAAACAGACAGCATTGCAACAAATAAATGAAGTGTGCAATGACTTTCA GACCATGAGAAAACAATTGGAAGAGACCTATTCACAGACCCGTTTTATGG AACAA |
| *Bacteroides vulgatus ATCC 8482* | 1877 | TTCCGCCTCCGCACGGGTAAGGGGCAGCGTATTGTCGGGTCGGGTGAAAG CCACCAGGATTTTCACCGAATGTCCGCTGGCGCCCATAAAGGCGCACCGC GTCTGCGGCAACTTCCATGCCTCCCGTTTCACCTGCTCCACTTCCGCCCT GCCCGCCAAGGG |
| *Bacteroides vulgatus ATCC 8482* | 1878 | TGATGGTTCCCCACACTCTGAAACGGTGTGGCGTGGATACATCGCACAGG GAGAATATGGCTGGAACCCAACAGCACGGACAGTCGAAGCCTTCAAAGCC GCACATGCCCAACGAGAATTTGGCTTTCATCCAAATGACAACCATATGGC TTTTCT |
| *Parabacteroides distasonis ATCC 8503* | 1879 | GGTTAGTTATCCCAATATCCCGATCATCCGGAAATATCCGAATATGACGG GCTTTTATGATAAACCGGATTATAAGAGGAATATAGAATTGATACGGCGG CTTGTTGGTAATTGCATTGTCATAAGAGTCTCGGACGATACCTTTCAGGA TAATATGATGCTGG |
| *Parabacteroides distasonis ATCC 8503* | 1880 | AAAGATAAGGGTTCCCCATGAACCTCTTATCATCCGTTCGGATCATTTCC GGTTGACGCAGGTTTGTACCAATTTCATTAATAATGCGGTGAAGTTTACC GCTAAGGGATATATCGAGATCGGATACGAACTTAGCGCAGATGGAAAATC GATCCTTATTT |
| *Parabacteroides distasonis ATCC 8503* | 1881 | ATTACCAAAAACAGTGACCTGTTGCTTAAGCTGATCAACGATATCTTGGA TCTATCACGCATAGAATCCGGTAGCATGTCTTTCTCTTATGAGAACCTCG ATCTGAGTAAACTGATGGGAGATATCTTCCATACGCAT |
| *Parabacteroides distasonis ATCC 8503* | 1882 | CGTGAACAATATATATTCCTTGGATCGGGTACGTTTATCCGGAAAGAACG GGATTTCGATATCGGATATACCTAAGATAAAGCCGGATACGATGTATATA AGCACACTCAGTACGAAATCGGCGAATGCCCTGATTAAGGGATTT |
| *Lactobacillus delbrueckii subsp. bulgaricus ATCC BAA-365* | 1883 | GCAGGAGATTCCGGCGGTTGAACATGGGGAAAATGGGCATTTTCCAGTTA AAGACACGGACAAACGCAAGCTTTTTAAAGGCAAGATCAATTACCGGCAA GCCCAAGTGGACTTAATCGACCGCTTGCACGACTTTGTCGAAGATGCCGG GCAAAGGGTC |
| *Lactobacillus delbrueckii subsp. bulgaricus ATCC BAA-365* | 1884 | TTTGCTCAAGCAGGATCCGGTTTACCGGGAATTTATCACTTCTCTGGCCA GCCGCTACCAAAACCGCCCAAGCGAACTGCCCTTGATCTTGGCTGAAGGA AATTTCGCTTTTGGCCAGCTTTATCCCTGCCAGGGAGATTACGTGACTAA TCCCGATGCTTT |
| *Lactobacillus delbrueckii subsp. bulgaricus ATCC BAA-365* | 1885 | TAGCCCAGTACCTGCGCCAGGAGAGCCAGGACCGGGACTTCCGCCCGGCC AGCTACCGGATGACGGAAGACTTGTCTAATTGTGAAGAGGTCATCTTGGA CCTGCTCCGTGATGAAGACGGCAACCTCTGTTTTGTCGGAGCGACTGGGT CTTTGGAACC |
| *Campylobacter curvus* 525.92 | 1886 | GACCGATGTAGGCGGTATAGTAGGCTTGTAAAAATGTGCCTAAATTCTTA GCGACATAGACTATCACGATGCCAAGGGGCAGCAGATAAAGCCATGTTTT TTCTTTTTCGATGAAAATTTCATTTAGCACGGGCTTTACCATATATGCAG TCGCCGCCGTGCCT |
| *Campylobacter hominis ATCC BAA-381* | 1887 | GTTCCTGTAATTACAACAGTTTTTTTTGTGAAAACATTTTGTGAAATTTC AGTTTTTTGGGCGCTTAAATGCAAAAAAGAAAGTAAATTTTCAATTTTTT CGCGATTTATCTCACAAAAACCGACAAAACTT |
| *Campylobacter hominis ATCC BAA-381* | 1888 | ATTTTTAAAATTTCATCAAAACTTGCATTCAGCCAATTTTCGCCAAAAAT TTCAGCGATTTTTCTGGCGGCCACTTCGCCTATATGTTCGATTCCAAGTG CTGTAATAAACCTATAAAGT |
| *Campylobacter concisus 13826* | 1889 | ATAAGTGCGGGTGCTAGCACACCTGACTGGATCATACAAAAAGTCGTTGA CAGAATCAAAAAAGTATAA |
| *Akkermansia muciniphila ATCC BAA-835* | 1890 | AGGGCTTCCTTGTACTTGCCTTCGCGGTACAGGTTGCGGCCTTCCGCAAG GAGCTGCATGGCTTCCTGGGTTTGCGCTTCGCGGCGGGCCATGGCTGTGC GC |
| *Akkermansia muciniphila ATCC BAA-835* | 1891 | ATTTGTTTGGCGTCTCCCGGAATCCGGAAGTCGGAACCTTCCTCTACTTC AAAGCTGGTATCCCGTTCGGGGCCG |
| *Atopobium parvulum DSM 20469* | 1892 | CTTTTCATACGAGAAAATATTATCAACTGATTGCTCCCCTATATATTCCG CAGCTTTATGTTTTAAAAAATCAAGATTTCGCTCTTTAACCGCTTCGGGT CTAAACCAACTGCATATGAATACAGAGGTTATAAG |
| *Atopobium parvulum DSM 20469* | 1893 | TGAATATAAAGCGGCATCTTGCTCGATTGTTCCAATGATTTCTCCATTAA CACTTACAACCCACAAAATACCACCGTCTAATAATTCATTGTTTTTCTCT ACAATTGATAAAATTGGACCAGATATATACAC |
| *Veillonella parvula DSM 2008* | 1894 | AGCTATGGAACGCTCTTTGCGTGCTCTAAAAGGTTTTGTACACATGGCTG ATGCAATGGAGGCAAATACGATTAAAGCTGTTGCTACCGCAGCAGTACGT TT |
| *Veillonella parvula DSM 2008* | 1895 | AATGGTACTAGCGTGTACTATTGCACCATGGCCAATTGTTACGTAATCTC CAAGAATGCAAGCCCTGTCGTCATCAA |
| *Veillonella parvula DSM 2008* | 1896 | TATCCGCTTTTAATGTATATGTAATTATACGATTTTGGCGTTCTTATCGT ATTCCTCGCCACCCGCATCAGGAAGCA |
| *Citrobacter rodentium ICC168* | 1897 | ATGAATAAAATTTATTTCTCTCAAGACCCGGTGGGTTTTTATATCGAAGG TGTGTCTGCGGTTCCCTCCAATGCTATTGAAGTTAGCGCGGATATTTATA ATGAGTTTGCCGGAGTGGCGTGGCCTGATGGGAAAGTACTAGGTGCTGAT GATTCAGGAT |
| *Citrobacter rodentium ICC168* | 1898 | GCCATGATGAATTGATTGCACAAGCGGAAGCCGAAAAACAACGGTTGATT GATGAGACCAACGTCTGGATAAACGGGCAGCAATGGCCGTCTAAATTAGC GCTGGGCCGCCTCTCTGAGGATGAAAAAGCGCAGTTTAACGAATGGCTGG ACTATCTGGACGCG |
| *Streptococcus gallolyticus UCN34* | 1899 | CGATATTGATGATACTTCTTTTTAAAGTTGCCATTTTGATTTCCTCCTTC TAGTGATTAATAG |
| *Streptococcus gallolyticus UCN34* | 1900 | GGTTCATAATAGGCACAAAGCGGCTGCCACTTGTAAAGTCAGAAATAAAC GATAATGGATTAAAAGAATAGCCTTGGATACCGATATTTTTCAGCAAAAT CACATAAATCAAAA |
| *Enterococcus faecium TX0133a04* | 1901 | CGATCGAACCTTTTATCATCTTCATTCCTCCAATATTCTTGTCCATTCAT GAACTGCTGGGCCAGCCATTTGTATCGCATCCTCACCAGAGATCATCAAG TCTCCTTGTTCCAAGTGAACAACTACCTGATCTGCTGAAATTTTCCCAGT TTCCTTAGCAA |
| *Enterococcus faecium TX0133a04* | 1902 | GGGAATCAGCACTGGAAACAATTTTTTCTGTTTTTGTTTCCGTTACTTTT TCCATTCCAAAGTGATTGGACAAATTAGCGACGATCAAACTTAGTGAAGC GACGAAGATTAGTCCGAAAATCAAAGATAAAAAGGTTTGCCATGTT |
| *Enterococcus faecium TX0133a04* | 1903 | GAAAATATCTCTGCTAATATATTGGTTTTCTTTTGATAAAATATAGTGAA TCGAAACGGCGCTTGGAAAAACATTCGTGCGGATGGATAATTGACCGCTT CTCTAACCGTACCGAACAAAAGATAATCTTTTAAGGCTTCAACTGCCAAA CGTCCGCTG |
| *Peptostreptococcus stomatis DSM 17678* | 1904 | TTTAGTAGTTAACCTAAACCTGGTCATCTACCATCTGTACGTACTCTAAC ACATCAAATCCTAGTCTTTTATAAAGGTCTATAGCCCTCTTATTAGTTCC ACAAACTTCTAACCTATACCTTACTGCTTCAGGAAAGTTTTCCT |
| *Peptostreptococcus stomatis DSM 17678* | 1905 | GTATTCTTCACTTATATAAAGGTCCTCTAGCTGTATGGTGATTCCTGCCA CCTCTGAAGCATAATAGCTGGTTACTATACCAAACCCAACCAAATTTCCA TCATACCTTGCTTCATATCCCCTTATAGAATGCTCTCTTGATAAGAT |
| *Mycoplasma fermentans JER* | 1906 | TTTGTTGAACCTTCAAAAAGAAATAACTTCACAAAAAATGTTTTTTCTTA CATTACAAGAGATAGATTTGATTTTGGTGAAGCTTTTAATAATAACTATG ATTTCTTTTCAACTATTTTTGAATACCTAATTTCAGATTAC |
| *Mycoplasma fermentans JER* | 1907 | AGATGTTGCATTCAAATATGAAGATACAATTGATTATTTAGTTGGATACA TTAATGAAGATGATTTTTATCAAAAATTTGACGATACATTAATTAGAATT TCAGATTATAAACAAAATGAAGCTTTTAATGTTGATACT |
| *Eubacterium limosum KIST612* | 1908 | ACCGTCGAGGAGGTCACGCAGGCCCTGGCGCAGCTCCTCGGCGTAGGCAG ACTGGACCGGCAGGGTCTCCAGGGCCTGGTACAAAGACTGGGCAAGGGCG GAATCCATCATGATAAAGCTTGCGTTTTTTTTCATCTGGGTTCCTCCTTT CTAGAT |
| *Eubacterium limosum KIST612* | 1909 | AAGGCAGAGGTATCAACACCCGGAGGGCAGTCTGCCGCCTGGGGATACGG CGGGCACGCCGCCTGCCCGCAGGCCTGCTTTTCTGATCGTTTTGTCGGTT CCGCTGTTACTGCAACTGTCCAAG |
| *Parabacteroides merdae ATCC 43184* | 1910 | AGATGCAGAGGGGAGAAAAGAATTGGAAGAATAATGTTTTAAAGGCATTC TCGTCGTCGTTTACAGCGATGTTCCAAAACAACTTGTCGATTGTATGTGG CAGTGTCTCCTTCATGTTTTCGTTTGATGACAACAAATATTTACTAAGTT CTCATA |
| *Parabacteroides merdae ATCC 43184* | 1911 | GTGTAATTTTAGAAACAAGGCCATAAGCGCTTGACAATTCCACCTGTTCG ATCGGCTGATTTTCCAATCTGTAGATACGCTGGTAAAAGTAGATACTGCT AATTAAGGCTGGAATGAGCAATATGGCTGCCGTGTAACGAAAATAGCGAC CGATCTTTTGT |
| *Faecalibacterium prausnitzii M21*/*2* | 1912 | TCGGTGCAGATCTTGTTCCGGGTCTCTGTATCCAGCGTTTCTCCGTTGGA AAGCAGATTGCTGGCATTGCCGGAAATGGAGGTCAGCGGGGTGCGCAGGT CATGGGAGATCGTGCGCAGCAGGTTTGCCCGCAG |
| *Faecalibacterium prausnitzii M21*/*2* | 1913 | GCCCACGGGGGTGTATTTGATGGCGTTATCCACCAGATTGACAACGACCT GCATGATCAGCCGTGCATCCACATTGACCAGGAGGATCTCGTCCCCATAC TGTGTTGTGATGGTGTGTTCGCAGCTTTTCCGGTTGACATGATGCAGTGC TTC |
| *Faecalibacterium prausnitzii M21*/*2* | 1914 | GTCTTATCCATCTTGTCGGACACGACAACGCCGACGCGGGTCTTTCTCAG ATTACGTTCTTCCATCGTTTAACCTCCTTACTGCTTCTCAGCCAGAACGG TCATCACGCGGGCGATGTCCTTCTTGACTGCTTCGATGCGGCCGGGGTTC TCCAGCTGGTTGA |
| *Faecalibacterium prausnitzii M21*/*2* | 1915 | AGAATCCTCTTTTTCAATGCCCAAAAGCGTGGTAGAATAGGGAAAAGATT CAGTATCTGAAAAACGGAGCGCGCAACAATGAAGATTCTGGTCAGCGCCT GCCTGCTGGGCGAAAACTGCAAATACAGCGGCGGAAACAATTACAATCAG GCGGTCTGT |
| *Parvimonas micra ATCC 33270* | 1916 | ATATGCCCTAACAAGTCCTCCCGCTCCTAGTAAGATTCCACCGAAATATC TAGTGGAAATCACCAAGCAATTAGTTAAGTCCTTTTTTCTTAAAACTTCA AGCATTGGAATTCCGGCAGTTCCTGAAGG |
| *Parvimonas micra ATCC 33270* | 1917 | ATTAATTTTTCTCTATCATTTATAAAATTTTTCATAAATTCAATCTTTTT ATTTTCCATAATTATCCCCAAAATATTATTATAAAATCGCATTGATTATA TCATATATATTAGTTAGAATCAATTTTTGAATCTTTTTTAATTTCATAAA |
| *Bi fidobacterium bifidum NCIMB 41171* | 1918 | ATTGGCCGCGTACTTGCGGTTCAGGCGGTGGCATACGTGGTGCTATTCGG CGTGACGGGCGTGCTGGAGTTCACGTTCGGGAACCAGGGCGGTGTGGCGC AAGGGGTTGCGATCGCGCTCGCCGTGGTGCTGCTCGTGCTATTCCAGGTT GGGTGGCCGTTCC |
| *Bifidobacterium bifidum NCIMB 41171* | 1919 | TGCCAATTGCCGGCGTGCGTTGCGTGTGATTCAGGAGGGGACGGATTCGT CGATGGAAACGCGCACCCGACTCGTTCCCCTCAAATACGGGATAGACGCG CCCTGCGTCAATTACAAGATTGGAGTGAAGCGTCTGAACCGG |
| *Collinsella stercoris DSM 13279* | 1920 | TAGGGACTGTCCCTAAATTAAAGTTCTAAATTGAGGTTGACAGCCCTAAT CCTCTTCGATGCCTAAAAACACCTGTGTGCGAGGGTAGTAAAGCACCGTC GACCCAGCCTCGGCGCTGGTGAGCTCGGAAAGGACTCGCGGCCAATCGCC GCGCTTGACCGA |
| *Roseburia intestinalis L1-82* | 1921 | GATCTCCCCCTCCACATCAAGAAGCACACCGCGGGACGCATTCTGCCTTA ACTGTACCAGATAATCCGCATACTCCATGACGAATCTGCGGAAATGTGAA AGGCTTCCTCGGATCATATGTACCTGAAACGCATTTTCGCAGATATACTG TAAC |
| *Roseburia intestinalis L1-82* | 1922 | CCATCGCAAGGGAAACCAGACGGTACAGGAGATAATTCATGTTCATCTGT ACCAATTCCGGGTTCATTCCCGCCTCATTCATCTCCTCATAGATCGCTGC AACATTATCCTCGATCCTTTTCTTGTCATTCTCTTCCACACTC |
| *Enterococcus gallinarum EG2* | 1923 | TGTATCAAGAGTGGTCAGGTCGACAGGTTCAACCTTCTTTACTGCATGGT GACTTTTGGCGCGGCAATGTGCTGTTTG |
| *Enterococcus gallinarum EG2* | 1924 | ATTGCTTGCCCAGTTTGGGTTATCCGGAACATTAACGCCGATCAGCGGGG GTGACGTGAATCAGACGTTCCGGTTG |
| *Enterococcus gallinarum EG2* | 1925 | GGCGTCTTGTTTGCATGTATGAGGTATGGTTCTAAAAAAGCAAGAAATGG GGCGACTGCTGCTGCATTTGTGCAAGCA |
| *Prevotella copri DSM 18205* | 1926 | TATAATCATACTATAGGCATTAGAGCATCGACGACTTTCGGTATAGGCAA TTGGATGAACGGAAGTGTTTCGGCAACAGGAATCTACAGACATGACAAGA GTAACGATTTCTTTGACTTACCCTTCAATCGCAAACATATCTCTGCCATT CT |
| *Prevotella copri DSM 18205* | 1927 | GAAGCCATCACATCCATTTCATACTTAACCCATTCTATCAGTCGAAAGCC ATTCAAGGACTCTATGACATCAAATCAGTCTTTCTCTTGATTGCTATGTT GAGATGGGCTTCCGATAATGATAAATGGAGCATTGTGGTTAAAGGCAGCA ACATCT |
| *Prevotella copri DSM 18205* | 1928 | GTTCTTCTGCTCAATATTGTGCTGTATTCACCTCGTCTGCAGTATTTCTT GCTTTGGCTGCCTTCAACTATTGGGCATCCTACAAGCTCTTCACCCGTAT GCAGGTTATCTGCAACAAGTGGATCAACATCTAAGAATTAAAATTT |
| *Prevotella copri DSM 18205* | 1929 | CAGTCTACTCCTTCCTGATATTTGCCCATTCCTTCGCAACCCTGGGAGGA GCCTTCTACCGTAAGTTTCCTGTGCTTCTGACAGCATGTACGGGATTGGC ACTTTGTCTGATTTTGGGTTATATTATCAACGAACTGGGTGAAGCCGGAT G |
| *Holdemania filiformis DSM 12042* | 1930 | CGGCAACAGGAAACATATTGCATCTCACTCAGCAAAAAAACACGCCTGCA TATCAACCTCAAATCCCTGAATCCTTTTGCTTTACTGGGATTACCTAGAC AGCAGGCAAACCGTTTCCACATGGCACGAAGCTTCAGATGAATGTCAGCG CATCTG |
| *Holdemania filiformis DSM 12042* | 1931 | TTTTTTCTTTTTCCAACCCTATACGATTTTATAGCTCATTTTCTAAAAAC CCTACAAGATTTTATATTACCGAAAAACTTTAAATTAATAAGGTGTCTTT CAATGCACTTATTGCTTAAGACCTATCCCCATTTTCCTGACTAATGATAT CCTGACTTTTC |
| *Helicobacter bilis ATCC 43879* | 1932 | TTTGATTGACTTCATCTTTGGTGAAAATTTGATTTATGATTTACTAACAG ATTCTGCTGAAAATCTTATTAAC |
| *Helicobacter bilis ATCC 43879* | 1933 | ACATGATTTTCAAGCAGTCCCGCAGGATACTGACTTTTATGCTGAGATTG ATGAAAAATTTATCGCCCCATTAC |
| *Anaerococcus vaginalis ATCC 51170* | 1934 | AAGAGAATAAATAGAAAATGGAATTAATAATATAGGAGTTTAAAATGTTA ATTAATATGAAAGAAATGTTGAAAGTTGCTAACGAGAATAATTTTGCTGT ACCAGCATTTAATA |
| *Anaerococcus vaginalis ATCC 51170* | 1935 | GCAGAGTATTTCCTGTGGCGGCTATTAAGGTTATGTTGCTGTCTAATAAT TTACTTACTAATTTTTTTATATTATTTATATTATCTTCTTTGTTAATAGT GTTTATTAAACTTTTTTTTCTATTTAAAAAAAATTCTAT |
| *Anaerococcus vaginalis ATCC 51170* | 1936 | CCAATATCTCTTAAAAGTAAAATTTTCATTTGGTAAAAACCGTCAAAACC AGAATGTTGACACATTCTAACAACAGTTGCATCGCTTACTTTTGTTTCGT TTGCTATTTCTTTTACACTCATAAGAGTTACTTTGT |
| *Collinsella aerofaciens ATCC 25986* | 1937 | TTGCAATGCGATTGTCCCTGTGTCGCCCTGACCGATTATGATTGGCGAAA CCAGCTTTCGTCCGTTCATGACTCGATTGTATTTGTCGATGAGGGCTTAA AAGAGATCCATTCTGATGAGTTTGCCCATCATGTGCTGTATTCCTCGAAT TATTTCGTGCT |
| *Dorea formicigenerans ATCC 27755* | 1938 | TGGAAAATCCGAAAAGTAAAATTAAAGGTAGTAATACGGAAAATACGGAA ACAAAAGAAGGGGCTGTTCGCTTTGATATTATTTTTTATGTTCGAATGAA AGATGGAATTTCTCAGATTATTGTGAATATAGAAGCTCAAAAGAATAGTT CGCC |
| *Dorea formicigenerans ATCC 27755* | 1939 | TGAAAGCGCAGTATGACGAGAATGCCAAAAAGCTTTTAAGTAACAAAATT TTTCTGGCACATATTTTAAAAGGAACAGTAACGGAATTTAAAGATGCGAA TCCAAGAGATATCATTTCTTTGATTGAAGGAGAACCATATGTATCTAC |
| *Ruminococcus gnavus ATCC 29149* | 1940 | GATACTGTTTCGGGACAGTAACCAGCGTATGATCGAAGGGAAGCAGGTAC TGATCCTGACAGATTCTGTGACGACCGGTGCTTTGCTTGCAAAAGCCGTG GAAGCAGTGCTGTATTACGGTGGACGTGTATGTGGTATCTGTGCAGTATT CAGTGCGG |
| *Ruminococcus gnavus ATCC 29149* | 1941 | TATGGAACTCACTGGCATATAATGGTGCCAGATGGATTGCATCCGGGCGA TATCATTACAATATTGAGACTGTGTTGGATGAGAGGATCCCTTTCATACC CTGGACATTGGTAATCTATTTTGGATGTTATCTGTTTTGGGGAATCAATT ACATTTTGAT |
| *Campylobacter rectus RM3267* | 1942 | ATATGCGGATTTTAAATTTTGTGTATTTCTCATAATATATCCTTGAGAAA GAGGTTAAAATATAAAAACATAATAATAAATTAATGTTTAATGTAAGCTT AAGGGATTAGTAAAATTTAATATGAAATATAAATTCTTATAT |
| *Campylobacter rectus RM3267* | 1943 | CAAATTTATCTTTTTTTGAGTGCCGGTAAAATAATAATATTTTTGAGCGG TGGTGCCGTTTTCTATGTTTGAGTTTATCGTAGCGAAATCGGCCGCAAAA TTTGAAACGGCGAGTAAAATAGCCGTTGCCGCAACCGAACT |
| *Campylobacter rectus RM3267* | 1944 | TATCAAAAACGTAAACGGTATCGGAGAAAAGACATTTGAAAATTTAAAAG GCGATATCTCGATAAGCGGCGAAAACGTGATGCCTGCAAGCAGTAAAGTG TCTAAAAAAGTAAAAGAAGCTA |
| *Actinomyces viscosus C505* | 1945 | CACCCCCGGCTTCGACGACGCCTAAGTCTTCCGAGGTGTACATCCAGTGC TGGACATCGAGGTCAGGAGGACCGAGAGGAGCGTCGACACCAGCTGCTGT CCACGCCAGAGCGTTCGCCGAACGGCGGACGTCGGCCCCGATCCAGGCAA GCTCGTCAAGCGCCGC |
| *Actinomyces viscosus C505* | 1946 | GTGTTCTACCGAACGGCCAGAACCACATCACTGGAAGGGACAGGATGAGG GCAAGAAACACCGTCACATTGAGAATAAAATTCAAAGAAGAGGTAAACGC CTTCAGAACCCGAACAATCGATCTCAACACATTCATCTCTTTTCAAGTGG ATGACGATGCCACC |
| *Actinomyces viscosus C505* | 1947 | TTCAGTTCCGCGTTCACTTCTGAAATATTCATCGGTTGTATTCGTTTCTG GGATTCGTGGGGTTGGACTTTTGACATGCAGTCGCACCGAGTATCAGACT CCCGGGAAGGTCCCGTCCCCGAGGAATTCTCTGCGCCTCGGCGCATATGG ACCGATGCCCGC |
| *Campylobacter gracilis RM3268* | 1948 | AAGCTCAGTGCTTTCAAAAAACCTTAAATTTTTGCGCGGAATTTCGGCTA AAATTTTATCTTTAAAGCCCTCATAATCGTTAGTTAAAACGATCTGATTT ATGATTTTCAAAGCACCACT |
| *Campylobacter gracilis RM3268* | 1949 | TTGTCTAAAATTTTAAAAAGCTGAATCAGTTTGAGATCTAAGCTCGGATC GCTCTCGCTCATGTAAAAGCTATTTTGTAGCCTCTCATCGATGAGCCACA GATAGCTATCCTCGTGGCTTTTGGCGATTTTGGCTAACGGATGGTTGCCG TTGCC |
| *Campylobacter gracilis RM3268* | 1950 | GACATTAGTATTATCTAAAAACGAGATTACGATGTCAAATTTACGACTTT TTAGAAGCCTGTGCAAGGCTAAAATTTTATCATAGCGCTTTTTTATATTT CCAAAAACTCCTAAATCGCCTACGCCCAAATCCAAGCTAATAAGCTCGAT CTTAGGATC |
| *Campylobacter gracilis RM3268* | 1951 | TGACTTTGTTTTTTGCCTGAAGCACAGCTAGGCCTAAATTTTGAATCAAC GGCACGCTTACTGGAAGCACCAAAATAAGCGCGATTGCGTATGAGATCTC GTAATTCGCGCCCGCCCATA |
| *Peptostreptococcus anaerobius 653-L* | 1952 | GAGGCCCACAATATGTATCACGGTGAGAAGGTTGCCTTTGGAACTTGTGT ACAGCTTATATTAGAAGATGCTCCTTTGGAAGAAATAGAAGAAATATATA ATTT |
| *Peptostreptococcus anaerobius 653-L* | 1953 | CACTATACACAATATGCCATTCGAAGTTAACGAAAAGAAGGTATACAGTG CAATTATCGCTGCTGATAATATGGGAAGGAAGTATTTAGGCAAATAATCG AGAATTAGGAGGTAATAAAATGTCAAATGTAGAGTCAACAAAATACAGGT GTA |
| *Prevotella histicola F0411* | 1954 | TTGTGCTGGCCATCATCACTACGCGTGTCAGGCATTAGCACAACATGTGT TGATTATCAGCTCCAATCCAACTAAACATAGTCTGATTTATAATAACTTA CTACAATAACTATAAAAGATGAATTATTAT |
| *Prevotella histicola F0411* | 1955 | TCCGGCTCCGTTTTTATCAATCCTGTTGTATGGATCGCGTTCTGTTCATT ATCTTATGATGGTCTTTTTGCCGTTGATGACATAGACGCCCTTGCCCAAT GACTTCAGACTCTTCGCATCTTTGAGAACCTGTTTGCCGTCGAGTGTGTA GACATCATATAGAA |
| *Helicobacter bizzozeronii CIII-1* | 1956 | TCTGGGGTCTCTATGCCCCAACATGTTGAGGTTAGGTCCTTGAATCACCA AAATTTTCATGCCATTCTCCTTACCAAGTGAAAGATTAAAGAGGTCATTA TAGCATAAAACTCCCGTTTAAAGCCCAAAGGCTTAGAGTGTGAAATTATG G |
| *Helicobacter bizzozeronii CIII-1* | 1957 | GTAAGTTTTGATACAACCAATGGTGTTAAAAGCCGTCCAAGGACTATCAA TATGCAACTTAGCCTGTTTCATACCCGACTCAATTTGCACCAAATTATCT AAAAACTTATGGTTGAAGTCTTGGATTTTTAAAATATAAAGGTGCAAAA |
| *Helicobacter bizzozeronii CIII-1* | 1958 | AAAAGAATATTACCATGAAGTTCAGTTATAGCAAACCCACGCCCAAGCAC ATGGTGGATTTACTCACCAAAGTTTGGGTTTTTTACATGTGTTTGTCCTT GTGTCTGATTTGGGGACTAGCCTATTTTTTACGCCACTACACCAAAGCC |
| *Enterococcus hirae ATCC 9790* | 1959 | ACCAAAGAAACAATTCCTTTTGCGGCAATCGTCATAGTGGATTCCGACGA CATTTTAGATTCGAAAGCCTATCTTGAAAACTATGCCAGCTTCGGTGGGT ATTTTGATTTTTCGCTGAGCGATGAAATGATTTATGGCTTC |
| *Enterococcus hirae ATCC 9790* | 1960 | ATATTATTTAGGATGGATCGAAAGCCAAAAAGTAGAAGCTGATTTAACAA ATGAGGATAAGCAAAAGTAGACGAAGAAGGAGCGGGAAAGGATAGTTATC TCGCTCTTCTTCGTCTATTTGGTTAGGATTTATCAGGGTTAA |
| *Bacteroides nordii CL02T12C05* | 1961 | TTCAAGTCATTGATACGTACCTGATAGTTGGACACGTCGTCTATATTCTT TGTGTTGTCCTTCTCCGTAGGCACATAATTCGCATCTGTCGTCTCTTTCC AGCAAAAGCCGGAAAGGAACATTTCACCTCCTCCGTCATCCAGCACAGAA GTAGATACAATAA |
| *Bacteroides nordii CL02T12C05* | 1962 | TTTTAAGAAAGCATTCTTGGGCCAGATAGAACTACCCAACACAAAACAGG ACTGGAAAAAGAAATATCCTCCAATAACCAACATAAAATCTTTCAACGTA CGGGTCAATGTATAATAGGGAACGTTTCCTCCGACACAATGTGACAACGG AAC |
| *Bacteroides nordii CL02T12C05* | 1963 | TAATTTAATATCTTGTGATACCATTATCAACAAAATGCAGATAAACACAG ATTAATGCATATTAAAACCATTGATTCCTTGTACTTCCCACACTGGGAAG TTCTCCAGGCGGTGTTTACGTTGGTTCCCCACAGATTGGCACCGAGTTTC ACAG |
| *Bacteroides nordii CL02T12C05* | 1964 | TTAGGGAGGATATAATAAATAGTTAATAGCGTTTCAATATAGAGTTTTAT ACAATATTACTTCTTGATTTTCAGAATTTCTGTGAATTGTTTTCAGTGTT TTCTTTATAGTATCACAAAT |
| *Barnesiella intestinihominis YIT 11860* | 1965 | ACAATTCTCACTGATTTTTACTGTACCGAGGTAATTCCCATCAAAAAAAA AGACACCCCATGACAAAGATACCACATATCTAAACAAAAAAATGCGACAG ATTTTTCTGTCGCATTCGTAGCCCATAGGAGAATCGAA |
| *Lactobacillus murinus ASF361* | 1966 | GATCACTTTCATGTCGGTGCCTGAGCGGGCCTTTCTCTCAGACTGGTCAT ATGCGATCGGATCATTAGTTATCATCCCGATCATCCCGATCTTAAATCAT TATTATGTCCCTTTCTTTCGC |
| *Lactobacillus murinus ASF361* | 1967 | CCACACATGCTCGCGGATCTGGTCTCTTGTTAAGACTTGTCCTCGATTGC GACATAAATATTCCAACACTTCGTATTCTTTAGCTGTCAGCTCGATCAAA GTCGCTTCCTTTTTCACCTGCTTTTTAGCAATATTTATCGTTATATCACC TATTT |
| *Eubacterium rectale CAG: 36* | 1968 | TATTTTTTCTCAACCTCTGTGAGGAACTTGTCATCAAGCTCCGCCTTTAT AAGTACGTTCTCATCA |
| *Cloacibacillus porcorum* | 1969 | GTGCTATGTTCAGAGGAGATGTTGTAGATACTCCCGATGATGAATGGCTT AAACTTTTTGATATTAATGTTCACGCGGTCTTTTATCTTTCTAGGGAGGC CATACGGCTTATGCGGGAACATAAAATAGCGGGGAACATTGTACA |
| *Cloacibacillus porcorum* | 1970 | AAGGGGCATATCGCCTACGCAACTACAAAAGGAGCAGTAGTGCAAATGAC ACGTTGCATGGCTTTAGACTGTGCCTCAGATGGAATACGCATAAACGCTG TATGCCCTGGTGCAACTGATACTGCGATGCCAATGTCAAAGCATAGTGC |
| *Blautia coccoides* | 1971 | GGCACTTGCCGCACTACAGGTGCCATAGCCTGCACTGTATATACTGCAAT CCTTTCCTGTTCTGGCCGCCGGAGGATTTATGTTGGTTATAGTATATACG GTAATGCCCAAGGGTAAAATGCTGTATTCTGCT |
| *Ruminococcus bromii* | 1972 | TTACGGCTTAATATTAAGACTTGTTTCATACGGAATCGATACAGGGCTTA TTGAAAAAGAGGATATAATCTACACGCAAAATAGGCTTTTATCGTTGTTC CATATGGATGAACCCGATGACAGTTGTACTTGCCTTACAGCAGATAACGA |
| *Phascolarctobacterium faecium DSM 14760* | 1973 | CTAATAAGAAAGATAAGAAGTATAAAAAGGAAAAGAAGGTTATTAAAAAC CCATTTTTCTAAATTTATAATAAAGGAAGACATACGATGTATATCAAACG CCATTTGGAGACGACAATTGAAAAACTGAGCGGTTGTTGTAAGG |
| *Phascolarctobacterium faecium DSM 14760* | 1974 | AGGAAATGGTTTATCTGCGCAATATTGCCAGGGATAAGTTTGGCATGAAA TTTTTGTAGTTTTACATGAAAATTTTGAATTTTATTTTCACTGTTGGTAT GTAGTACAACAGATTGCTTTTAAGATAGCTGCTAGTTGATT |
| *Helicobacter salomonis* | 1975 | TCCAAGTATTTGCCAAAGAACCCTTTGTGGGCATCAAAAGCGCGCACAAA CTCCAGAGTTGGATGAAAAATTCTCTCCAAGTGTGCGATCATCACTGGAG GCATGTTGACTAGGGCAAGTTTGGCGAGATTTTCTGCACTCAAGAG |
| *Helicobacter salomonis* | 1976 | TTGCATGTCTTTTTGAATACTGGAAATGGCGCGTTGCTGGTCATTTGTGA GAACAAAGGGTAGGGCCTGTAGAAATTCTCTAAGGAGCTGGGCATTAGCA TGCCCACCAAACTTGCTTTGAAAATTCCACTTTTTGGCATTCAAGGAGAG CATAT |
| *Gardnerella vaginalis* | 1977 | GCTATCCGCTATGGGATTACGCTTATTCCCACATGACCATTCCATCTCTA CATGCAGAGTCAGGAATAATTGTCATAGCTGCTCTTATCCACTTTATACT GGTGCTTATCTGCATATTCGATAAG |
| *Gardnerella vaginalis* | 1978 | TTTCCTTTTTGCTTTCTTCCAAGGAGTAGTAAATCCACCATTCCAAACAG TATTTATTCATGCAGTAGATGATAATCTCGTTGGACAGGTAATGTCTGTA T |
| *Gardnerella vaginalis* | 1979 | GGAATGGCAACATTTGTAAAAAACCAAACGTACAGGACTTTAGCTCCATT TGCAATACTCGAAGCTATCGCATCTACTGGCATAAGCTTTGCTGGAGTCC TCTTATTCTCAATGGTTCTACATAGTGCAGAAGGATATGGCTGGTATTTA |

## Claims

1. A computer-implemented method, comprising:
receiving a plurality of nucleic acid sequence reads, wherein the sequence reads include a plurality of 16S sequence reads;
first mapping the plurality of 16S sequence reads to a plurality of compressed 16S reference sequences, wherein each compressed 16S reference sequences include a set of hypervariable segments for a corresponding strain of a species;
generating a read count matrix containing read counts of 16S sequence reads mapped to each hypervariable segment in the set of hypervariable segments, wherein rows of the read count matrix correspond to strains of species and columns correspond the hypervariable segments;
reducing the read count matrix by applying thresholding to the read counts to form a reduced read count matrix;
compressing a database of full-length 16S reference sequences to form a reduced set of full-length 16S reference sequences based on the reduced read count matrix, the reduced set of full-length 16S reference sequences stored in a memory;
second mapping the plurality of 16S sequence reads to the reduced set of full-length 16S reference sequences;
counting the 16S sequence reads that mapped to each full-length reference in the reduced set of full-length 16S reference sequences to form a second set of read counts;
normalizing the read counts in the second set of read counts to form normalized counts;
aggregating the normalized counts for a given level to form aggregated counts, wherein the given level is a species level, a genus level or a family level; and applying a threshold to the aggregated counts to detect a presence of a microbe at the given level in a sample.

2. The computer-implemented method of claim 1, wherein the reducing the read count matrix further comprises eliminating rows of the read count matrix when a sum of read counts within the row are less than a row sum threshold to form a first reduced read count matrix.

3. The computer-implemented method of claim 2, wherein the reducing the read count matrix further comprises:
adding the read counts of the rows of the first reduced read count matrix that correspond to identical expected signatures for a corresponding species to form column sums; and
adding the column sums to form a combined sum, wherein an expected signature comprises binary values corresponding to the hypervariable segments in the set of hypervariable segments expected to be present (=1) or absent (=0) in the strain, preferably,
wherein the reducing the read count matrix further comprises eliminating the rows of the first reduced read count matrix when the combined sum is less than a combined sum threshold to form a second reduced read count matrix.

4. The computer-implemented method of claim 2, wherein the reducing the read count matrix further comprises:
adding the read counts of the rows of the first reduced read count matrix that correspond to identical expected signatures for a corresponding species to form column sums; and
adding the column sums to form a combined sum, wherein an expected signature comprises binary values corresponding to the hypervariable segments in the set of hypervariable segments expected to be present (=1) or absent (=0) in the strain,
wherein the reducing the read count matrix further comprises applying a signature threshold to the column sums to assign binary values to form an observed signature for each row of the second reduced read count matrix, the observed signature and expected signature each having a total number of categories, in particular,
wherein the compressing further comprises determining a ratio of the categories that have matching binary values in the observed signature and the expected signature to the total number of categories, preferably,
wherein the compressing further comprises selecting a corresponding full-length 16S reference sequence from the database of full-length 16S reference sequences stored in memory for a first reduced set of full-length 16S reference sequences when the ratio is greater than a ratio threshold.

5. The computer-implemented method of claim 1, wherein the plurality of nucleic acid sequence reads further include a plurality of targeted species sequence reads, preferably,
further comprising mapping the targeted species sequence reads to segmented reference sequences to form targeted species mapped reads, wherein each segmented reference sequence comprises segments corresponding to expected amplicons for a strain of the targeted species.

6. The computer-implemented method of claim 1, wherein the plurality of 16S sequence reads correspond to amplicons produced by amplifying a nucleic acid sample in the presence of one or more primer pairs targeting one or more hypervariable regions of a prokaryotic 16S rRNA gene.

7. The computer-implemented method of claim 1, wherein the plurality of nucleic acid sequence reads further include a plurality of targeted species sequence reads, and
wherein the plurality of targeted species sequence reads correspond to amplicons produced by amplifying a target nucleic acid sequence contained within a genome of a microorganism that is outside a hypervariable region of a prokaryotic 16S rRNA gene, wherein different primer pairs amplify different target nucleic acid sequences contained within the genome of different microorganisms in the nucleic acid sample.

8. A computer-implemented method, comprising:
receiving a plurality of nucleic acid sequence reads at a processor, wherein the sequence reads include a plurality of 16S sequence reads;
first mapping the plurality of 16S sequence reads to a plurality of compressed 16S reference sequences, wherein each compressed 16S reference sequence includes a set of hypervariable segments for a corresponding strain of a species;
counting the 16S sequence reads mapped to each hypervariable segment in the set of hypervariable segments to form a first set of read counts;
compressing a database of full-length 16S reference sequences to form a reduced set of full-length 16S reference sequences based on the first set of read counts of the 16S sequence reads mapped to the compressed 16S reference sequences, the reduced set of full- length 16S reference sequences stored in a memory;
second mapping the plurality of 16S sequence reads to the reduced set of full-length 16S reference sequences;
counting the 16S sequence reads that mapped to each full-length reference sequence in the reduced set of full-length 16S reference sequences to form a second set of read counts; and
detecting a presence of a microbe at a species level, a genus level or a family level in a sample based on the second set of read counts.

9. The computer-implemented method of claim 8, wherein the plurality of nucleic acid sequence reads further include a plurality of targeted species sequence reads, preferably, further comprising mapping the targeted species sequence reads to segmented reference sequences to form targeted species mapped reads, wherein each segmented reference sequence comprises segments corresponding to expected amplicons for a strain of the targeted species.

10. The computer-implemented method of claim 9, further comprising aggregating counts of the targeted species mapped reads to form aggregated read counts per species preferably, further comprising detecting a presence of the targeted species in the sample based on the aggregated read counts per species.

11. The computer-implemented method of claim 9, further comprising generating the segmented reference sequences by applying an in silico PCR based on primers of a species primer pool.

12. The computer-implemented method of claim 8, further comprising generating the compressed 16S reference sequences by applying an in silico PCR based on primers of a 16S primer pool, or , wherein the plurality of 16S sequence reads correspond to amplicons produced by amplifying a nucleic acid sample in the presence of one or more primer pairs targeting one or more hypervariable regions of a prokaryotic 16S rRNA gene.

13. The computer-implemented method of claim 8, wherein the plurality of nucleic acid sequence reads further include a plurality of targeted species sequence reads, and wherein the plurality of targeted species sequence reads correspond to amplicons produced by amplifying a target nucleic acid sequence contained within a genome of a microorganism that is outside a hypervariable region of a prokaryotic 16S rRNA gene, wherein different primer pairs amplify different target nucleic acid sequences contained within the genome of different microorganisms in the nucleic acid sample.

14. A system, comprising:
a machine-readable memory; and
a processor configured to execute machine-readable instructions, which, when executed by the processor, cause the system to perform a method, comprising:
receiving a plurality of nucleic acid sequence reads at the processor, wherein the sequence reads include a plurality of 16S sequence reads;
first mapping the plurality of 16S sequence reads to a plurality of compressed 16S reference sequences, wherein each compressed 16S reference sequences include a set of hypervariable segments for a corresponding strain of a species;
generating a read count matrix containing read counts of 16S sequence reads mapped to each hypervariable segment in the set of hypervariable segments, wherein rows of the read count matrix correspond to strains of species and columns correspond the hypervariable segments;
reducing the read count matrix by applying thresholding to the read counts to form a reduced read count matrix;
compressing a database of full-length 16S reference sequences to form a reduced set of full-length 16S reference sequences based on the reduced read count matrix, the reduced set of full-length 16S reference sequences stored in the memory;
second mapping the plurality of 16S sequence reads to the reduced set of full-length 16S reference sequences;
counting the 16S sequence reads that mapped to each full-length reference in the reduced set of full-length 16S reference sequences to form a second set of read counts;
normalizing the read counts in the second set of read counts to form normalized counts;
aggregating the normalized counts for a given level to form aggregated counts, wherein the given level is a species level, a genus level or a family level; and applying a threshold to the aggregated counts to detect a presence of a microbe at the given level in a sample.

15. A system, comprising:
a machine-readable memory; and
a processor configured to execute machine-readable instructions, which, when executed by the processor, cause the system to perform a method, comprising:
receiving a plurality of nucleic acid sequence reads at the processor, wherein the sequence reads include a plurality of 16S sequence reads;
first mapping the plurality of 16S sequence reads to a plurality of compressed 16S reference sequences, wherein each compressed 16S reference sequence includes a set of hypervariable segments for a corresponding strain of a species;
counting the 16S sequence reads mapped to each hypervariable segment in the set of hypervariable segments to form a first set of read counts;
compressing a database of full-length 16S reference sequences to form a reduced set of full-length 16S reference sequences based on the first set of read counts of the 16S sequence reads mapped to the compressed 16S reference sequences, the reduced set of full- length 16S reference sequences stored in the memory;
second mapping the plurality of 16S sequence reads to the reduced set of full-length 16S reference sequences;
counting the 16S sequence reads that mapped to each full-length reference sequence in the reduced set of full-length 16S reference sequences to form a second set of read counts; and
detecting a presence of a microbe at a species level, a genus level or a family level in a sample based on the second set of read counts.

## Patentansprüche

1. Computerimplementiertes Verfahren, umfassend:
Empfangen einer Vielzahl von Nukleinsäuresequenz-Reads, wobei die Sequenz-Reads eine Vielzahl von 16S-Sequenz-Reads umfassen;
erstes Abbilden (Mapping) der Vielzahl von 16S-Sequenz-Reads auf eine Vielzahl von komprimierten 16S-Referenzsequenzen, wobei jede komprimierte 16S-Referenzsequenz einen Satz von hypervariablen Segmenten für einen entsprechenden Stamm einer Art umfasst;
Erzeugen einer Read-Count-Matrix, die Read-Counts von 16S-Sequenz-Reads enthält, die auf jedes hypervariable Segment in dem Satz von hypervariablen Segmenten abgebildet wurden, wobei Zeilen der Read-Count-Matrix Stämmen von Arten und Spalten den hypervariablen Segmenten entsprechen;
Reduzieren der Read-Count-Matrix durch Anwenden eines Schwellenwerts auf die Read-Counts, um eine reduzierte Read-Count-Matrix zu bilden;
Komprimieren einer Datenbank von 16S-Referenzsequenzen voller Länge, um einen reduzierten Satz von 16S-Referenzsequenzen voller Länge basierend auf der reduzierten Read-Count-Matrix zu bilden, wobei der reduzierte Satz von 16S-Referenzsequenzen voller Länge in einem Speicher gespeichert wird;
zweites Abbilden (Mapping) der Vielzahl von 16S-Sequenz-Reads auf den reduzierten Satz von 16S-Referenzsequenzen voller Länge;
Zählen der 16S-Sequenz-Reads, die jeweils auf einer Referenzsequenz voller Länge in dem reduzierten Satz von 16S-Referenzsequenzen voller Länge abgebildet wurden, um einen zweiten Satz von Read-Counts zu bilden;
Normalisieren der Read-Counts im zweiten Satz von Read-Counts, um normalisierte Read-Counts zu bilden;
Aggregieren der normalisierten Counts für eine gegebene Ebene, um aggregierte Read-Counts zu bilden, wobei die gegebene Ebene eine Artebene, eine Gattungsebene oder eine Familienebene ist; und Anwenden eines Schwellenwerts auf die aggregierten Counts, um das Vorhandensein eines Mikroorganismus auf der gegebenen Ebene in einer Probe nachzuweisen.

2. Das computerimplementierte Verfahren nach Anspruch 1, wobei das Reduzieren der Read-Count-Matrix ferner das Eliminieren von Zeilen der Read-Count-Matrix umfasst, wenn eine Summe der Read-Counts innerhalb der Zeile kleiner als ein Zeilensummen-Schwellenwert ist, um eine erste reduzierte Read-Count-Matrix zu bilden.

3. Das computerimplementierte Verfahren nach Anspruch 2, wobei das Reduzieren der Read-Count-Matrix ferner umfasst:
Addieren der Read-Counts der Zeilen der ersten reduzierten Read-Count-Matrix, die identischen erwarteten Signaturen für eine entsprechende Art entsprechen, um Spaltensummen zu bilden; und
Addieren der Spaltensummen, um eine kombinierte Summe zu bilden, wobei eine erwartete Signatur Binärwerte umfasst, die den hypervariablen Segmenten in dem Satz von hypervariablen Segmenten entsprechen, von denen erwartet wird, dass sie in dem Stamm vorhanden (=1) oder abwesend (=D) sind, vorzugsweise,
wobei das Reduzieren der Read-Count-Matrix ferner das Eliminieren der Zeilen der ersten reduzierten Read-Count-Matrix umfasst, wenn die kombinierte Summe kleiner als ein Schwellenwert für die kombinierte Summe ist, um eine zweite reduzierte Read-Count-Matrix zu bilden.

4. Das computerimplementierte Verfahren nach Anspruch 2, wobei das Reduzieren der Read-Count-Matrix ferner umfasst:
Addieren der Read-Counts der Zeilen der ersten reduzierten Read-Count-Matrix, die identischen erwarteten Signaturen für eine entsprechende Art entsprechen, um Spaltensummen zu bilden; und
Addieren der Spaltensummen, um eine kombinierte Summe zu bilden, wobei eine erwartete Signatur Binärwerte umfasst, die den hypervariablen Segmenten in dem Satz von hypervariablen Segmenten entsprechen, von denen erwartet wird, dass sie in dem Stamm vorhanden (=1) oder abwesend (=D) sind,
wobei das Reduzieren der Read-Count-Matrix ferner das Anwenden eines Signatur-Schwellenwerts auf die Spaltensummen umfasst, um Binärwerte zuzuweisen und so eine beobachtete Signatur für jede Zeile der zweiten reduzierten Read-Count-Matrix zu bilden, wobei die beobachtete Signatur und die erwartete Signatur jeweils eine Gesamtzahl von Kategorien aufweisen, insbesondere,
wobei das Komprimieren ferner das Bestimmen eines Verhältnisses der Kategorien, die übereinstimmende Binärwerte in der beobachteten Signatur und der erwarteten Signatur aufweisen, zur Gesamtzahl der Kategorien umfasst,
wobei das Komprimieren ferner das Auswählen einer entsprechenden 16S-Referenzsequenz voller Länge aus der im Speicher abgelegten Datenbank von 16S-Referenzsequenzen voller Länge für einen ersten reduzierten Satz von 16S-Referenzsequenzen voller Länge umfasst, wenn das Verhältnis größer als ein Verhältnis-Schwellenwert ist.

5. Das computerimplementierte Verfahren nach Anspruch 1, wobei die Vielzahl von Nukleinsäuresequenz-Reads ferner eine Vielzahl von Sequenz-Reads gezielter Spezies umfasst,
ferner umfassend das Abbilden der Sequenz-Reads Zielarten auf segmentierte Referenzsequenzen, um abgebildete Reads Zielarten zu bilden, wobei jede segmentierte Referenzsequenz Segmente umfasst, die den erwarteten Amplikons für einen Stamm der gezielten Art entsprechen.

6. Das computerimplementierte Verfahren nach Anspruch 1, wobei die Vielzahl von 16S-Sequenz-Reads Amplikons entspricht, die durch Amplifizieren einer Nukleinsäureprobe in Gegenwart von einem oder mehreren Primerpaaren erzeugt wurden, die auf eine oder mehrere hypervariable Regionen eines prokaryotischen 16S-rRNA-Gens abzielen.

7. Das computerimplementierte Verfahren nach Anspruch 1, wobei die Vielzahl von Nukleinsäuresequenz-Reads ferner eine Vielzahl von Sequenz-Reads Zielarten umfasst, und
wobei die Vielzahl von Sequenz-Reads Zielarten Amplikons entspricht, die durch Amplifizieren einer Ziel-Nukleinsäuresequenz erzeugt wurden, die innerhalb eines Genoms eines Mikroorganismus außerhalb
einer hypervariablen Region eines prokaryotischen 16S-rRNA-Gens liegt, wobei verschiedene Primerpaare verschiedene Ziel-Nukleinsäuresequenzen amplifizieren, die im Genom verschiedener Mikroorganismen in der Nukleinsäureprobe enthalten sind.

8. Computerimplementiertes Verfahren, umfassend:
Empfangen einer Vielzahl von Nukleinsäuresequenz-Reads durch einen Prozessor, wobei die Sequenz-Reads eine Vielzahl von 16S-Sequenz-Reads umfassen;
erstes Abbilden (Mapping) der Vielzahl von 16S-Sequenz-Reads auf eine Vielzahl von komprimierten 16S-Referenzsequenzen, wobei jede komprimierte 16S-Referenzsequenz einen Satz von hypervariablen Segmenten für einen entsprechenden Stamm einer Art umfasst;
Zählen der 16S-Sequenz-Reads, die auf jedes hypervariable Segment in dem Satz von hypervariablen Segmenten abgebildet wurden, um einen ersten Satz von Read-Counts zu bilden;
Komprimieren einer Datenbank von 16S-Referenzsequenzen voller Länge, um einen reduzierten Satz von 16S-Referenzsequenzen voller Länge basierend auf dem ersten Satz von Read-Counts der 16S-Sequenz-Reads, die auf die komprimierten 16S-Referenzsequenzen abgebildet wurden, zu bilden, wobei der reduzierte Satz von 16S-Referenzsequenzen voller Länge in einem Speicher gespeichert wird;
zweites Abbilden (Mapping) der Vielzahl von 16S-Sequenz-Reads auf den reduzierten Satz von 16S-Referenzsequenzen voller Länge;
Zählen der 16S-Sequenz-Reads, die jeweils auf einer Referenzsequenz voller Länge in dem reduzierten Satz von 16S-Referenzsequenzen voller Länge abgebildet wurden, um einen zweiten Satz von Read-Counts zu bilden; und
Nachweisen des Vorhandenseins eines Mikroorganismus auf einer Artebene, einer Gattungsebene oder einer Familienebene in einer Probe basierend auf dem zweiten Satz von Read-Counts.

9. Das computerimplementierte Verfahren nach Anspruch 8, wobei die Vielzahl von Nukleinsäuresequenz-Reads ferner eine Vielzahl von Sequenz-Reads Zielarten umfasst, vorzugsweise ferner umfassend das Abbilden der Sequenz-Reads Zielarten auf segmentierte Referenzsequenzen, um abgebildete Reads Zielarten zu bilden, wobei jede segmentierte Referenzsequenz Segmente umfasst, die den erwarteten Amplikons für einen Stamm der gezielten Arten entsprechen.

10. Das computerimplementierte Verfahren nach Anspruch 9, ferner umfassend das Aggregieren von Counts der abgebildeten Reads Zielarten, um aggregierte Read-Counts pro Art zu bilden, vorzugsweise ferner umfassend das Nachweisen des Vorhandenseins der gezielten Art in der Probe basierend auf den aggregierten Read-Counts pro Art.

11. Das computerimplementierte Verfahren nach Anspruch 9, ferner umfassend das Erzeugen der segmentierten Referenzsequenzen durch Anwenden einer in-silico-PCR basierend auf Primern eines Art-Primer-Pools.

12. Das computerimplementierte Verfahren nach Anspruch 8, ferner umfassend das Erzeugen der komprimierten 16S-Referenzsequenzen durch Anwenden einer in-silico-PCR basierend auf Primern eines 16S-Primer-Pools, oder wobei die Vielzahl von 16S-Sequenz-Reads Amplikons entspricht, die durch Amplifizieren einer Nukleinsäureprobe in Gegenwart von einem oder mehreren Primerpaaren erzeugt wurden, die auf eine oder mehrere hypervariable Regionen eines prokaryotischen 16S-rRNA-Gens abzielen.

13. Das computerimplementierte Verfahren nach Anspruch 8, wobei die Vielzahl von Nukleinsäuresequenz-Reads ferner eine Vielzahl von Sequenz-Reads Zielarten umfasst, und wobei die Vielzahl von Sequenz-Reads Zielarten Amplikons entspricht, die durch Amplifizieren einer Ziel-Nukleinsäuresequenz erzeugt wurden, die innerhalb eines Genoms eines Mikroorganismus außerhalb einer hypervariablen Region eines prokaryotischen 16S-rRNA-Gens liegt, wobei verschiedene Primerpaare verschiedene Ziel-Nukleinsäuresequenzen amplifizieren, die im Genom verschiedener Mikroorganismen in der Nukleinsäureprobe enthalten sind.

14. System, umfassend:
einen maschinenlesbaren Speicher; und
einen Prozessor, der so konfiguriert ist, dass er maschinenlesbare Anweisungen ausführt, die, wenn sie von dem Prozessor ausgeführt werden, das System veranlassen, ein Verfahren durchzuführen, das Folgendes umfasst:
Empfangen einer Vielzahl von Nukleinsäuresequenz-Reads durch den Prozessor, wobei die Sequenz-Reads eine Vielzahl von 16S-Sequenz-Reads umfassen;
erstes Abbilden (Mapping) der Vielzahl von 16S-Sequenz-Reads auf eine Vielzahl von komprimierten 16S-Referenzsequenzen, wobei jede komprimierte 16S-Referenzsequenz einen Satz von hypervariablen Segmenten für einen entsprechenden Stamm einer Art umfasst;
Erzeugen einer Read-Count-Matrix, die Read-Counts von 16S-Sequenz-Reads enthält, die auf jedes hypervariable Segment in dem Satz von hypervariablen Segmenten abgebildet wurden, wobei Zeilen der Read-Count-Matrix Stämmen von Arten und Spalten den hypervariablen Segmenten entsprechen;
Reduzieren der Read-Count-Matrix durch Anwenden eines Schwellenwerts auf die Read-Counts, um eine reduzierte Read-Count-Matrix zu bilden;
Komprimieren einer Datenbank von 16S-Referenzsequenzen voller Länge, um einen reduzierten Satz von 16S-Referenzsequenzen voller Länge basierend auf der reduzierten Read-Count-Matrix zu bilden, wobei der reduzierte Satz von 16S-Referenzsequenzen voller Länge im Speicher gespeichert wird;
zweites Abbilden (Mapping) der Vielzahl von 16S-Sequenz-Reads auf den reduzierten Satz von 16S-Referenzsequenzen voller Länge;
Zählen der 16S-Sequenz-Reads, die jeweils auf einer Referenzsequenz voller Länge in dem reduzierten Satz von 16S-Referenzsequenzen voller Länge abgebildet wurden, um einen zweiten Satz von Read-Counts zu bilden;
Normalisieren der Read-Counts im zweiten Satz von Read-Counts, um normalisierte Read-Counts zu bilden;
Aggregieren der normalisierten Counts für eine gegebene Ebene, um aggregierte Read-Counts zu bilden, wobei die gegebene Ebene eine Artebene, eine Gattungsebene oder eine Familienebene ist; und Anwenden eines Schwellenwerts auf die aggregierten Counts, um das Vorhandensein eines Mikroorganismus auf der gegebenen Ebene in einer Probe nachzuweisen.

15. System, umfassend:
einen maschinenlesbaren Speicher; und
einen Prozessor, der so konfiguriert ist, dass er maschinenlesbare Anweisungen ausführt, die, wenn sie von dem Prozessor ausgeführt werden, das System veranlassen, ein Verfahren durchzuführen, das Folgendes umfasst:
Empfangen einer Vielzahl von Nukleinsäuresequenz-Reads durch den Prozessor, wobei die Sequenz-Reads eine Vielzahl von 16S-Sequenz-Reads umfassen;
erstes Abbilden (Mapping) der Vielzahl von 16S-Sequenz-Reads auf eine Vielzahl von komprimierten 16S-Referenzsequenzen, wobei jede komprimierte 16S-Referenzsequenz einen Satz von hypervariablen Segmenten für einen entsprechenden Stamm einer Art umfasst;
Zählen der 16S-Sequenz-Reads, die auf jedes hypervariable Segment in dem Satz von hypervariablen Segmenten abgebildet wurden, um einen ersten Satz von Read-Counts zu bilden;
Komprimieren einer Datenbank von 16S-Referenzsequenzen voller Länge, um einen reduzierten Satz von 16S-Referenzsequenzen voller Länge basierend auf dem ersten Satz von Read-Counts der 16S-Sequenz-Reads, die auf die komprimierten 16S-Referenzsequenzen abgebildet wurden, zu bilden, wobei der reduzierte Satz von 16S-Referenzsequenzen voller Länge im Speicher gespeichert wird;
zweites Abbilden (Mapping) der Vielzahl von 16S-Sequenz-Reads auf den reduzierten Satz von 16S-Referenzsequenzen voller Länge;
Zählen der 16S-Sequenz-Reads, die jeweils auf einer Referenzsequenz voller Länge in dem reduzierten Satz von 16S-Referenzsequenzen voller Länge abgebildet wurden, um einen zweiten Satz von Read-Counts zu bilden; und
Nachweisen des Vorhandenseins eines Mikroorganismus auf einer Artebene, einer Gattungsebene oder einer Familienebene in einer Probe basierend auf dem zweiten Satz von Read-Counts.

## Revendications

1. Procédé mis en œuvre par ordinateur comprenant :
La réception de plusieurs lectures de séquence d'acide nucléique, les lectures de séquence comprenant une série de lectures de séquence 16S ;
La première cartographie de la série de lectures de séquence 16S en une série de séquences de référence 16S comprimées, dans laquelle chaque séquence de référence 16S comprimée comprend un ensemble de segments hypervariables pour une souche correspondante d'une espèces ;
La génération d'une matrice de nombre de lectures contenant les nombres de lectures de séquence 16S associée à chaque segment hypervariable dans l'ensemble des segments hypervariables, dans laquelle les lignes de la matrice de nombres de lectures correspondent aux souches d'espèces et les colonnes correspondent aux segments hypervariables ;
La réduction de la matrice de nombre de lectures en appliquant un seuil aux nombres de lectures pour former une matrice de nombre de lectures réduite ;
La compression d'une base de données de séquences de référence 16S de longueur complète pour former un ensemble réduit de séquences de référence 16S de longueur complète basée sur la matrice de nombres de lectures réduite, l'ensemble réduit de séquences de référence 16S de longueur complète étant stocké dans une mémoire ;
La seconde cartographie de la série de lectures de séquence 16S dans l'ensemble réduit de séquences de référence 16S de longueur complète ;
Le comptage des lectures de séquence 16S, qui sont associées à chaque référence de longueur complète dans l'ensemble réduit de séquences de référence 16S de longueur complète pour former un deuxième ensemble de nombres de lectures ;
La normalisation des nombres de lectures dans le deuxième ensemble de nombres de lectures pour former des nombres normalisés ;
l'agrégation des nombres normalisés pour un niveau donné pour former des nombres agrégés, le niveau donné étant un niveau d'espèce, un niveau de genre ou un niveau de famille ; et l'application d'un seuil aux nombres agrégés pour détecter la présence d'un microbe à un niveau donné dans un échantillon.

2. Procédé mis en œuvre par ordinateur selon la revendication 1, dans lequel la réduction de la matrice de nombres de lectures comprend en outre l'élimination des lignes de la matrice de nombres de lectures lorsque la somme de nombres de lectures dans la ligne est inférieure à un seuil de somme des lignes pour former une première matrice de nombres de lectures réduite.

3. Procédé mis en œuvre par ordinateur selon la revendication 2, dans lequel la réduction de la matrice de nombres de lectures comprend en outre :
l'ajout des nombres de lectures des lignes de la première matrice de nombres de lectures réduite, qui correspond aux signatures attendues identiques pour une espèce correspondante pour former la somme des colonnes ; et
l'ajout des sommes des colonnes pour former une somme combinée, où une signature attendue comprend des valeurs binaires correspondant aux segments hypervariables dans l'ensemble des segments hypervariables censés être présents (= 1) ou absent (= 0) dans la souche, de préférence,
dans lequel la réduction de la matrice de nombres de lectures comprend en outre l'élimination des lignes de la première matrice de nombres de lectures réduite lorsque la somme combinée est inférieure à un seuil de somme combinée pour former une deuxième matrice de nombres de lectures réduite.

4. Procédé mis en œuvre par ordinateur selon la revendication 2, dans lequel la réduction de la matrice de nombres de lectures comprend en outre :
l'ajout des nombres de lectures des lignes de la première matrice de nombres de lectures réduite, qui correspond aux signatures attendues identiques pour une espèce correspondante pour former la somme des colonnes ; et
l'ajout des sommes des colonnes pour former une somme combinée, où une signature attendue comprend des valeurs binaires correspondant aux segments hypervariables dans l'ensemble des segments hypervariables censés être présents (= 1) ou absent (= 0) dans la souche,
dans lequel la réduction de la matrice de nombres de lectures comprend en outre l'application d'un seuil de signature aux sommes de colonnes pour attribuer des valeurs binaires afin de former une signature observée pour chaque ligne de la deuxième matrice de nombres de lectures réduite, la signature observée et la signature attendue ayant chacune un nombre total de catégories, en particulier,
dans lequel la compression comprend en outre la détermination d'un rapport entre les catégories qui ont des valeurs binaires correspondantes dans la signature observée et la signature attendue par rapport au nombre total de catégories, de préférence,
dans lequel la compression comprend en outre la sélection d'une séquence de référence 16S de longueur complète correspondante dans la base de données de séquences de référence 16S de longueur complète stockées en mémoire pour un premier ensemble réduit de séquences de référence 16S de longueur complète lorsque le rapport est supérieur à un seuil de rapport.

5. Procédé mis en œuvre par ordinateur selon la revendication 1, dans lequel la série de lectures de séquence d'acide nucléique comprend en outre une série de lectures de séquence des espèces ciblées, de préférence,
comprenant en outre la cartographie des lectures de séquence des espèces ciblées en séquences de référence segmentées pour former des lectures alignées des espèces ciblées, dans lequel chaque séquence de référence segmentée comprend des segments correspondant aux amplicons attendus pour une souche des espèces ciblées.

6. Procédé mis en œuvre par ordinateur selon la revendication 1, dans lequel la série de lectures de séquence 16S correspond à des amplicons produits par l'amplification d'un échantillon d'acide nucléique en présence d'une ou plusieurs paires d'amorce ciblant une ou plusieurs régions hypervariables d'un gène d'ARNr 16S procaryote.

7. Procédé mis en œuvre par ordinateur selon la revendication 1, dans lequel la série de lectures de séquence d'acide nucléique comprend en outre une série de lectures de séquence des espèces ciblées, et
dans lequel la série de lectures de séquence d'espèces ciblées correspond à des amplicons produits par l'amplification d'une séquence d'acide nucléique cible contenue dans le génome d'un micro-organisme, qui se trouve hors
d'une région hypervariable d'un gène d'ARNr 16S procaryote, dans laquelle différentes paires d'amorces amplifient différentes séquences d'acide nucléique cibles contenues dans le génome de différents micro-organismes dans l'échantillon d'acide nucléique.

8. Procédé mis en œuvre par ordinateur comprenant :
La réception de plusieurs lectures de séquence d'acide nucléique dans un processeur, les lectures de séquence comprenant une série de lectures de séquence 16S ;
La première cartographie de la série de lectures de séquence 16S en une série de séquences de référence 16S comprimées, dans laquelle chaque séquence de référence 16S comprimée comprend un ensemble de segments hypervariables pour une souche correspondante d'une espèces ;
Le comptage des lectures de séquence 16S associées à chaque segment hypervariable dans l'ensemble des segments hypervariables pour former un premier ensemble de nombres de lectures ;
La compression d'une base de données de séquences de référence 16S de longueur complète pour former un ensemble réduit de séquences de référence 16S de longueur complète basée sur le premier ensemble de nombres de lectures de séquence 16S associées aux séquences de référence 16S compressées, l'ensemble réduit de séquences de référence 16S de longueur complète étant stocké dans une mémoire ;
La seconde cartographie de la série de lectures de séquence 16S dans l'ensemble réduit de séquences de référence 16S de longueur complète ;
Le comptage des lectures de séquence 16S, qui sont associées à chaque référence de longueur complète dans l'ensemble réduit de séquences de référence 16S de longueur complète pour former un deuxième ensemble de nombres de lectures ; et
La détection de la présence d'un microbe au niveau de l'espèce, au niveau du genre ou au niveau de la famille dans un échantillon sur la base du deuxième ensemble de nombres de lectures.

9. Procédé mis en œuvre par ordinateur selon la revendication 8, dans lequel la série de lectures de séquence d'acide nucléique comprend en outre une série de lectures de séquence d'espèces ciblées, de préférence, comprenant en outre la cartographie des lectures de séquence des espèces ciblées en séquences de référence segmentées pour former des lectures alignées des espèces ciblées, dans lequel chaque séquence de référence segmentée comprend des segments correspondant aux amplicons attendus pour une souche de l'espèce ciblée.

10. Procédé mis en œuvre par ordinateur selon la revendication 9, comprenant en outre l'agrégation des nombres de lectures alignées des espèces ciblées pour former des nombres de lectures agrégés par espèces, de préférence, comprenant en outre la détection de la présence des espèces ciblées dans l'échantillon sur la base des nombres de lectures agrégés par espèces.

11. Procédé mis en œuvre par ordinateur selon la revendication 9, comprenant en outre la génération des séquences de référence segmentées en appliquant une PCR sur silice à partir d'amorces d'un groupe d'amorces d'espèce.

12. Procédé mis en œuvre par ordinateur selon la revendication 8, comprenant en outre la génération des séquences de référence 16S compressées en appliquant une PCR sur silice basée sur des amorces d'un groupe d'amorces 16 S, ou dans lequel la série des lectures de séquence 16S correspond aux amplicons produits par l'amplification d'un échantillon d'acide nucléique en présence d'une ou plusieurs paires d'amorces ciblant une ou plusieurs régions hypervariables d'un gène d'ARNr 16S procaryote.

13. Procédé mis en oeuvre par ordinateur selon la revendication 8, dans laquelle la série de lectures de séquence d'acide nucléique comprend en outre une série de lectures de séquence d'espèces ciblées, et dans lequel la série de lectures de séquence d'espèces ciblées correspond à des amplicons produits par l'amplification d'une séquence d'acide nucléique cible contenue dans le génome d'un micro-organisme situé en dehors d'une région hypervariable d'un gène d'ARNr 16S procaryote, dans lequel différentes paires d'amorces amplifient différentes séquences d'acide nucléique cibles contenues dans le génome de différents micro-organismes présents dans l'échantillon d'acide nucléique.

14. Système comprenant :
une mémoire lisible par machine ; et
un processeur configuré pour exécuter des instructions lisibles par une machine, qui, lorsqu'elles sont exécutées par le processeur, amènent le système à effectuer un procédé comprenant :
La réception de plusieurs lectures de séquence d'acide nucléique dans le processeur, les lectures de séquence comprenant une série de lectures de séquence 16S ;
La première cartographie de la série de lectures de séquence 16S en une série de séquences de référence 16S comprimées, dans laquelle chaque séquence de référence 16S comprimée comprend un ensemble de segments hypervariables pour une souche correspondante d'une espèces ;
La génération d'une matrice de nombre de lectures contenant les nombres de lectures de séquence 16S associée à chaque segment hypervariable dans l'ensemble des segments hypervariables, dans laquelle les lignes de la matrice de nombres de lectures correspondent aux souches d'espèces et les colonnes correspondent aux segments hypervariables ;
La réduction de la matrice de nombre de lectures en appliquant un seuil aux nombres de lectures pour former une matrice de nombre de lectures réduite ;
La compression d'une base de données de séquences de référence 16S de longueur complète pour former un ensemble réduit de séquences de référence 16S de longueur complète basée sur la matrice de nombres de lectures réduite, l'ensemble réduit de séquences de référence 16S de longueur complète étant stocké dans la mémoire ;
La seconde cartographie de la série de lectures de séquence 16S dans l'ensemble réduit de séquences de référence 16S de longueur complète ;
Le comptage des lectures de séquence 16S, qui sont associées à chaque référence de longueur complète dans l'ensemble réduit de séquences de référence 16S de longueur complète pour former un deuxième ensemble de nombres de lectures ;
La normalisation des nombres de lectures dans le deuxième ensemble de nombres de lectures pour former des nombres normalisés ;
l'agrégation des nombres normalisés pour un niveau donné pour former des nombres agrégés, le niveau donné étant un niveau d'espèce, un niveau de genre ou un niveau de famille ; et l'application d'un seuil aux nombres agrégés pour détecter la présence d'un microbe à un niveau donné dans un échantillon.

15. Système comprenant :
une mémoire lisible par machine ; et
un processeur configuré pour exécuter des instructions lisibles par une machine, qui, lorsqu'elles sont exécutées par le processeur, amènent le système à effectuer un procédé comprenant :
La réception de plusieurs lectures de séquence d'acide nucléique dans le processeur, les lectures de séquence comprenant une série de lectures de séquence 16S ;
La première cartographie de la série de lectures de séquence 16S en une série de séquences de référence 16S comprimées, dans laquelle chaque séquence de référence 16S comprimée comprend un ensemble de segments hypervariables pour une souche correspondante d'une espèces ;
Le comptage des lectures de séquence 16S associées à chaque segment hypervariable dans l'ensemble des segments hypervariables pour former un premier ensemble de nombres de lectures ;
La compression d'une base de données de séquences de référence 16S de longueur complète pour former un ensemble réduit de séquences de référence 16S de longueur complète basée sur le premier ensemble de nombres de lectures de séquence 16S associées aux séquences de référence 16S compressées, l'ensemble réduit de séquences de référence 16S de longueur complète étant stocké dans la mémoire ;
La seconde cartographie de la série de lectures de séquence 16S dans l'ensemble réduit de séquences de référence 16S de longueur complète ;
Le comptage des lectures de séquence 16S, qui sont associées à chaque référence de longueur complète dans l'ensemble réduit de séquences de référence 16S de longueur complète pour former un deuxième ensemble de nombres de lectures ; et
La détection de la présence d'un microbe au niveau de l'espèce, au niveau du genre ou au niveau de la famille dans un échantillon sur la base du deuxième ensemble de nombres de lectures.
